# EUROPEAN PATENT APPLICATION

(11) **EP 4 714 943 A2**
(43) Date of publication of application: **25.03.2026**
(21) Application number: 25225505.4
(22) Date of filing: 15.03.2021
(51) Int. Cl.: C07D 213/02

(54) **METHODS OF TREATING RESPIRATORY DISEASE WITH DEUPIRFENIDONE**

(30) Priority: 13.03.2020 US 202062988919 P; 19.03.2020 US 202062992106 P; 08.04.2020 US 202063007304 P; 26.05.2020 US 202063030067 P; 28.05.2020 US 202063031412 P; 04.06.2020 US 202063034892 P; 17.06.2020 US 202063040454 P; 01.09.2020 US 202063073209 P; 02.10.2020 US 202063087051 P; 02.10.2020 US 202063087089 P; 23.10.2020 US 202063104967 P; 23.10.2020 US 202063104978 P; 03.12.2020 US 202063121139 P; 03.12.2020 US 202063121157 P
(62) Divisional of application: 21766819.3
(71) Applicant: PURETECH LYT 100, INC., Boston, Massachusetts 02210 (US)
(72) Inventor: The designation of the inventor has not yet been filed
(74) Representative: Murgitroyd & Company

(57) **Abstract**

Disclosed herein is a method of treating a viral, e.g., SARS-CoV-2, respiratory disease or infection, such as COVID-19 related pneumonia, that includes administering deuterium-enriched pirfenidone, e.g., daily for 3 months or more, such that the viral respiratory disease or infection is treated. The treating is effective in preventing the development of, halting the progression of, or slowing the progression of one or more of pulmonary fibrosis, respiratory complications of the viral respiratory disease or infection, respiratory symptoms of the viral respiratory disease or infection or pulmonary dysfunction.

## Description

### TECHNICAL FIELD

The present disclosure is directed to methods for treating or preventing COVID-19 respiratory infection or complications thereof, in a subject. The methods include administration of an effective amount of a deuterium-enriched pirfenidone to the subject.

### BACKGROUND

Coronavirus Disease 2019 (COVID-19), previously designated 2019-nCoV, is an acute respiratory illness caused by the coronavirus. SARS-CoV-2. SARS-CoV-2 is a novel member of the beta-coronavirus genus, which further includes four additional human virus species: HCoV-HKU1, HCoV-OC43, Middle East respiratory syndrome coronavirus (MERS-CoV), and the severe acute respiratory syndrome coronavirus (SARS-CoV). COVID-19 was identified as the cause of a cluster of pneumonia cases in Wuhan, a city in the Hubei Province of China, at the end of 2019. It subsequently spread throughout China and elsewhere, becoming a global health emergency. On the 11th of March 2020, the World Health Organization (WHO) declared COVID-19 as a pandemic. As of May 6, 2020, nearly 3.7 million people have been infected and around 260,000 people have died from COVID-19 worldwide. (Spagnolo et al., Lancet Respiratory Medicine, published online May 15, 2020 https://doi.org/10.1016/S2213-2600(20)30222-8).

There are currently no drugs or other therapeutic agents approved to treat acute COVID-19. Current clinical management of COVID-19 includes infection prevention and control measures and supportive care of acute infection, including supplemental oxygen and mechanical ventilator support when indicated.

There is increasing evidence of complications that persist in survivors after the acute infection has resolved. These post-acute sequelae of COVID-19 are collectively being referred to as "Long Covid." Long Covid is a complex pathophysiology that can involve tissue damage, residual inflammation, and fibrosis in the lung. These mechanisms can lead to lasting respiratory complications. There is presently a shadow pandemic of these Long Covid complications that may contribute to the enormous health, social, and economic cost of the pandemic. There are virtually no treatment options being developed that focus on treating or preventing these post-acute sequelae of COVID-19, including respiratory complications. Accordingly, there is an unprecedented medical need for such Long Covid treatments, as well as treatments that address inflammation, cellular or organ modification or injury, and the like in related infectious diseases including SARS and MERS.

### SUMMARY OF THE DISCLOSURE

The COVID-19 clinical course can progress from viral infection through mild, moderate, severe disease, and acute respiratory distress syndrome (ARDS). It is becoming clear through both survivors' stories and emerging clinical research that of the millions of COVID-19 patients that recover, at least a fraction may experience serious long-term pulmonary dysfunction as a result of interstitial pneumonia and chronic fibrotic sequelae. In outbreaks of related illnesses such as SARS and MERS, a third of patients had long-term fibrosis and lung dysfunction, often lasting well after their recovery from the acute phase of the disease. A need exists for therapeutic strategies to treat and/or prevent the inflammatory/fibrotic respiratory aftermath of viral respiratory diseases, such as those resulting from human influenza or beta-coronaviruses, including SARS-CoV-2.

Provided herein are methods of treating infection a subject. The methods generally include administering to a subject in need thereof an effective amount of a deuterium-enriched pirfenidone compound, wherein the subject is treated. In some embodiments, the infection is an inflammation-inducing infection. In some embodiments, the infection is a fibrosis-inducing infection. In some embodiments, the infection is viral. In some embodiments the virus is a coronavirus. In some embodiments, the virus is a novel coronavirus. In some embodiments, the coronavirus is COVID-19.

Accordingly, in one aspect is provided a method of treating various aspects of viral respiratory infection or disease, e.g., COVID-19, such as the acute phase of the infection, complication(s) of the infection, recovery from the infection, and the respiratory aftermath of the infection. The method comprises administering to a patient in need an effective amount of a deuterium-enriched pirfenidone having the structure: 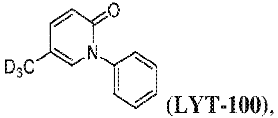 referred to herein as LYT-100. In order to help patients to recover from the viral respiratory infection or disease (e.g., COVID-19), treatment can begin at the first onset of symptoms. For example, treatment can begin less than 24 hours, 48 hours, 72 hours, or 96 hours after onset of a first symptom of the viral respiratory disease. Alternatively, treatment can be initiated after a longer period of time from onset of a first symptom or symptoms, e.g., 5 days, 7 days, or ~9-10 days. Accordingly, either or both of the acute infection and long term effects of the infection are treated.

In another aspect, a method of treating a subject having a viral respiratory disease or having viral respiratory complication(s) is provided. The method includes administering to a subject in need thereof an effective amount of LYT-100, wherein the viral respiratory disease or viral respiratory complication(s) is treated in the subject.

In another aspect, a method of treating a subject having a viral respiratory disease or a viral respiratory infection is provided. The method includes administering to a subject in need thereof an effective amount of LYT-100, wherein the treating is effective in ameliorating respiratory symptoms of the viral respiratory disease or infection.

In another aspect, a method of preventing a viral respiratory disease or a viral respiratory complication(s) in a subject is provided. The method includes administering to a subject in need thereof an effective amount of LYT-100, wherein the viral respiratory disease or viral respiratory complication(s) is prevented in the subject.

In another aspect, a method of preventing a viral respiratory disease or a viral respiratory complication(s) in a subject is provided. The method includes administering to a subject in need thereof an effective amount of LYT-100, wherein the treating is effective in preventing the development of, halting the progression of, slowing the progression of, or otherwise ameliorating one or more of lung fibrosis, respiratory complications of the viral respiratory disease or infection, or pulmonary dysfunction in the subject.

In some embodiments, the method is effective in preventing the development of pulmonary fibrosis, pulmonary dysfunction, or both in the subject. In some embodiments, the treating is effective in preventing the development of pulmonary fibrosis in the subject. In some embodiments, the treating is effective in preventing the development of pulmonary dysfunction in the subject.

In some embodiments, the method is effective in halting the progression of pulmonary fibrosis pulmonary dysfunction, or both in the subject. In some embodiments, the method is effective in halting the progression of pulmonary fibrosis in the subject. In some embodiments, the method is effective in halting the progression of pulmonary dysfunction in the subject.

In some embodiments, the method is effective in slowing the progression of pulmonary fibrosis, pulmonary dysfunction, or both in the subject. In some embodiments, the method is effective in slowing the progression of pulmonary fibrosis in the subject. In some embodiments, the method is effective in slowing the progression of pulmonary dysfunction in the subject.

In some embodiments, the method is effective in ameliorating respiratory symptoms of the viral respiratory disease or viral respiratory infection. In some embodiments, the method is effective in preventing respiratory symptoms of the viral respiratory disease or infection. In some embodiments, the respiratory symptoms are cough, dyspnea, or both. In some embodiments, the subject exhibits reduced cough, dyspnea, or both, relative to a subject that has not been treated with LYT-100.

In some embodiments, the viral respiratory infection is caused by infection with human influenza virus H1N1, human influenza virus H7N9, SARS-CoV, SARS-CoV-2, MERS-CoV, HCoV-HKU1, or HCoV-OC43.

In some embodiments, the viral respiratory disease or viral respiratory complication(s) is caused by a human influenza virus. In some embodiments, the human influenza virus is an H1N1 or H7N9 strain.

In some embodiments, the viral respiratory disease or viral respiratory complication(s) is caused by a beta-coronavirus. In some embodiments, the beta-coronavirus is SARS-CoV, SARS-CoV-2, MERS-CoV, HCoV-HKU1, or HCoV-OC43.

In some embodiments, the viral respiratory disease is COVID-19 or a complication thereof. In some embodiments, the viral respiratory disease is COVID-19 related pneumonia. In some embodiments, the viral respiratory disease or complication thereof is non-critical COVID-19 related pneumonia. In some embodiments, the complication is COVID-19 related acute respiratory distress syndrome (ARDS). In some embodiments, the viral respiratory complication excludes idiopathic pulmonary fibrosis (IPF).

In some embodiments, tissue and/or organ damage is treated or prevented in the subject. In some embodiments, inflammation is treated or prevented in the subject. In some embodiments, tissue remodeling or insult is treated or prevented in the subject. In some embodiments, fibrosis is treated or prevented in the subject. In some embodiments, lung remodeling is reversed or prevented, lung remodeling is resolved, lung function is improved and/or lung damage is treated. In some embodiments, lung dysfunction is reduced or improved, oxygen saturation is improved, CT scans are improved, mortality is reduced, comorbid disease is reduced and/or sepsis is prevented or treated.

In some embodiments, the treatment is initiated within a few hours or days of diagnosis. In some embodiments, treatment continues after the acute infection, e.g., post-discharge to treat long term effects of the infection. In some embodiments the infection is a moderate-to-severe, or severe, infection. In some embodiments, the infection is mild or moderate. In some embodiments, treatment begins less than 24 hours, 48 hours, 72 hours, or 96 hours after onset of a first symptom of the infection. In other embodiments, treatment begins 10 days after onset of a first symptom of the infection.

In some embodiments, the treatment is initiated within 90 days from a confirmed viral respiratory disease diagnosis. In some embodiments, the treatment is initiated within 60 days from a confirmed viral respiratory disease diagnosis. In some embodiments, the treatment is initiated within about 90, about 80, about 70, about 60, about 50, about 45, about 40, about 35, about 30, about 28, about 21, about 14, about 7, or about 1 day from a confirmed viral respiratory disease diagnosis. In some embodiments, the treatment is initiated between 90 and 42 days from a confirmed viral respiratory disease diagnosis. In some embodiments, the treatment is initiated within 42 days from a confirmed viral respiratory disease diagnosis. In some embodiments, the treatment is initiated within 30 days, within 14 days, within 10 days, or within 7 days from a confirmed viral respiratory disease diagnosis. In some embodiments, the treatment is initiated within 14 days from a confirmed viral respiratory disease diagnosis. In some embodiments, the treatment is initiated within from 1 day to 14 days from a confirmed viral respiratory disease diagnosis. In some embodiments, the treatment is initiated within 1 day, 2 days, 3 days, 4 days, 5 days, 6 days, 7 days, 10 days, or 14 days from a confirmed viral respiratory disease diagnosis. In some embodiments, the treatment is initiated between 14 and 28 days from a confirmed viral respiratory disease diagnosis.

In some embodiments, the treatment is initiated during the acute phase of the viral respiratory disease. In some embodiments, the treatment is initiated during the post-acute phase of the viral respiratory disease. In some embodiments, the treatment is initiated during the acute phase of the viral respiratory disease, and continued during the post-acute phase of the viral respiratory disease.

In some embodiments, treating comprises administering an effective amount of LYT-100 daily for up to 3 months or 91 days. In some embodiments, treating comprises administering an effective amount of LYT-100 daily for up to 12 months.

In some embodiments, treating comprises daily administration of a total daily dose of between 200 mg and 2250 mg LYT-100. In some embodiments, treating comprises daily administration of a total daily dose of 1000 mg of LYT-100. In some embodiments, treating comprises daily administration of a total daily dose of 1500 mg of LYT-100. In some embodiments, treating comprises daily administration of a total daily dose of 2000 mg of LYT-100.

In some embodiments, the daily administration is once daily administration. In some embodiments, the daily administration is twice daily administration. In some embodiments, the daily administration is three times daily.

In some embodiments, the LYT-100 is administered without regard to food.

In some embodiments, the LYT-100 is administered without food.

In some embodiments, the LYT-100 is administered with solid food or with a nutritional supplement.

In some embodiments, the administration is two daily doses of 500 mg LYT-100. In some embodiments, the administration is two daily doses of 750 mg of LYT-100. In some embodiments, the administration is three daily doses of 500 mg LYT-100. In some embodiments, the administration is two daily doses of 1000 mg of LYT-100. In any of these embodiments, the LYT-100 is administered with food. In any of these embodiments, the LYT-100 is administered without food. In any of these embodiments, the LYT-100 is administered without regard to food.

In some embodiments, the LYT-100 is administered orally.

In some embodiments, the LYT-100 is administered orally without food in two daily doses of 100 mg each.

In some embodiments, the LYT-100 is administered orally without food in three daily doses of 100 mg each.

In some embodiments, the LYT-100 is administered orally without food in two daily doses of 250 mg each. In some embodiments, the LYT-100 is administered orally without food in three daily doses of 250 mg each.

In some embodiments, the LYT-100 is administered orally without food in two daily doses of 500 mg each. In some embodiments, the LYT-100 is administered orally without food in three daily doses of 500 mg each.

In some embodiments, the LYT-100 is administered orally without food in two daily doses of 750 mg each. In some embodiments, the LYT-100 is administered orally without food in three daily doses of 750 mg each.

In some embodiments, the LYT-100 is administered orally without food in two daily doses of 1000 mg each.

In some embodiments, the LYT-100 is administered without dose escalation.

In some embodiments, the LYT-100 is administered orally without food in two daily doses of 1000 mg each without dose escalation.

In some embodiments, the LYT-100 is administered in tablet or capsule form. In some embodiments, the LYT-100 is formulated as powder in 250 mg capsules.

In some embodiments, the LYT-100 is administered and the subject is also maintaining another treatment relating to the viral respiratory disease. In some embodiments, the another treatment comprises one or more non-pharmacological treatments selected from the group consisting of mechanical ventilation, non-invasive ventilation, O₂ supplementation, extra corporeal membrane oxygenation (ECMO), continuous positive airway pressure (CPAP), respiratory rehabilitation, and exercise. In some embodiments, the LYT-100 is administered in conjunction with at least one additional therapeutic agent selected from the group consisting of antivirals, antibiotics, analgesics, convalescent plasma, immune globulins, immunomodulators, corticosteroids, anti-inflammatories, nonsteroidal anti-inflammatories, and immunosuppressants.

In some embodiments, the LYT-100 is administered in two daily doses in an amount of 500 mg each on days 1 through 3, and in two daily doses in an amount of 750 mg each thereafter, e.g., on day 4 and thereafter. In some embodiments, the LYT-100 is administered in two daily doses in an amount of 500 mg each on days 1 through 3, and in two daily doses in an amount of 750 mg each thereafter, e.g., on days 4 through 91. In some embodiments, the LYT-100 is administered in two daily doses in an amount of 500 mg each on days 1 through 3, and in two daily doses in an amount of 750 mg each thereafter, e.g., for up to 3 months or about 3 months. In some embodiments, the LYT-100 is administered in two daily doses in an amount of 500 mg each on days 1 through 3, and in two daily doses in an amount of 750 mg each thereafter, e.g., for up to 12 months or about 12 months.

In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., on days 4 through 91. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., for up to 3 months or about 3 months. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., for up to 12 months or about 12 months.

In some embodiments, the LYT-100 is administered in two daily doses in an amount of 500 mg each on days 1 through 3, and in two daily doses in an amount of 1000 mg each thereafter, e.g., on day 4 and thereafter. In some embodiments, the LYT-100 is administered in two daily doses in an amount of 500 mg each on days 1 through 3, and in two daily doses in an amount of 1000 mg each thereafter, e.g., on days 4 through 91. In some embodiments, the LYT-100 is administered in two daily doses in an amount of 500 mg each on days 1 through 3, and in two daily doses in an amount of 1000 mg each thereafter, e.g., for up to 3 months or about 3 months. In some embodiments, the LYT-100 is administered in two daily doses in an amount of 500 mg each on days 1 through 3, and in two daily doses in an amount of 1000 mg each thereafter, e.g., for up to 12 months or about 12 months.

In some embodiments, the LYT-100 is administered in two daily doses in an amount of 250 mg each for two days; in two daily doses in an amount of 500 mg each for two days; and in two daily doses in an amount of 750 mg each thereafter, e.g., on days 7 through 91. In some embodiments, the LYT-100 is administered in two daily doses in an amount of 250 mg each for two days: in two daily doses in an amount of 500 mg each for two days; and in two daily doses in an amount of 750 mg each thereafter, e.g., for up to 3 months or about 3 months. In some embodiments, the LYT-100 is administered in two daily doses in an amount of 250 mg each for two days; in two daily doses in an amount of 500 mg each for two days; and in two daily doses in an amount of 750 mg each thereafter, e.g., for up to 12 months or about 12 months.

In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., on days 7 through 91. In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., for up to 3 months or about 3 months. In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., for up to 12 months or about 12 months.

In some embodiments, administering the LYT-100 does not comprise an initial titration. In some embodiments, the LYT-100 is administered in two daily doses in an amount of 500 mg each for up to 3 months or about 3 months. In some embodiments, the LYT-100 is administered in two daily doses in an amount of 500 mg for up to 12 months or about 12 months. In some embodiments, the LYT-100 is administered in two daily doses in an amount of 750 mg each for up to 3 months or about 3 months. In some embodiments, the LYT-100 is administered in two daily doses in an amount of 750 mg each for up to 12 months or about 12 months. In some embodiments, the LYT-100 is administered in two daily doses in an amount of 1000 mg each for up to 3 months or about 3 months. In some embodiments, the LYT-100 is administered in two daily doses in an amount of 1000 mg each for up to 12 months or about 12 months.

In some embodiments, the method further includes administering at least one antiviral therapy. In some embodiments, the method further includes administering an antiviral, an antibacterial compound, and/or anti-inflammatory agent.

In some embodiments, one or more of the following applies to the subject prior to treatment with LYT-100: the subject has a positive COVID-19 RT-PCR or SARS-CoV-2 RNA diagnostic test result; the subject is or was hospitalized for COVID-19 respiratory disease for at least 1-day, and required at least one of the listed treatment modalities for at least 24 hours: mechanical ventilation (MV), extra-corporeal membrane oxygenation (ECMO), non-invasive ventilation (NIV), high flow nasal oxygen (HFNO), and/or other high flow oxygen with FiO2 ≥35% and flow rates ≥ 8 lpm; the subject has a confirmed diagnosis of COVID-19; the subject exhibits COVID-19 pneumonia as confirmed by imaging, including by chest X-ray or high-resolution computerized tomography with a minimum of two lobe involvement.

In some embodiments, the subject exhibits shortness of breath ≥ grade 3 on a Modified Borg Dyspnea Scale (mBDS).

In some embodiments, the subject prior to treatment with LYT-100, does not require extra-corporeal membrane oxygenation (ECMO) or mechanical ventilation (MV) for 72 hours before starting administration of LYT-100, or has been off of ECMO/MV for at least 48 hours before starting administration of LYT-100. In some embodiments, the subject, prior to treatment with LYT-100, may require extra-corporeal membrane oxygenation (ECMO) or mechanical ventilation (MV).

In some embodiments, the subject exhibits one or more of the following prior to treatment: shortness of breath ≥ grade 3 on mBDS dyspnea scale, fever >38C°; cough; elevated C-Reactive Protein (CRP) ≥ 10.0 mg/dL; oxygen saturation ≤93% at rest; requires supplemental oxygen; requires high-flow oxygen or non-invasive ventilation.

In some embodiments, the subject does not have one or more of the following: a pre-existing respiratory condition unrelated to the viral respiratory disease; a resting heart rate of ≥ 120 bpm, systolic blood pressure of > 180 mm Hg and a diastolic blood pressure of > 100 mm Hg; a fall in pulse oximetry oxygen saturation (SpO₂) to <80% upon ambulation; a body mass index ≥ 40 kg/m²; a lactate dehydrogenase (LDH) value >360; a history of anaphylactic reaction including reactions to general anesthetic agents; a history of allergic reaction to any drug which led to significant morbidity; a history of prior allergic reaction to pirfenidone or LYT-100; sepsis; or septic, hypovolemic, cardiogenic, or neurogenic shock.

In some embodiments, the subject is not intubated or mechanically ventilated.

In some embodiments, the subject does not require extracorporeal membrane oxygenation (ECMO).

In some embodiments, the subject has not been intubated, mechanically ventilated, or received ECMO within 72 hours prior to initiating treatment.

In some embodiments, the subject is not undergoing dialysis.

In some embodiments, the subject does not have a history of dialysis.

In some embodiments, the subject is not currently taking any of the following: fluvoxamine, enoxacin, or ciprofloxacin; nonsteroidal anti-inflammatory drugs (NSAIDs); inhibitors of CYP1A2; inducers of CYP1A2, CYP2C9 or 2C19; nicotine; and any drug associated with substantial risk for prolongation of the QTc interval.

In some embodiments, the subject is not currently using tobacco or nicotine products.

In some embodiments, an improvement of 25 meters or more in a 6-minute walk test (6-MWT) over monthly increments through day 91 of treatment is achieved. In some embodiments, the improvement of 25 meters or more is achieved at a time point which is earlier than that achieved in a subject which has not been treated with LYT-100. In some embodiments, a rate of improvement in in the distance walked on the 6MWT in a subject which has been treated with LYT-100 is greater than a rate of improvement in a subject which has not been treated with LYT-100. In some embodiments, the subject exhibits one or more of: a statistically significant increase in the distance walked on the six-minute walk test (6MWT) as compared to a baseline 6MWT result in the treated subject; a statistically significant increase in the distance walked on the six-minute walk test (6MWT) as compared to a subject who has not been treated with LYT-100; a statistically significant rate of improvement in the distance walked on the 6MWT as compared to a rate of improvement in the distance walked on the 6MWT in a subject who has not been treated with LYT-100. In some embodiments, when a distance walked on the 6MWT is measured at two or more time points after beginning treatment with LYT-100, the subject exhibits a difference in the distance walked in the 6MWT between the two or more time points (ΔD) which is greater than a ΔD in a subject who has not been treated with LYT-100.

In some embodiments, at least a 1.5-point decrease in the Modified Borg Dyspnea Scale is achieved.

In some embodiments, one or more of the following is achieved in the subject: an improvement in blood oxygenation level in the human subject over 91 days of treatment, as determined by pulse oximetry; a 10 unit drop in an overall score in the Short Form Health Survey (SF-36 v2) over 91 days of treatment; an increase of ≥2 units in the overall St. George's Respiratory Questionnaire-Idiopathic Pulmonary Fibrosis (SGRQ-I) score over 91 days of treatment; an improvement in pulmonary inflammation; an improvement in fibrosis; an improvement in other radiographic abnormalities, as measured by determining at least a 2-point score improvement over 91 days of treatment on high resolution computed tomography (HRCT) scans, from the level of the upper thoracic inlet to the inferior level of the costophrenic angle over 28, 56, and 91 days of treatment utilizing the following scoring system: a) number of involved segments; and b) lung lobe involvement for each of five lobes via the semi- Quantitative Lung Fibrosis (QLF) added and Computer-Aided Diagnosis (CAD) QLF Scores.

In some embodiments, the subject exhibits reduced evidence of lung fibrosis, respiratory complications, or pulmonary dysfunction relative to a subject who has not been treated with LYT-100, as measured by a chest X-ray or a CT scan, or as evidenced by a pulmonary function test result.

In some embodiments, the subject exhibits reduced evidence of lung fibrosis, respiratory complications, or pulmonary dysfunction relative to a subject who has not been treated with LYT-100, as measured by a pulmonary function test result, wherein the pulmonary function test result is a statistically significant improvement in the distance walked on the six-minute walk test (6MWT) compared to a baseline 6MWT result in the treated subject.

In some embodiments, at least a 1.5-point decrease in the Modified Borg Dyspnea Scale is achieved.

In some embodiments, the subject exhibits an improvement in the score on one or more of the following: Dyspnea-12; Patient-reported quality of life (SF-36 v2).

In some embodiments, a reduction in fibrosis or fibrotic lesions on high resolution computed tomography (HRCT) is achieved in the subject. In some embodiments, the fibrotic lesion reduced in the subject is one or more of ground glass opacity, reticular patterning, honeycombing, traction bronchiectasis, bronchiolectasis, interlobular septal thickening. ill-defined margins, air bronchogram, "crazy-paving" pattern, thickening of the adjacent pleura, nodules, cystic changes, pleural effusion, or lymphadenopathy.

In some embodiments, at least a 25% reduction in one or more of the following biomarkers is achieved: C-Reactive Protein (CRP); d-dimer; cardiac troponin; ferritin; lactate dehydrogenase (LDH); interleukin-6 (IL-6); TGF-β1; TNF-α; IL-1β; PDGF- β; GCSF; VEGF.

In some embodiments, an improvement by 2 points or higher on the WHO Ordinal Scale for Clinical Improvement is achieved.

In some embodiments, mechanical ventilation of the subject is prevented. In some embodiments, intubation of the subject is prevented. In some embodiments, use of one or more of the following in the subject is prevented: non-invasive ventilation, O₂ supplementation, extra corporeal membrane oxygenation (ECMO).

In another aspect is provided a method of treating post-acute sequelae of infection with SARS-CoV-2, the method comprising administering to a subject in need thereof an effective amount of: 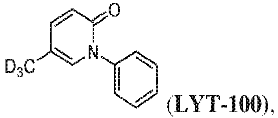 wherein the post-acute sequelae of infection are treated in the subject.

In some embodiments, the post-acute sequelae comprise one or more of post-acute respiratory complications, impaired lung function, pulmonary dysfunction, and pulmonary fibrosis.

In some embodiments, the post-acute sequelae comprise progressive pulmonary fibrosis.

In some embodiments, a reduction in fibrosis or fibrotic lesions on high resolution computed tomography (HRCT) is achieved in the subject.

In some embodiments, the administering is initiated during an acute phase of the SARS-CoV-2 infection. In some embodiments, the administering is initiated during an acute phase of the SARS-CoV-2 infection and continued during a post-acute phase of the SARS-CoV-2 infection. In some embodiments, the administering is initiated during a post-acute phase of the SARS-CoV-2 infection.

In some embodiments the subject with one or more post-acute respiratory complications was previously treated with, but no longer requires mechanical ventilation (MV), extracorporeal membrane oxygenation (ECMO), non-invasive ventilation (NIV), high-flow nasal oxygen therapy (HFNO), or high flow supplemental oxygen with FiO2 ≥35%.

In some embodiments, the subject has not been treated with mechanical ventilation or extracorporeal membrane oxygenation.

In some embodiments, the subject requires regular nasal cannula O₂ supplementation.

In some embodiments, the subject does not require regular nasal cannula O₂ supplementation.

In some embodiments, the administering begins while the subject is hospitalized for SARS-CoV-2 infection, at the time of discharge from the hospital for SARS-CoV-2 infection, or post-discharge from the hospital for SARS-CoV-2 infection.

In some embodiments, the LYT-100 is administered orally without food in two daily doses of 500 mg each, 750 mg each, or 1000 mg each. In some embodiments, the LYT-100 is administered orally with food in two daily doses of 500 mg each, 750 mg each, or 1000 mg each. In some embodiments, the LYT-100 is administered orally without regard to food in two daily doses of 500 mg each, 750 mg each, or 1000 mg each.

In some embodiments, the LYT-100 is administered orally with or without food in an initial dose of 500 mg BID for the first 3 days of dosing, followed by titration up to 750 mg BID thereafter for at least three months.

In some embodiments, the LYT-100 is administered orally with or without food in an initial dose of 500 mg BID for the first 3 days of dosing, followed by titration up to 1000 mg BID thereafter for at least three months.

In some embodiments, the LYT-100 is administered orally with or without food in an initial dose of 500 mg BID for the first 3 days of dosing. followed by titration up to 750 mg BID for days 4 through 7, followed by titration up to 1000 mg BID thereafter for at least three months.

In any of these embodiments, the subject is a human subject. In any of these embodiments, the subject is an adult human.

In any of these embodiments, the subject does not have idiopathic pulmonary fibrosis.

In any of these embodiments, the effective amount is a clinically proven effective amount, *e.g.,* as demonstrated in a U.S. FDA or EMA clinical trial.

### BRIEF DESCRIPTION OF THE FIGURES

**FIG. 1** depicts representative micrographs of Sirius-red stained liver sections illustrating that LYT-100 significantly reduced the area of fibrosis.
**FIG. 2** illustrates the percent fibrosis area for LYT-100 versus vehicle and control.
**FIG. 3A** illustrates that LYT-100 does not induce survival of Primary Mouse Lung Fibroblasts (PMFL); **FIG 3B****.** and **FIG. 3C** illustrate LYT-100 reduced TGF-β-induced total collagen level in PMFL a 6 well and 96 well format, respectively; and **FIG. 3D** and **FIG. 3E** illustrate LYT-100 reduced TGF-β-induced soluble fibronectin levels and soluble collagen levels.
**FIG.4A** illustrates that LYT-100 does not affect survival of L929 cells. **FIG. 4B****,** illustrates that LYT-100 inhibits TGF-induced collagen synthesis. **FIG. 4C** illustrates that LYT-100 significanly inhibits TGF-β-induced total collagen levels. **FIG. 4D** is a graph illustrating that LYT-100 significantly inhibits TGF-β-induced soluble collagen levels. **FIG. 4E** illustrates that LYT-100 signficantly reduced soluble fibronectin levels, in the absence and presence of TGF-β-induction.
**FIG. 5** depicts plasma TNF-alpha concentration 90 minutes after LPS infusion in rats pre-dosed with vehicle, LYT-100, or pirfenidone.
**FIG. 6** is a table of Day 1 toxicokinetic parameters from a toxicology study comparing LYT-100 to pirfenidone for parent compound and major metabolites.
**FIG. 7** is a table of Day 28 toxicokinetic parameters from a toxicology study comparing LYT-100 to Pirfenidone for parent compound and major metabolites.
**FIGS. 8A-B** are tables of Day 91 toxicokinetic parameters from a toxicology study comparing LYT-100 to pirfenidone for parent compound and major metabolites.
**FIG. 9** is a chart of mean plasma concentration of LYT-100 following single and repeat oral administration of deupirfenidone in female Sprague Dawley rats.
**FIG. 10** is a chart of mean plasma concentration of pirfenidone (SD-559) following single and repeat oral administration of pirfenidone in female Sprague Dawley rats.
**FIG. 11** is a chart of mean plasma concentrations following single and repeat oral administration of 750 mg/kg LYT-100 and pirfenidone in female Sprague Dawley rats on day 28.
**FIG. 12** and **FIG. 13** are charts depicting the AUC₀₋₂₄ dose relationship following single and repeat oral administration of deupirfenidone in female and male Sprague Dawley rats, respectively.
**FIG. 14** and **FIG. 15** are charts depicting the AUC₀₋₂₄ dose relationship following single and repeat oral administration of pirfenidone in female and male Sprague Dawley rats, respectively.
**FIGS. 16A-16D** are charts of mean plasma concentrations in four cohorts (Cohorts 1-4) following oral administration of LYT-100 in human subjects in a multiple ascending dose (MAD) study at 100 mg, 250 mg, 500 mg, and 750 mg BID, respectively.
**FIG. 17A** and **FIG. 17B** are charts depicting the concentration of LYT-100 and the three major metabolites in plasma following repeat oral administration of 750 mg BID LYT-100 in human subjects in the MAD study.
**FIG. 18** is a table providing Tₘₐₓ, Cₘₐₓ, AUC, and accumulation ratio for the parent drug (LYT-100) and its major metabolites quantitated for Cohort 4 in the MAD study.
**FIG. 19** is a table providing Tₘₐₓ, Cₘₐₓ, AUC, and accumulation ratio for the parent drug (LYT-100) and its major metabolites quantitated for Cohorts 1-4 in the MAD study.
**FIGS. 20A** and **20B** (linear and log scale concentration axes, respectively) are charts depicting the concentration of LYT-100 and the three major metabolites (SD-789, SD-790, and SD-1051) in plasma following repeat oral administration of 1000 mg BID LYT-100 (Cohort 6) in human subjects in the MAD study.
**FIGS. 21A-21E** are charts of mean plasma concentrations for LYT-100 and each metabolite (SD-789, SD-790, and SD-1051) in five cohorts (Cohorts 1-4 and 6) following oral administration of LYT-100 in human subjects in a multiple ascending dose (MAD) study at 100 mg, 250 mg, 500 mg, 750 mg, and 1000 mg BID, respectively.
**FIGS. 22A-22F** are charts of mean plasma concentrations for LYT-100 and each metabolite (SD-789, SD-790, and SD-1051) in each of the six subjects in Cohort 6.
**FIG. 23** is a table of pharmacokinetic data for Cohort 6 (Tₘₐₓ, Cₘₐₓ, AUC₀₋₁₂, AUC₁₂₋₂₄, AUC₉₆₋₁₀₈, and AUC accumulation ratio (AUC₉₆₋₁₀₈/AUC₀₋₁₂)) for the active and each metabolite.
**FIG. 24** is a table of pharmacokinetic data for LYT-100 and each metabolite (SD-789, SD-790, and SD-1051) for dosing Cohorts 1-4 and 6 (100 mg, 250 mg, 500 mg, 750 mg, and 1000 mg BID, respectively).
**FIG. 25** is a chart depicting the dose proportionality (AUC₉₆₋₁₀₈ versus dose) for LYT-100 and major metabolite SD-789 across Cohorts 1-4 and 6 in the MAD study.
**FIG. 26** is a table comparing pharmacokinetic data for LYT-100 and each metabolite (SD-789, SD-790, and SD-1051) with data extrapolated from Huang et al. (200 mg BID pirfenidone).
**FIGS. 27A** and **27B** are charts of mean plasma concentrations for LYT-100 and each metabolite (SD-789, SD-790, and SD-1051) in plasma following single oral administration of 500 mg LYT-100 in fasted and fed **(****FIGS. 27A** and **27B****,** respectively) human subjects in Cohort 5 of the MAD study.
**FIG. 28** is a table of pharmacokinetic data for LYT-100 and each metabolite (SD-789, SD-790. and SD-1051) for Cohort 5 (500 mg single dose; fed vs fasted).
**FIG. 29** is a table of pharmacokinetic data for LYT-100 and the major metabolite (SD-789) extrapolated from the single ascending dose study and compared to the 750 mg cohort in the MAD study.

### DETAILED DESCRIPTION

Fibrosis and inflammation are a common mechanism across several lung diseases, and there is increasing data that respiratory complications of COVID-19, including shortness of breath, begin during the acute phase of illness and may persist as lung fibrosis. According to a research letter published in the Journal of the American Medical Association (JAMA), more than 40% of COVID-19 survivors assessed in an Italian study still reported shortness of breath an average of 60 days following symptom onset. Carfi, A., Bernabei, R., & Landi, F. (2020). Persistent Symptoms in Patients After Acute COVID-19. Jama, 324(6), 603. doi:10.1001/jama.2020.12603. These data suggest that a significant percentage of COVID-19 survivors may be at risk for long-term sequelae, a condition that is now referred to as "Long COVID." Similar complications caused by the Severe Acute Respiratory Syndrome (SARS) epidemic lasted for years, leading to impaired lung function in many survivors.

Pirfenidone (Deskar^{®}), CAS# 53179-13-8, Pirespa, AMR-69, Pirfenidona, Pirfenidonum, Esbriet, Pirfenex, 5-methyl-1-phenyl-1*H*-pyridin-2-one, 5-Methyl-1-phenyl-2-(1*H*)-pyridone, 5-methyl-1-phenylpyridin-2(1*H*)-one, is an orally administered small molecule anti-inflammatory and antifibrotic properties. Pirfenidone is an approved therapy for the treatment of IPF in 30 European countries, Japan, South Korea, China, India, Argentina, and Mexico. Worldwide, pirfenidone has been available commercially for IPF since late 2008. Pirfenidone, under the brand name Esbriet^{®}, was approved by the FDA in the United States in 2014 for the treatment of IPF. Pirfenidone has been shown to be generally well tolerated; the most common adverse events (AEs) were gastrointestinal symptoms, fatigue, rash, and photosensitivity reactions.

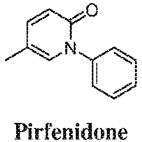

Pirfenidone has been shown to slow the progression of idiopathic pulmonary fibrosis (IPF) in clinical trials (Noble et al. Pirfenidone in patients with idiopathic pulmonary fibrosis (CAPACITY): two randomized trials. Lancet. 2011:377(9779):1760-1769; Taniguchi et al., Pirfenidone in idiopathic pulmonary fibrosis. Eur Respir J. 2010:35(4):821-829; King et al. A phase 3 trial of pirfenidone in patients with idiopathic pulmonary fibrosis. N Engl J Med. 2014;370(22):2083-2092.; Rafii et al., A review of current and novel therapies for idiopathic pulmonary fibrosis. J Thorac Dis. 2013;5:48-73).

In addition to the treatment of IPF, pirfenidone has been clinically evaluated for its safety and efficacy for the treatment of other chronic fibrotic disorders, including renal fibrosis, hepatic fibrosis, and myelofibrosis. Tada et al., Clin. Exper. Pharmacol. Physiol. 28:522-527 (2001); Cho et al., Clin. J. Am. Soc. Nephrol. 2:906-913 (2007); Nagai et al., Intern. Med.65 41:1118-1123 (2002); Raghu et al., Am. J. Respir. Crit. Care Med. 159:1061-1069 (1999); Gahl et al., Mal. Genet. Metab. 76:234-242 (2002); Armendariz-Borunda et al., Gut 55:1663-1665 (2006); Angulo et al., Dig. Dis. Sci. 47:157-161 (2002); Mesa et al., Brit. J. Haematol. 114:111-113(2001).

It is likely that multiple mechanisms contribute to the unique profile of pirfenidone. Pirfenidone attenuates fibroblast proliferation, production of fibrosis-associated proteins and cytokines, and biosynthesis and accumulation of extracellular matrix in response to cytokine growth factors (Schaefer et al., Antifibrotic activities of pirfenidone in animal models, Eur Respir Rev. 2011;20:85-97.; InterMune UK, Ltd. Esbriet^{®} Summary of Product Characteristics. 2011). Pirfenidone blocks the production and activity of TGF-β, a key growth factor that increases collagen production while decreasing its degradation. Moreover, administration of pirfenidone reduces the production of other fibrogenic factors that are induced by TGF-β, such as fibronectin and connective tissue growth factor (Schaefer, 2011). Pirfenidone is capable of blocking bleomycin-induced PDGF production as well as fibroblast and hepatic stellate cell proliferation in response to PDGF (DiSario et al. Effect of pirfenidone on rat hepatic stellate cell proliferation and collagen production, J of Hepatol. 2002 Nov. 37.5.584-591). Pirfenidone inhibits the expression of TNF-α, IL-1, and intercellular adhesion molecule 1 (ICAM-1) (Schaefer, 2011). In a murine macrophage-like cell line, pirfenidone suppressed TNF-a production or secretion through mitogen-activated protein kinase and c-Jun N-lerminal kinase-independent mechanisms and increased the levels of IL-10, an anti-inflammatory cytokine (Schaefer, 2011).

In fibrotic diseases such as Idiopathic Pulmonary Fibrosis (IPF), fibrotic pathogenesis is thought to arise from epithelial injury, proliferation of lung fibroblasts, and excessive or inappropriate deposition of connective tissue matrix with endothelin, PDGF, and TGF-β playing critical roles.

Among many other demonstrated effects, pirfenidone attenuates fibroblast proliferation, production of fibrosis-associated proteins and cytokines, and biosynthesis and accumulation of extracellular matrix in response to cytokine growth factors such as TGF-β and platelet-derived growth factor, or PDGF (Schaefer et al., Antifibrotic activities of pirfenidone in animal models, Eur Respir Rev. 2011;20:85-97.; InterMune UK, Ltd. Esbriet^{®} Summary of Product Characteristics. 2011). Studies have shown that pirfenidone's anti-fibrotic and anti-inflammatory activity is due, at least in part, to inhibition of production and activity of TGF-B. Oku et al., Eur. J. Pharmacol. 590:400-408 (2008); Tian et al., Chin. Med. Sci. J. 21:145-151 (2006); Schaefer et al., Eur. Respir. Rev. 20:85-97 (2011). Pirfenidone is capable of blocking bleomycin-induced PDGF production as well as fibroblast and hepatic stellate cell proliferation in response to PDGF (DiSario et al. Effect of pirfenidone on rat hepatic stellate cell proliferation and collagen production, J of Hepatol. 2002 Nov. 37.5.584-591). Pirfenidone also blocks the production and activity of TGF-β, a key growth factor that increases collagen production while decreasing its degradation. Moreover, administration of pirfenidone reduces the production of other fibrogenic factors that are induced by TGF-β, such as fibronectin and connective tissue growth factor (Schaefer, 2011).

Pirfenidone has demonstrated activity in nonclinical models for multiple fibrotic conditions, including those of the lung, kidney and liver (Schaefer, 2011; Scriabine et al., New developments in the therapy of pulmonary fibrosis, Adv Pharmacol. 2009; 57:419-464). In animal models, pretreatment with pirfenidone reduces inflammation caused by a number of inciting agents, such as lipopolysaccharide (LPS), bleomycin, amiodarone, and carbon tetrachloride, and has demonstrated activity in preclinical models of lymphedema and radiation-induced fibrosis. Pirfenidone has also shown activity investigational clinical studies in patients with unclassifiable interstitial lung disease (ulLD), focal segmental glomerulosclerosis (FSGS), and other indications, and has been granted an FDA Breakthrough Therapy designation in ILDs.

Despite pirfenidone's desirable pharmacological profile, it suffers from poor tolerability and pharmacokinetic deficits that limit its use in IPF, and perhaps in other ILDs as well. Pirfenidone is also associated with significant tolerability issues and dose-limiting toxicities.

Pirfenidone has a short half-life in humans and consequently relatively frequent dosing may be required. For the treatment of some conditions, the recommended daily maintenance dose of pirfenidone is 801 mg three times per day (2403 mg·day-1) (a total of nine (9) pills per day at full dose) with a 14-day titration period upon treatment initiation.

In addition, to obtain the maximum benefits of pirfenidone treatment, the adverse events (AEs) associated with pirfenidone require management. The most common AEs are gastrointestinal (GI) and skin-related adverse events, for example, nausea, rash, diarrhea, fatigue, dyspepsia, anorexia, dizziness, gastroesophageal reflux disease, decreased appetite, decreased weight, photosensitivity, and cough. In addition, several treatment-emergent adverse events have been reported, including upper respiratory infection and bronchitis. A recent study in patients treated with pirfenidone under a compassionate use program demonstrated that 44% of the patients had an adverse event with pirfenidone, with only half of them continuing on pirfenidone after a dose-reduction. Raghu & Thickett. Thorax; 68: 605-608 (2013). Adverse events common with pirfenidone at 2403 mg/day include nausea, rash, fatigue, diarrhea, vomiting, dyspepsia, photosensitivity, and anorexia. Noble et al. Lancet; 377: 1760-69 (2011).

The results of several expanded clinical trials are summarized in Lancaster et al., Eur Resp Rev 2017:26:170057 which reports treatment-emergent adverse events (TEAEs) as rates per 100 PEY (equivalent to the frequency at which a physician might expect these TEAEs to occur if 100 patients with IPF were followed for 1 year). Herein, it is noted that the most common reported AEs leading to discontinuation are nausea, fatigue, diarrhea, and/or rash with frequencies as high as 62.1 per 100 PEY (nausea), 27.6 per 100PEY (diarrhea), 52.4 per 100PEY (fatigue). In a single-center, retrospective, observational study of 351 patients who were receiving pirfenidone, 75% of reported AEs were GI-related, with loss of appetite (17%) and nausea/vomiting (15%) being most frequent, similar to what was observed in the phase III trials. The highest number of treatment discontinuations occurred with appetite loss and nausea/vomiting. The incidence of AEs and discontinuation increases with age. The proportion of patients with ADRs leading to dose modification/interruption or discontinuation increased with increasing age: an ADR leading to dose modification/interruption occurred in 32.7% of patients aged ≥80 years and in 18.0% of patients aged <65 years, while an ADR leading to discontinuation occurred in 20.9% of patients aged >80 years and in 7.5% of patients aged <65 years.

A long-term observational safety study found that adverse drug reactions led to permanent treatment discontinuation in 28.7% of patients taking pirfenidone. Cottin et al. (2018). Long-term safety of pirfenidone: Results of the prospective, observational PASSPORT study. ERJ Open Research, 4(4), 00084-2018. doi:10.1183/23120541.00084-2018. Real-world experience with pirfenidone in the IPF treatment setting highlights significant problems with treatment compliance, resulting in about half of the patients starting therapy either discontinuing therapy, reducing does, or switching to another therapy, all of which lead to suboptimal disease management. For example, in a large, multinational post-marketing study that analyzed treatment practice of about 11,000 patients diagnosed with IPF, only about 13% of patients were receiving pirfenidone after about a 5-year follow-up period. A high frequency of gastrointestinal adverse events (e.g., nausea, vomiting, and diarrhea) was a major reason for low compliance. Approximately 73% of patients in the study experienced an adverse event, including 38% with gastrointestinal symptoms, leading to the high discontinuation rate.

Several methods for managing AEs associated with pirfenidone are used, including varying the dose titration schedule by using a slower titration schedule and employing dose modifications, including reductions or interruptions (e.g., dose reductions and interruptions may occur in patients receiving pirfenidone, e.g., 28 days' reduction and 14 days' interruption may occur during treatment). In addition, modification of eating habits of the patient may be required when adjusting the pirfenidone dose. Taking pirfenidone with a substantial amount of food, specifically the full dose at the end of a substantial meal or spreading out the three capsules during the meal, may reduce the rate of pirfenidone absorption and mitigate the onset of GI-related AEs.

Although slower titration and dose modification may assist in addressing patient AEs, employing such measures has significant therapeutic impact, notably patients who received pirfenidone 1197 mg/day were reported to experience greater lung function decline than patients who were receiving the full dose of 2403 mg/day.

In addition, pirfenidone treatment has liver function AE's, therefore, monitoring liver function is also important during pirfenidone treatment. Elevations of aspartate transaminase (AST) and alanine transaminase (ALT) levels to >3x the upper limit of normal (ULN) occurred in the phase III trials (3.2%), which were managed by dose modifications or discontinuation. If AST and ALT elevations (>3x to ≤5x ULN) occur without symptoms or hyperbilirubinemia, the dose may be reduced or interrupted until values return to normal. However, in cases in which the AST and ALT elevations (>3x to<5x ULN) are accompanied by hyperbilirubinemia or if patients exhibit >5x ULN, pirfenidone must be permanently discontinued.

In addition, patients must be monitored for drug-drug interactions, because the patients taking other oral medications at the same time, may significantly affect pirfenidone metabolism by inhibiting or inducing hepatic enzyme systems (cytochrome P450 1A2 (CYP1A2), CYP3A4, P-glycoprotein). For example, for strong CYP1A2 inhibitors such as fluvoxamine and enoxacin, pirfenidone should be reduced to 267 mg three times daily (801 mg·day-1). For moderate CYP1A2 inhibitors, such as ciprofloxacin at a dosage of 750 mg twice daily, pirfenidone should be reduced to 534 mg three times daily (1602 mg·day-1). Patients should also be assessed for GI intolerance, skin reactions and liver enzyme elevations.

Accordingly, limitations of pirfenidone include: a short half-life of only about 2.5 hours; a high pill burden (of 9 capsules per day (TID dosing); poor tolerability including nausea, diarrhea and photosensitivity; a high dose required for efficacy that induces side effects; and significant interpatient variability. Moreover, pirfenidone treatment requires various AE management strategies, including a slower dose titration for initiating treatment, taking pirfenidone with substantial meals, spacing doses throughout the meal, diet modification, weight-based dosing regimens and dose reductions and interruptions, as well as continual liver function monitoring.

LYT-100, a new chemical entity, is a deuterated, oral small molecule designed to overcome the noted challenges associated with pirfenidone (e.g., compliance, dosing and tolerability issues). Specifically, LYT-100 is the deuterium-enriched pirfenidone, 5-(methyl-d₃)-1-phenylpyridin-2-(1*H*)-one. LYT-100 has the following structure:

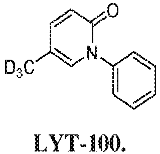

Reference to "LYT-100" herein further includes any hydrate, solvate, crystalline polymorph, amorphous form, or the like, of 5-(methyl-d₃)-1-phenylpyridin-2-(1*H*)-one. The preparation of LYT-100 has been disclosed in, for example, U.S. Patent Nos 8,383,823 and 9,018,232, and U.S. Patent Application Publication Nos 2009/0131485 and 2013/0018193, each of which is incorporated by reference herein. As described therein, the level of deuterium enrichment of the pirfenidone may vary. The natural abundance of deuterium is 0.015%, such that each hydrogen atom in non-deuterated pirfenidone naturally comprises about 0.015% deuterium. However, as used herein, reference to "deuterated" means that each atom referred to as "D" is enriched over the natural abundance of deuterium. In some embodiments, the deuterium enrichment at each position designated as D is of no less than about 1%, no less than about 5%, no less than about 10%, no less than about 20%, no less than about 50%, no less than about 70%, no less than about 80%, no less than about 90%. In particular embodiments, each atom referred to as "D" is enriched over the natural abundance of deuterium by at least a factor of 6000 (i.e., a given atom designated as D contains at least about 90% deuterium, with the remainder being hydrogen along with the trace amount of tritium naturally present. For example, each atom designated as D contains at least about 90%, at least about 95%, at least about 96%, at least about 97%, at least about 98%, at least about 99%, at least about 99.5%, or at least about 99.9% deuterium. Thus, while non-isotopically enriched pirfenidone will inherently contain small amounts of deuterated isotopologues, the concentration of naturally abundant stable hydrogen isotopes is small and immaterial as compared to the degree of stable isotopic substitution of compounds of this disclosure. See, for instance, Wada E et al., Seikagaku 1994, 66:15; Ganes L. Zet al., Comp Biochem. PhysiolMol Integr Physiol. 1998, 119:725.

LYT-100 retains the pharmacology of pirfenidone, but has a differentiated pharmacokinetic (PK) profile, which is designed to enable improved tolerability, less frequent dosing, and potentially increased efficacy. An advantage of LYT-100 is that it can be administered on a twice-a-day dosing schedule, in contrast to pirfenidone, which requires a three-times-a-day dosing schedule (801 mg three times a day for a total daily dose of 2403 mg, representing a total pill burden of 9 capsules). The anti-fibrotic and anti-inflammatory properties of LYT-100, along with the improved PK profile, may be beneficial in treating a range of fibrotic and inflammatory diseases. Particularly, respiratory conditions, including those associated with COVID-19- both in the early phases of the disease and as part of the often-long recovery- as well as interstitial lung diseases (ILDs), including progressive fibrosing ILDs (PF-ILDs) such as idiopathic pulmonary fibrosis (IPF), may benefit from treatment with LYT-100.

### Metabolites and Pharmacokinetics

Both LYT-100 and pirfenidone share a common major metabolite. An *in vitro* comparative mass balance study of pirfenidone and LYT-100 in human and rat liver microsomes and a comparative study of pirfenidone and LYT-100 metabolites in rat, dog, mouse, and monkey liver microsomes revealed that metabolism of both compounds was qualitatively similar and showed that deuteration did not introduce novel metabolites (although metabolism of LYT-100 occurred more slowly).

Following administration of either LYT-100 or pirfenidone in both rats and humans, the most abundant measured circulating metabolite was 5-carboxy-pirfenidone (LYT-105; SD-789), believed to result primarily through metabolic oxidation by cytochrome P450 subtype 1A2 (CYP1A2). Other metabolites detected include 4'-hydroxy-pirfenidone and the corresponding 4'-hydroxy-deupirfenidone (SD-1051), not quantifiable in humans; as well as 5-hydroxymethyl-pirfenidone (SD-788) and the corresponding 5-(hydroxymethyl-*d*₂)-pirfenidone (SD-790), both minor metabolites in humans.

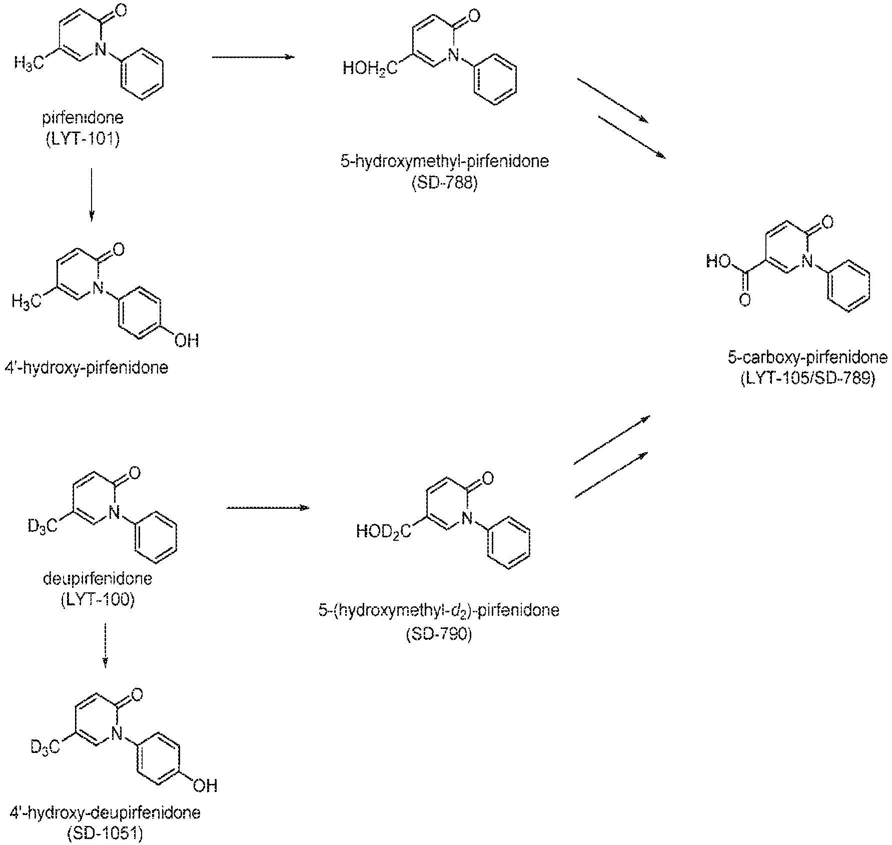

In humans, approximately 80% of an oral pirfenidone dose is excreted in urine, with the majority of recovered drug material (>95%) excreted as the 5-carboxy-pirfenidone metabolite and less than 1% as unchanged pirfenidone (InterMune UK, 2011; Rubino, 2009). In healthy volunteers, the half-life of pirfenidone is approximately 2 to 3 hours (Huang, 2013; Rubino, 2009; InterMune UK, 2011). Plasma exposure to 5-carboxy-pirfenidone is approximately 50% of the exposure to pirfenidone (Huang, 2013). Although the 5-hydroxymethyl-pirfenidone metabolite plasma levels are near or below level of quantification in the majority of human subjects after oral administration of 801 mg pirfenidone (Rubino, 2009), this pharmacologically active metabolite is prevalent in rat (PMDA, 2008). The 5-hydroxymethyl-pirfenidone metabolite has demonstrated anti-inflammatory and anti-fibrotic activity (PMDA, 2008; Togami et al., Possible involvement of pirfenidone metabolites in the antifibrotic action of a therapy for idiopathic pulmonary fibrosis. Biol Pharm Bull. 2013:36:1525-1527) while the 5-carboxy-pirfenidone metabolite has generally been described as pharmacologically inactive (PMDA, 2008; InterMune UK, 2011).

Referring to Example 6, in a single ascending dose (SAD) study, the pharmacokinetics of the active LYT-100 (deupirfenidone) and non-deuterated 5-carboxypirfenidone metabolite (LYT-105) were evaluated. Administration in the fed state of a single 801 mg dose of LYT-100 resulted in overall greater exposure (AUC, Cₘₐₓ) than observed with administration of an 801 mg dose of pirfenidone. No appreciable difference in the apparent elimination T_{½} or time to Cₘₐₓ was observed for the 2 compounds. Administration of the 801 mg dose of LYT-100 resulted in greater drug exposure than with the same pirfenidone dose, but surprisingly, the incidence of gastrointestinal and nervous system adverse events was not increased with LYT-100 administration as compared to pirfenidone.

Referring to Table 1, e.g., on average, after administration of LYT-100, the 5-carboxy-pirfenidone metabolite (LYT-105) represents 43.3% of the parent in comparison to 68.1% of the parent after administration of pirfenidone (Cₘₐₓ). On average, after administration of LYT-100, the 5-carboxy-pirfcnidonc metabolite (LYT-105) represented 43.8% of the parent in comparison to 65.9% of the parent after administration of pirfenidone (AUC). This difference in exposure was not associated with a change in half-life, suggesting formation, and not clearance, of this non-deuterated metabolite is affected by the deuterium substitution in the parent molecule.

| **Table 1: Summary of Metabolite Ratio** | | |
|---|---|---|
| **Pharmacokinetic Parameter** | **Metabolite/Parent Ratio %** | |
| | **Pirfenidone (LYT-101)** | **LYT-100** |
| | **LYT-105/ LYT-101** | **LYT-105/ LYT-100** |
| Cₘₐₓ (ng/mL) | 68.1% | 43.3% |
| AUC_{0-∞} | 65.9% | 43.8% |

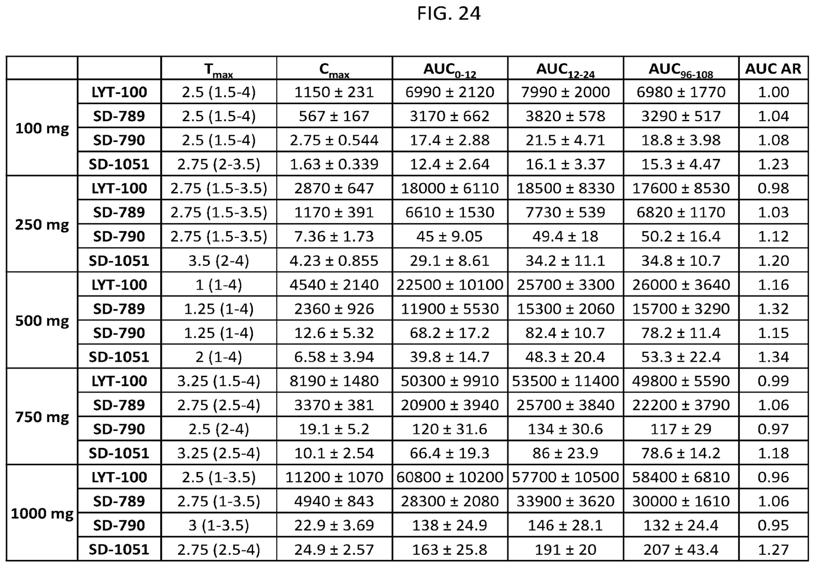

Referring to Example 7, in a multiple ascending dose (MAD) study in human subjects, similar results were observed across all dose Cohorts. The major metabolite was 5-carboxy-pirfenidone (SD-789) at all doses, and the average ratio of metabolite to the parent (M/P) by AUC was 0.45. Pharmacokinetic data for LYT-100 and each metabolite (SD-789, SD-790, and SD-1051) are provided for each dosing Cohort (100 mg, 250 mg, 500 mg, 750 mg, and 1000 mg BID) in **FIG. 24****,** which demonstrated similar accumulation ratios (~1) across all dose groups.

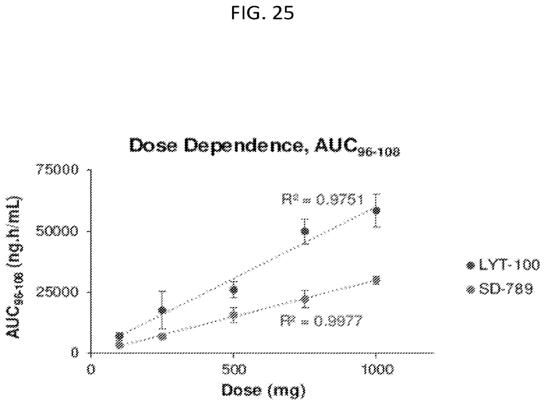

The dose dependence of AUC was evaluated for LYT-100 and 5-carboxypirfenidone across all dose Cohorts using the AUC₉₆₋₁₀₈ data points. The data **(****FIG. 25****)** demonstrated linear dose proportionality for both parent and major metabolite. Surprisingly, however, the linear trend of each had different slopes; the parent exposure increased with dose more rapidly than 5-carboxypirfenidone metabolite exposure. The 5-carboxypirfenidone metabolite is generally considered pharmacologically inactive (*See, e.g.,* FDA CDER NDA No. 022535Orig1s000; Clinical Pharmacology and Biopharmaceutical Review, and European Medicines Agency CHMP assessment report EMEA/H/C/002154, December 16, 2020); however, the Cₘₐₓ of pirfenidone and 5-carboxy-pirfendione have been correlated with GI adverse events (Rubino et al., Pulmonary Pharmacology & Therapeutics 22(2009), 279-285).

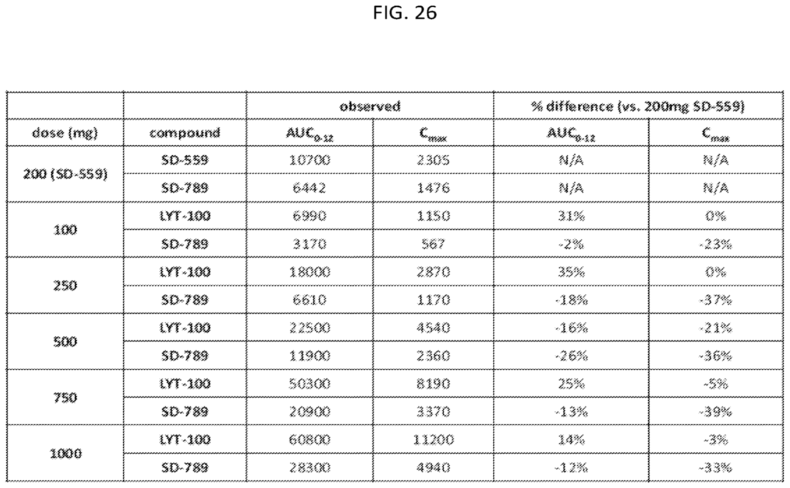

Data across the Cohorts was compared against data from Huang et. al. ("Pharmacokinctics, Safety and Tolerability of Pirfenidone and its Major Metabolite after Single and Multiple Oral Doses in Healthy Chinese Subjects under Fed Conditions." Drug Res (Stuttg) 63, 388-395; 2013; 200 mg BID pirfenidone), extrapolated to 100, 250, 500, 750, 1000 mg, assuming dose proportionality and comparing to AUC₀₋₁₂ and Cₘₐₓ, LYT-100 and 5-carboxy metabolite only **(Table 2;** **FIG. 26****).** With the exception of the 500 mg dose, there was an increase for LYT-100 AUC over that of pirfenidone and a decrease for the Cₘₐₓ of the 5-carboxy metabolite over that of same metabolite from pirfenidone.

| **Table 2: Comparison and Extrapolation of Data** | | | |
|---|---|---|---|
| | | **observed** | **% difference (vs. 200mg SD-559)** |
| **dose (mg)** | **compound** | **Cₘₐₓ** | **Cₘₐₓ** |
| **200 (SD-559)** | **SD-559** | 2305 | N/A |
| | **SD-789** | 1476 | N/A |
| **100** | **LYT-100** | 1150 | 0% |
| | **SD-789** | 567 | -23% |
| **250** | **LYT-100** | 2870 | 0% |
| | **SD-789** | 1170 | -37% |
| **500** | **LYT-100** | 4540 | -21% |
| | **SD-789** | 2360 | -36% |
| **750** | **LYT-100** | 8190 | -5% |
| | **SD-789** | 3370 | -39% |
| **1000** | **LYT-100** | 11200 | -3% |
| | **SD-789** | 4940 | -33% |

Also surprising is that LYT-100 demonstrated minimal food effect. Referring to **Table 3,** in the fed state, the AUC of LYT-100 was decreased 19% relative to that achieved when subjects were dosed in the fasted state, and in the fed state, the Cₘₐₓ of LYT-100 was decreased 23% relative to that achieved when subjects were dosed in the fasted state. There was a small increase for Tₘₐₓ.

**Table 3** further provides comparative data for pirfenidone (801 mg dose) under fed and fasted conditions as obtained in a prior study (See, e.g., Rubino et al., Pulmonary Pharmacology and Therapeutics. 22 (2009), 279-285). In the fed state, the AUC of pirfenidone was decreased 15% relative to that achieved when subjects were dosed in the fasted state (similar to LYT-100), the Cₘₐₓ was decreased 49% relative to that achieved when subjects were dosed in the fasted state. There was a large increase of 500% in Tₘₐₓ for pirfenidone.

**Table 3: LYT-100 and Pirfenidone, Fed vs Fasted**

| **Pharmacokinetic Parameter** | **Fasted** | **Fed** | **% Change Fed vs Fasted** |
|---|---|---|---|
| | **LYT-100 @500 mg** | **LYT-100 @500 mg** | |
| AUC | 60900 | 49500 | -19% |
| Cₘₐₓ | 10300 | 7900 | -23% |
| Tₘₐₓ | 1 hr | 1.25 hr | +25% |

| | **Pirfenidone @801 mg** | **Pirfenidone @801 mg** | |
|---|---|---|---|
| AUC | 69.7 | 59.3 | -15% |
| Cₘₐₓ | 15.7 | 7.87 | -49% |
| Tₘₐₓ | 0.5 hr | 3 hr | +500% |

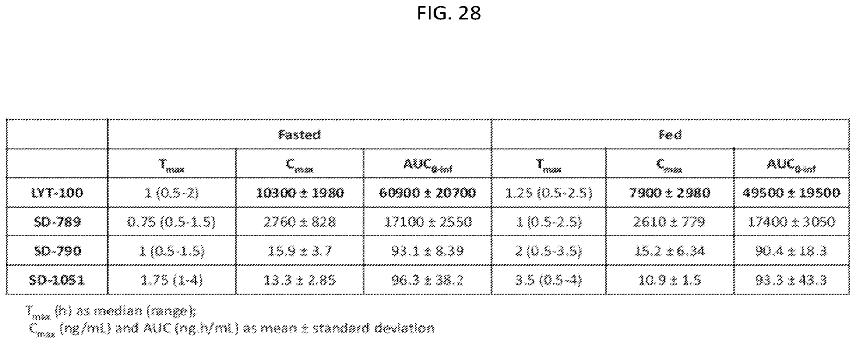

With respect to the metabolite food effect in the prior MAD study, surprisingly, there was no food effect on Cₘₐₓ or AUC for LYT-100 or the major metabolite 5-carboxy-pirfenidone **(Table 4).** Pharmacokinetic data for Cohort 5 (Tₘₐₓ, Cₘₐₓ, AUC_{0-inf}) for the active and each metabolite in fasted and fed subjects following a single 500 mg dose is provided in **FIG. 28****.** The data demonstrated that in the fed state, the Cₘₐₓ and AUC of LYT-100 were decreased relative to that achieved when subjects were dosed in the fasted state (23% and 19% decrease in Cₘₐₓ and AUC, respectively). Pirfenidone demonstrates a much greater food effect on Cₘₐₓ (49% decrease in fed state). For LYT-100, there was no food effect on Cₘₐₓ or AUC of LYT-100 metabolites. In contrast to food effects on pirfenidone (median Tₘₐₓ shift from 0.5 to 3), there was no food effect on the Tₘₐₓ of either LYT-100 or its metabolites.

**Table 4: 5-carboxypirfenidone for LYT-100 and Pirfenidone, Fed vs Fasted**

| **Pharmacokinetic Parameter** | **Fasted** | **Fed** | **% Change Fed vs Fasted** |
|---|---|---|---|
| | **LYT-100 @500 mg 5-CO₂ metabolite** | **LYT-100 @500 mg 5-CO₂ metabolite** | |
| AUC | 2760 | 2610 | -5% |
| Cₘₐₓ | 17100 | 17400 | -1.7% |
| Tₘₐₓ | 0.75 | 1 | +33% |

In some embodiments, the deuterated pirfenidone, e.g., LYT-100, has at least one of the following properties: a) decreased inter-individual variation in plasma levels of the compound or a metabolite thereof as compared to pirfenidone; b) increased average plasma levels of the compound per dosage unit thereof as compared to pirfenidone; c) decreased average plasma levels of at least one metabolite of the compound per dosage unit thereof as compared to pirfenidone; d) increased average plasma levels of at least one metabolite of the compound per dosage unit thereof as compared to pirfenidone; and e) an improved clinical effect during the treatment in the subject per dosage unit thereof as compared to pirfenidone. Thus, disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound, e.g., LYT-100; so as to effect one or more of a) - e) above during the treatment of the disorder as compared to pirfenidone. In some embodiments, the deuterium-enriched pirfenidone compound has at least two of the properties a) through e) above. In some embodiments, the deuterium-enriched pirfenidone compound has three or more of the properties a) through e) above. In one embodiment, administration of LYT-100 has the properties of increased AUC and Cₘₐₓ compared to pirfenidone; and decreased average plasma levels of 5-carboxy-pirfenidone as compared to pirfenidone at the same dose. Additionally, in some embodiments, administration of LYT-100 has minimal or no adverse events, or significantly reduced adverse events relative to pirfenidone. In some embodiments, the adverse events are one or more of headache, nausea, abdominal discomfort, abdominal distension, or headache. Additionally, in some embodiments, LYT-100 has an improved clinical effect during the treatment in the subject as compared to pirfenidone.

Disclosed herein are methods of reducing the level of 5-carboxypirfenidone in a subject, comprising administering a pirfenidone derivative, or a pharmaceutically acceptable salt thereof, to a subject in need thereof. In some embodiments, the level of 5-carboxypirfenidone is relative to that in a subject treated with pirfenidone. A pirfenidone "derivative" as used herein generally refers to a pirfenidone molecule that has been functionalized or chemically altered. In some embodiments, the functionalization or chemical alteration includes deuteration, fluorination, or the bioisosteric replacement of one or more functional groups. The term "bioisosteric groups" or "bioisosteres," or the like, as used herein, refers to substituents or functional groups with similar physical or chemical properties that confers broadly similar biological properties to a chemical compound, as generally understood in medicinal chemistry or pharmaceutical sciences.

In some embodiments, the functionalization or chemical alteration can include replacement of one or more hydrogen atoms of the 5-methyl group of pirfenidone with deuterium, as described herein above.

In some embodiments, the functionalization or chemical alteration can include replacement of one or more hydrogen atoms of the 5-methyl group of pirfenidone with an alkyl group. As used herein, "alkyl" refers to any saturated (i.e., having all *sp³* carbon atoms) straight chain or branched chain hydrocarbon. In some embodiments, an alkyl group contains one to twelve carbon atoms (C₁-C₁₂). In some embodiments, an alkyl group contains one to eight carbon atoms (C₁-C₈). In some embodiments, an alkyl group contains one to six carbon atoms (C₁-C₆). In some embodiments, an alkyl group contains one to four carbon atoms (C₁-C₄). Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl.

In some embodiments, the functionalization or chemical alteration can include replacement of one or more hydrogen atoms of the 5-methyl group of pirfenidone with an alkenyl group. As used herein, "alkenyl" refers to any unsaturated (i.e., having at least one site of unsaturation) straight chain or branched chain hydrocarbon with at least one carbon-carbon double bond (i.e., at least two *sp*²-hybridized carbon atoms). In some embodiments, an alkenyl group contains two to six carbon atoms (C₂-C₆). In some embodiments, an alkenyl group contains two to four carbon atoms (C₂-C₄). Alkenyl groups may have E or Z orientations. As used herein, the term "unsaturated" refers to the presence of a carbon-carbon double or triple bond in one or more positions within the hydrocarbon. Unsaturated hydrocarbons may be mono- or polyunsaturated. Representative alkenyl groups include, but are not limited to, ethylene or vinyl, allyl, 1-butenyl, 2-butenyl, isobutylenyl, 1-pentenyl, 2-pentenyl. 3-methyl-1-butenyl, 2-methyl-2-butenyl, 2,3-dimethyl-2-butenyl, and the like.

In some embodiments, the functionalization or chemical alteration can include replacement of one or more hydrogen atoms of the 5-methyl group of pirfenidone with an alkynyl group. As used herein, "alkynyl" refers to an unsaturated straight chain or branched chain hydrocarbon with at least one carbon-carbon triple bond (i.e., at least two sp-hybridized carbon atoms). In some embodiments, an alkynyl group contains two to six carbon atoms (C₂-C₆). In some embodiments, an alkynyl group contains two to four carbon atoms (C₂-C₄). Representative alkynyl groups include, but are not limited to, ethynyl, propargyl, and 1-butynyl.

Alkyl, alkenyl, and alkynyl groups can be unsubstituted or substituted. "Substituted" as used herein means that one or more hydrogen atoms are each independently replaced with a substituent. Typical substituents include, but are not limited to, -Cl, -Br, -F, -alkyl, -OH, -OCH₃, NH₂, -NHCH₃, -N(CH₃)₂, -CN, -NC(=O)CH₃, -C(=O)-, -C(=O)NH₂, and -C(=O)N(CH₃)₂.

In some embodiments, the functionalization or chemical alteration can include replacement of one or more hydrogen atoms of the 5-methyl group of pirfenidone with an electron-withdrawing substituent. Electron-withdrawing substituents include, but are not limited to, halogen (e.g., fluorine, chlorine, bromine), haloalkyl (e.g., -CF₃, -CF₂H), and -C(O)OR, wherein R is an alkyl, alkenyl, alkynyl, or aryl group. The term "aryl" as used herein refers to an aromatic hydrocarbon group. An aryl group can be unsubstituted or substituted as defined herein above. Examples of unsubstituted and substituted aryl groups include, but are not limited to, phenyl, 2-fluorophenyl, 3-fluorophenyl, 4-fluorophenyl, 2-methylphenyl, 3-methylphenyl, 4-methylphenyl, 2-chlorophenyl, 3-chlorophenyl, 4-chlorophenyl, 2,6-dichlorophenyl, 3,4-difluorophenyl, 2-hydroxyphenyl, 3-hydroxyphenyl, 4-hydroxyphenyl, 2-methoxyphenyl, 3-methoxyphenyl, 4-methoxyphenyl, 2-cyanophenyl, 3-cyanophenyl, 4-cyanophenyl, 2-dimethylaminophenyl, 3-dimethylaminophenyl, 4-dimethylaminophenyl, 3-methylsulfonylphenyl, 4-methylsulfonylphenyl, 3-aminophenyl, 3-methylaminophenyl, 3-(2-hydroxyethoxy)phenyl, 2-trifluoromethylphenyl, 3-trifluoromethylphenyl, 4-trifluoromethylphenyl, 2-isopropylphenyl, 3-isopropylphenyl, 4-isopropylphenyl, 1-naphthyl, and 2-naphthyl.

Exemplary pirfenidone derivatives include, without limitation, a halogenated derivative of pirfenidone, a deuterium-enriched derivative of pirfenidone, a pirfenidone further substituted on the phenyl ring, the pyridone ring, or both, and the like.

Further disclosed herein is a method of reducing adverse events arising from pirfenidone treatment, comprising administering a pirfenidone derivative, or a pharmaceutically acceptable salt thereof, to a subject in need thereof. In some embodiments, the adverse events are reduced by reducing the level of 5-carboxypirfenidone in a subject. In some embodiments, the functionalization or chemical alteration includes deuteration, fluorination, or bioisosteric replacement of one or more functional groups. In some embodiments, the functionalization or chemical alteration can include replacement of one or more hydrogen atoms of the 5-methyl group of pirfenidone with deuterium. In some embodiments, the functionalization or chemical alteration can include replacement of one or more hydrogen atoms of the 5-methyl group of pirfenidone with an alkyl group. In some embodiments, the functionalization or chemical alteration can include replacement of one or more hydrogen atoms of the 5-methyl group of pirfenidone with an electron-withdrawing functional group.

Disclosed herein is a pharmaceutical composition comprising a (a) means for reducing the levels of 5-carboxyperfenidone in a subject relative to those found in a subject treated with pirfenidone, and (b) a pharmaceutically acceptable carrier.

Also disclosed herein is a pharmaceutical composition comprising a (a) means for reducing the adverse effects associated with pirfenidone treatment, and (b) a pharmaceutically acceptable carrier.

### Adverse Event Profile

Without wishing to be bound by any particular theory, it is believed that the improved metabolic stability and/or the attenuated formation of the 5-carboxy-prifenidone major metabolite seen with LYT-100 may contribute to improved tolerability, less frequent dosing, reduced pill burden, and better treatment compliance with LYT-100 vs. pirfenidone, all of which may translate to an overall improvement in treatment outcomes. Accordingly, adverse events observed in the MAD study were evaluated and considered with respect to the exposure levels to LYT-100 and the metabolite. Based on the blinded data obtained in the MAD study, all adverse events were mild and transient. Referring to Example 7, the most common events were headache and mild abdominal discomfort. Further, in contrast to pirfenidone (Phase I study, PIPF-005), surprisingly, LYT-100 did not demonstrate a dose-related increase in adverse events based on the MAD data. For example, the low 250 mg BID dose had the most AEs, while the high dose (1000 mg) had the lowest (two incidences of headache). There were no dose limiting toxicities observed, and no maximally tolerated dose was reached.

### Definitions

While the terms used herein are believed to be well understood by one of ordinary skill in the art, definitions are set forth herein to facilitate explanation of the presently-disclosed subject matter.

The term "adverse event" or "AE" refers to any any event, side-effect, or other untoward medical occurrence that occurs in conjunction with the use of a medicinal product in humans, whether or not considered to have a causal relationship to this treatment. An AE can, therefore, be any unfavorable and unintended sign (that could include a clinically significant abnormal laboratory finding), symptom, or disease temporally associated with the use of a medicinal product, whether or not considered related to the medicinal product. Events meeting the definition of an AE include: (1) Exacerbation of a chronic or intermittent pre-existing condition including either an increase in frequency and/or intensity of the condition; (2) New conditions detected or diagnosed after study drug administration that occur during the reporting periods, even though they may have been present prior to the start of the study; (3) Signs, symptoms, or the clinical sequelae of a suspected interaction; (4) Signs, symptoms, or the clinical sequelae of a suspected overdose of either study drug or concomitant medications (overdose per se will not be reported as an AE/SAE).

Events that do not meet the definition of an AE include: (1) Medical or surgical procedure (e.g., endoscopy, appendectomy); the condition that leads to the procedure should be reported as an AE if it meets the criteria of an AE; (2) Situations where an untoward medical occurrence did not occur (e.g., social and/or convenience admission to a hospital); (3) Anticipated day-to-day fluctuations of pre-existing disease(s) or condition(s) present or detected at the start of the study that do not worsen. Severity (intensity) of AEs may be graded as one of: Mild (Grade 1): A type of AE that is usually transient and may require only minimal treatment or therapeutic intervention. The event does not generally interfere with usual activities of daily living. Moderate (Grade 2): A type of AE that is usually alleviated with additional specific therapeutic intervention. The event interferes with usual activities of daily living, causing discomfort but poses no significant or permanent risk of harm to the research participant. Severe (Grade 3): A type of AE that interrupts usual activities of daily living, or significantly affects clinical status, or may require intensive therapeutic intervention. Life-threatening (Grade 4): A type of AE that places the participant at immediate risk of death. Death (Grade 5): Events that result in death. AEs may be categorized as not related (the event is clearly related to other factors such as the participant's environment or clinical state, therapeutic interventions or concomitant drugs administered to the participant), possibly related (the event follows a reasonable temporal sequence from the time of study drug administration or follows a known response to the study drug but could have been produced by other factors such as the participant's clinical state, other therapeutic interventions, or concomitant drugs administered to the participant), or probably related (the event follows a reasonable temporal sequence from the time of study drug administration and follows a known response to the study drug and cannot be reasonably explained by other factors such as the participant's clinical state, other therapeutic interventions, or concomitant drugs administered to the participant.).

An "anti-angiotensin agent" is a molecule that inhibits the activity of AngI or AngII, or a molecule that inhibits AngI to AngII conversion (e.g., ACE inhibitor or ACE agonist). Examples of anti-angiotensin agents include captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099.

An "anti-T cell agent" is a molecule that reduces T cell-mediated inflammation, T cell activation, T cell differentiation, and/or T cell proliferation. Classes of anti-T cell agents include calcineurin inhibitors and IL-2 inhibitors. Examples of small molecule anti-T cell agents include tacrolimus, teriflunomide, leflunomide, cyclosporine, and pimecrolimus. Examples of macromolecule anti-T cells agents include denileukin diftitox and Basiliximab.

An "anti-TGF-β1 agent" is a molecule that inhibits the expression, secretion, activation, signaling, or activity of transforming growth factor beta 1. Pirfenidone and deuterium-enriched pirfenidone are examples of small molecule anti-TGF-β1 agents.

As used herein, the term "clinically effective amount," "clinically proven effective amount," and the like, refer to an effective amount as shown through a clinical trial, e.g., a U.S. Food and Drug Administration (FDA) clinical trial.

As used herein, the term "clinical efficacy" refers to the ability to produce a desired effect in humans as shown through a Food and Drug Administration (FDA) clinical trial.

The term "collagen infiltration" refers to the entry of the connective tissue collagen into cells or into the extracellular matrix around cells. This occurs in organs and tissues naturally and under normal circumstances but can occur excessively and accompany or cause disease.

The terms "collagen-infiltration modulator" or "modulating collagen infiltration" are meant to be interchangeable and refer to the ability of a compound disclosed herein to alter the occurrence and/or amount of collagen infiltration. A fibrosis modulator may increase the occurrence or level of collagen infiltration, may increase or decrease the occurrence and/or amount of collagen infiltration depending on the concentration of the compound exposed to the adrenergic receptor, or may decrease the occurrence and/or amount of collagen infiltration. Such activation or inhibition may be contingent on the occurrence of a specific event, such as activation of a signal transduction pathway, and/or may be manifest only in particular cell types.

The term "collagen-mediated disorder" refers to a disorder that is characterized by abnormal or undesired collagenic infiltration, that when collagen infiltration activity is modified, leads to the desired responses depending on the route of administration and desired end result. A collagen-mediated disorder may be completely or partially mediated through the modulation of collagen infiltration. In particular, a collagen-mediated disorder is one in which modulation of collagen infiltration activity results in some effect on the underlying disorder, e.g., administering a collagen-infiltration modulator results in some improvement in at least some of the patients being treated. In some embodiments, the "collagen-mediated disorder" relates to or originates from a viral infection. In some embodiments, the "collagen-mediated disorder" relates to or originates from a corona virus infection, e.g., COVID-19. In some embodiments, the "collagen-mediated disorder" does not relate to or does not originate from a corona virus infection. In some embodiments, the "collagen-mediated disorder" does not relate to or does not originate from COVID-19.

The term "COVID-19" refers to a respiratory illness caused by the beta-coronavirus, severe acute respiratory syndrome coronavirus 2 (SARS-CoV-2). COVID-19 is characterized especially by fever, cough, fatigue, and shortness of breath, and which may progress to pneumonia and respiratory failure, such as acute respiratory distress syndrome (ARDS).

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods, such as mass spectrometry and nuclear magnetic resonance spectroscopy.

The term "is/are deuterium." when used to describe a given variable position in a molecule or formula, or the symbol "D," when used to represent a given position in a drawing of a molecular structure, means that the specified position is enriched with deuterium above the naturally occurring distribution of deuterium. In some embodiments, deuterium enrichment is of no less than about 1%, no less than about 5%, no less than about 10%, no less than about 20%, no less than about 50%, no less than about 70%, no less than about 80%, no less than about 90%, no less than about 98%, or in some embodiments no less than about 99% of deuterium at the specified position. In some embodiments, the deuterium enrichment is above 90% at each specified position. In some embodiments, the deuterium enrichment is above 95% at each specified position. In some embodiments, the deuterium enrichment is about 99% at each specified position.

"Disorder", "condition", "disease", "syndrome" is meant to be used as interchangeable terms to refer to a biological state that differs from the normal and/or healthy state at the cellular, tissue, organ and/or organism level, e.g., the tissue(s) of one or more organ(s) is affected such that by appearance and/or function it differs from normal and/or healthy tissue. In some embodiments, it refers to an abnormal biological state at the cellular, tissue, organ and/or organism level.

The term "dyspnea" refers to difficult or labored breathing, and may be quantitated using, for example, the modified Borg dyspnea scale (MBDS). Dyspnea is common in ARDS and viral pneumonia patients (Cavallazzi and Ramirez, Clin Chest Med 39, 703-721(2018)), and is a common symptom of COVID-19 (Goyal et al., New England J. Med. 382, 2372-2374 (2020)) that typically develops after the first symptoms (Wang et al. JAMA, 2020; 323(11):1061-1069). It is related to disease severity and is predictive of mortality (Xie et al., 2020). Dyspnea is also one of the most common reported symptoms in the emerging post-discharge data in COVID-19, consistent with the lingering dyspnea present in nearly a third of SARS survivors a year after infection (Ong et. al., CHEST 128, 1393-1400 (2005)).

An "effective amount" of a composition as disclosed herein is an amount sufficient to carry out a specifically stated purpose. An "effective amount" can be determined empirically and in a routine manner, in relation to the stated purpose, route of administration, and dosage form.

The term "fibrosis" refers to the deposition of extracellular matrix components, excessive fibrous connective tissue, or scarring within an organ or tissue.

The term "fibrosis-inducing infection" refers to any viral or bacterial infection that can contribute to tissue remodeling, inflammation or fibrosis, e.g., by triggering an immune response and/or sustained inflammation.

The term "fibrotic-mediated disorder" refers to a disorder that is characterized by abnormal or undesired fibrotic activity, that when fibrosis activity is modified, leads to the desired responses depending on the route of administration and desired end result. A fibrosis-mediated disorder may be completely or partially mediated through the modulation of fibrosis. In particular, a fibrosis-mediated disorder is one in which modulation of fibrosis activity results in some effect on the underlying disorder, e.g., administering a fibrosis modulator results in some improvement in at least some of the patients being treated. In some embodiments, the "fibrotic-mediated disorder" relates to or originates from a viral infection. In some embodiments, the "fibrotic-mediated disorder" relates to or originates from a corona virus infection, e.g., COVID-19. In some embodiments, the "fibrotic-mediated disorder" does not relate to or does not originate from a corona virus infection. In some embodiments, the "fibrotic-mediated disorder" does not relate to or does not originate from COVID-19.

The terms "fibrosis modulator" or "modulating fibrosis" are meant to be interchangeable and refer to the ability of a compound disclosed herein to alter the occurrence and/or amount of fibrosis. A fibrosis modulator may increase the occurrence or level of fibrosis, may increase or decrease the occurrence and/or amount of fibrosis depending on the concentration of the compound exposed to the adrenergic receptor, or may decrease the occurrence and/or amount of fibrosis. Such activation or inhibition may be contingent on the occurrence of a specific event, such as activation of a signal transduction pathway, and/or may be manifest only in particular cell types.

The term "idiopathic pulmonary fibrosis (IPF)" refers to a type of lung disease that results in scarring of the lungs (pulmonary fibrosis) for unknown reasons (i.e., the disease state is of unknown origin). In some embodiments, the method of treating a viral respiratory disease disclosed herein specifically excludes treatment of IPF.

The term "improved clinical effect" as used herein broadly encompasses any clinical effect that confers a therapeutic benefit over current and/or standard and/or approved therapeutic methods. For example, in some instances, it can refer to an improvement of a combination therapy over a monotherapy; an improvement relating to a method or route of administration over another route of administration; and/or the use of one compound over another compound, including e.g., the use of salts. pro-drugs, variants, analogs, derivatives, etc of one compound over a parent compound. Non-limiting examples of an improved clinical effect include therapeutic methods and/or compounds that: a) reduce or eliminate unwanted metabolites, b) increase the half-life of the parent drug, c) decrease the number of doses needed to achieve a desired effect, d) decrease the amount of a dose needed to achieve a desired effect, e) increase the formation of active metabolites, if any are formed, f) decrease the production of deleterious metabolites in specific tissues and/or create a more effective drug and/or a safer drug for polypharmacy, whether the polypharmacy be intentional or not, g) maintain the beneficial aspects of a corresponding compound while substantially increasing the maximum tolerated dose, h) decrease toxicity, i), lower the maximum plasma concentration (Cₘₐₓ) of the minimum efficacious dose (MED), j) lower the efficacious dose and thus decrease the non-mechanism-related toxicity and/or lower the probability of drug-drug interactions, k) decrease pill burden; 1) increased patient tolerability; m) a lower efficacious dose; n) increased bioavailability; o) increase Cₘₐₓ; m) increase Tₘₐₓ; and n) effect an increase in systemic exposure during the treatment in the subject per dosage unit thereof as compared to parent compound. "Prevent" or "prevention" refers to prophylactic or preventative measures that obstruct, delay and/or slow the development of a targeted pathologic condition or disorder or one or more symptoms of a targeted pathologic condition or disorder. Thus, those in need of prevention include those at risk of or susceptible to developing the disorder. Subjects that are at risk of or susceptible to developing lymphedema include, but are not limited to, cancer patients undergoing radiation therapy, chemotherapy, and/or surgical lymph node dissection. In some embodiments, a disease or disorder is successfully prevented according to the methods provided herein if the patient develops, transiently or permanently, e.g., fewer or less severe symptoms associated with the disease or disorder, or a later onset of symptoms associated with the disease or disorder, than a patient who has not been subject to the methods of the invention.

The terms "infection", "infection-related disorder", "infection-related disease", infection-related condition", and "infection-related syndrome" refers to a disorder, disease, condition, or syndrome arising as the result of an infection, which is an invasion of a host organism's bodily tissues by a disease-causing organism (e.g., bacteria, virus, fungus, protozoon, worm, etc.) and their multiplication, and the reaction of host tissues to these organisms and the toxins they produce. Infections are caused by microorganisms such as viruses, prions, bacteria, and viroids, and larger organisms like parasites and fungi. Hosts can fight infections using their immune system, e.g., mammalian hosts react to infections with an innate response, often involving inflammation, followed by an adaptive response. Thus, inflammation is typically associated with an infection and infection-related diseases, disorders, conditions. In some cases, an infection-related disease or disorder may develop into an interstitial lung disease or some other pulmonary disorder and therefore the terms "infection-related disorder/disease/condition/syndrome" can encompass an interstitial lung disease, such as ILD, IPF, chILD, and any of the other interstitial lung disorders discussed herein, or another pulmonary disorder which results in lung tissue injury, damage, and/or remodeling. In some embodiments, the "infection" or "infection-related disorder" is a viral infection or relates to a viral infection. In some embodiments, the "infection" or "infection-related disorder" is a corona virus infection or relates to a corona virus infection, e.g., COVID-19. In some embodiments, the "infection" or "infection-related disorder" is not a corona virus infection or does not relate to a corona virus infection. In some embodiments, the "infection" or "infection-related disorder" is not a SARS-CoV-2 infection or does not relate to a SARS-CoV-2 infection. The terms "inflammation-related disorder", "inflammatory-mediated disorder", "inflammatory-mediated condition" and any other similar terms used herein referring to inflammation, are meant to be used interchangeably in this disclosure to refer to any disease, disorder, condition, or syndrome related to an inflammation state or an inflammatory response that is characterized by abnormal or undesired inflammatory activity. Inflammation is part of a complex biological response of vascular tissues to harmful stimuli, such as pathogens, damaged cells, or irritants as a protective attempt by an organism to remove the injurious stimuli and to initiate the healing process. Thus, an inflammatory disease, disorder, condition, or syndrome can arise from an inflammation state and/or an inflammatory response and/or arises as a result of one or more pathogens or infectious agents and/or one or more inflammatory agents or mediators of the host organism. The classical signs of acute inflammation are pain, heat, redness, swelling, and loss of function. As used herein, the inflammation-related or inflammatory-mediated disease, disorder, condition, or syndrome is not meant to be limited to a condition relating to or resulting from acute inflammation, but rather is not defined or limited by time or duration and can also relate to a condition that develops over a longer period of time from an inflammation state or inflammatory response. Thus, an inflammation-related disease or inflammatory-mediated disorder and the like can be a temporary and/or acute disease, disorder, condition, or syndrome, or can be a chronic disease, disorder, condition, or syndrome arising or originating from an inflammatory response and/or is maintained by an inflammatory response or inflammation state. In some cases, an inflammation-related disease or inflammatory-mediated disorder may develop into an interstitial lung disease or some other pulmonary disorder and therefore the terms "inflammation-related disorder/disease/condition/syndrome" or "inflammatory-mediated disorder/disease/condition/syndrome" can encompass an interstitial lung disease, such as ILD, IPF, chILD, and any of the other interstitial lung disorders discussed herein, or another pulmonary disorder which results in lung tissue injury, damage, and/or remodeling. In some embodiments, the "inflammation-related disorder" or "inflammatory-mediated disorder" is related to or originates from a viral infection. In some embodiments, the "inflammation-related disorder" or "inflammatory-mediated disorder" is related to or originates from a corona virus infection, e.g., COVID-19. In some embodiments, the "inflammation-related disorder" or "inflammatory-mediated disorder" is not related to or does not originate from a corona virus infection. In some embodiments, the "inflammation-related disorder" or "inflammatory-mediated disorder" is not related to or does not originate from a SARS-CoV-2 infection.

The term "interstitial pneumonia" refers to an inflammatory condition of the lungs affecting the interstitium (the tissue and space around the alveoli. Interstitial pneumonia is one subtype of pneumonia. It concerns alveolar epithelium, pulmonary capillary endothelium, basement membrane, and perivascular and perilymphatic tissues. Interstitial pneumonia is characterized by specific clinical, radiologic and pathologic features. Clinical characterization includes dyspnea and cough that may progress to respiratory failure. Histologically, this condition is characterized by diffuse alveolar inflammation and damage attributed to bacteria, viruses or fungi that can infect the lung parenchymal alveolar and extra-alveolar functional units. Interstitial pneumonia can result in pulmonary fibrosis that reduces lung function, even after the acute insult has resolved.

The term "isotopic enrichment" refers to the percentage of incorporation of a less prevalent isotope of an element at a given position in a molecule in the place of the more prevalent isotope of the element. The term "non-isotopically enriched" refers to a molecule in which the percentages of the various isotopes are substantially the same as the naturally occurring percentages.

The term "non-critical" as used herein refers to a subject who is, or a disease population where the subjects are, not intubated, and not receiving invasive mechanical ventilation or extracorporeal membrane oxygenation (ECMO) at the time of initiating the treatment. The subject may still be in need of supplemental oxygen, e.g., via nasal cannula. Non-critical subjects or patients include those who have acute COVID-19 respiratory illness, exhibiting pneumonia, dyspnea, cough, radiologic evidence of lung inflammatory processes, and/or oxygenation deficits. The subject may or may not be hospitalized. Non-critical subjects or patients further include those in the recovery ("chronic") phase of COVID-19 respiratory illness. The subject may or may not be hospitalized. Such subjects include those who are not hospitalized, including, for example, those who were previously hospitalized with acute COVID-19 respiratory illness, but which have been discharged, referred to herein is "post-discharge" patients, and those with persistent impairment of pulmonary function subsequent to an acute COVID-19 respiratory illness.

The term "pharmaceutical composition" refers to a preparation that is in such form as to permit the biological activity of the active ingredient to be effective, and which contains no additional components that are unacceptably toxic to a subject to which the composition would be administered. Pharmaceutical compositions can be in numerous dosage forms, for example, tablet, capsule, liquid, solution, soft gel, suspension, emulsion, syrup, elixir, tincture, film, powder, hydrogel, ointment, paste, cream, lotion, gel, mousse, foam, lacquer, spray, aerosol, inhaler, nebulizer, ophthalmic drops, patch, suppository, and/or enema. Pharmaceutical compositions typically comprise a pharmaceutically acceptable carrier, and can comprise one or more of a buffer (e.g. acetate, phosphate or citrate buffer), a surfactant (e.g. polysorbate), a stabilizing agent (e.g. human albumin), a preservative (e.g. benzyl alcohol), a penetration enhancer, an absorption promoter to enhance bioavailability and/or other conventional solubilizing or dispersing agents. Choice of dosage form and excipients depends upon the active agent to be delivered and the disease or disorder to be treated or prevented, and is routine to one of ordinary skill in the art.

The term "pneumonia" as used herein refers to an inflammatory condition of the lung affecting primarily the small air sacs ("alveoli") in one or both lungs. The air sacs may fill with fluid or pus, causing cough with phlegm or pus, fever, chills, and difficulty breathing. A variety of organisms, including bacteria, viruses and fungi, can cause pneumonia; however. as used herein, pneumonia refers to the inflammation caused by viral infection (e.g., with a beta-coronavirus such as SARS-CoV, SARS-CoV-2, MERS-CoV, HCoV-HKU 1, or HCoV-OC43. In particular embodiments, the causative virus is SARS-CoV-2. In particular embodiments, the pneumonia is interstitial pneumonia.

The terms "related" and "mediated" as used herein are meant to be used interchangeably to refer to an association or involvement, whether direct or indirect association/involvement. Thus, for example, the term "infection-related disorder" and the term "inflammatory-mediated disorder" are meant to refer broadly to disorders that are associated with and/or have some involvement with infection or inflammation, respectively.

The term "SF-36" refers to a well-established patient reported outcome (PRO) quality of life measure. The SF-36 questionnaire is a self-administered questionnaire containing 36 items that measures health on functional status, well-being and overall evaluation of health in 8 domains.

The term "Six (6) Minute Walk Test (6MWT)" refers to a test which measures the distance a patient can quickly walk on a flat, hard surface in a period of 6 minutes. It evaluates the global and integrated response of all the systems involved during exercise.

Terms such as "treating" or "treatment" or "to treat" or "alleviating" or "to alleviate" or "ameliorate" refer to therapeutic measures that cure, slow down, ameliorate or lessen one or more symptoms of, halt progression of, and/or ameliorate or lessen a diagnosed pathologic condition or disorder. Thus, those in need of treatment include those already with the disorder. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence. In some embodiments, a subject is successfully "treated" for a disease or disorder according to the methods provided herein if the patient shows, e.g., total, partial, or transient alleviation or elimination of symptoms associated with the disease or disorder. For example, "treating" an infection-related disorder or an inflammation-related disorder or a fibrotic-related disorder or a collagen-related disorder refers to a total, partial, or transient alleviation or elimination of symptoms associated with each of those diseases or disorders, as described herein. In some embodiments, "treating" refers to one or more of reducing uncontrolled inflammation, alleviating the consequence of immune dysregulation, alleviating the consequence of fibroproliferative response to the viral-induced inflammation, and preventing the emergence or progression of pulmonary fibrosis. In some embodiments, "treating" refers to totally, partially, or transiently alleviating or eliminating the respiratory symptoms associated with the pulmonary fibrosis that develops subsequent to COVID-19 induced pneumonia. Non-limiting example of respiratory symptoms to treat or prevent include cough, dyspnea, and exercise intolerance. In some embodiments, "treating" refers to preventing the development of, halting the progression of, slowing the progression of, or otherwise ameliorating the pulmonary fibrosis that develops subsequent to viral (e.g., COVID-19) induced pneumonia. In some embodiments, "treating" refers to preventing the development of, halting the progression of, slowing the progression of, or otherwise ameliorating chronic or permanent lung fibrosis, scarring, and/or pulmonary dysfunction that develops subsequent to viral (e.g., COVID-19) induced pneumonia.

The term "viral respiratory disease" as used herein refers to a respiratory illness or disease or complication subsequent to, precipitated by, caused by, or related to, a viral infection. The causative agent may include a virus such as a human influenza virus (e.g., H1N1 or H7N9 strains), or a beta-coronavirus, including but not limited to, SARS-CoV-2. The viral respiratory disease may include COVID-19, or a viral pneumonia, including, but not limited to, COVID-19 related pneumonia.

Compounds of the present invention include those described generally herein, and are further illustrated by the classes, subclasses, and species disclosed herein. As used herein, the following definitions shall apply unless otherwise indicated. For purposes of this invention, the chemical elements are identified in accordance with the Periodic Table of the Elements, Handbook of Chemistry and Physics, 98th Ed. Additionally, general principles of organic chemistry are described in "Organic Chemistry," Thomas Sorrell, University Science Books, Sausalito: 1999, and March's Advanced Organic Chemistry: Reactions, Mechanisms, and Structure, M. B. Smith and J. March, 7th Edition, John Wiley & Sons, 2013, the entire contents of which are hereby incorporated by reference.

Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a 13C- or 14C-enriched carbon are within the scope of this invention. Such compounds are useful, for example, as analytical tools, as probes in biological assays, or as therapeutic agents in accordance with the present invention.

Disclosed compounds, as well as pharmaceutically acceptable compositions comprising a disclosed compound and a pharmaceutically acceptable excipient, adjuvant, diluent, or carrier, are useful for treating a variety of diseases, disorders, and conditions. Such diseases, disorders, and conditions include those described herein.

All method steps described herein can be performed in any suitable order unless otherwise indicated herein or otherwise clearly contradicted by context. The use of any and all examples, or exemplary language (e.g., "such as") provided herein, is intended merely to better illuminate the materials and methods and does not pose a limitation on the scope unless otherwise claimed. No language in the specification should be construed as indicating any non-claimed element as essential to the practice of the disclosed materials and methods.

It will be readily apparent to one of ordinary skill in the relevant arts that suitable modifications and adaptations to the compositions, methods, and applications described herein can be made without departing from the scope of any embodiments or aspects thereof. The compositions and methods provided are exemplary and are not intended to limit the scope of the claimed embodiments. All of the various embodiments, aspects, and options disclosed herein can be combined in all variations. The scope of the compositions, formulations, methods, and processes described herein include all actual or potential combinations of embodiments, aspects, options, examples, and preferences herein.

One of ordinary skill in the art will recognize that each of the therapeutic agents described herein are known to be associated with treatment of one or more diseases, disorders, or conditions. Accordingly, it will be understood that, in certain embodiments, the present disclosure provides a method of treating a disease, disorder, or condition in a patient in need thereof, comprising administering to the patient an effective amount of a disclosed compound, combination of compounds, or pharmaceutical composition thereof.

Disclosed herein are methods for the treatment, prevention, and/or amelioration of an infection, an infection-related disorder, an inflammation-related or inflammatory-mediated disorder, fibrotic-mediated disorder, and/or a collagen-mediated disorder comprising administering to a subject in need thereof a deuterium-enriched pirfenidone compound disclosed herein, e.g., LYT-100. Such infections, and related diseases and disorders, are described further herein below.

### Infection, Inflammation, and Fibrosis

The biological response to an infection, e.g. a viral or bacterial infection, includes inflammation which takes place in the affected organ, such as in the heart, pancreas, liver, kidney, lung, brain, intestinal tract and reproductive system, potentially leading to tissue damage or disease. At the tissue level, infection-induced inflammation is characterized by redness, swelling, heat, pain, and loss of tissue function, which result from local immune, vascular and inflammatory cell responses to infection. Regardless of the location of the infection in the body, the inflammatory response has certain key steps in common. The infectious virus, bacterium or other pathogen is recognized by cell surface pattern receptors, and this triggers intracellular inflammatory signaling pathways, such as the NF-κB, MAPK, and JAK-STAT signaling pathways. Consequently, pro-inflammatory cytokines are released, leading to the recruitment of pro-inflammatory immune cells to the site of infection. As part of the local immune response, activated macrophages, neutrophils, monocytes, and other cells are recruited to the site of infection. Inflammation-mediated immune cell alterations are associated with many diseases, conditions and disorders, including respiratory disorders, interstitial lung diseases, asthma, cancer, chronic inflammatory diseases, atherosclerosis, diabetes, fibrotic diseases, and autoimmune and degenerative diseases.

Infection also plays a role in fibrosis. Inflammation of infectious origin can become chronic and promote the development of fibrosis. The persistence of these infectious stimuli and the resulting sustained injury drives fibrosis through the promotion of changes in immune as well as structural cells. Persistent viral infections have also been found to promote and enhance fibrosis.

Tissue injury caused by infection and resulting inflammation is followed by the formation of a new extracellular matrix as part of the tissue repair process. Fibroblast activation and proliferation are key components in the tissue repair process, and activation of these cells is characterized by the production and deposition of ECM proteins such as collagen type I, collagen type III, fibronectin, elastin, proteoglycans, and laminin. If the tissue repair process is dysregulated, fibrosis occurs as a result. Persistent stimuli derived from pathogens (e.g., bacteria, viruses, fungi, and multicellular parasites) promote fibrosis in many organs, including those associated with the pulmonary, cardiovascular, integumentary, and alimentary systems. Major microorganisms that have been associated directly and/or indirectly with organ fibrosis include, but are not limited to, bacteria (e.g., Mycobacterium tuberculosis, Mycobacterium Leprae, Pseudomonas aeruginosa), viruses (e.g., HCV, HBV, respiratory syncytial virus, Herpes virus (EBV, CMV, HHVB, and MHV-68)), parasites (e.g., Schistosoma manosi, Toxoplasma gondii, Pneumocystis carinii, Cryptosporidium parvum, Leishmania spp., Thrichinella spiralis, Brugia malayi, and Taenia spp.), and fungi (e.g., Aspergillus fumigatus, Candida albicans, and Cryptococcus neoformans) (Meneghin et al., Infectious disease, the innate immune response, and fibrosis, J Clin Invest. 2007 Mar 1; 117(3): 530-538, and references therein).

Inflammatory processes, such as those experienced by a viral infection, can result in lung injury that leads to pulmonary fibrosis. Qiao et al., Pulmonary fibrosis induced by H5N1 viral infection in mice, Respir Res 10(1): 107 (2009). Acute lung inflammation can result from tissue exposure to bacterial and viral pathogens, and/or environmental pollutants. Excessive acute inflammation, tissue remodeling, and subsequent lung injury can cause pulmonary fibrosis and impair gas exchange. Unresolved lung injury and chronic inflammation are frequently observed in acute respiratory distress syndrome, cystic fibrosis, chronic obstructive pulmonary disease (COPD), and asthma. A correlation has been shown between respiratory viral infections and the development of pulmonary fibrosis in humans. Animal models suggest that virus infection either alone or in combination with immune modulation could be triggers for the onset of fibrosis. A number of viruses, such a s influenza virus (e.g., influenza A (H1N1)), hepatitis C virus, TT virus, adenovirus, human cytomegalovirus, Epstein-Barr virus, and gamma herpes viruses have been studied as a link in the pathogenesis of IPF (Sharma and Singh, Flu and pulmonary fibrosis, Lung India. 2013 Apr-Jun; 30(2): 95-96).

Bacterial infections may also play a role in the pathogenesis of pulmonary fibrosis, and antiviral or antibiotic drugs may modify the course of fibrosis. Molyneaux and Maher, The role of infection in the pathogenesis of idiopathic pulmonary, fibrosis, European Respiratory Review 2013 22: 376-381. Knippenberg et al., Streptococcus pneumoniae triggers progression of pulmonary fibrosis through pneumolysin, Thorax 2015; 0:1-11.

In several organs, including the lung, the persistence viral or bacterial antigens promotes the development pro-inflammatory cytokine (called Th2 type) environment. Within this polarized Th2 tissue environment, the organ seems to be at particular risk for a secondary viral infection, and this second hit might then trigger amplification of innate immune and tissue remodeling events. Viral-associated bacterial super infections are also responsible for enhancement of the effect on induction of pulmonary fibrosis.

Accordingly, deuterium-enriched pirfenidone can be used to treat the serious lung/respiratory complications associated with viral and/or bacterial infections, such as any of those described herein, e.g., SARS-CoV-2. For example, the compounds and methods described herein can be used to treat inflammation-induced lung injury(ies) associated with viral and/or bacterial infections, e.g., interstitial lung injury, tissue damage, tissue remodeling, etc.

The infection can be, e.g., any viral or bacterial or mycobacterial infection, protozoan infection, parasite infection or fungal infection. The infection may be an infection of the lungs (with e.g., a coronavirus such as SARS-CoV-2, or an influenza such as H1N1), of any other organ, e.g., heart, kidney or liver (e.g., Hepatitis A, B, C, D), multiple organs or tissues, or of the entire body (e.g., an HIV). In some embodiments, the infection may be elsewhere in the body, e.g., Helicobacter pylori (H. pylori), Chlamydia pneumonia (C. pneumoniae), Cytomegalovirus (CMV), hepatitis C virus (HCV) and may have an effect on a particular organ, e.g., the heart.

Examples of infectious diseases and conditions, and infection-related diseases, disorders, and syndromes that can be treated, prevented, and/or ameliorated by deuterium-enriched pirfenidone include, but are not limited to, Acute Flaccid Myelitis (AFM), Anaplasmosis, Anthrax, Babesiosis, Botulism, Brucellosis, Campylobacteriosis, Carbapenem-resistant Infection (CRE/CRPA), Chancroid, Chikungunya Virus Infection (Chikungunya), Chlamydia, Ciguatera (Harmful Algae Blooms (HABs)), Clostridium Difficile Infection, Clostridium Perfringens (Epsilon Toxin), Coccidioidomycosis fungal infection (Valley fever, COVID-19 (Coronavirus Disease 2019), Creutzfeldt-Jacob Disease, transmissible spongiform encephalopathy (CJD), Cryptosporidiosis (Crypto), Cyclosporiasis, Dengue, 1,2,3,4 (Dengue Fever), Diphtheria, E. coli infection, Shiga toxin-producing (STEC), Eastern Equine Encephalitis (EEE), Ebola Hemorrhagic Fever (Ebola), Ehrlichiosis, Encephalitis, Arboviral or parainfectious, Enterovirus Infection, Non-Polio (Non-Polio Enterovirus), Enterovirus Infection , D68 (EV-D68), Giardiasis (Giardia) , Glanders, Gonococcal Infection (Gonorrhea), Granuloma inguinale, Haemophilus Influenza disease, Type B (Hib or H-flu), Hantavirus Pulmonary Syndrome (HPS), Hemolytic Uremic Syndrome (HUS), Hepatitis A (Hep A), Hepatitis B (Hep B), Hepatitis C (Hep C), Hepatitis D (Hep D), Hepatitis E (Hep E), Herpes, Herpes Zoster, zoster VZV (Shingles). Histoplasmosis infection (Histoplasmosis), Human Immunodeficiency Virus/AIDS (HIV/AIDS), Human Papillomavirus (HPV), Influenza (Flu), Lead Poisoning, Legionellosis (Legionnaires Disease), Leprosy (Hansens Disease), Leptospirosis, Listeriosis (Listeria), Lyme Disease, Lymphogranuloma venereum infection (LGV), Malaria, Measles, Melioidosis, Meningitis, Viral (Meningitis, viral), Meningococcal Disease, Bacterial (Meningitis, bacterial), Middle East Respiratory Syndrome Coronavirus (MERS-CoV), Mumps, Norovirus, Paralytic Shellfish Poisoning (Paralytic Shellfish Poisoning, Ciguatera), Pediculosis (Lice, Head and Body Lice), Pelvic Inflammatory Disease (PID). Pertussis (Whooping Cough). Plague; Bubonic, Septicemic, Pneumonic (Plague). Pneumococcal Disease (Pneumonia), Poliomyelitis (Polio), Powassan, Psittacosis (Parrot Fever), Pthiriasis (Crabs; Pubic Lice Infestation). Pustular Rash diseases (Small pox, monkeypox, cowpox) Q-Fever, Rabies. Ricin Poisoning, Rickettsiosis (Rocky Mountain Spotted Fever), Rubella, Including congenital (German Measles), Salmonellosis gastroenteritis (Salmonella), Scabies Infestation (Scabies), Scombroid, Septic Shock (Sepsis), Severe Acute Respiratory Syndrome (SARS), Shigellosis gastroenteritis (Shigella), Smallpox, Staphyloccal Infection, Methicillin-resistant (MRSA), Staphylococcal Food Poisoning, Enterotoxin - B Poisoning (Staph Food Poisoning), Staphylococcal Infection, Vancomycin Intermediate (VISA). Staphylococcal Infection, Vancomycin Resistant (VRSA), Streptococcal Disease, Group A (invasive) (Strep A (invasive)), Streptococcal Disease, Group B (Strep-B), Streptococcal Toxic-Shock Syndrome, STSS, Toxic Shock (STSS, TSS) Syphilis , primary, secondary, early latent, late latent, congenital, Tetanus Infection, tetani (Lock Jaw), Trichomoniasis (Trichomonas infection), Trichonosis Infection (Trichinosis), Tuberculosis (TB), Tuberculosis (Latent) (LTBI) Tularemia (Rabbit fever), Typhoid Fever, Group D, Typhus Vaginosis, bacterial (Yeast Infection), Vaping-Associated Lung Injury (e-Cigarette Associated Lung Injury), Varicella (Chickenpox),Vibrio cholerae (Cholera), Vibriosis (Vibrio), Viral Hemorrhagic Fever (Ebola, Lassa, Marburg), West Nile Virus, Yellow Fever, Yersenia (Yersinia), and Zika Virus Infection (Zika).

In some embodiments, the infection is a respiratory infection. In some embodiments, the respiratory infection is bacterial. In some embodiments, the respiratory infection is viral. In some embodiments, the viral pathogen is selected from one of the following categories: filamentous, isometric (or icosahedral), enveloped, and head and tail. In some embodiments, the viral pathogen is an enveloped virus. In some embodiments, the viral pathogen is a non-enveloped virus. In some embodiments, the viral pathogen is an RNA Virus. In some embodiments, the viral pathogen is a positive strand ssRNA virus. In some embodiments, the viral pathogen is a negative strand ssRNA virus. In some embodiments, the viral pathogen is a dsRNA virus. In some embodiments, the viral pathogen is a DNA virus. In some embodiments, the viral pathogen is a single stranded DNA virus. In some embodiments, the viral pathogen is a double stranded DNA virus. In some embodiments, the viral pathogen is a retrovirus. In some embodiments, the viral pathogen is a positive-sense single-stranded RNA virus that replicates through a DNA intermediate. In some embodiments, the viral pathogen is a double-stranded DNA virus that replicates through a single-stranded RNA intermediate.

In some embodiments, the viral pathogen is selected from, but not limited to, Adenoviridae, Arenaviridae, Astroviridae, Caliciviridae, Coronaviridae, Filoviridae, Flaviviridae, Hanta viridae, Hepadnavirus, Herpesviridae, Nairoviridae, Orthomixoviridae, Papillomaviridae, Paramyxoviridae, Parvoviridae, Peribunyaviridae, Phenuiviridae, Picornaviridae, Poxviridae, Pneumoviridae, Polyomaviridae, Retroviridae, Rhabdoviridae, and Togaviridae.

In some embodiments, the infection is related to one or more viral pathogens selected from HEV-B, SINV, WNV,YFV, ZIKV, FLUAV, HMPV, JUNV, LASV. LCMV, RVFV. HSV-2, SARS-Cov-2, CHIKV, DENV, HATV, HAV, HCoV-229E, HCoV-NL63, HCoV-OC43, HCV, HEV, HEV-A, HEV-C, HRV-A, HRV-B, JEV, KUNV, MERS-CoV, NoV, Par-A3, POWV, RAVBV, RRV, RuV, SAFV, SARS-CoV, TBEV, ANDV, BUNV, CCHFY, EBOV, FLUBV, FLUCV, HDV, HENVM, HPIV-1, HPIV-2, HTV, MARV, MeV, NiV, RABV, RAVV, RSV, SFTSV, SNV, VSV, ADV, BKV, CMV, EBV, HHV-6, HHV-7, HPV, HSV-1, JCV, KSHV, MCV, SV40, VACV, VARY, VZV, HBV, RV, B19V, HIV-1, and HIV-2.

In some embodiments, the infection is related to one or more of following pathogens: Swine flu (H1N1) infection, LPAI (low pathogenic avian flu) H7N9, HPAI (high pathogenic avian flu), H5N1, Non-polio enterovirus, influenza A or B, bacterial pneumonia, e.g., Mycoplasma pneumoniae, Viral pneumonia (e.g., influenza A or B, respiratory syncytial virus (RSV), parainfluenza, or adenovirus), corona virus (e.g., SARS-CoV, MERS-CoV, or COVID-19, HCoV-229E, HCoV-NL63, or HCoV-OC43 ), Hendra virus, Human Metapneumovirus, Hantavirus, Nipah virus, Sin NOmbre Virus, HIV-1 or HIV-2, Group A Streptoccoccus, Anthrax. Brucellosis, Hantavirus, Psittacosis, Plague, Q Fever, Tularemia, Whooping Cough, Tuberculosis, Pneumococcal meningitis, Rubella, Mumps, Measles, Influenza, Legionair's disease, Middle East Respiratory Syndrome (MERS), Severe Acute Respiratory Syndrome (SARS), Diphtheria, Haemophilus influenzae type b, or Influenza (flu).

In some embodiments, the infection, infection-related disorder, inflammatory-mediated disorder, fibrotic-mediated disorder, collagen-mediated disorder, and/or one or more symptoms of any thereof, arises from a coronavirus infection. In some embodiments, the corona virus is a beta-corona virus. In some embodiments, the beta-corona virus is SARS-CoV, SARS-CoV2, or MERS-CoV. In particular embodiments, the method treats a SARS-CoV-2 infection and/or one or more symptoms thereof. As used herein, reference to "SARS-CoV-2" is intended to contemplate all variants, including, but not limited to, 201/501Y.V1, 20H/501Y.V2, and P.1.

### Dual inhibition of inflammation and fibrosis in COVID-19 patients

Fibrosis and inflammation are a common mechanism across several lung diseases and respiratory illnesses, including viral respiratory diseases such as Coronavirus Disease 2019 (COVID-19). COVID-19 is an acute respiratory illness caused by the beta-coronavirus, SARS-CoV-2. The beta-coronavirus genus includes five human virus species: HCoV-HKU1, HCoV-OC43, Middle East respiratory syndrome coronavirus (MERS-CoV), and the severe acute respiratory syndrome coronavirus (SARS-CoV or SARS), and SARS-CoV-2. SARS shares genetic similarities with SARS-CoV-2, the virus underlying COVID-19. In SARS-CoV, patients can develop persistent pulmonary fibrosis and up to 1/3 of MERS patients have pulmonary fibrosis after recovery.

The clinical course of beta-coronavirus diseases, including COVID-19, can progress from viral infection through mild, moderate, severe disease, and acute respiratory distress syndrome (ARDS). Patients who survive the acute phase of ARDS may either suffer from lingering long-term pulmonary complications or even develop progressive forms of pulmonary fibrosis (Meduri et al., Chest 1995, 107, 1062-1073). Pulmonary fibrosis is an early event in > 50% of ARDS patients, even evident on CT scans on the first day of respiratory distress, and in the vast majority after five days. (Cabrera-Benitez et al., Anesthesiology. 2014;121(1):189-198). Early fibrosis is also a valuable predictor of mortality in some ARDS studies, where death rate was 57% in patients with lung fibrosis, compared to zero mortality in those without fibrosis. (Burnham et al., Eur Respir J. 2014;43(1):276-285). Notably, many patients who develop ARDS survive the acute phase of the illness, while a substantial proportion fatally succumb to subsequent progressive pulmonary fibrosis. George et al., "Pulmonary fibrosis and COVID-19: the potential role for anti-fibrotic therapy," Lancet Respiratory Medicine (May 15, 2020).

Lung scarring remains for months or years in > 45% of ARDS patients discharged from the intensive care unit, emphasizing that, ramifications of the infection on long term lung function need to be adequately therapeutically addressed in a timely manner. (Ichikado, et al., BMJ Open. 2012;2(2):e000545).

In the early 2000s, SARS affected 8422 individuals and killed 916 (NIH: National Institute of Allergy and Infectious Diseases). In SARS, autopsies performed on patients who succumbed in the acute phase of infection confirmed lung fibrosis in various stages of progression (Zuo et al., Molecular Biology of the SARS-Coronavirus, 247-258 (July 22, 2009), Venkataraman et al. Antiviral Res 143:142-150 (2017); Tse et al., J. Clin. Pathol. 2004, 57, 260-265).

ACE2 and TGF-β mediated lung fibrosis has been documented in the SARS-CoV infection. Multiple cohorts of SARS survivors, after discharge from the hospital, had lung fibrosis, pulmonary function deficits, or both. At one year of follow-up, >27% of survivors showed decreased lung function and increased lung fibrosis. Specific pulmonary deficits included forced expiratory volume in the first second (FEV1) and lung diffusing capacity for carbon monoxide (DLCO; Ong et al., CHEST 128, 1393-1400 (2005)). Long-term follow-ups in SARS patients have found that both imaging and pulmonary functioning abnormalities persist for years. Patients with fibrosis and GGO imaging findings had much worse long-term outcomes than patients with GGO imaging abnormalities alone (Hsu et al., CHEST 2004, 126, 149-158). A small study of 11 SARS patients found that GGO had transitioned to more reticulation predominant features on CT imaging, a sign of fibrosis that persisted up to 7 years after SARS (Wu et al.. Med. Sci. Monit. 22, 2793-2799 (2016)). 82% of the patients studied still had low DLCO despite years of recovery. These changes persist for nearly 15-years post-infection, with 38% of patients studied still having reduced diffusion capacity, a proportion essentially unchanged from 3-years post-discharge (Zhang et al., Bone Research 8, 1-8 (2020)). In the 15-year follow-up studies on SARS patients, there was little additional recovery in function or resolution in fibrosis after the first few months or year. Neither CT imaging abnormalities nor DLCO abnormalities substantially changed between 2006 and 2018, and DLCO did not significantly change since the initial infection. The pulmonary recovery was limited to the immediate 1-2 years after the infection, if at all, and remained an essentially life-long scar on pulmonary system.

SARS is the most similar to COVID-19, but other viruses lead to pulmonary complications as well. Another similar coronavirus, Middle East Respiratory Syndrome (MERS) affected 2519 individuals and killed 866 (NIH: National Institute of Allergy and Infectious Diseases). MERS had an even higher case fatality rate than SARS, and survivors of MERS also had clear deficits. For example, 66% of patients had ground-glass opacity (GGO) (Das et al., Am. J. Roentgenology 205. W267-S274 (2015)), and 33% of survivors had pulmonary fibrosis (Das et al.. Indian J. Radiol. Imaging 27. 342-349 (2017)). H7N9 influenza affected more individuals than MERS, and although the case fatality rate was lower than SARS and MERS, survivors still had residual pulmonary complications (Chen et al., Scientific Reports 7, 1-8 (2017)). At 3, 6. and 12-month follow-ups, patients had pulmonary function abnormalities. While these were pulmonary deficits were most severe in the months immediately after discharge, the majority of patients still had abnormal DLCO even at 1- and 2-years post-discharge. CT imaging revealed improvements after discharge, but pulmonary fibrosis persisted in 42% of patients for a year after hospital discharge.

While SARS and MERS affected far fewer individuals than COVID-19, their genetic and clinical similarities (pneumonia, dyspnea, fever) offer a useful point of comparison for the potential longer-term respiratory complications of COVID-19 (Park et al., 2020). SARS, MERS, and COVID-19 are caused by closely related viruses and have similar clinical presentations to COVID-19, specifically cough, dyspnea, fever, fatigue, and potentially pneumonia, with radiological signs including ground glass opacification (GGO) on chest computed tomography (CT) scans.

H1N1 (i.e., "swine flu") had much lower fatality rates than SARS or MERS, but nonetheless caused long-term sequelae. In H1N1, 3-month follow-ups of survivors revealed DLCO% reductions and imaging abnormalities in 62% of patients , and 12-months post discharge, 33% of patients still had severe abnormal pulmonary diffusion disorder (Bai et al., CHEST 139, 1156-1164 (2011)). In H1N1 ARDS patients, 43% had below-normal DLCO% at 1 year (Luyl et al., I 142, 583-592 (2012).

### Acute COVID-19 Disease

Currently, subjects who test positive for COVID-19 are generally those who are tested within 10 days of the first symptom. Respiratory symptoms generally start to appear at 3 weeks, including shortness of breath and tightness of chest. Other symptoms fluctuate in the type and severity, the most prevalent symptoms being: loss of taste, elevated temperature (slight elevation was more commonly reported than temperatures exceeding 101°F), dry cough, shortness of breath, headache, sinus congestion, body ache, fatigue, brain fog/concentration challenges, chills, sweats, sleep loss, elevated heart rate, tachycardia, lung burn, dizziness, and loss of appetite. (Assaf et al., An Analysis of the Prolonged COVID-19 Symptoms Survey, https://docs.google.com/document/d/1KmLkOArlJem-PArnBMbSp-S_E3OozD47UzvRG4qM5Yk/edit; Report Released: May 11, 2020). Secondary infections have occurred in COVID-19 patients, pneumonia being the most common. Current clinical management of beta-coronavirus respiratory diseases, including COVID-19, is generally directed to supportive care and symptomatic treatment, reduction of viral load, and suppression of inflammatory response, most commonly addressed with acetaminophen, vitamin C, D and zinc supplements, inhaler or nebulizer breathing treatments (e.g., Flonase or other corticosteroids), and electrolytes.

Resolution of symptoms of acute COVID-19 may be gradual. Symptoms including cough, shortness of breath on exertion, loss of smell or taste, sinus congestion, and headache may linger for an extended period, and commonly remain for 3 weeks or more. (O'Keefe et al., "Visual Representation of Symptom Course in COVID-19 Outpatients", MedRxiv, published online June 7, 2020; https://doi.org/10.1 101/2020.06.05.20123471). It has been reported that a large proportion of patients are symptomatic for 5-7 weeks, and the chance of full recovery by 50 weeks is only 20%. Some neurological symptoms (brain fog, memory loss, trouble concentrating) have been reported as lasting up to 8 weeks. Gastrointestinal symptoms are less common but may also be similarly prolonged.

Recent studies reveal that the lung is the organ most affected by COVID-19. Like other viral respiratory disease, COVID-19 causes respiratory complications. In a subgroup of patients, COVID-19 is marked by rapid respiratory and systemic decline which can lead to mortality and significant morbidity and potentially also resulting in lasting disabilities in survivors. In survivors, both the disease severity and the invasive treatment contribute towards a persistently diminished lung function, a phenomenon also observed in SARS-CoV and MERS. Venkataraman et al. Antiviral Res 143: 142 (2017).

It is believed that the mortality associated with COVID-19, as well longer term sequelae, are the result of multiple inflammatory, fibrotic and vasculature pathologies affecting the lungs. Disease severity and mortality are largely driven by the raging inflammation and consequential destruction and fibrosis of the pulmonary parenchyma and other organ systems, with vascular pathology as another important driver. Conti et al., J Biol Regul Homeost Agents 34(2): 31(2020). Vasculopathy in COVID-19 may not only contribute to pulmonary complications, but also coagulopathy that can lead to mortality. (Levi et al., Lancet Haematol. 2020;7(6):e438-e440). Such vascular pathologies include diffuse alveolar epithelium destruction, capillary damage/bleeding, hyaline membrane formation, alveolar septal fibrous proliferation, and pulmonary consolidation. Mo et al., Eur Respir 2001217 (Jan 2020). Concomitant damage of the vascular endothelium and increased permeability and pro-coagulation state favor vascular leakage and microvascular thrombosis.

The acute disease course of COVID-19 can be modeled in three stages: 1) an initial response mounted by the immune system to the virus, 2) a secondary interferon-driven immune response leading to tissue damage, and 3) a final hyper-inflammation in which an inflammatory macrophage response leads to aberrant tissue repair and ultimately fibrosis (Merad and Martin, 2020; Siddiqi and Mehra, 2020).

Infection with SARS-CoV2 triggers a host inflammatory response and immune dysregulation, which induces a release of pro-inflammatory cytokines and engagement of cognate cell mediators of innate immunity (e.g. alveolar macrophages). The SARS-CoV-2 virus recognizes angiotensin-converting enzyme 2 (ACE2) receptors on human respiratory epithelial and endothelial cells, and infects them via clathrin-mediated endocytosis. (Wan et al., J Virol. 2020;94(7)). The ensuing disease course is driven by both the pathogen and the host cellular and humoral immune response. The blueprint of lung destruction in COVID-19 is indicative of early signs of inflammatory and exuberant immune response, prevalent bilateral lung involvement (>75% of cases), rapid progression to consolidation lesions (>65% of cases; Wang et al., Radiology. 2020:200843), as well as evidence of fibrotic areas on radiological imaging. (Li et al. J Transl Med. 2020;18(1):154; Shi et al. Lancet Infect Dis. 2020 20(4), P425-434).

In earlier stages, the presence of serum cytokines and other inflammatory markers are predictors of disease severity and death (Chen et al., 2020a; Ruan et al., 2020; Zhou et al., 2020) and can be used to predict patients at critical risk (Yan et al., 2020). Propagation of the inflammatory signal culminates in the form of a "cytokine storm", characterized by an exuberant release of a myriad of pathogenic molecules such as tumor necrosis factor-α (TNF-α), interleukins (e.g., IL-6, IL-8, 1L1-ß), vascular cndothclial growth factor (VEGF); platelet derived growth factor (PDGF); interferon-y (IFN-y); intercellular Adhesion Molecule 1 (ICAM-1); monocyte chemoattractant protein-1 (MCP-1), transforming growth factor-ß (TGF-8); matrix metalloproteases (MMP2/9) and the like. This triggers an avalanche of reactions such as: i) recruitment of immune cells, ii) induction of vascular permeability, leakage and clotting, iii) pulmonary dysfunction and tissue damage.

In some patients, this uncontrolled inflammatory response (Mehta et al. Lancet 2020, 395, 1033-1034) results in the acute respiratory distress syndrome (ARDS), which leads to the need for mechanical ventilation and poor outcome. (Wang et al. JAMA, 2020; 323(11):1061-1069). ARDS in coronaviral infection likely results from inflammation, fluid accumulation in the lungs, and progressive fibrotic changes that interfere with gas exchange necessary for respiration. Fibrosis is relatively rare less than a week into ARDS. but by the 3^{rd} week. 24% of patients have fibrosis, and the percentage is substantially greater (61%) beyond three weeks (Thille et al., The Lancet Respiratory Medicine 2013, 1, 395-401). In ARDS, the immune system transitions from repelling the initial insult to repair, but inflammation can lead instead to fibrosis or scarring. Specifically, the initial viral replication generates an immune response, including increases in pro-inflammatory cytokines like IL-6, TNFα, and IL-1β, which recruit immune cells that cause local tissue damage and fibrosis (Gralinski, L.E., https://doi.org/10.1128/mBio.01753-182018; Sun et al. J Med Virol. 2020, 92:612). Ultimately, progressive lung damage from this inflammation and tissue re-modelling contributes to respiratory failure and is thought to be the ultimate cause of death in most patients.

Mechanical ventilation is a crucial tool for supporting respiratory function in critically ill patients, yet this intervention can cause fibrosis as well (Cabrera-Benitez et al., 2014), either directly via mechanotransduction of signaling cascades in pulmonary epithelial and endothelial cells (Frank and Matthay, 2002; Rocco et al., 2012), or by activating immune mechanisms like increasing circulating macrophages that can shift the lung tissue milieu into a fibroproliferative state (Quesnel et al., 2010).Cytokine storm and a heightened state of an abnormal immune response are known triggers of pulmonary fibrosis. (George et al., Lancet Respir Med. 2020; DOI:https://doi.org/10.1016/S2213-2600(20)30225-3). Concurrently, the pro-fibrotic cascade is also triggered, reflected in fibroproliferation (fibroblast activation and propagation), with increased collagen synthesis and deposition.

Compromised respiratory function and lung fibrosis, seen following SARS and anticipated in the aftermath of COVID-19, may strongly resemble idiopathic pulmonary fibrosis (IPF). It has been reported that pulmonary fibrosis is generally present at autopsy in fatal cases of COVID-19, and there are anecdotal reports of severe fibrotic organizing pneumonia in COVID-19 patients. George et al., "Pulmonary fibrosis and COVID-19: the potential role for antifibrotic therapy," Lancet Respiratory Medicine (May 15, 2020). The composition and distribution of increased extracellular matrix deposited in the lungs of ARDS and in patients with IPF compared to normal human lungs was documented 35 years ago. (Raghu et al., Am Rev Respir Dis. 1985;131(2):281-289). Preliminary reports from COVID-19 suggest a similar process, with striking increases in pro-inflammatory cytokines like IL-6 and TNF and development of radiological signs of inflammatory and fibrotic lesions in the lungs (Shi et al., The Lancet Infectious Diseases 2020 (20)4, P425-434).

The transition from inflammation to fibrosis can be visualized in COVID-19 patients with pneumonia. Early imaging shows inflammation-related opacification, while later tests reveal a characteristic pattern of lung damage due to interstitial lung remodeling-likely alveolar epithelial cell injury. (Shi et al., The Lancet Infectious Diseases 2020 (20)4, P425-434). Chest X-rays show evidence of pneumonia, while CT scans show ground glass opacity, smooth or irregular, interlobular septal thickening, ill-defined margins, air bronchogram, "crazy-paving" pattern, thickening of the adjacent pleura, nodules, cystic changes, bronchiolectasis, pleural effusion, or lymphadenopathy.

Severe cases of SARS-CoV-2 pneumonia show ground-glass opacities and other signs on CT imaging indicative of interstitial lung damage and emerging risk of fibrosis and long-term pulmonary remodeling. Post-mortem samples from patients with COVID-19 show diffuse alveolar damage suggestive of ARDS, which has previously been identified in patients of related coronaviral infections like SARS and MERS (Xu et al., 2020). ARDS is characterized by an acute inflammatory storm in the lung parenchyma, leading to alveolar edema, decreased lung compliance and, ultimately, hypoxemia. ARDS in coronaviral infection results from runaway inflammation, fluid accumulation in the lungs, and progressive fibrotic that interferes with gas exchange necessary for respiration. Specifically, the initial viral replication generates an immune response, including increases in pro-inflammatory cytokines like IL-6, TNFα, and IL-1β, which recruit immune cells that cause local tissue damage and fibrosis (Gralinski and Baric, 2015). Ultimately, progressive lung damage from this inflammation and tissue remodeling contributes to respiratory failure and is thought to be the ultimate cause of death. As in viral-related ARDS, mitigation strategies for COVID-19 fall in two broad categories: anti-viral drugs designed to dampen viral replication (i.e., reduce viral load) and improve recovery times; and anti-inflammatory drugs designed to reduce the cytokine storm (e.g., low-dose methylprednisone), although this later approach is controversial

Additional studies are under way to explore the efficacy of antiviral or anti-inflammatory agents on the course of COVID-19. A large number of clinical trials (> 500 by current count) of antivirals, anti-inflammatory and immuno-modulatory, and other drugs (anti-malarials, anti-coagulants, vasodilators, anti-angiogenics etc.) are in progress, in conjunction with a myriad of symptom control and invasive supportive measures being deployed for COVID-19. (Thorlund et al., *The Lancet.* DOI:https://doi.org/10.1016/S2589-7500(20)30086-8; Lythgoe et al., Trends Pharmacol Sci. 2020 Jun;41(6):363-382, doi: 10.1016/j.tips.2020.03.006).

Initial studies suggest that remdesivir, an anti-viral agent previously used for Ebola and other viral infections, may be effective in reducing the time of hospitalization from COVID-19 (Beigel et al., New England Journal of Medicine 2020, DOI: 10.1056/NEJMoa2007764; Wang et al., The Lancet 2020, 395, 1569-1578; Gilead Press Release, https://www.gilead.com/news-and-press/press-room/press-releases/2020/5/gileads-investigational-antiviral-remdesivir-receives-us-food-and-drug-administration-emergency-use-authorization-for-the-treatment-of-covid19) and alleviating symptoms for mild and moderate COVID-19 (lopinavir-ritonavir, and ribavirin; The Lancet 2020, https://doi.org/10.1016/). However, current anti-viral approaches may not modulate the immune dysregulation and fibroproliferation driven by the infection, the consequence of which have adverse outcomes.

Investigation of anti-inflammatory drugs (Stebbing et al., The Lancet Infectious Diseases 2020, 20, 400-402) has been prompted by the noted inflammatory excess in COVID-19 patients. The therapeutics under investigation include both biological agents and small molecules, some of which are investigational and others approved for the treatment of anti-inflammatory and autoimmune diseases (e.g. chloroquine and hydroxychloroquine, adalimumah, baricitinib, ruxolitinib, siltuximab, tocilizumab, thalidomide, tofacitinib, anakinra and emapalumab, apremilast, calcium release-activated calcium channels inhibitor, ibudilast, leronlimab, etc.). *See, e.g.,* Taccone et al., Lancet Respir Med. 2020; Feldmann et al. Lancet. 2020, Volume 395, ISSUE 10234, P1407-1409, May 02, 2020; Stebbing et al. Lancet Infect Dis. 2020;20(4):400-402; McKee, PharmaTimes online 2020; http://www.pharmatimes.com/news/positive_early_data_from_siltuximab_covid-19_trial_1334145; https://wwwglobenewswirecom/news-release/2020/04/09/2014265/0/en/FDA-Grants-CalciMedica-Permission-to-Begin-Dosing-CM4620-IE-in-Patients-with-Severe-COVID-J9-Pneumonia-under-a-Newly-Opened-INDhtml.2020; *https:*//*wwwbiospacecom*/*article*/*releases*/*medicinova-announces-plans-to-initiate-a-clinical-trial-of-mn-166-ibudilast-for-covid-19-acute-respiratory-distress-syndrome-ards-*/*.*2020; *https:*//*billy*/*39RZQCB Accessed April 6, 2020;* Blocentury. 2020;https://www.biocentury.com/trial-timeline.

A pharmacologic intervention that can prevent long-term lung injury, e.g., by targeting both inflammation and fibrosis, may disrupt the molecular cascade that leads to lung scarring and loss of lung function before the damage has occurred. Early onset elevation (<3 days from first symptoms) in the above mentioned cytokines/markers, and relatively persistent high levels even in the third week post symptom onset (Liu et al., EBioMedicine 55:102763 (2020)), suggest that a sustained, effective, yet safe neutralization of the key inflammatory and fibrotic factors, may be of paramount importance in COVID-19. The need for early intervention is supported by the growing acknowledgement that profound damage of the respiratory system can peak already around day ten post COVID-19 symptom onset, resulting in ARDS. (Feng et al., Radiology. 2020; https://doi.org/10.1148/radiol.2020200370). The clinical time course and similarity of COVID-1 to SARS suggest a limited window of recovery, with no additional recovery of fibrotic lesions or diffusion capacity after a year (Zhang et al., Bone Research 8. 1-8(2020)). Therefore, it would be desirable to provide therapeutic strategies able to attenuate the inflammatory/fibrotic respiratory aftermath of acute COVID-19 infection. (Spagnolo et al. Lancet Respir Med. 2020, doi: 10.1016/S2213-2600(20)30222-8 [Epub ahead of print]).

### "Long Hauler" COVID-19

Serious post-acute respiratory complications are an emerging issue for those who survive COVID-19. Follow-up studies of patients from the first wave of COVID-19 in the Spring of 2020 have revealed a pattern of persistent respiratory symptoms in survivors. Specifically, post-recovery, longer-term impaired lung function and fibrosis are being observed, similar to that following infection with other coronaviruses, including SARS and MERS. Fibrosis in the lungs can impair lung capacity, pulmonary function, and gas exchange. Recent COVID-19 post-discharge studies indicate that, as with other related viral pneumonias, a subset of survivors of acute infection may have persistent and potentially progressive lung fibrosis, long-term pulmonary dysfunction, and diminished of quality of life, continuing long after resolution of the acute infection, despite improvement in clinical and radiological parameters such as chest radiographs and blood results. Spagnolo et al., "Pulmonary fibrosis secondary to COVID-19: a call to arms?" Lancet Respiratory Medicine (May 15, 2020). Amid the increasing evidence of this significant minority of patients suffering from debilitating long-term impacts from the disease, the labels "Long Covid" and "Covid Long-hauler" are gaining traction in categorizing those patients experiencing long term residual pulmonary function abnormalities. While a formal definition of long COVID has not yet been accepted within the medical community, the term "long COVID" is generally accepted. and is used herein, to refer to individuals having a medical diagnosis of COVID-19, based on symptoms and/or diagnostic testing, and which have not yet returned to a pre-COVID-19 level of health and function following resolution of the acute phase of infection with the SAR-CoV-2 virus. Symptoms and dysfunctions include, but are not limited to, fatigue, dyspnea, cough. arthralgia, chest pain, cognitive impairment, depression, anxiety, myalgia, headache, fever, gastrointestinal issues, palpitations, pulmonary function abnormalities, acute kidney injury, myocardial inflammation, and ventricular dysfunction.

In COVID-19, the rate of lung fibrosis and pulmonary dysfunction post-recovery ranges from 30-50% (Li et al., Eur Radiol. 2020; Mo et al., Eur. Resp. J. 55: 2001217 (2020); Yu et al., Korean J Radiol. 2020;21(6):746-755), in line with the approximately one-third of SARS and MERS patients with long-term lung sequelae (Das et al., Indian J. Radiol. Imaging 27, 342-349 (2017); Ong et al., CHEST 128, 1393-1400 (2005); Zhang et al., Bone Research 8, 1-8 (2020)).

Currently, a high proportion of mild, moderate, and severe COVID-19 patients (17-53%) already show signs of lung fibrosis at three-weeks post-symptom onset. (Li et al. Eur Radiol. 2020, https://doi.org/10.1007/s00330-020-06817-6; Ye et al., Eur Radiol. 2020, Mar 19;1-9, doi: 10.1007/s00330-020-06801-0). CT scans of COVID-19 patients show clear evidence of interstitial thickening, coarse reticular patterns and parenchymal bands, all early signs and predictors of underlying processes, which culminate in frank pulmonary fibrosis. (Yu et al., Korean J Radiol. 2020;21(6):746-755). Of COVID-19 patients with pneumonia, 44% of patients had fibrosis on CT imaging at 9-days post-discharge (Yu et al., Korean J. Radiol. 21, 746-755 (2020)). The group of patients with fibrosis had higher CRP and IL-6 during their hospitalization, supporting the link between inflammation in the acute phase and fibrosis during the transition to recovery. Post-discharge is further dominated by restrictive ventilation disorder and small airway obstruction (Dongqing, Research Square; http://orchidorg/0000-0002-5681-3663, 2020), with reduced forced vital capacity (FVC) below 80% and maximum expiratory volume (MEF25, MEF50, MEF75) below 70%.

Studies of COVID-19 patients at the time of discharge from hospital and at two weeks post discharge have reported persistently and significantly compromised lung functions. Namely, anomalies in the lung diffusing capacity for carbon monoxide (DLCO%; an objective measure of gas exchange) and restrictive ventilatory defects (e.g., abnormal ratio of forced expiratory volume in the first second (FEV1) to forced vital capacity (FVC); FEV1/FVC%) appear to be predominant (present in 47.2% of patients at discharge), and directly correlate with severe acute disease clinical course and high inflammatory indicators. (Mo et al, European Respiratory Journal (in Press) 2020; https://doi.org/10.1183/13993003.01217-2020). Reduced DLCO% is a finding that has been consistently documented in COVID-19 as well as in previous coronavirus pneumonias (e.g., SARS). DLCO% deficits in related viral pneumonia were accompanied by persistent imaging abnormalities, including fibrosis, and respiratory symptoms that last for years after the initial hospitalization. Diffusion abnormalities translate to deficits in physical functioning and quality of life. For example, more than a third of SARS patients had deficits in DLCO% and reductions in maximum aerobic capacity (Hui et al., Chest. 2005;128(4):2247-2261), and in idiopathic pulmonary fibrosis (IPF), DLCO% has been correlated with performance in the 6-minute walk tests (6MWT) and with arterial oxygen saturation during exercise (Caminati et al., Respiratory Medicine 103, 117-123 (2009)).

According to a research letter published in the Journal of the American Medical Association (JAMA), in a study of 143 survivors, more than 40% of COVID-19 survivors assessed in an Italian study still reported shortness of breath an average of 60 days following symptom onset with greater than 20% reporting chest pain and greater than 15% reporting cough. Carfi, A., Bemabei, R., & Landi, F. (2020). Persistent Symptoms in Patients After Acute COVID-19. Jama, 324(6), 603. doi:10.1001/jama.2020.12603. Notably, this was a relatively non-severe population, as 87.4% had not been in the ICU and 4/5th had not been ventilated. Patients with more severe clinical cases and more invasive respiratory interventions like ventilation are expected to have more post-acute respiratory complications. This experience out of Italy is not unique. More than 40% of survivors in a UK-based sample had long-term dyspnea (Halpin et al.). The Centers for Disease Control reported over 40% of patients with cough and nearly 30% with dyspnea in a US-based sample (Tenforde et al., 2020). The longest study to date, out of France, followed patients an average of 111-days post-admission and found over 40% still had shortness of breath (Garrigues et al., 2020)

In view of the similar pathophysiology to other viral pneumonias (e.g., SARS and MERS), residual pulmonary dysfunction in the COVID-19 aftermath may similarly be expected to persist for many months or years. Further, residual non-progressive fibrosis may result in considerable future morbidity and mortality in patients who have recovered from COVID-19, particularly older patient populations and/or those with preexisting pulmonary conditions.

Hundreds of clinical trials are underway to combat COVID-19, but the vast majority are focused on vaccines or the acute treatment of severe patients. Surprisingly few clinical trials are targeting the long-term sequelae of COVID-19. In view of the unprecedented scale of the current pandemic, patients who have recovered from the acute symptoms of COVID-19, yet have persistent pulmonary dysfunction, have the potential to create an enormous and unprecedented public health burden. (George et al., Lancet Respir Med. 2020; DOI:https://doi.org/10.1016/S2213-2600(20)30225-3). With an overall hospitalization rate of 89.3 per 100,000 and nearly 2.5 million cases in the United States alone (CDC, 2020), there is a potentially large patient population at risk for longer-term reductions in pulmonary function due to COVID-19. Accordingly, the death and disability from COVID-19-related chronic lung disease could be several-fold the expected 50,000 to 100.000 deaths from acute COVID-19 infection. Accordingly, there is a potentially enormous unmet medical need for new therapeutic strategies able to attenuate the inflammatory/fibrotic respiratory aftermath of COVID-19 pneumonia, prevent subsequent disability related to lung injury, and address the longer-term respiratory complications and sequelae of COVID-19 in "long-haulers."

### Deuterated Pirfenidone for the Treatment of COVID-19

There are considerable patho-biological and clinical similarities between acute exacerbations of chronic interstitial lung diseases (AE-ILDs) and acute interstitial pneumonias (AIPs) with chronic consequences (such as COVID-19 and ARDS). Both COVID-19 and AE-ILDs are mediated by the same pro-inflammatory and pro-fibrotic factors. Swigris et al., Semin Respir Crit Care Med 27(6): 659 (2006). A sustained, effective, yet safe neutralization of the key inflammatory and fibrotic factors would be of paramount importance in COVID-19. Further, a need exists for therapeutic strategies to treat and/or prevent the inflammatory/fibrotic respiratory aftermath of viral respiratory diseases, such as those resulting from human influenza or beta-coronaviruses, including SARS-CoV-2. Additionally, or alternatively, initiating or maintaining treatment for survivors of acute beta-coronavirus infection such as COVID-19 with lung function impairment and evidence of interstitial pneumonia on imaging, with an agent that has anti-inflammatory/anti-fibrotic effect, may yield improved, clinically meaningful outcomes, in the post-hospital discharge setting.

Treatments are urgently needed to prevent aberrant tissue remodeling and the deleterious repair processes that lead to lung scarring in the aftermath of acute beta-coronavirus infection, including COVID-19. There is a window of intervention in COVID-19 and other viral pneumonias to resolve fibrosis and prevent long-term complications. In view of the similarities between SARS and COVID-19, what happens in the initial months after contracting COVID-19 and other viral pneumonias will likely determine a patient's risk for potentially life-long complications. This emphasizes the importance and urgency of intervening with a treatment before and/or during the recovery period. Accordingly, we envision a two-pronged approach to both improve outcomes in the acute setting as well as ameliorate the longer term, fibrotic sequelae of COVID-19.

First, administration starting in the acute COVID-19 setting, where applying brakes to the inflammatory and fibrotic mediators early in the disease could be crucial for tipping the course from progression to resolution. The need for early intervention is supported by the growing acknowledgement that profound damage of the respiratory system can peak around day ten, post COVID-19 symptom onset, resulting in ARDS. Feng Pan et al., Radiology 295(3):715-721 (Feb 13, 2020). The composition and distribution of increased extracellular matrix deposited in the lungs of ARDS and in patients with IPF compared to normal human lungs has been well documented. Raghu et al., Am Rev Respir Dis 131, 281 (1985). Pulmonary fibrosis is an early event in > 50% of ARDS patients, even evident on CT scans on the first day of respiratory distress, and in the vast majority after five days. Cabrera-Benitez et al., Anesthesiology 121, 189-198 (2014). Early fibrosis is also a valuable predictor of mortality in some ARDS studies, where death rate was 57% in patients with lung fibrosis, compared to zero mortality in those without fibrosis. Burnham et al., Eur Respir J 43, 276-285 (2014). Equally, lung scarring remains for months or years in > 45% of ARDS patients discharged from the intensive care unit, emphasizing that, ramifications of the infection on long term lung function need to be adequately therapeutically addressed in a timely manner. Ichikado et al., BMJ Open 2(2), e000545 (Mar 2, 2012).

Second, we believe that a maintained treatment strategy for survivors of COVID-19 with lung function impairment and evidence of interstitial pneumonia on imaging, with an agent that has anti-inflammatory/anti-fibrotic effect, may yield improved and clinically meaningful outcomes, in the post-hospital discharge setting, i.e. a scenario more akin to a chronic fibrotic ILD. Although fibrosis is chronic and progressive in IPF/progressive fibrotic ILD, compared to a chronic and persisting pattern of fibrosis following post-acute pneumonias, the shared underlying mechanisms, described above, raise the potential that therapies which now treat IPF/ILDs could also be beneficial for not only acute, but also chronic COVID-19 and other post infectious associated lung fibrosis.

By implementing this two-pronged approach, we envisage a prolonged exposure to and efficacy of the pharmacological, dual, anti-inflammatory/anti-fibrotic intervention in a subgroup of survivors of COVID-19. This would be in addition to-antivirals/antibiotics/anticoagulants and other supportive measures. In this regards, a compound like pirfenidone would not act as an immune suppressive medication, and unlike corticosteroids, or blockades of specific cytokines (e.g. TNF- a, IL-6) that are being investigated as potential treatments for COVID-19, would not pose or create the risk for superimposed infection.

The recognition of parallel roles of inflammation and fibrosis in both diseases has suggested a therapeutic strategy to combat both inflammation/immune dysregulation and fibroproliferative response to COVID-19 with a single agent. To this end, pirfenidone and nintedanib, two drugs available for the treatment of IPF and other fibrotic ILDs, are currently being evaluated in clinical trials for acute COVID-19 (NCT04338802, NCT04282902, ChiCTR2000031138, ChiCTR2000030892). Lythgoe et al., Trends Pharmacol Sci 41(6):363-382 (April 9, 2020).

Although pirfenidone has been predominantly benchmarked as an anti-fibrotic for IPF, it was initially characterized as an anti-inflammatory agent. Ruwanpura et al., Am J Respir Cell Mol Biol 62(4): 413 (2020). Not only does it improve upon respiratory function and survival in established IPF, but it also slows down the disease progression and increases survival in patients manifesting with acute exacerbations of chronic fibrotic lung diseases. Ruwanpura et al., Am J Respir Cell Mol Biol 62(4): 413 (2020), Vianello et al., Curr Med Res Opin 35(7):1187 (2019). It does so by exerting suppression on three catalyst events of lung damage and dysfunction: oxidative stress, inflammation and fibrosis. Pirfenidone modulates adhesion molecules (e.g. ICAM-1), inhibits chemokines and cytokines targeting pulmonary epithelium, as well as the vascular endothelium (IL6, IL-8, IL-1ß, IL-4/IL-13, IL-12, TNF-a, TGF-ß, MCP-1, IFN-y) and suppresses excessive matrix/collagen deposition (TGF-8, PDGF, TIMP1, MMP2/9, TNF-a, collagen, fibronectin). Ruwanpura et al., Am J Respir Cell Mol Biol 62(4): 413 (2020). Pirfenidone can also impact the hemodyscrasias, which are adverse clinical predictors in AE-ILDs, as well as in COVID-19, such as the neutrophil-to-lymphocyte ratio imbalance. Id.

There is overlap between the pathobiology of lung fibrosis, where remodeling of vascular structures occurs, and COVID-19, where deleterious changes in lung vasculature are mediated by VEGF, IL-8 and TGF-ß, which pirfenidone inhibits. Id., Barratt et al., QJM 107(7): 515 (2014). In this regard, pirfenidone also upregulates claudin-5, a critical controller of pericellular permeability and leakage. Kaneko et al., Clin Exp Immunol 113(1): 72 (1998), Yamagami et al., Am J Physiol Heart Circ Physiol 309(3):H512-522 (2015). In addition, pirfenidone also potently suppresses macrophages in acute and chronic ILDs (Xuan et al., European Respiratory Society Annual Congress 2013 Abstract Number: 3209 (2013)), which have now been associated as central mediators of the COVID-19 cytokine storm, dubbed the macrophage activation syndrome. McGonagle et al., Autoimmun Rev 102537 (2020). Through the multi-faceted mode of action, pirfenidone has the potential, therefore, to correct the imbalance between pro- and anti-inflammatory and fibrotic responses, back into a regulated tissue and systemic immune response. Macias-Barragan et al., Fibrogenesis Tissue Repair 3, 16 (2010).

Clinicians have reported experiences with pirfenidone in non-viral acute respiratory distress, both in a pre-treatment and treatment regimen. IPF patients who are already receiving pirfenidone treatment have better outcomes measured by survival when admitted into respiratory intensive care units with progression to ARDS, and this pre-treatment with pirfenidone may prevent inflammatory and fibrotic processes and lead to chronic lung damage (Vianello, et al. (2019). Pirfenidone improves the survival of patients with idiopathic pulmonary fibrosis hospitalized for acute exacerbation. Current Medical Research and Opinion 35, 1187-1190). In a separate case study, a patient with fibrotic-stage ARDS was given pirfenidone 200 mg three times a day for 12 days, even while remaining on ventilation. Pirfenidone treatment was followed by improvement in oxygenation and transition off of ventilation (Chen, H.C. (2019). Pirfenidone can enhance the patient's recovery from fibrotic phase of ARDS: a case report. CHEST 155, 138A.These case reports suggest the underlying mechanism of pirfenidone may play a role in ameliorating the progressive course of ARDS.

Pirfenidone is known for its antifibrotic, anti-inflammatory and antioxidative effects to reduce collagen synthesis (Raghu G, Striker LJ, Hudson LD, Striker GE. Extracellular matrix in normal and fibrotic human lungs. Am Rev Respir Dis. 1985; 131:281-289), deposition and suppression of pro-inflammatory cytokines, among other effects (Collins BF, Raghu G. Antifibrotic therapy for fibrotic lung disease beyond idiopathic pulmonary fibrosis. Eur Respir Rev. 2019;28), including targeting TGF-β synthesis. Pirfenidone has been used in non-viral ARDS and acute exacerbation of IPF (Raghu G. Idiopathic pulmonary fibrosis: shifting the concept to irreversible pulmonary fibrosis of many entities. Lancet Respir Med. 2019; 7:926-929). In a case series of HIN1 virus-related ARDS patients requiring mechanical ventilation, pirfenidone was used to treat persistent symptoms and hypoxemia, resulting in symptomatic improvement, regression of opacities, and no further progression of fibrosis seen on CT scans (Mineo G, et al., Post-ARDS pulmonary fibrosis in patients with H1N1 pneumonia: role of follow-up CT. Radiol Med. 2012;117:185-200; Saha, A., et al. (2018). Combined pirfenidone, azithromycin and prednisolone in post-H1N1 ARDS pulmonary fibrosis. Sarcoidosis Vasculitis and Diffuse Lung Disease 35, 85-90). Accordingly, pirfenidone may have utility in treating the serious lung/respiratory complications associated with viral and/or bacterial infections, such as any of those described herein, e.g., SARS-CoV-2.

However, pirfenidone's use could be hampered by certain limitations associated with its use. These include: 1) frequent dosing, which results in high peak to trough fluctuations in plasma concentrations, 2) photosensitivity, 3) liver toxicity and above all, and 4) predominant gastrointestinal side-effects leading to >25% treatment discontinuation rate. A therapeutic compound which improves upon metabolism and dose exposure of pirfenidone, while retaining or exceeding its efficacy, would therefore be a welcomed addition to the armamentarium of agents in acute and chronic interstitial lung diseases (ILDs), including those associated with viral infections, and specifically, with COVID-19.

One such therapeutic agent is the deuterium-enriched pirfenidone as disclosed herein (deupirfenidone, LYT-100), which benefits from the deuterium kinetic isotope effect in suppressing metabolic enzyme kinetics. LYT-100 has unique pharmacokinetic properties that improve on the tolerability of pirfenidone, while retaining the proven anti-fibrotic and anti-inflammatory properties that have the potential to treat COVID-19 and the respiratory complications resulting therefrom. Preclinical data disclosed herein demonstrate the antifibrotic and anti-inflammatory activity of LYT-100 (See, e.g., Examples 1-4). For instance, LYT-100 at a dose of 60 mg/kg/day significantly reduced the area of liver fibrosis in a streptozocin-induced non-alcoholic steatohepatitis (NASH) mouse model; and pretreatment with oral doses of 100 and 300 mg/kg LYT-100 inhibited TNFα and IL-6 in a rat lipopolysaccharide (LPS) model of systemic inflammation. LYT-100 also reduced pro-inflammatory cytokines and suppressed TGF-β and downstream signaling to inhibit fibrosis in Primary Mouse Lung Fibroblasts. LYT-100 can potentially act to address acute and unique anti-inflammatory activity on multiple cytokines that have not been tested with pirfenidone.

One advantage of LYT-100 is that it can be administered on a twice-a-day dosing schedule, in contrast to pirfenidone, which requires a three-times-a-day dosing schedule. Thus, at least because of a simplified dosing schedule. LYT-100 can engender increased patient compliance and reduced pill burden relative to pirfenidone and can ultimately be a more effective therapeutic agent. Study results disclosed herein show that a 1000 mg dosing of LYT-100 BID produces exposure levels that are similar to those produced by an 801 mg dosing of pirfenidone TID. Thus, at least because of a decrease in adverse events and a simplified dosing schedule, LYT-100 can engender increased patient compliance and reduced pill burden relative to pirfenidone and can ultimately be a more effective therapeutic agent.

Without wishing to be bound by any particular theory, we believe LYT-100 can effectively treat subjects to both improve outcomes in the acute setting as well as ameliorate the longer term, fibrotic sequelae of viral respiratory infections, e.g., infection with the beta-coronavirus SARS-CoV2, which results in the viral respiratory disease COVID-19. Initiating administration of LYT-100 in the acute phase, applying brakes to the inflammatory and fibrotic mediators early in the disease, could be crucial for tipping the course from progression to resolution. Again without wishing to be bound by theory, it is believed that beginning LYT-100 treatment early in the course of COVID-19, prior to irreversible injury, as well as during recovery, may allow resolution of inflammation and prevent fibrosis and the long term pulmonary deficits which impact quality of life.

In order to help patients to recover from the viral respiratory infection or disease (e.g., COVID-19), treatment can begin at the first onset of symptoms. For example, treatment can begin less than 24 hours, 48 hours, 72 hours, or 96 hours after onset of a first symptom of the viral respiratory disease. Alternatively, treatment can be initiated after a longer period of time from onset of a first symptom or symptoms, e.g., 5 days, 7 days, or ~9-10 days, or longer. Accordingly, either or both of the acute infection and long-term effects of the infection are treated.

The methods of treating COVID-19 and the respiratory complications resulting therefrom are described further herein below.

### Methods and Dosing for Treating Infection, Inflammation, and Related Conditions

In some embodiments, the disclosure provides methods of treating a subject having a viral respiratory disease or having viral respiratory complication(s). The method includes administering to a subject in need thereof an effective amount of the deuterium-enriched pirfenidone LYT-100, 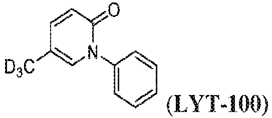 wherein the viral respiratory disease or viral respiratory complication(s) is treated in the subject.

In some embodiments, the disclosure provides methods of treating a subject having a viral respiratory disease or a viral respiratory infection. The method includes administering to a subject in need thereof an effective amount of LYT-100, wherein the treating is effective in ameliorating respiratory symptoms of the viral respiratory disease or infection.

In some embodiments, the disclosure provides methods of preventing a viral respiratory disease or a viral respiratory complication(s) in a subject. The method includes administering to a subject in need thereof an effective amount of LYT-100, wherein the viral respiratory disease or viral respiratory complication(s) is prevented in the subject.

In some embodiments, the disclosure provides methods of preventing a viral respiratory disease or a viral respiratory complication(s) in a subject. The method includes administering to a subject in need thereof an effective amount of LYT-100, wherein the treating is effective in preventing the development of, halting the progression of, slowing the progression of, or otherwise ameliorating one or more of lung fibrosis, respiratory complications of the viral respiratory disease or infection, or pulmonary dysfunction in the subject.

In some embodiments, the disclosure provides methods of treating the respiratory aftermath or complication(s) of a viral respiratory disease, e.g., a COVID-19 related infection, by administration of an effective amount of LYT-100. In some embodiments, the disclosure provides methods for treating, preventing, and/or ameliorating tissue damage and/or tissue injury in a subject, including a human subject, having or suspected of having an infection (e.g., a bacterial or viral infection, e.g., a corona virus infection, e.g., COVID-19) and/or an infection-related condition (e.g., from a bacterial or viral infection, e.g., a corona virus infection, e.g., COVID-19), comprising administering to the subject a therapeutically effective amount of LYT-100. In some embodiments, the infection and/or infection-related condition is a viral infection, e.g., corona virus infection, e.g., COVID-19. In some embodiments, the infection and/or infection-related condition is a respiratory infection, e.g.. from corona virus infection, e.g., SARs or SARS-CoV-2 infection. In some embodiments, the infection and/or infection-related condition is a lung or pulmonary infection, e.g., from corona virus infection. e.g., SARs or SARS-CoV-2 infection. In some embodiments, the tissue is lung or pulmonary tissue. In some embodiments, the tissue is interstitial tissue, e.g., interstitial lung tissue. In some embodiments, wherein the tissue is lung tissue, the treating, preventing, and/or ameliorating of tissue damage and/or injury is demonstrated by decreasing lobe score and/or lobe involvement.

In some embodiments, the disclosure provides methods for treating, preventing, and/or ameliorating tissue remodeling in a subject, including a human subject, having or suspected of having an infection (e.g., a bacterial or viral infection, e.g., a corona virus infection. e.g., COVID-19) and/or an infection-related condition (e.g., from a bacterial or viral infection, e.g., a corona virus infection, e.g., COVID-19), comprising administering to the subject a therapeutically effective amount of LYT-100. In some embodiments, the infection and/or infection-related condition is a viral infection, e.g.. corona virus infection, e.g., COVID-19. In some embodiments, the infection and/or infection-related condition is a respiratory infection, e.g., from corona virus infection, e.g., SARs or SARS-CoV-2 infection. In some embodiments, the infection and/or infection-related condition is a lung or pulmonary infection, e.g., from corona virus infection, e.g., SARs or SARS-CoV-2 infection. In some embodiments, the tissue is lung or pulmonary tissue. In some embodiments, the tissue is interstitial tissue, e.g., interstitial lung tissue. In some embodiments, wherein the tissue is lung tissue, the treating, preventing, and/or ameliorating of tissue remodeling is demonstrated by decreasing lobe score and/or lobe involvement.

In some embodiments, the disclosure provides methods for maintaining and/or improving the stability and/or integrity of a tissue in a subject, including a human subject, having or suspected of having an infection (e.g., a bacterial or viral infection, e.g., a corona virus infection, e.g., COVID-19) and/or an infection-related condition (e.g., from a bacterial or viral infection, e.g., a corona virus infection, e.g., COVID-19), comprising administering to the subject a therapeutically effective amount of LYT-100. In some embodiments, the infection and/or infection-related condition is a viral infection, e.g., corona virus infection, e.g., COVID-19. In some embodiments, the infection and/or infection-related condition is a respiratory infection, e.g., from corona virus infection, e.g., SARs or SARS-CoV-2 infection. In some embodiments, the infection and/or infection-related condition is a lung or pulmonary infection, e.g., from corona virus infection, e.g., SARs or SARS-CoV-2 infection. In some embodiments, the tissue is lung or pulmonary tissue. In some embodiments, the tissue is interstitial tissue, e.g., interstitial lung tissue. In some embodiments, wherein the tissue is lung tissue, the maintaining and/or improving the stability and/or integrity of the tissue is demonstrated by decreasing lobe score and/or lobe involvement.

In some embodiments, the disclosure provides methods for reducing the progression of, a respiratory disease and/or one or more symptom(s) thereof, in a subject, including a human subject, having or suspected of having an infection (e.g., a bacterial or viral infection, e.g., a corona virus infection, e.g., COVID-19) and/or an infection-related disease or one or more symptoms thereof, comprising administering to the subject a therapeutically effective amount of LYT-100. In some embodiments, the respiratory disease is acute respiratory distress syndrome (ARDS). In some embodiments, the one or more acute symptom(s) is one or more acute symptom(s) of ARDS.

In some embodiments, the preventing or reducing the progression of a respiratory disease comprises preventing or reducing the progression from a mild respiratory disease to a severe respiratory disease. In some embodiments, the preventing or reducing the progression of a respiratory disease comprises preventing or reducing the progression from none to mild, none to moderate, none to severe, mild to moderate, from mild to severe, from moderate to severe.

In some embodiments, the treating is effective in preventing the development of pulmonary fibrosis, pulmonary dysfunction, or both in the subject. In some embodiments, the treating is effective in preventing the development of pulmonary fibrosis in the subject. In some embodiments, the treating is effective in preventing the development of pulmonary dysfunction in the subject.

In some embodiments, the treating is effective in halting the progression of pulmonary fibrosis pulmonary dysfunction, or both in the subject. In some embodiments, the treating is effective in halting the progression of pulmonary fibrosis in the subject. In some embodiments, the treating is effective in halting the progression of pulmonary dysfunction in the subject.

In some embodiments, the treating is effective in slowing the progression of pulmonary fibrosis, pulmonary dysfunction, or both in the subject. In some embodiments, the treating is effective in slowing the progression of pulmonary fibrosis in the subject. In some embodiments, the treating is effective in slowing the progression of pulmonary dysfunction in the subject.

In some embodiments, the treating is effective in ameliorating respiratory symptoms of the viral respiratory disease or viral respiratory infection. In some embodiments, the treating is effective in preventing respiratory symptoms of the viral respiratory disease or infection. In some embodiments, the respiratory symptoms are cough, dyspnea, or both. In some embodiments, the subject exhibits reduced cough, dyspnea, or both, relative to a subject that has not been treated with LYT-100.

In one embodiment, the viral respiratory disease or viral respiratory complication(s) is caused by a human influenza virus. In some embodiments, the viral respiratory infection is caused by infection with human influenza virus H1N1, human influenza virus H7N9, SARS-CoV, SARS-CoV-2, MERS-CoV, HCoV-HKU1, or HCoV-OC43.

In some embodiments, the viral respiratory disease or viral respiratory complication(s) is caused by a beta-coronavirus. In some embodiments, the beta-coronavirus is SARS-CoV, SARS-CoV-2, MERS-CoV, HCoV-HKU1, or HCoV-OC43. In some embodiments, the viral respiratory disease is COVID-19. In some embodiments, the viral respiratory disease is COVID-19 related pneumonia. In some embodiments, the viral respiratory disease is COVID-19 or a complication thereof. In some embodiments, the complication is COVID-19 related pneumonia. In some embodiments, the viral respiratory disease or complication thereof is non-critical COVID-19 related pneumonia. In some embodiments, the complication is COVID-19 related acute respiratory distress syndrome (ARDS). In some embodiments, the viral respiratory complication excludes idiopathic pulmonary fibrosis (IPF).

In some embodiments, the treatment is initiated within 90 days from a confirmed viral respiratory disease diagnosis. In some embodiments, the treatment is initiated within 60 days from a confirmed viral respiratory disease diagnosis. In some embodiments, the treatment is initiated within about 90, about 80, about 70, about 60, about 50, about 45, about 40, about 35, about 30, about 28, about 21, about 14, about 7, or about 1 day from a confirmed viral respiratory disease diagnosis. In some embodiments, the treatment is initiated between 90 and 42 days from a confirmed viral respiratory disease diagnosis. In some embodiments, the treatment is initiated within 42 days from a confirmed viral respiratory disease diagnosis. In some embodiments, the treatment is initiated within 30 days, within 14 days, within 10 days, or within 7 days from a confirmed viral respiratory disease diagnosis. In some embodiments, the treatment is initiated within 14 days from a confirmed viral respiratory disease diagnosis. In some embodiments, the treatment is initiated within from 1 day to 14 days from a confirmed viral respiratory disease diagnosis. In some embodiments, the treatment is initiated within 1 day, 2 days, 3 days, 4 days, 5 days, 6 days, 7 days, 10 days, or 14 days from a confirmed viral respiratory disease diagnosis. In some embodiments, the treatment is initiated between 14 and 28 days from a confirmed viral respiratory disease diagnosis.

In some embodiments, the treatment is initiated during the acute phase of the viral respiratory disease. In some embodiments, the treatment is initiated during the acute phase of the viral respiratory disease, and continued during the post-acute phase of the viral respiratory disease. In some embodiments, the treatment is initiated during the post-acute phase of the viral respiratory disease.

In some embodiments, the disclosure provides a method of treating post-acute sequelae of infection with SARS-CoV-2 (i.e., COVID-19). As used herein the term "post-acute sequelae" refers to one or more persistent symptoms, dysfunctions, or disorders remaining after resolution of acute COVID-19 illness.

Accordingly, in some embodiments, the disclosure provides a method of treating post-acute sequelae of infection with SARS-CoV-2, the method comprising administering to a subject in need thereof an effective amount of: 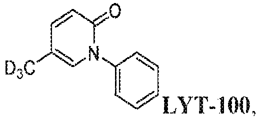 wherein the post-acute sequelae of infection are treated in the subject.

In some embodiments, the post-acute sequelae comprise one or more of post-acute respiratory complications, impaired lung function, pulmonary dysfunction, and pulmonary fibrosis. In some embodiments, the post-acute sequelae comprise progressive pulmonary fibrosis. In some embodiments, a reduction in fibrosis or fibrotic lesions on high resolution computed tomography (HRCT) is achieved in the subject.

In some embodiments, the post-acute sequelae comprise one or more of fatigue, dyspnea, cough, arthralgia, chest pain, cognitive impairment, depression, anxiety, myalgia, headache, fever, gastrointestinal issues, palpitations, pulmonary function abnormalities, acute kidney injury, myocardial inflammation, and ventricular dysfunction

In some embodiments, the administering is initiated during the acute phase of the SARS-CoV-2 infection. In some embodiments, the administering is initiated during the acute phase of the SARS-CoV-2 infection, and continued during a post-acute phase of the SARS-CoV-2 infection. In some embodiments, the administering is initiated during the post-acute phase of the SARS-CoV-2 infection.

### Dosing with LYT-100

In any of the methods and embodiments described in the present disclosure, the deuterium-enriched pirfenidone compound. e.g., LYT-100, is administered at a total daily dose of 100-2500 mg. In some embodiments, the daily dose is 100, 200, 250, 300, 400, 500, 600, 700, 750, 800, 900, 1000, 1100, 1200, 1250, 1300, 1400, 1500, 1600, 1700, 1800, 1900, 2000, 2100, 2200, 2250, 2300, 2400, or 2500 mg. In some embodiments, the daily dose is 2500 mg. In some embodiments, the daily dose is 2000 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg.

In some embodiments, the deuterium-enriched pirfenidone, e.g., LYT-100, is administered twice daily. In some embodiments, the deuterium-enriched pirfenidone, e.g., LYT-100, is administered three times daily.

In some embodiments, the deuterium-enriched pirfenidone, e.g., LYT-100, is administered 250 mg twice daily. In some embodiments, the deuterium-enriched pirfenidone, e.g., LYT-100, is administered 500 mg twice daily. In some embodiments, the deuterium-enriched pirfenidone, e.g., LYT-100, is administered 750 mg twice daily. In some embodiments, the deuterium-enriched pirfenidone, e.g., LYT-100, is administered 1000 mg twice daily. In some embodiments, the deuterium-enriched pirfenidone compound, e.g., LYT-100, is administered 1500 mg once daily. In some embodiments, the deuterium-enriched pirfenidone, e.g., LYT-100, is administered 1000 mg once daily. In some embodiments, the deuterium-enriched pirfenidone, e.g., LYT-100, is administered 750 mg once daily. In some embodiments, the deuterium-enriched pirfenidone, e.g., LYT-100, is administered 500 mg once daily. In some embodiments, the deuterium-enriched pirfenidone, e.g., LYT-100, is administered 250 mg once daily.

In some embodiments, the deuterium-enriched pirfenidone compound, e.g., LYT-100 is administered 500 mg three times daily. In some embodiments, the deuterium-enriched pirfenidone compound, e.g., LYT-100. is administered 250 mg three times daily. In some embodiments, the deuterium-enriched pirfenidone is administered 333 mg three times daily. In some embodiments, the deuterium-enriched pirfenidone is administered 166 mg three times daily. In some embodiments, the deuterium-enriched pirfenidone is administered orally up to 2403 mg/day. In some embodiments, the deuterium-enriched pirfenidone is administered at 801 mg, either once daily, twice daily, or three times daily. In any of the above embodiments, the deuterium-enriched pirfenidone is administered orally. In any of the above embodiments, the deuterium-enriched pirfenidone is administered orally in tablet form. In any of the above embodiments, the deuterium-enriched pirfenidone is administered orally in capsule form.

In any of the above embodiments, the deuterium-enriched pirfenidone is taken orally with food. In any of the above embodiments, the deuterium-enriched pirfenidone is administered without regard to food. In any of the above embodiments, the deuterium-enriched pirfenidone is administered with food. In any of the above embodiments, the deuterium-enriched pirfenidone is administered without food. In any of the above embodiments, the deuterium-enriched pirfenidone, e.g., LYT-100, is administered without dose escalation.

In some embodiments, the deuterium-enriched pirfenidone, e.g., LYT-100, is administered orally without food in two daily doses of 100 mg each. In some embodiments, the deuterium-enriched pirfenidone, e.g., LYT-100, is administered orally without food in three daily doses of 100 mg each.

In some embodiments, the deuterium-enriched pirfenidone, e.g., LYT-100, is administered orally without food in two daily doses of 200 mg each. In some embodiments, the deuterium-enriched pirfenidone, e.g., LYT-100, is administered orally without food in three daily doses of 200 mg each.

In some embodiments, the deuterium-enriched pirfenidone, e.g., LYT-100, is administered orally without food in two daily doses of 500 mg each. In some embodiments, the deuterium-enriched pirfenidone, e.g., LYT-100, is administered orally without food in three daily doses of 500 mg each.

In some embodiments, the deuterium-enriched pirfenidone, e.g., LYT-100, is administered orally without food in two daily doses of 750 mg each. In some embodiments, the deupirfenidone is administered orally without food in three daily doses of 750 mg each.

In some embodiments, the deuterium-enriched pirfenidone, e.g., LYT-100, is administered orally without food in two daily doses of 1000 mg each. In some embodiments, the deuterium-enriched pirfenidone, e.g., LYT-100, is administered orally without food in two daily doses of 1000 mg each, without dose escalation.

The duration of treatment may vary. For example, in some embodiments, the treatment duration is selected from 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, and 25 weeks, and any increment therein. In some embodiments, the treatment duration is selected from 1 month, 2 months, 3 months, 4 months, 5 months, 6 months, 7 months, 8 months, 9 months, 10 months, 11 months, or 12 months, and any increment therein. In some embodiments, treating comprises administering an effective amount of LYT-100 daily for up to 3 months or 91 days. In some embodiments, treating comprises administering an effective amount of LYT-100 daily for up to 12 months. In some embodiments, the treatment duration is one year, 2 years, 3 years, 4 years, 5 years or greater.

In some embodiments, the LYT-100 is administered in two daily doses in an amount of 500 mg each on days 1 through 3, and in two daily doses in an amount of 750 mg each thereafter, e.g., on day 4 and thereafter. In some embodiments, the LYT-100 is administered in two daily doses in an amount of 500 mg each on days 1 through 3, and in two daily doses in an amount of 750 mg each thereafter, e.g., on days 4 through 91. In some embodiments, the LYT-100 is administered in two daily doses in an amount of 500 mg each on days 1 through 3, and in two daily doses in an amount of 750 mg each thereafter, e.g., for up to 3 months or about 3 months. In some embodiments, the LYT-100 is administered in two daily doses in an amount of 500 mg each on days 1 through 3, and in two daily doses in an amount of 750 mg each thereafter, e.g., for up to 12 months or about 12 months.

In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., on days 4 through 91. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., for up to 3 months or about 3 months. In some embodiments, the LYT-100 is administered in two daily doses in an amount of 500 mg each on days 1 through 3, in two daily doses in an amount of 750 mg each on days 4 through 7, and in two daily doses in an amount of 1000 mg thereafter, e.g., for up to 12 months or about 12 months.

In some embodiments, the LYT-100 is administered in two daily doses in an amount of 500 mg each on days 1 through 3, and in two daily doses in an amount of 1000 mg each thereafter, e.g., on day 4 and thereafter. In some embodiments, the LYT-100 is administered in two daily doses in an amount of 500 mg each on days 1 through 3, and in two daily doses in an amount of 1000 mg each thereafter, e.g., on days 4 through 91. In some embodiments, the LYT-100 is administered in two daily doses in an amount of 500 mg each on days 1 through 3, and in two daily doses in an amount of 1000 mg each thereafter, e.g., for up to 3 months or about 3 months. In some embodiments, the LYT-100 is administered in two daily doses in an amount of 500 mg each on days 1 through 3, and in two daily doses in an amount of 1000 mg each thereafter, e.g., for up to 12 months or about 12 months.

In some embodiments, the LYT-100 is administered in two daily doses in an amount of 250 mg each for two days; in two daily doses in an amount of 500 mg each for two days; and in two daily doses in an amount of 750 mg each thereafter, e.g., on days 7 through 91. In some embodiments, the LYT-100 is administered in two daily doses in an amount of 250 mg each for two days; in two daily doses in an amount of 500 mg each for two days; and in two daily doses in an amount of 750 mg each thereafter, e.g., for up to 3 months or about 3 months. In some embodiments, the LYT-100 is administered in two daily doses in an amount of 250 mg each for two days; in two daily doses in an amount of 500 mg each for two days; and in two daily doses in an amount of 750 mg each thereafter, e.g., for up to 12 months or about 12 months.

In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., on days 7 through 91. In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., for up to 3 months or about 3 months. In some embodiments, the LYT-100 is administered in two daily doses in an amount of 250 mg each for two days; in two daily doses in an amount of 500 mg each for two days; in two daily doses in an amount of 750 mg each for two days; and in two daily doses in an amount of 1000 mg each thereafter, e.g., for up to 12 months or about 12 months.

In some embodiments, administering the LYT-100 does not comprise an initial titration. In some embodiments, the LYT-100 is administered in two daily doses in an amount of 500 mg each for up to 3 months or about 3 months. In some embodiments, the LYT-100 is administered in two daily doses in an amount of 500 mg for up to 12 months or about 12 months. In some embodiments, the LYT-100 is administered in two daily doses in an amount of 750 mg each for up to 3 months or about 3 months. In some embodiments, the LYT-100 is administered in two daily doses in an amount of 750 mg each for up to 12 months or about 12 months. In some embodiments, the LYT-100 is administered in two daily doses in an amount of 1000 mg each for up to 3 months or about 3 months. In some embodiments, the LYT-100 is administered in two daily doses in an amount of 1000 mg each for up to 12 months or about 12 months.

In some embodiments, one or more of the following applies to the subject prior to treatment with LYT-100: the subject has a positive COVID-19 RT-PCR or SARS-CoV-2 RNA diagnostic test result; the subject is or was hospitalized for COVID-19 respiratory disease for at least 1-day, and required at least one of the listed treatment modalities: mechanical ventilation (MV), extra-corporeal membrane oxygenation (ECMO), non-invasive ventilation (NIV), high flow nasal oxygen (HFNO) during hospitalization; the subject has a confirmed diagnosis of COVID-19; the subject exhibits COVID-19 pneumonia as confirmed by imaging, including by chest X-ray or high-resolution computerized tomography with a minimum of two lobe involvement.

In some embodiments, the subject exhibits shortness of breath ≥ grade 3 on a Modified Borg Dyspnea Scale (mBDS).

In some embodiments, the subject prior to treatment with LYT-100, does not require extra-corporeal membrane oxygenation (ECMO) or mechanical ventilation (MV) for 72 hours before starting administration of LYT-100, or has been off of ECMO/MV for at least 48 hours before starting administration of LYT-100. In some embodiments, the subject, prior to treatment with LYT-100, may require extra-corporeal membrane oxygenation (ECMO) or mechanical ventilation (MV).

In some embodiments, the subject exhibits one or more of the following prior to treatment: shortness of breath ≥ grade 3 on mBDS dyspnea scale, fever >38C°; cough; elevated C-Reactive Protein (CRP) ≥ 10.0 mg/dL; oxygen saturation ≤93% at rest; requires supplemental oxygen; requires high-flow oxygen or non-invasive ventilation.

In some embodiments, the subject does not have one or more of the following: a preexisting respiratory condition unrelated to the viral respiratory disease; a resting heart rate of ≥ 120 bpm, systolic blood pressure of > 180 mm Hg and a diastolic blood pressure of > 100 mm Hg; a fall in pulse oximetry oxygen saturation (SpO₂) to <80% upon ambulation; a body mass index ≥ 40 kg/m²; a lactate dehydrogenase (LDH) value >360; a history of anaphylactic reaction including reactions to general anaesthetic agents; a history of allergic reaction to any drug which led to significant morbidity; a history of prior allergic reaction to pirfenidone or LYT-100; sepsis; or septic, hypovolemic, cardiogenic, or neurogenic shock.

In some embodiments, the subject is not intubated or mechanically ventilated. In some embodiments, the subject has not been intubated, mechanically ventilated, or received ECMO within 72 hours prior to initiating treatment. In some embodiments, the subject does not require extracorporeal membrane oxygenation (ECMO).

In some embodiments, the subject is not undergoing dialysis. In some embodiments, the subject does not have a history of dialysis.

In some embodiments, the subject is not currently taking any of the following: fluvoxamine, enoxacin, or ciprofloxacin; corticosteroids or nonsteroidal anti-inflammatory drugs (NSAIDs); inhibitors of CYP1A2; inducers of CYP1A2, CYP2C9 or 2C19; nicotine; and any drug associated with substantial risk for prolongation of the QTc interval. In some embodiments, the subject is not currently using tobacco or nicotine products.

In some embodiments, an improvement of 25 meters or more in a 6-minute walk test (6-MWT) over monthly increments through day 91 of treatment is achieved. In some embodiments, the improvement of 25 meters or more is achieved at a time point which is earlier than that achieved in a subject which has not been treated with LYT-100. In some embodiments, a rate of improvement in in the distance walked on the 6MWT in a subject which has been treated with LYT-100 is greater than a rate of improvement in a subject which has not been treated with LYT-100. In some embodiments, the subject exhibits one or more of: a statistically significant increase in the distance walked on the six-minute walk test (6MWT) as compared to a baseline 6MWT result in the treated subject; a statistically significant increase in the distance walked on the six-minute walk test (6MWT) as compared to a subject who has not been treated with LYT-100; a statistically significant rate of improvement in the distance walked on the 6MWT as compared to a rate of improvement in the distance walked on the 6MWT in a subject who has not been treated with LYT-100. In some embodiments, when a distance walked on the 6MWT is measured at two or more time points after beginning treatment with LYT-100, the subject exhibits a difference in the distance walked in the 6MWT between the two or more time points (AD) which is greater than a AD in a subject who has not been treated with LYT-100.

In some embodiments, at least a 1.5-point decrease in the Modified Borg Dyspnea Scale is achieved.

In some embodiments, one or more of the following is achieved in the subject: an improvement in blood oxygenation level in the human subject over 91 days of treatment, as determined by pulse oximetry; a 10 unit drop in an overall score in the Short Form Health Survey (SF-36 v2) over 91 days of treatment; an increase of ≥2 units in the overall St. George's Respiratory Questionnaire-Idiopathic Pulmonary Fibrosis (SGRQ-I) score over 91 days of treatment; an improvement in pulmonary inflammation; an improvement in fibrosis; an improvement in other radiographic abnormalities, as measured by determining at least a 2-point score improvement over 91 days of treatment on high resolution computed tomography (HRCT) scans, from the level of the upper thoracic inlet to the inferior level of the costophrenic angle over 28, 56, and 91 days of treatment utilizing the following scoring system: a) number of involved segments; and b) lung lobe involvement for each of five lobes via the semi- Quantitative Lung Fibrosis (QLF) added and Computer-Aided Diagnosis (CAD) QLF Scores.

In some embodiments, the subject exhibits reduced evidence of lung fibrosis, respiratory complications, or pulmonary dysfunction relative to a subject who has not been treated with LYT-100, as measured by a chest X-ray or a CT scan, or as evidenced by a pulmonary function test result.

In some embodiments, the subject exhibits reduced evidence of lung fibrosis, respiratory complications, or pulmonary dysfunction relative to a subject who has not been treated with LYT-100, as measured by a pulmonary function test result, wherein the pulmonary function test result is a statistically significant improvement in the distance walked on the six-minute walk test (6MWT) compared to a baseline 6MWT result in the treated subject.

In some embodiments, at least a 1.5-point decrease in the Modified Borg Dyspnea Scale is achieved.

In some embodiments, the subject exhibits an improvement in the score on one or more of the following: Dyspnoea-12; Patient-reported quality of life (SF-36 v2).

In some embodiments, a reduction in fibrosis or fibrotic lesions on high resolution computed tomography (HRCT) is achieved in the subject. In some embodiments, the fibrotic lesion reduced in the subject is one or more of ground glass opacity, reticular patterning, honeycombing, traction bronchiectasis, bronchiolectasis, interlobular septal thickening, ill-defined margins, air bronchogram, "crazy-paving" pattern, thickening of the adjacent pleura, nodules, cystic changes, pleural effusion, or lymphadenopathy.

In some embodiments, at least a 25% reduction in one or more of the following biomarkers is achieved: C-Reactive Protein (CRP); d-dimer; cardiac troponin; ferritin; lactate dehydrogenase (LDH); interleukin-6 (IL-6); TGF-β1; TNF-a; IL-1β; PDGF- β; GCSF; VEGF.

In some embodiments, an improvement by 2 points or higher on the WHO Ordinal Scale for Clinical Improvement is achieved.

In some embodiments, mechanical ventilation of the subject is prevented. In some embodiments, intubation of the subject is prevented. In some embodiments, use of one or more of the following in the subject is prevented: non-invasive ventilation, O₂ supplementation, extra corporeal membrane oxygenation (ECMO).

### Combination Therapies

The deuterium-enriched pirfenidone compound, e.g., LYT-100, as disclosed herein can be administered alone or in combination with one or more other therapeutic compounds and/or therapeutic processes. In some embodiments, LYT-100 is administered in combination with one or more anti-T cell agent(s), anti-TGF-β1, and/or anti-angiotensin agents. Examples of suitable agents include, but are not limited to, tacrolimus, teriflunomide, leflunomide, captopril, losartan, cyclosporine, pimecrolimus, denileukin, diftitox, basiliximab, LY550410, LY580276, SB-505124, and galunisertib (LY2157299 Monohydrate). In some embodiments, the anti-T cell agent is selected from tacrolimus, teriflunomide, leflunomide, cyclosporine, pimecrolimus, denileukin diftitox, and basiliximab. In some embodiments, the anti-TGF-β1 agent or anti-angiotensin agent is selected from pirfenidone, captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. In some embodiments, the anti-angiotensin agent is an ACE agonist, for example, an ACE-2 agonist.

In some embodiments, the deuterium-enriched pirfenidone compound, e.g., LYT-100, is administered in combination with one or more anti-inflammatory agent(s). Non-limiting examples of anti-inflammatory agents include etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, and tolmetin. Anti-inflammatory agents also include Cox-2 inhibitors, including but not limited to, celecoxib, apricoxib, robenacoxib, valdecoxib, anitrazafen, tilmacoxib, flumizole, cimicoxib, rofecoxib, mavacoxib, and firocoxib.

In some embodiments, the deuterium-enriched pirfenidone, e.g., LYT-100, is administered with one or more additional therapeutic agent(s) selected from sepsis agents. antibacterials, antifungals, antivirals, anti-pathogen, anti-coagulants, thrombolytics, steroidal drugs, anti-inflammatory drugs, non-steroidal anti-inflammatory drugs (NSAIDs), opioids, anesthetics, calcium channel blockers, Beta-blockers, nitrates or nitrites, ACE inhibitors, statins, platelet aggregation inhibitors, adenosine, digitoxin, anti-arrhythmic agents, sympathomimetic drugs, endothelin converting enzyme (ECE) inhibitors, thromboxane enzyme antagonists, potassium channel openers, thrombin inhibitors, growth factor inhibitors, platelet activating factor (PAF) antagonists, anti-platelet agents, Factor VIIa Inhibitors, Factor Xa Inhibitors, renin inhibitors, neutral endopeptidase (NEP) inhibitors, vasopepsidase inhibitors, HMG CoA reductase inhibitors, squalene synthetase inhibitors, fibrates, bile acid sequestrants, anti-atherosclerotic agents, MTP Inhibitors, potassium channel activators, alpha-PDE5 agents, beta-PDE5 agents, diuretics, anti-diabetic agents, PPAR-gamma agonists, mineralocorticoid enzyme antagonists, aP2 inhibitors, protein tyrosine kinase inhibitors, anti-inflammatories, antiproliferatives, chemotherapeutic agents, immunosuppressants, anticancer agents, cytotoxic agents, antimetabolites, farnesyl-protein transferase inhibitors, hormonal agents, microtubule-disruptor agents, microtubule-stabilizing agents, topoisomerase inhibitors, prenyl-protein transferase inhibitors, cyclosporins, TNF-alpha inhibitors, cyclooxygenase-2 (COX-2) inhibitors, gold compounds, antalarmin, Z-338 and platinum coordination complexes.

In some embodiments, the deuterium-enriched pirfenidone, e.g., LYT-100, is administered with one or more steroidal drug(s) selected from the group consisting of aldosterone, beclometasone, betamethasone, deoxycorticosterone acetate, fludrocortisone acetate, hydrocortisone (cortisol), prednisolone, prednisone, methylprenisolone, dexamethasone, and triamcinolone. Alvesco (ciclesonide) is a glucocorticoid steroid derivative for asthma and allergies. In some embodiments, the LYT-100 is administered with ciclesonide. In some embodiments, the LYT-100 is administered with an inhibitor of acetaldehyde dehydrogenase, e.g., Disulfiram.

In some embodiments, the deuterium-enriched pirfenidone, e.g., LYT-100, is administered with one or more non-steroidal anti-inflammatory agent(s) selected from the group consisting of aceclofenac, acemetacin, amoxiprin, aspirin, azapropazone, benorilate, bromfenac, carprofen, celecoxib, choline magnesium salicylate, diclofenac, diflunisal, etodolac, etoracoxib, faislamine, fenbuten, fenoprofen, flurbiprofen, ibuprofen, indometacin, ketoprofen, ketorolac, lomoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinprazone, suprofen, tenoxicam, tiaprofenic acid, and tolmetin.

In some embodiments, the deuterium-enriched pirfenidone, e.g., LYT-100, is administered with one or more antiviral agent(s) selected from Favipiravir, Nitazoxanide, Remdesivir, Mycophenolic acid, Chloroquine, Cidofovir. Niclosamide, Brincidofovir, EIPA (amiloride), BCX4430 (Galdecivir), Gemcitabine, Rapamycin (Sirolimus), ABT-263, Berberine, Cyclosporine, Emetine, Amodiaquine, Brequinar, Obatoclax, Ribavirin, Luteolin, Tilorone (Amixin), Glycyrrhizin, Eflornithine, Sorafenib, Suramin, Monensin, Arbidol (Umifenovir), Sunitinib, Labyrinthopeptin A2, Silvestrol, Emodin, Amiodarone, Raloxifene, Azithromycin, Labyrinthopeptin A1, Mitoxantrone, Ganciclovir, Letermovir, Artesunate, Dasatinib, Ivermectin, Foscarnet. Simvastatin, Bortezomib, Camptothecin, Itraconazole, Lopinavir, Azacitidine, Leflunomide, Nelfinavir, Valacyclovir, 4-HPR (Fenretinide), Aprotinin, Topotecan, Oritavancin, Novobiocin, Pentosan polysulfate, Ezetimibe, Hydroxychloroquine, Ritonavir, Filociclovir, Isolanid (lanatoside C), Sofosbuvir, Manidipine, Lovastatin, Metformin, Minocycline, Dalbavancin, Teicoplanin, N-MCT, Roscovitine (Seliciclib), Caffeine, Genistin, Regorafenib, Homoharringtonine, Alisporivir, Cepharanthine, Erlotinib, Gefitinib, Hexachlorophene, Imatinib, Lobucavir, Verapamil, Apoptozole, Fluoxetine, Fluvastatin, Posaconazole, Nafamostat, Tamoxifen, Aciclovir, Chlorpromazine, Acetylsalicylic acid, Camostat, Memantine, Tenofovir, Indomethacin, Flavopiridol, Bithionol, Doxycycline, Salinomycin, Saracatinib, Bepridil, Quinine, Diphyllin, Telavancin, Promethazine, Kasugamycin, Trametinib, Zanamivir, CYT107, Lamivudine, Thymalfasin, Pegasys (Peginterferon alpha 2a), Danoprevir, ACE2, and Mefloquine. In some embodiments, the deuterium-enriched pirfenidone, e.g., LYT-100, is administered with niclosamide or nitazoxonide. In some embodiments, the deuterium-enriched pirfenidone, e.g., LYT-100, is administered with one or more antiviral agent(s) selected from lopinavir, ritonavir and remdesivir.

In some embodiments, the combination therapy takes the form of a fixed combination, e.g., a composition comprising the deuterium-enriched pirfenidone in combination with one or more other therapeutic compounds as described herein. As used herein, the term "combination," "combined," and related terms refers to the simultaneous or sequential administration of therapeutic agents in accordance with the present disclosure. For example, a compound or a pharmaceutically acceptable salt or pharmaceutical composition thereof may be administered with another therapeutic agent simultaneously or sequentially in separate unit dosage forms or together in a single unit dosage form. Accordingly, the present disclosure provides a single unit dosage form comprising a disclosed compound, an additional therapeutic agent, and a pharmaceutically acceptable excipient, carrier, adjuvant, diluent, or vehicle. In some embodiments, the additional agent is formulated in a separate composition from the disclosed deuterium-enriched pirfenidone. In some embodiments, the combination therapy comprises the administration of the deuterium-enriched pirfenidone and one or more other therapeutic compounds, which can be administered independently of one another at the same time or at different times. In some embodiments, the deuterium-enriched pirfenidone is administered as a combined administration of fixed combinations with the one or more other therapeutic compounds.

Embodiments of the present disclosure can be further defined by reference to the following non-limiting examples. It will be apparent to those skilled in the art that many modifications, both to materials and methods, can be practiced without departing from the scope of the present disclosure.

### EXEMPLIFICATION

### Example 1: LYT-100 Significantly Reduced Area of Fibrosis in Mouse Model

Non-alcoholic steatohepatitis (NASH) is characterized by lobular inflammation, hepatocyte ballooning and degeneration progressing to liver fibrosis. LYT-100 was orally administered at 0 mL/kg (Vehicle only: 0.5% CMC) or 10 mL/kg twice daily from 6-9 weeks of age in 18 male mice in which NASH mice was induced by a single subcutaneous injection of 200 µg streptozotocin solution 2 days after birth and feet with a high fat diet after 4 weeks of age. LYT-100 was administered at an oral dose of 30 mg/kg twice daily (60 mg/kg/day). In addition, nine non-NASH mice were fed with a normal diet and monitored.

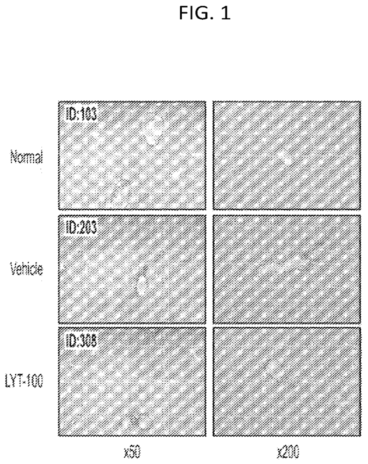

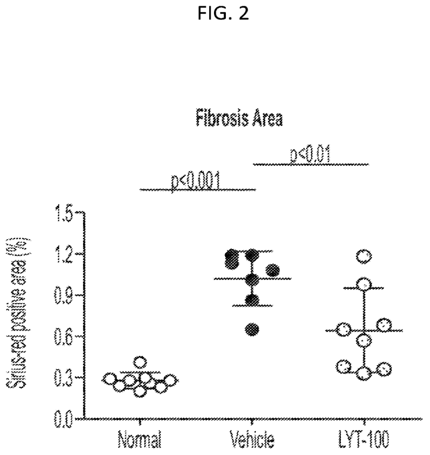

**FIG. 1** depicts representative micrographs of Sirius-red stained liver sections illustrating that LYT-100 significantly reduced the area of fibrosis. Specifically, liver sections from the vehicle group exhibited collagen deposition in the pericentral region of the liver lobule. And the LYT-100 group showed a significant reduction in the fibrosis area compared to the vehicle group. These results demonstrate that LYT-100 has a potential to inhibit the progression of fibrosis. **FIG. 2** illustrates the percent fibrosis area for LYT-100 versus vehicle and control. The results are also summarized **Table 5** below.

**Table 5: Fibrosis Area**

| Parameter (mean ± SD) | Normal (n=9) | Vehicle (n=7) | LYT-100 (n=8) |
|---|---|---|---|
| Fibrosis Area (%) | 0.27 ± 0.06 | 1.02 ± 0.20 | 0.64 ± 0.31* |

| | | | |
|---|---|---|---|
| *p<0.01, Vehicle vs LYT-100 | | | |

Liver sections from the Vehicle group exhibited severe micro- and macro vesicular fat deposition, hepatocellular ballooning and inflammatory cell infiltration. While LYT-100 hepatocyte ballooning was similar to Vehicle, scores were lower for lobular inflammation and steatosis. **(Table 6 and 7).**

**Table 6: NAFLD Activity Score**

| Group | n | Score | | | | | | | | | | | NAS (mean ± SD) |
|---|---|---|---|---|---|---|---|---|---|---|---|---|---|
| | | Steatosis | | | | Lobular Inflammation | | | | Hepatocyte ballooning | | | |
| | | 0 | 1 | 2 | 3 | 0 | 1 | 2 | 3 | 0 | 1 | 2 | |
| Normal | 9 | 9 | - | - | - | 9 | - | - | - | 9 | - | - | 0.0 ± 0.0 |
| Vehicle | 7 | 2 | 5 | - | - | - | 2 | 1 | 4 | - | 1 | 6 | 4.9 ± 1.2 |
| LYT-100 | 8 | 4 | 3 | 1 | - | - | 5 | 3 | - | - | - | 8 | 4.0 ± 1.1 |

**Table 7: Definition of NAS Components**

| **Item** | **Score** | **Extent** |
|---|---|---|
| | | |
| Steatosis | 0 | <5% |
| | 1 | 5-33% |
| | 2 | >33%-66% |
| | 3 | >66% |
| | | |
| Hepatocyte Ballooning | 0 | None |
| | 1 | Few balloon cells |
| | 2 | Many cells/prominent ballooning |
| | | |
| Lobular Inflammation | 0 | No foci |
| | 1 | <2 foci/200x |
| | 2 | 2-4 foci/200x |
| | 3 | >4 foci/200x |

As evidenced above, LYT-100 significantly reduced the area of fibrosis, reduced inflammation, and reduced accumulation of fat (steatosis), as compared to the untreated NASH mice.

### Example 2: LYT-100 Reduction of TGF-β-induced proliferation and collagen levels in Primary Mouse Lung Fibroblasts

LYT-100 was evaluated for an ability to reduce the TGF-β-induced proliferation of, and collagen levels in, Primary Mouse Lung Fibroblasts (PMLF).

Inhibition of p38 members by LYT-100 is important as p38 members are activated by TGF-β signaling pathway. TGF-β activation, in turn plays a significant role in transcriptional induction of the collagen type IA2. The collagen type IA2 makes up the majority of extracellular matrix, which accumulates during progression of, e.g., IPF. Deposition of collagen is one of the most important components of fibrotic lung tissue, a process primarily induced by TGF-β. Since accumulation of insoluble collagen encroaches on the alveolar space, it plays pivotal role in distortion of lung architecture and progression of IPF. Therefore, inhibition of TGF-β -induced collagen synthesis is an important target for IPF. In addition to insoluble (structural) collagen, fibrotic lungs of IPF patients also show high levels of non-structural (soluble) collagen.

Although this type of collagen may eventually become insoluble collagen, until then, soluble collagen can serve as a ligand for integrin receptors of lung fibroblasts and epithelial cells. Binding of soluble collagen to these receptors induces proliferation and migration of these cells. Fibronectin is another important component of fibrotic lungs as it is induced by TGF-β and functions both as a structural component of extra cellular matrix (ECM), as well as a ligand for integrin receptors. Just like soluble collagen, binding of fibronectin to integrin receptors induces the proliferation of fibroblast and epithelial cells of the lungs and plays significant role in progression of IPF.

### Preparation of Primary Mouse Lung Fibroblast

Primary Mouse lung fibroblast were prepared as follows. One lung was removed from 2 months old male BalbC Mouse, perfused with sterile PBS, minced and incubated in 2 ml of serum free Dulbecco's Modified Eagle's Medium (DMEM) containing 100 µg/ml of collagenase I for one hour at 37°C. Each sample was centrifuged at 1500 r.p.m (revolution per minute) for 5 minutes, washed three times with PBS and the final cellular pellet was resuspended in DMEM supplemented with 10% serum and Pen/Strep, and incubated in 150 mm plates at 37oC with 80% humidity and %% CO2. The growth medium was removed and fresh medium was added every day for 10 days.

### Testing the effect of LYT-100 on Survival of Primary Mouse Lung Fibroblast

LYT-100 was evaluated for an ability to alter TGF-β-induced proliferation of PMLF. At the end of 10-day incubation period above, lung fibroblasts were confluent. Before testing the effect of LYT-100 on survival of these cells, fibroblasts were tripsinized and five thousand cells were plated into 96 well plate in 200 µL complete DMEM, and incubated until cells reached to 95-100% confluency, then the medium was removed and complete DMEM containing proline (10 µM) and Ascorbic acid (20 µg/ml) was added. LYT-100, dissolved in pure ethanol, was added to the plates at a final concentration of 500 µM 1 h prior to addition of TGF-β (5ng/ml), and cells were further incubated for 72 hrs. One hundred µL of the growth medium was removed and 20 µL of MTT stock solution (prepared in PBS at 5.5 mg/ml concentration) was added and cells were incubated for 4 hours, then 100 µ1 of DMSO was added, and absorbance of developed color was monitored at 540-690 nm.

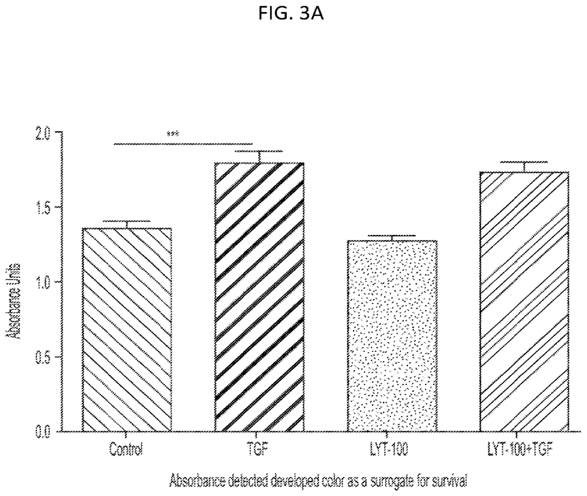

As shown at **FIG. 3A****,** LYT-100 did not affect the survival of PMLF alone. TGF-β (5 ng/ml) significantly induced the proliferation of PMLF by nearly 45% (p=0.001), and LYT-100 did appear to diminish TGF-β-induced proliferation of PMLF by 10%, but this effect was not statistically significant (p=0.19).

### TGF-β-induced Insoluble Collagen Synthesis using 6-well plate format

The effect of LYT-100 on inhibition of TGF-β-induced collagen synthesis was evaluated in PMLF in a 6-well format. One hundred thousand Primary Mouse Lung Fibroblasts were plated in 6-well plates and incubated in complete DMEM until they reached confluency. The incubation medium was removed and complete DMEM containing proline (10 µM) and Ascorbic acid (20 µg/ml) was added. LYT-100 was added to the plates at a final concentration of 500µM 1 h prior addition of TGF-β (5ng/ml), and cells were further incubated for 72 hrs.

Supernatant was removed, cells were washed with cold PBS, 1 ml Sircol reagent was added. The Sircol reagent contains the collagen binding dye Sirius red. The cells were scraped off with Sircol reagent and samples were shaken for 5 h at room temperature (RT), centrifuged at 10,000 rpm for 5 min, supernatant was removed, the pellet was washed in 0.5 M acetic acid to remove unbound dye, and recentrifuged at 10,000 rpm for 5 min, supernatant was removed and the final pellet was dissolved in 1 ml 0.5M NaOH and shaken at RT for 5 h. A sample of 100 µl of resultant solution was placed in 96-well. The color reaction was assessed by optical density at a wave length of 600 nm.

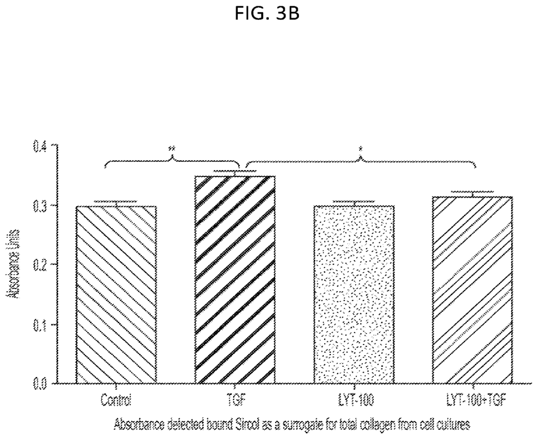

As shown in **FIG. 3B****,** PMLF responded to TGF-β with increased total collagen levels, (increase of 21%; p=0.0087). LYT-100 inhibited this induction by 15% (p=0.026), as compared to the TGF-β alone, without reducing the background level of collagen.

### TGF-β-induced Insoluble Collagen Synthesis using 96-well plate format

The effect of LYT-100 on TGF-β-induced collagen was confirmed in a high throughput collagen assay using 96-well plate format. Approximately 5,000 primary mouse fibroblasts were plated in complete DMEM in 96 well plates and incubated for 3 days at which time the cultures achieved confluency. After cells reached confluency, the medium was removed and fresh DMEM supplemented with ascorbic acid (20 µg /ml) and proline (10 µMol) was added. LYT-100 was then added to the appropriate cultures at a final incubation concentration of 500 µM. One hour later, TGF-β was added to the appropriate cultures at a final concentration of 5 ng/ml. After 72 hours, the media was replaced with a 0.5% glutaraldehyde solution. After 30 minutes, the adherent cells were washed and subsequently incubated with acetic acid at a final concentration of 0.5M. After a 30 min room temperature incubation, and subsequent washing steps, the wells were incubated with Sircol reagent. After 5 hours, the unbound dye was removed and the plates were washed and allowed to dry. To extract collagen-bound Sircol, 100 µL of alkaline solution (0.5M NaOH) was added and plates were shaken for 1 h on rotary shaker at room temperature. Absorbance at 600 nm was determined to detect bound collagen.

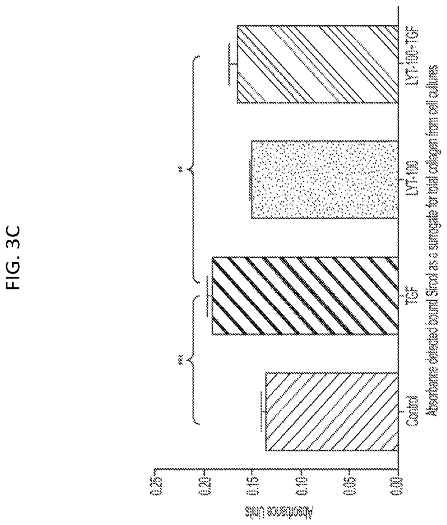

As shown in FIG. 3C, in the 6-well format, TGF-β induced insoluble collagen level by 40% (p=0.0002), LYT-100 diminished this TGF-β-stimulated collagen accumulation by 24% (p=0.0003) without reducing the background level of collagen.

### TGF-β-induced Soluble Fibronectin and Collagen Synthesis

LYT-100 was evaluated for its ability to modify TGF-β-induced soluble fibronectin and soluble collagen synthesis using a selective ELISA. Approximately 5,000 primary mouse lung fibroblasts were plated in complete DMEM in 96 well plates and incubated for 3 days at which time the cultures achieved confluency. After cells reached to confluency, medium was removed and fresh DMEM supplemented with ascorbic acid (20 µg /ml) and proline (10 µM) was added. LYT-100 was then added to the appropriate cultures at a final incubation concentration of 500 µM. One hour later, TGF-β (5 ng/ml) was added to the appropriate cultures at a final concentration. After 72 hours, 200 µl samples of the supernatant were placed onto an ELISA plate and incubated overnight. After blocking with %1 BSA for 2 h, plates were incubated with either an anti-collagen type I antibody or an anti-fibronectin antibody.

The plates were washed after 1 hour and incubated with secondary horseradish peroxidase-conjugated antibodies (anti-goat for the collagen antibody, anti-rabbit for the fibronectin antibody). After a series of washing steps the color reagent TMB (3,3',5,5'-Tetramethylbenzidine) was added and 15 minutes later the reactions were terminated with equal volumes of 2 N H2SO4. The levels of soluble collagen and fibronectin were determined by evaluating absorbance at 450 nm.

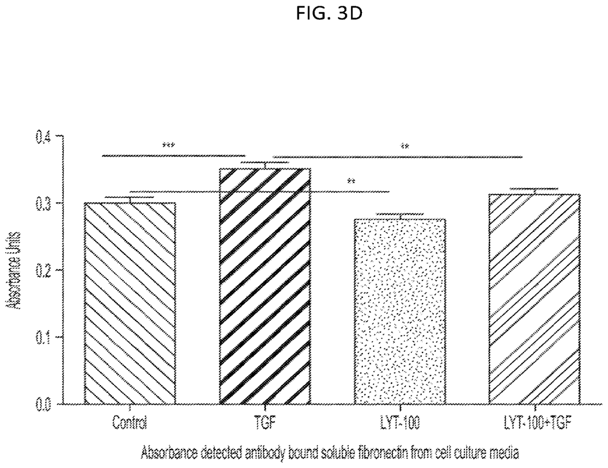

Referring to **FIG. 3D****,** TGF-β induced the level of soluble fibronectin by 16% (p=0.0021). LYT-100 inhibited TGF-β-dependent induction of fibronectin by 11% (p=0.0185). Moreover, LYT-100 also inhibited the background level of soluble fibronectin by 10% (p=0.03).

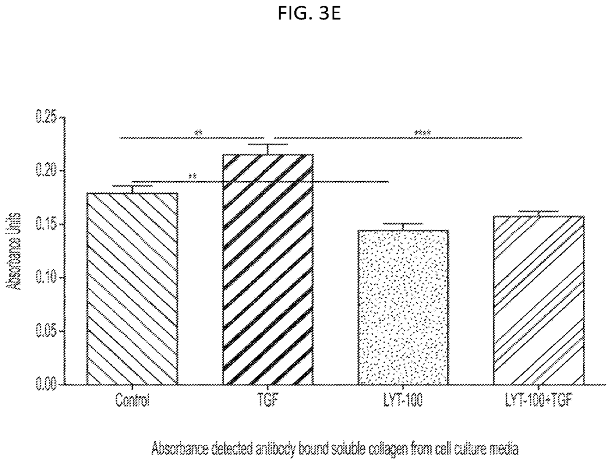

As shown in FIG. 3E, TGF-β induced the level of soluble collagen by 20% (p=0.0185). LYT-100 inhibited this TGF-β-dependent increase by 36% (p=0.0001). Moreover, it also inhibited background level of soluble collagen by 23% (p=0.0115).

In summary, LYT-100 was found to: (i) reduce TGF-β-induced cell proliferation, (ii) reduce both background and TGF-β-induced levels of insoluble (structural) collagen; (iii) reduce both background and TGF-β-induced levels of soluble collagen; and (iv) reduce both background and TGF-β-induced levels of soluble fibronectin.

During the progression of IPF, an accumulation of extra cellular matrix components such as collagen and an increase in the fibroblast population is observed. Persistent proliferation of fibroblasts is considered an important contributor to the lung architecture in IPF, including the diminished interstitial spaces of the alveoli. Thus, reducing TGF-β-induced proliferation of fibroblasts and structural collagen with LYT-100 has the potential to prolong lung function in IPF. In addition to inhibiting TGF-β-induced insoluble collagen level, LYT-100 also inhibits TGF-β-induced secreted collagen and fibronectin β. Secreted collagen and fibronectin not only increase the rate of formation of fibrotic foci in the lung, these proteins can also act as ligands for integrin receptors. When integrin receptors are activated they induce not only the proliferation of epithelial cells and fibroblasts of the lungs, but they also, along with TGF-β, induce epithelial mesenchymal transition (EMT) of the epithelial cells of the lungs. EMT causes these cells to migrate to different regions of the lungs. This migration is considered to be a very important contributor for the generation of new fibrotic foci in the lungs and progression of IPF.

LYT-100 has the ability to inhibit TGF-β-induced pro-fibrotic processes and to reduce basal factors, which have the potential to exacerbate ongoing fibrosis.

### Example 3: Effect of LYT-100 on L929 Cells

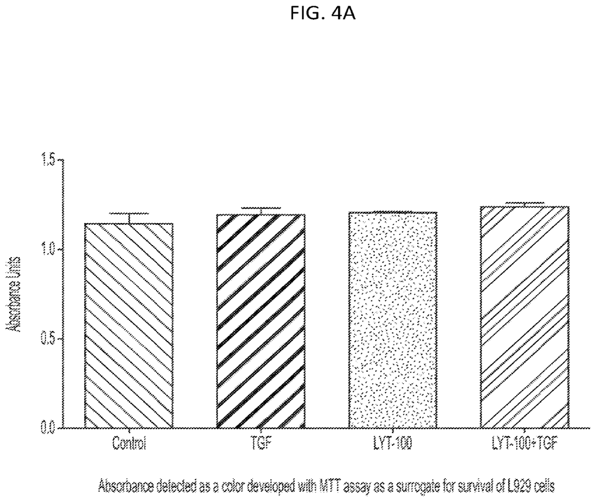

The effect of LYT-100 on survival of L929 Cells was determined. Five thousand L929 cells were plated in completed DMEN and incubated until confluency for 3 days. Medium was removed and complete DMEM containg Prolin (20 µg/ml) and ascorbic acid (10 uM) was added. LYT-100 was given at 500µM 1 h prior addition of TGFb (5ng/ml), and cells were further incubated for 72 hrs. 100µL of medium was removed, 20µL MTT solution was added for 4 hrs, then 100 µl of DMSO was added, and absorbance of developed dark pink color was determined at 54-690 nM. FIG. 4A illustrates that LYT-100 does not affect survival of L929 cells.

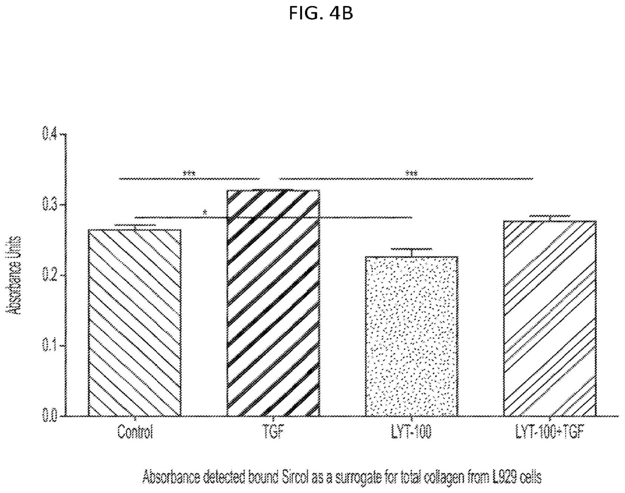

The effect of LYT-100 on TGF-induced collagen synthesis in 6-wells was determined. 100,000 L929 cells were plated in complete DMEN and incubated until confluency for 3 days. Medium was removed and complete DMEM containg Prolin (20 µg/ml) and ascorbic acid (10 µM) was added. LYT-100 was given at 500µM 1 hour prior addition of TGF-β (5ng/ml). Cells were further incubated for 72 hrs. Supernatant was removed, cells were washed with cold PBS, 1 ml SIRCOL reagent was added onto the cells and cells were scraped off, samples were shaken for 5 h.at RT, centrifuged at 10.000 rpm for 5 min, supernatant was removed, pellet was dissolved in 0.5 M acetic acid to remove unbound dye, and re-centrifuged at 10.000 rpm for 5 min, supernatant was removed and final pelet was dissolved in 1 ml 0.5M NaOH, shaken at RT for 5 h, 100 µl of resulted solution was placed in 96-well and O.D was determined at 600. The results are summarized in **FIG.** 4B, which illustrates that LYT-100 inhibits TGF-induced collagen synthesis. LYT-100 also significantly inhibits collagen synthesis in the absence of added TGF-β.

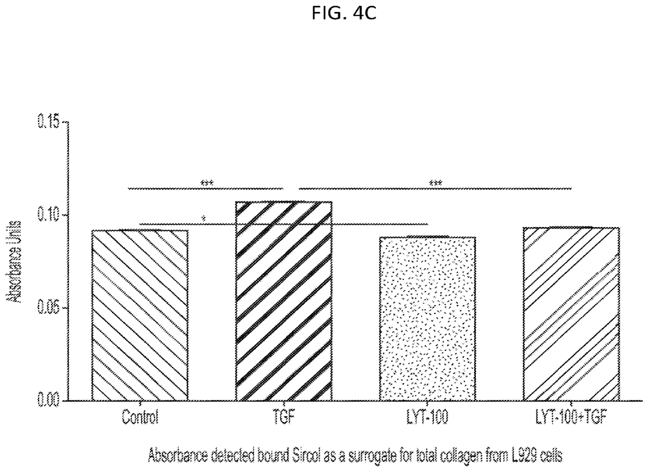

Next, the effect of LYT-100 onTGF-induced collagen synthesis was confimed using 96-well plate format. Five thousand L929 cells were plated in complete DMEN and incubated until confluency for 3 days. Medium was removed and complete DMEM containg Prolin (20 µg/ml) and ascorbic acid (10 µM) was added. LYT-100 was given at 500µM 1 h prior addition of TGF-β (5ng/ml). Cells were further incubated for 72 hrs. Supernatant was removed, 0.5% gluteraldehyde was added for 30 min at RT, removed, washed 3X with ddwater, 0.5 M acetic acid was added for 30 min at RT, removed, washed with water, air dried and 100 µl SIRCOL dye was added for 5 h at RT. Dye was removed, plate was washed extensively under running water, air dried and 200 µl of 0.5 M NaOH was added, plates were shaken at RT for 1 h, and OD was determined at 600 nm. The results summarized in **FIG. 4C** illustrate that LYT-100 significanly inhibited or reduced TGF-β-induced total collagen levels. LYT-100 also signficantly inhibited or reduced total collagen level in the absence of TGF-β induction.

The effect of LYT-100 on TGF-induced Soluble Collagen Synthesis was determined using a 96-well plate format. Five thousand L929 cells were plated in complete DMEN and incubated until confluency for 3 days. Medium was removed and complete DMEM containing Prolin (20 µg/ml) and ascorbic acid (10 µM) was added. LYT-100 was given at 500µM 1 h prior addition of TGF-β (5ng/ml). Cells were further incubated for 72 hrs. 200 µl supernatant of 96-well SIRCOL plate was placed onto ELISA plate and incubated O/N. Next day, supernatant was removed and 100 ul of 1%BSA in PBST was added and incubated for 2 h at RT, BSA was removed, plate was washed 3x with 200 µ1 of PBST, and anti-collagen type I a.b was added at 1:2000 dilution (prepared in %1 BSA in PBST), incubated at RT for 1 h, primary a.b was removed, plate was washed 3x with 200 µl PBST, and secondary anti-goat HRP was added at 1:2000 dilution, incubate at RT for 1 h, removed, plate was washed 3x with 200 µl PBST and 100 µl of TMB solution was added for color development for 15 min, then 100 µl of 2 N H2SO4 was added to stop the reaction and O.D of developed yellow color was determined at 450 nm.

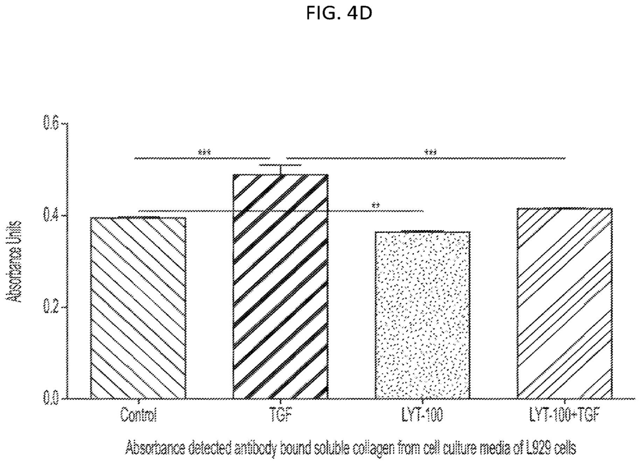

As illustrated in **FIG. 4D****,** LYT-100 significantly inhibits TGF-β-induced soluble collagen levels. LYT-100 also signficantly reduced soluble collagen levels in the absence of TGF-β-induction.

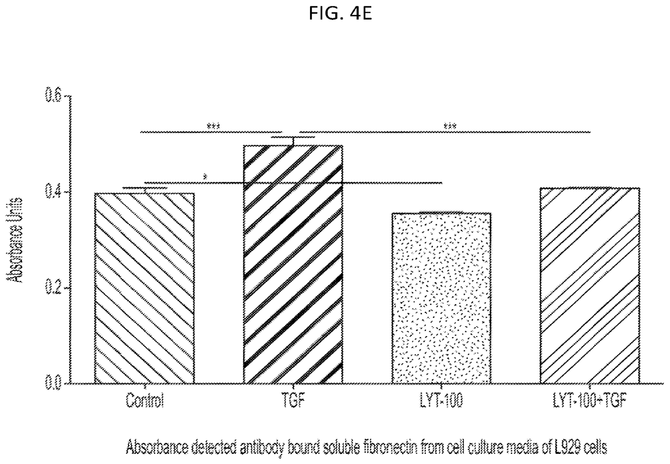

Fibronectin is another important component of fibrotic lungs as it is induced by TGF-β and functions both as a structural component of extra cellular matrix as well as well as a ligand for integrin receptors. Just like soluble collagen, binding of fibronectin to integrin receptors induces the proliferation of fibroblast and epithelial cells of the lungs. The effect of LYT-100 of TGF-induced soluble fibronectin synthesis was determined using a process similar to that described in the above paragraph for soluble collagen synthesis except that a fibronectin ELISA was used. As illustrated in **FIG. 4E****,** LYT-100 signficantly reduced soluble fibronectin levels, in the absence and presence of TGF-β-induction.

### Example 4: inhibition of lipopolysaccharide (LPS)-induced plasma TNF-a and IL-6 Concentrations Following Oral Dose SD-560 to Male Soraeue-Dawles Rats

LYT-100 and pirfenidone dosing solutions were prepared by dissolving each in a vehicle of 1% carboxymethyl cellulose and 0.2% Tween-80 in water. LPS was diluted with sterile saline to 2 mg/mL and sonicated at 40°C for 20 minutes to generate a stock solution and stored at 4°C. Each day of use, the stock solution was further diluted to 0.03 mg/mL in sterile saline.

Male Sprague-Dawley rats with indwelling femoral and jugular venous catheters (n=6-8 per dose and test article group) were used in this study. Rats were administered either vehicle (the dosing solution, 10 mL/kg), LYT-100 or pirfenidone at a concentration of 30, 100 or 300 mg/kg via syringe attached to an oral gavage needle. Sixty minutes after the oral dose. 0.03 mg/kg of LPS in 1 mL/kg saline was infused into the jugular vein.

Blood samples were collected from the femoral vein into heparin-coated syringes 15 minutes prior to the LPS infusion (45 minutes after oral doses of test article), 90 minutes after the LPS infusion and 4 hours after the LPS infusion. Plasma was prepared by centrifugation of the blood samples at 14,000 rpm for 10 minutes. Plasma samples were stored at -70 °C until analysis.

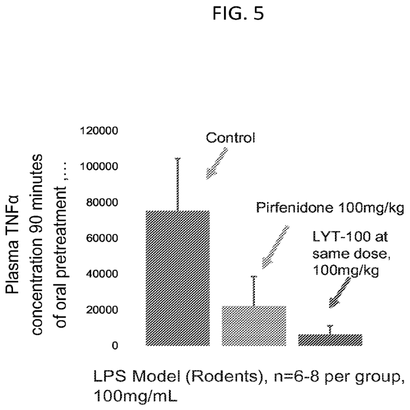

Ninety minutes after LPS injection, pretreatment with oral doses of 100 and 300 mg/kg LYT-100 (SD-560) inhibited TNFa and IL-6 **(Table 8, Table 9** and **FIG. 5****).**

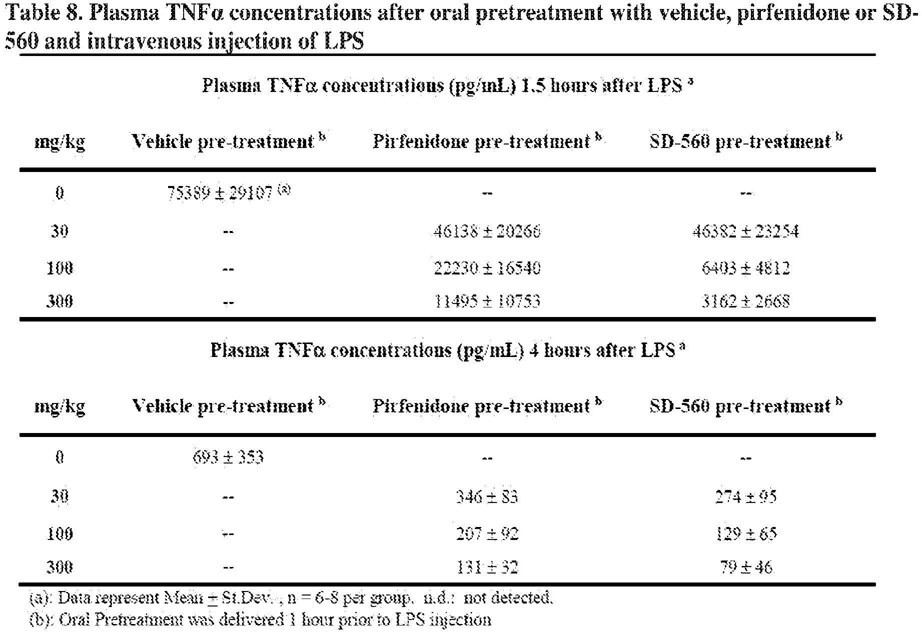

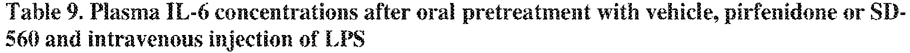

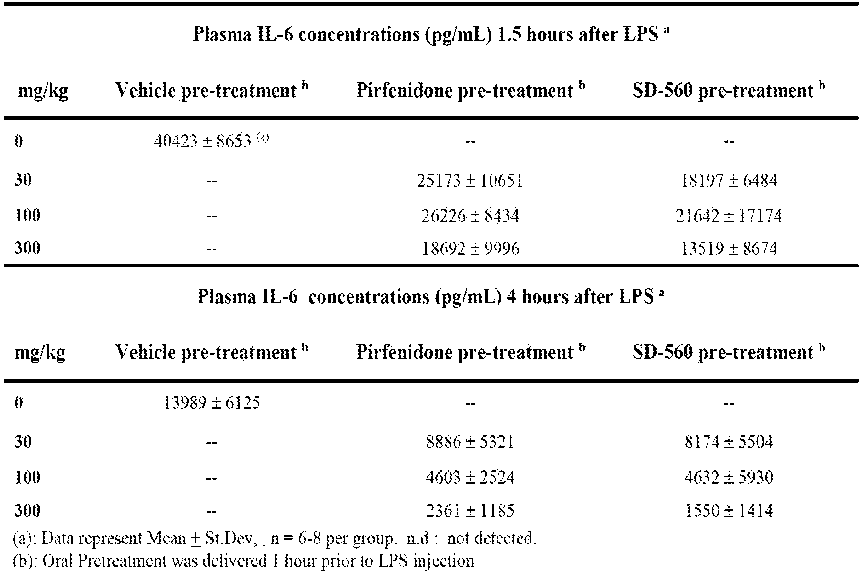

In other studies for pirfenidone mechanisms in acute and chronic interstitial lung diseases, LYT-100 downregulates ICAM-1, reduces vascular hyperpermeability, and suppresses TGF-β and downstream signaling.

### Examnle 5: Repeat dose oral toxicity and toxicokinetics studs of LYT-100 and airfenidone in Sprague Dawley rats followed by a 4-week recovery period

LYT-100 and pirfenidone were administered orally once daily for 91 consecutive days to Sprague Dawley rats to evaluate the potential reversibility of any findings following a 4-week recovery period. The profile of LYT-100, pirfenidone, and their metabolites were compared in order to understand the relationship between systemic exposure and their toxicity.

Male and female Sprague Dawley rats were separated in 6 different groups based on the 250, 500 and 750 mg/kg dose levels of LYT-100 and 750, and 875/1000 mg/kg (1000 mg/kg to male rats only on Day 1 only) dose levels of pirfenidone **(Table 10).**

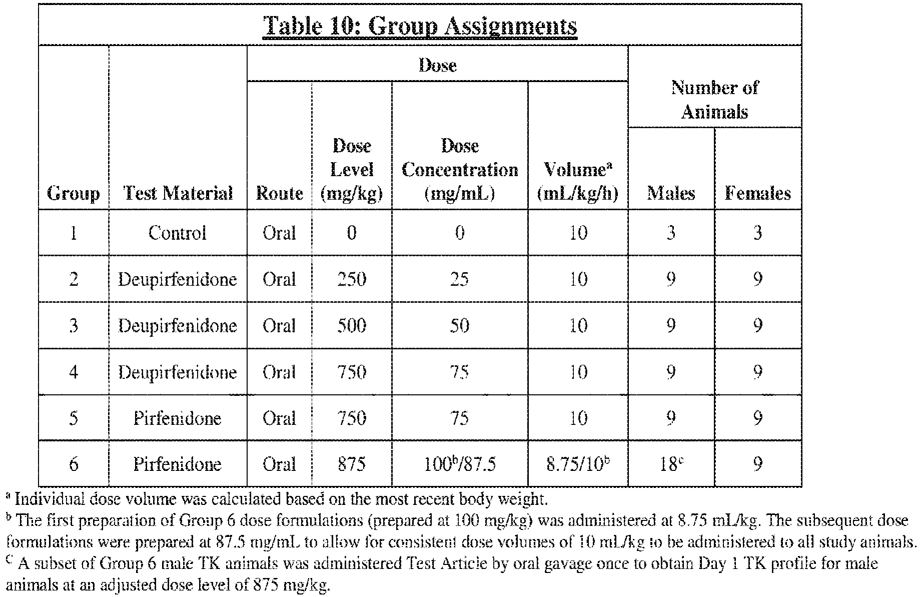

LYT-100 (deupirfenidone, SD-560) and SD-559 (pirfenidone) were administered via an oral gavage to fed male and female Sprague Dawley rats. Dose levels were administered once daily for 91 consecutive days with 250, 500, and 750 mg/kg (LYT-100) in Groups 2, 3, and 4, respectively and 750 and 875 mg/kg (SD-559) in Groups 5 and 6, respectively. The dose level of SD-559 for Group 6 was lowered from 1000 mg/kg to 875 mg/kg on Day 1 (Tox and TK females; Tox males) and Day 2 (TK males) due to test article-related clinical signs. The original male TK animals received 1000 mg/kg of SD-559 on Day 1 and continued on study. The protocol was amended to add additional TK male animals in Group 6 for Day 1 collection of TK samples at 875 mg/kg.

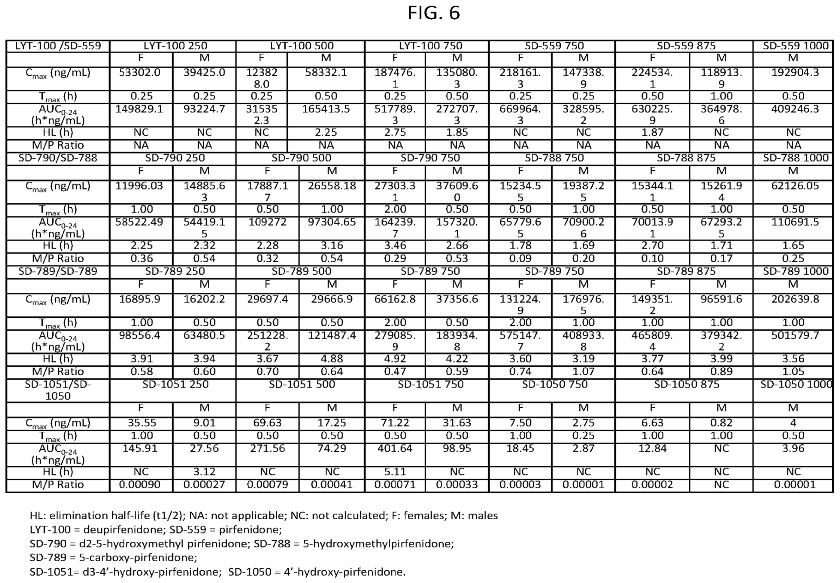

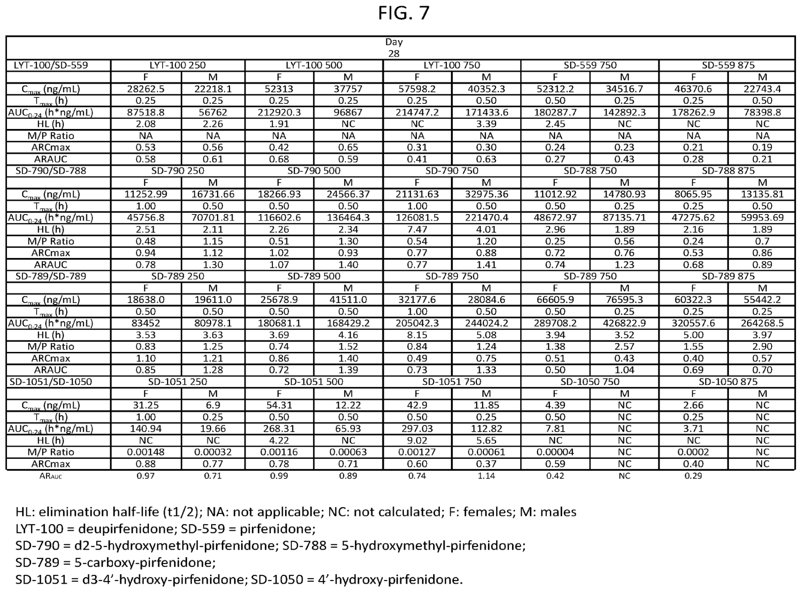

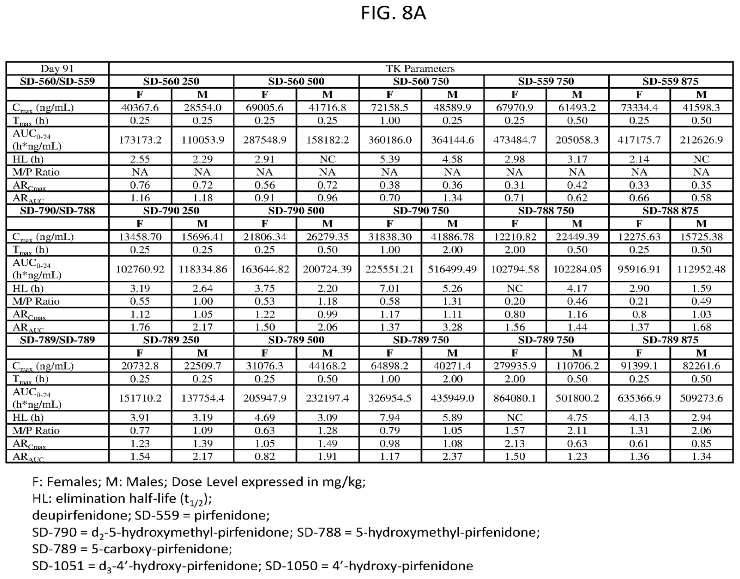

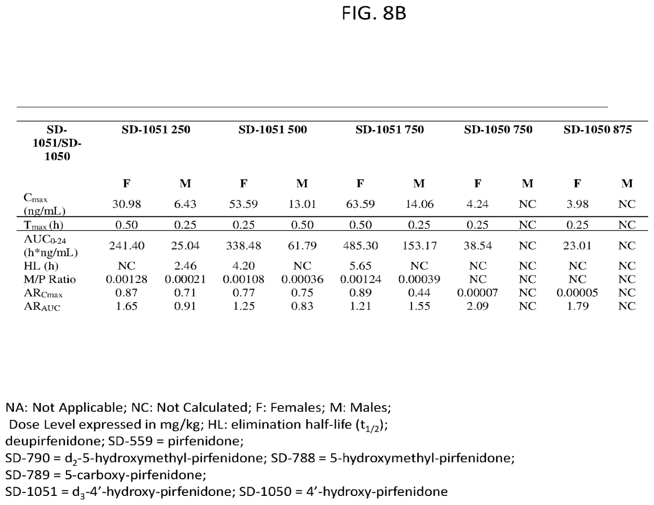

Blood samples were collected from 3 subsets of 3 animals/sex on Day 1 and Day 28 at pre-dose, 0.25, 0.5, 1, 2, 4. 8 and 24 hours (hr) post-dose for groups 2 to 6 and from one subset of 3 animals/sex at 0.25 hr post-dose for the vehicle group (group 1). Plasma was analyzed for LYT-100 and its metabolites d2-5-hydroxymethyl-pirfenidone (SD-790; active metabolite), d3-4'-hydroxy-pirfenidone (SD-1051) and 5-carboxy-pirfenidone (SD-789; inactive metabolite) (Groups 2 to 4) and for SD-559 and its metabolites 5-hydroxymethyl-pirfenidone (SD-788; active metabolite), 4'-hydroxy-pirfenidone (SD-1050) and 5-carboxy-pirfenidone (SD-789; inactive metabolite) (Groups 5 and 6) using validated LC-MS/MS bioanalytical methods. Analysis of samples from Group 1 for all analytes confirmed showed no exposure to any compound. Non-compartmental analysis (NCA) of plasma concentration data was conducted using Phoenix^{®} WinNonlin^{®}, version 8.0. A summary of the toxicokinetic (TK) parameters is presented in **FIGS. 6****,** **7****,** and **8A-B** for Days 1, 28 and 91, respectively. An assessment of similarity in exposure for parent and metabolites between Group 4 (LYT-100 at 750 mg/kg) and Group 5 (SD-559 at 750 mg/kg) is presented in **Table** 11 for Day 1 and Day 28.

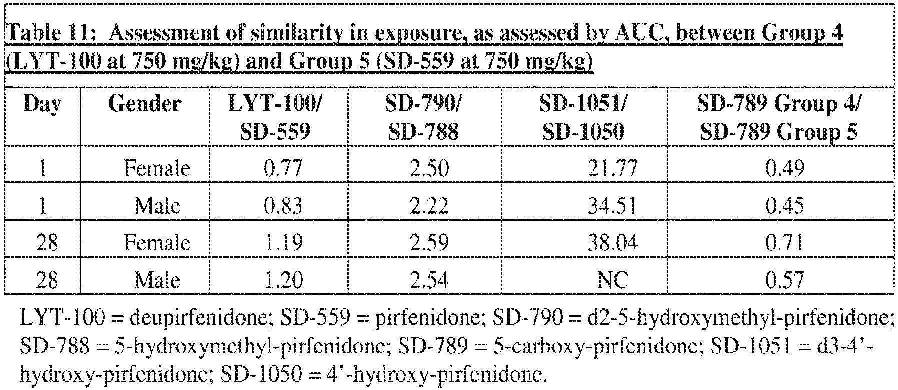

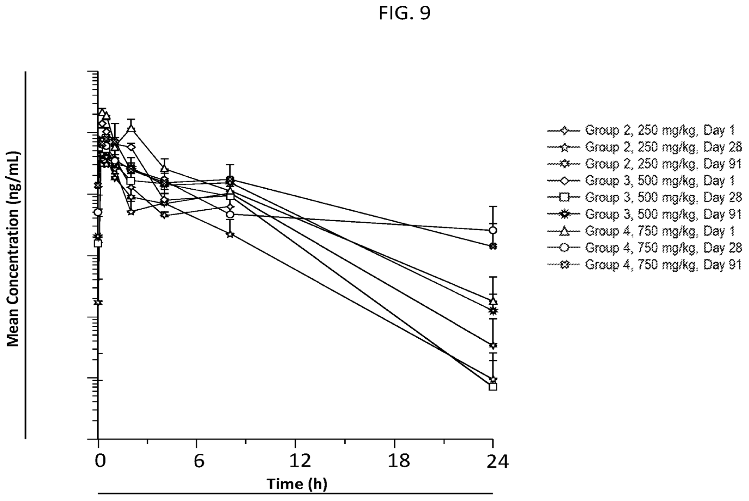

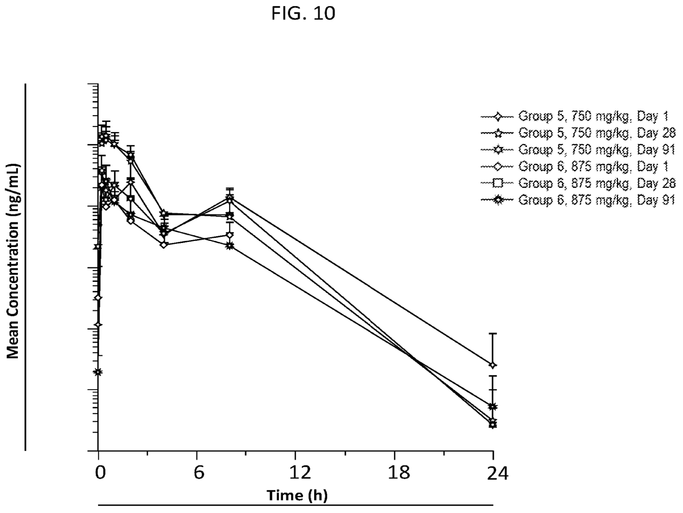

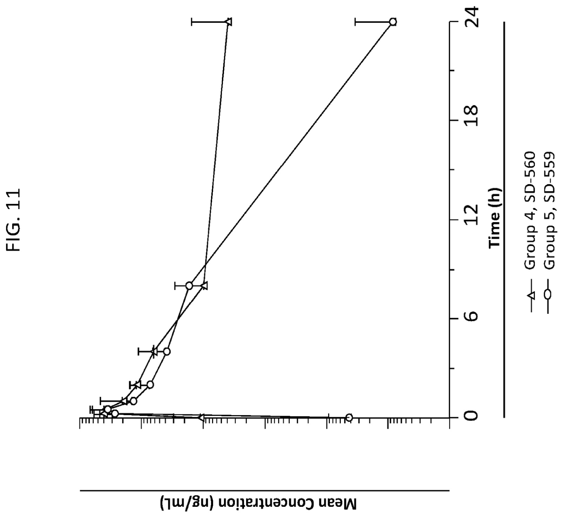

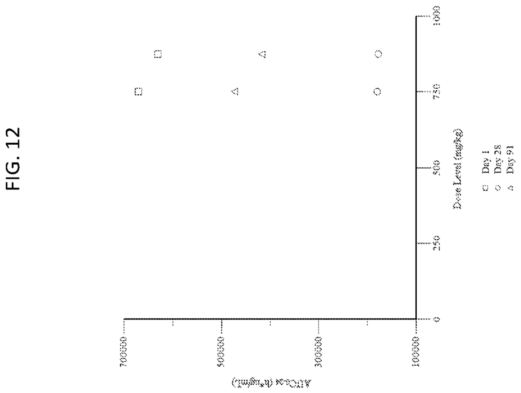

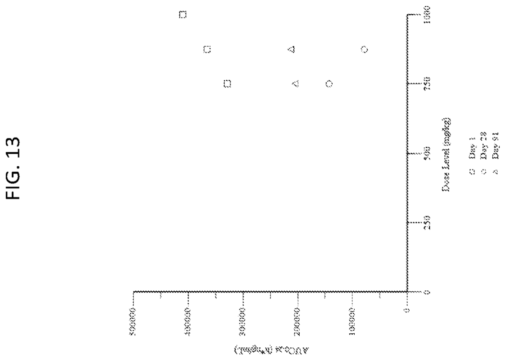

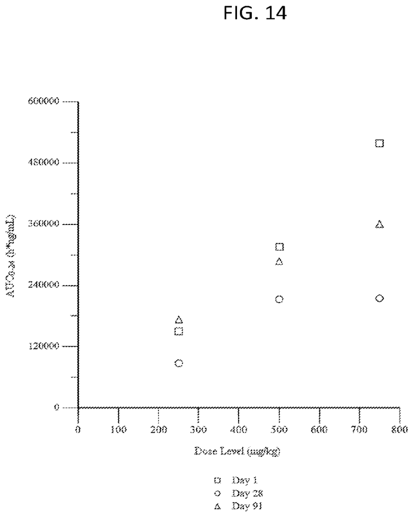

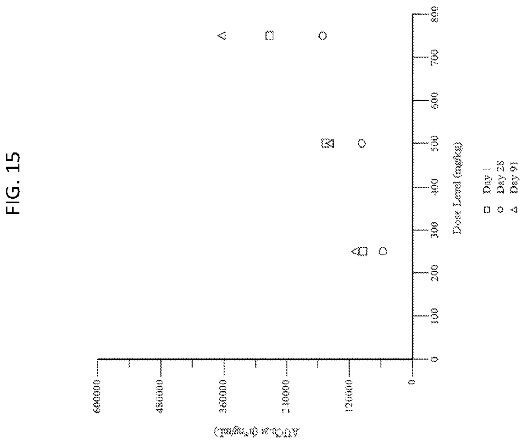

The increased exposure for LYT-100 relative to pirfenidone supports a less frequent dosing and/or lower dose than pirfenidone. Exemplary FIG. 9 and FIG. 10 depict the mean plasma concentration of LYT-100 over time following single and repeat oral administration of deupirfenidone and pirfenidone, respectively, in female Sprague Dawley rats on Days 1, 28 and 91. **FIG. 11** is a chart of mean plasma concentrations following single and repeat oral administration of 750 mg/kg LYT-100 and pirfenidone in female Sprague Dawley rats on day **28.** **FIG. 12** and FIG. **13** are charts depicting the AUC₀₋₂₄ dose relationship following single and repeat oral administration of deupirfenidone in of female and male Sprague Dawley rats, respectively. **FIG. 14** and **FIG. 15** are charts depicting the AUC₀₋₂₄ dose relationship following single and repeat oral administration of pirfenidone in of female and male Sprague Dawley rats, respectively.

In animals dosed with deupirfenidone (SD-560), females generally had higher Cₘₐₓ and AUC₀₋₂₄ values than males for analytes SD-560 and SD-1051 with differences being > 2-fold in some of the dose groups. No marked gender difference (≤ 2.0-fold) was observed for analytes SD-790 and SD-789 on all collection days, except Group 3 AUC₀₋₂₄ on Day 1 for SD-789 and AUC₀₋₂₄ in Group 4 on Day 91 for SD-790. Similarly, in animals dosed with pirfenidone (SD-559), females generally had higher Cₘₐₓ and AUC₀₋₂₄ values than males for analytes SD-559 and SD-1050 with differences being > 2-fold in some of the dose groups on all collection days except for the 750 and 875 mg/kg dose levels on Days 28 and 91due to the absence of TK parameters for males (BLQ values were observed over the complete sampling interval for all animals). No marked gender difference was observed for analytes SD-788 and SD-789 at the two dose levels on all collection days, except for SD-789 Cₘₐₓ in Group 5 on Day 91.

In animals dosed with pirfenidone (SD-559), females generally had higher Cmax and AUC₀₋₂₄ values than males for analytes SD-559 and SD-1050 with differences being > 2-fold in some of the dose groups on all collection days except for the 750 and 875 mg/kg dose levels on Days 28 and 91 due to the absence of TK parameters for males. No marked gender difference was observed for analytes SD-788 and SD-789 at the two dose levels on all collection days, except for SD-789 Cmax in Group 5 on Day 91.

Exposure of SD-560 in Groups 2 to 4, as assessed with Cmax and AUC₀₋₂₄. increased with dose where the increases were approximately less than dose proportional for Cmax and dose proportional for AUC₀₋₂₄ over the entire dose range of 250 to 750 mg/kg. Exposure of SD-790 increased with dose where the increases were, in general, dose proportional for Cmax and AUC₀₋₂₄ over the entire dose range of 250 to 750 mg/kg with the exception of Cmax in females and males on Day 28 (lower than dose proportional increase) and AUC₀₋₂₄ in males on Day 91 (higher than dose proportional increase). Exposure of SD-1051 in Groups 2 through 4, as assessed with Cmax. increased approximately in a less than dose proportional manner for Cmax over the entire dose range, except for males on Day 1. Exposure of SD-1051, as assessed with AUC₀₋₂₄, increased dose proportionally on Day 1 over the entire dose range. However, on Days 28 and 91, the increase in AUC₀₋₂₄ was less than dose proportional in females and greater than dose proportional in males. Exposure of SD-789 in Groups 2 through 4, as assessed with Cmax and AUC₀₋₂₄, increased with dose where the increases were generally approximately dose proportional for Cmax (with some variability) and were dose proportional for AUC₀₋₂₄ over the entire dose range.

Exposure of SD-559, and metabolites SD-788, SD-789 and SD-1050 in Groups 5 and 6, as assessed with Cmax and AUC₀₋₂₄ values, generally did not increase with increasing dose from 750 to 875 mg/kg and similar exposure for SD-559 and the different metabolites was observed between the two groups. The difference between dose levels is 14%, which may be masked by inter-individual variability of plasma concentrations. However, Cmax was lower for the 875 mg/kg dose level when compared to 750 mg/kg in females on Day 91 for SD-789 and in males on Day 1 for SD-1050.

No accumulation was observed for SD-560, SD-559 or the different metabolites at the different dose levels after multiple dosing for 28 Days. Exposure to parent SD-560 or SD-559 was lower on Day 28 compared to Day 1 with accumulation ratios between 0.68 and 0.21. Lower accumulation was observed for the high dose of SD-560 and the lowest was for SD-559. Following 91 days of dosing, exposure to parent SD-560 and SD-560 was higher than on Day 28 and lower or equal to exposure on Day 1 with the lowest accumulation ratio for SD-559 (0.58-0.71). After 91 days of dosing, little to moderate accumulation was observed for SD-790 and SD-788 based AUC₀₋₂₄, for SD-789 based on AUC₀₋₂₄ following SD-560 administration and for SD-789 based on Cₘₐₓ following SD-559 administration, and SD-1050 based on AUC₀₋₂₄.

It is interesting to note that, while on Day 1 exposure to LYT-100 (Cmax or AUC₀₋₂₄) was slightly lower (0.77 and 0.83 fold for females and males, respectively) than exposure of SD-559 at the same nominal dose (750 mg/kg); on Day 28 this is reversed (1.19 and 1.20 fold for females and males, respectively). This may be attributed to a slowing down of the metabolism of LYT-100 versus SD-559 because of deuterium incorporation. The stabilization imparted by deuterium substitution would manifest in a slower metabolism and therefore a higher exposure to the parent compound.

Assessment of similarity in exposure (AUC₀₋₂₄) and Cmax between SD-560 at 750 mg/kg and SD-559 at 750 mg/kg, suggested that SD-560 showed comparable Cmax and exposure to SD-559 on Days 1, 28 and 91 in both sexes with the exception of AUC₀₋₂₄, on day 91 for the 750 mg/kg dose. Metabolite SD-790 generated from SD-560 at 750 mg/kg showed almost twice the exposure and Cmax on all days and in both genders vs. the non-deuterated corresponding metabolite (SD-788) generated from SD-559 at 750 mg/kg. Metabolite SD-1051, deuterated analog of SD-1050, showed significantly higher levels and exposure than metabolite SD-1050 on all days and in both genders at 750 mg/kg of SD-560 versus 750 mg/kg of SD-559. However, SD-560 sequential metabolite, SD-789, formed from SD-790 showed approximately 50% lower Cmax and exposure than SD-789, sequential metabolite of SD-559. Increased exposure to the 5-hydroxy metabolite, SD-790 (deuterated) or SD-788 (non-deuterated), and decreased exposure to the 5-carboxy metabolite, SD-789, in animals dosed with deupirfenidone versus pirfenidone can be ascribed to deuterium stabilization against metabolism of deupirfenidone vs. pirfenidone. By slowing down the conversion of d2-5-hydroxymethyl pirfenidone (SD-790) to 5-carboxy pirfenidone (SD-789), deuterium in deupirfenidone would decrease Cmax and exposure to 5-carboxy pirfenidone while leading to accumulation of d2-5-hydroxy pirfenidone. Deuterium however does not appear to significantly slow down metabolism of deupirfenidone to d2-5-hydroxy pirfenidone in rat as exposure to deupirfenidone is similar to that of pirfenidone at the same dose level.

Similar trends in exposure and Cmax of SD-560 across all dose levels (250, 500 and 750 mg/kg), after adjusting for the different doses, were observed, when compared to SD-559 at 875 mg/kg or 1000 mg/kg (male rats on Day 1).

This data also provides relevant information to human dosing. Day 1 TK data, compared to the single dose human PK data, show that the highest human exposure to parent and metabolites is covered in the present toxicity study. Exposure to LYT-100 in the TK study, as assessed by AUC, is about 5-fold larger at 750 mg/kg, on Day 1 and in male rats, than its exposure in human at the high dose of 801 mg. Exposure to deuterated 5-hydroxymethyl pirfenidone (SD-790; active metabolite) is about 1250-fold higher in the rat and exposure to 5-carboxy-pirfenidone (SD-789; inactive metabolite) in the rat is about 8-fold higher than in human, using the TK data on Day 1 in male rats for the comparison since there is a gender effect and because the exposure in male is lower than in female. Finally, the deuterated 4'-hydroxy pirfenidone metabolite (SD-1051) was not quantifiable in human. A similar conclusion can be reached when comparing Day 1 Cmax between male rat and human, where Cmax of LYT-100, deuterated 5-hydroxymethyl-pirfenidone (SD-790; active metabolite), and 5-carboxy-pirfenidone (SD-789; inactive metabolite) are about 15-, 1825- and 9-fold, respectively, larger in rat than in human. When using data collected in male rat after 28 days of daily dosing, the conclusion is still applicable as exposures to LYT-100, deuterated 5-hydroxymethyl-pirfenidone (SD-790; active metabolite), and 5-carboxy-pirfenidone (SD-789; inactive metabolite) are about 5-, 1600-, and 7-fold, respectively, larger in rats than in humans.

### Example 6: LYT-100 Increases Systemic Exposure in Humans

LYT-100 was studied in a single dose, double-blinded, cross-over clinical trial of 24 healthy volunteers to assess safety and pharmacokinetics (PK). Following screening, eligible healthy volunteer subjects were admitted to a single clinical study site and were randomized to 1 of 2 treatment sequences. Subjects received a single 801 mg oral dose of either LYT-100 or pirfenidone in Period 1 and, following washout, crossed over to receive the other treatment in period 2. In each period, a standardized breakfast was provided to subjects prior to administration of study drug (to compare the PK profiles in the clinically relevant fed state) and plasma samples were collected over a 48-hour period after dosing for evaluation of PK. Dosing between the 2 periods was separated by a minimum of 7 days. Subjects completed the study upon completion of the 48-hour post-dose assessments following dosing period 2.

To avoid confounding the analysis of results with any influence of formulations, both study drugs (LYT-100 and pirfenidone) were synthesized using the same manufacturing process and were provided as unformulated powder in capsules: LYT-100 801 mg (267 mg capsules x 3); and pirfenidone 801 mg (267 mg capsules x 3).

All capsules were identical in size, shape, and external color. Both the LYT-100 and the pirfenidone used in this trial were provided in hard-shell gelatin capsules containing 267 mg of either LYT-100 or pirfenidone powder with no excipients. The order of the two treatments was assigned via a randomization schema in a 1:1 ratio such that half of the subjects received LYT-100 first and the other half received pirfenidone first.

The plasma concentrations of LYT-100, pirfenidone, and their respective associated metabolites (e.g., 5-carboxy-pirfenidone, 5-hydroxymethyl-pirfenidone, and 4'-hydroxy-pirfenidone) and sample collection times were used for calculation of the following pharmacokinetic parameters for each subject and treatment:

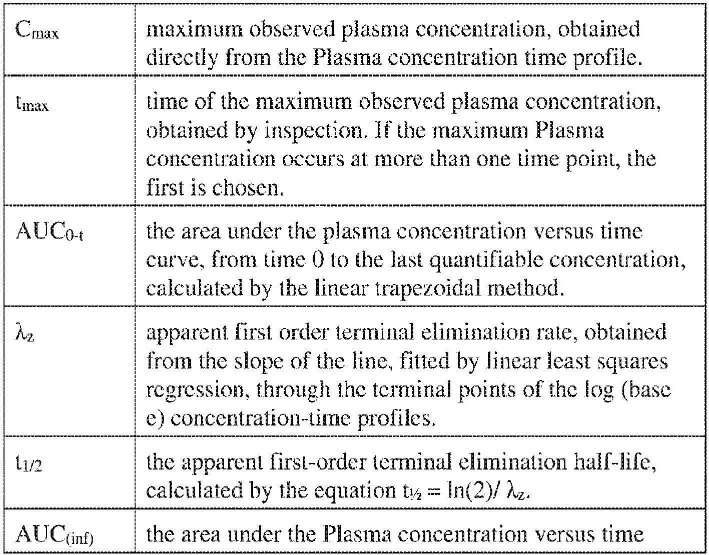

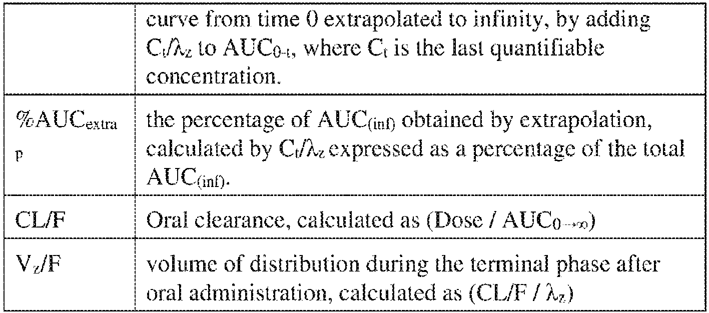

**Tables 12-14** summarize the pharmacokinetics over 24 hours of the active LYT-100 (deupirfenidone) and control pirfenidone (LYT-101), their partially active metabolites, deuterated 5-hydroxymethyl-pirfenidone (LYT-110) and non-deuterated 5-hydroxymethyl-pirfenidone (LYT-111), respectively; and common metabolite, non-deuterated 5-carboxy-pirfenidone (LYT-105).

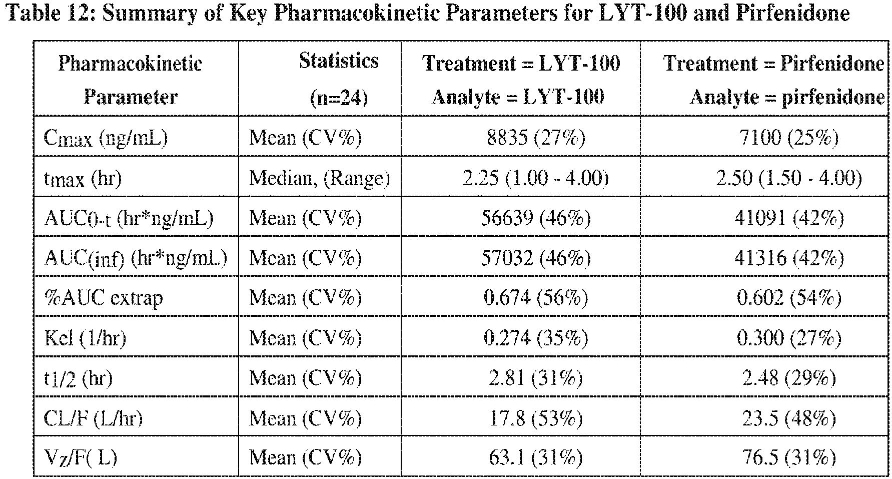

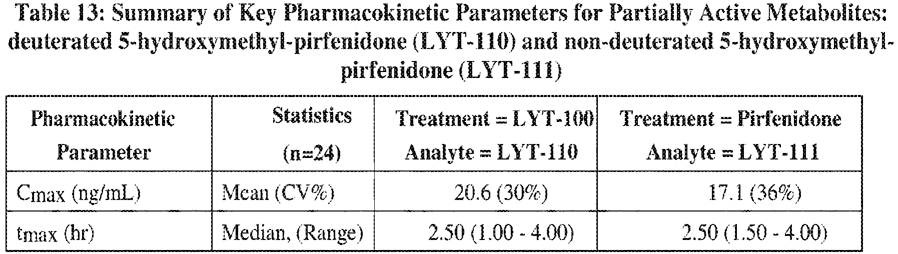

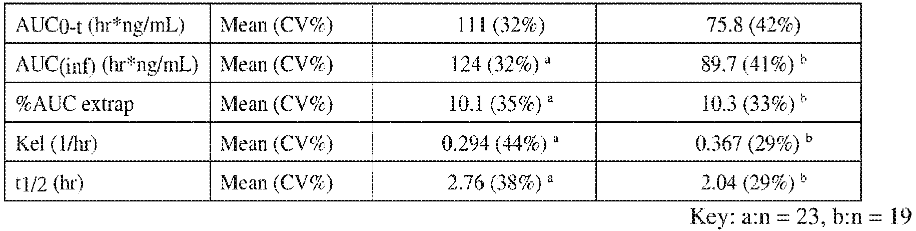

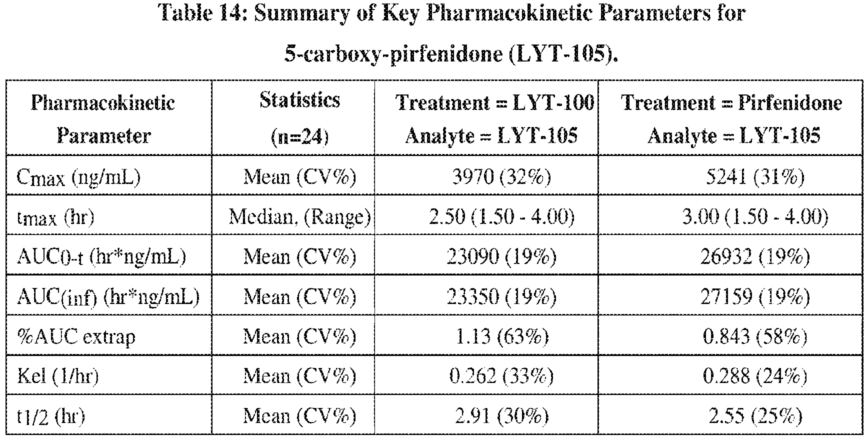

It was observed that the systemic exposure of LYT-100 was about 35% greater than for pirfenidone, and about 25% greater for Cₘₐₓ, with no appreciable difference in the apparent elimination half-life.

The increased systemic exposure to LYT-100 was accompanied by changes in the relative abundance of downstream metabolites. Following LYT-100 and pirfenidone, the most abundant measured circulating metabolite was 5-carboxy-pirfenidone (LYT-105). 5-carboxy-pirfenidone was reduced after LYT-100 relative to pirfenidone by approximately 15% and 25% for AUC and Cₘₐₓ, respectively. As a percent of the parent analyte AUC_{0-∞}, 5-carboxy-pirfenidone represented 43.8% for LYT-100 as compared to 65.9% for pirfenidone **(Table 15).** The remaining measured metabolites, 5-hydroxymethyl-pirfenidone (LYT-111) and 4'-hydroxy-pirfenidone (LYT-104), were far less abundant, representing less than 2% of parent in terms of AUC. The formation of the metabolite 5-hydroxymethyl-pirfenidone was approximately 50% greater in terms of overall systemic exposure (AUC) after administration of LYT-100. Similarly, 4'-hydroxy-pirfenidone was detectable more frequently after LYT-100 than after pirfenidone. Given the low plasma concentrations of these metabolites, however, these changes contributed little to the overall pharmacokinetic profile of LYT-100 relative to pirfenidone.

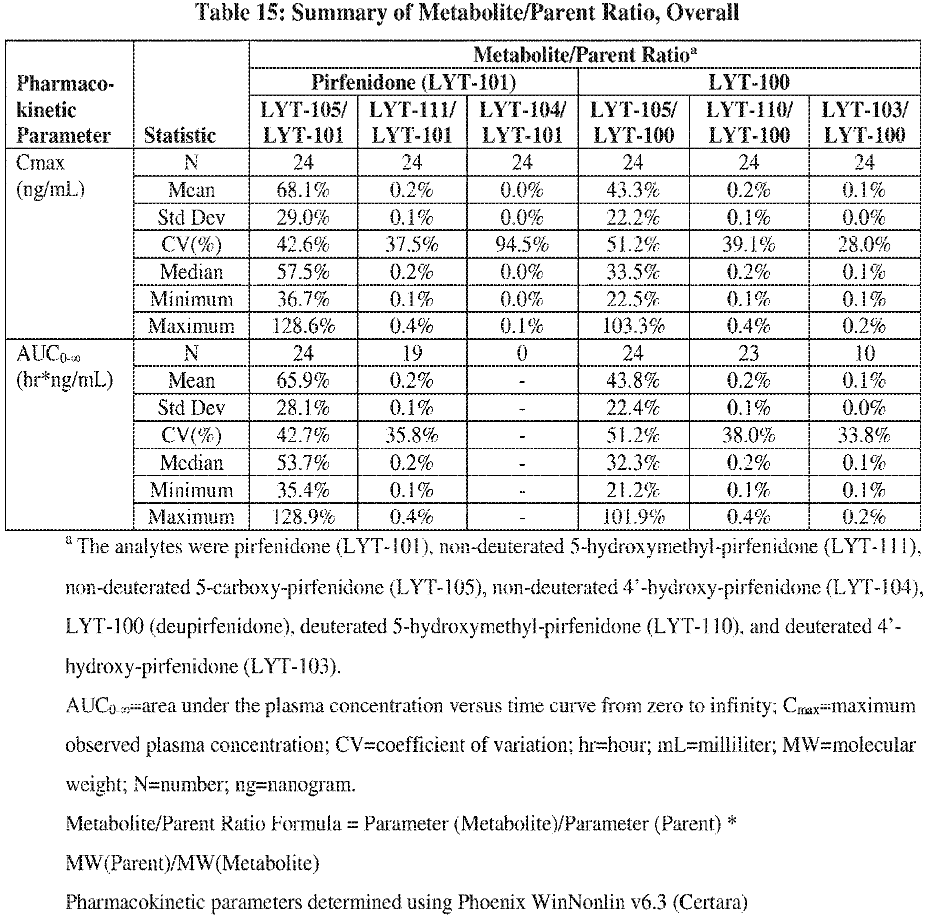

On average, after administration of LYT-100, the 5-carboxypirfenidone metabolite (LYT-105) represented 43.8% of the parent in comparison to 65.9% of the parent after administration of pirfenidone. This difference in exposure was not associated with a change in half-life, suggesting formation, and not clearance of this non-deuterated metabolite is affected by the deuterium substitution in the parent molecule.

Administration in the fed state of a single 801 mg dose of LYT-100 resulted in overall greater exposure (AUC, Cₘₐₓ) than observed with administration of an 801 mg dose of pirfenidone. No appreciable difference in the apparent elimination t_{½} or time to Cₘₐx was observed for the 2 compounds. The higher peak and overall exposure of LYT-100 was associated with a lower systemic exposure of the 5-carboxy-pirfenidone, suggesting the kinetic isotope effect at least partially protects against pre-systemic conversion of pirfenidone into 5-carboxy-pirfenidone.

The deuterium kinetic isotope effect appears independent of phenotype when comparing exposure between deuterated and non-deuterated pirfenidone. CYP1A2 has been reported as the main metabolizing enzyme for pirfenidone and higher enzyme activity in the hyperinduced CYP1A2 phenotype is associated with lower exposure of both deuterated and non-deuterated forms of pirfenidone relative to normal expression levels.

Overall, single doses of LYT-100 and pirfenidone were well tolerated and have a comparable safety profile. No clinically significant differences were observed between the 2 treatments in terms of type, severity, or frequency of treatment emergent adverse events. The most common adverse event following either treatment was headache. Of interest, although administration of the 801 mg dose of LYT-100 resulted in greater drug exposure than with the same pirfenidone dose, the incidence of gastrointestinal and nervous system adverse events was not increased with LYT-100 administration as compared to pirfenidone. No significant changes in laboratory parameters, vital signs, or ECGs were observed following either treatment.

### Example 7: LYT-100 Dosing and Food Effect Study

This study is a Phase 1 Multiple Ascending Dose and Food Effect Study in healthy volunteers to determine the pharmacokinetics and maximally tolerated dose of deupirfenidone (LYT-100) followed by a randomized double-blind placebo-controlled phase 1B in patients with breast cancer-related upper limb secondary lymphedema.

The Multiple ascending dose (MAD) and Food Effect (Parts 1 and 2) will be performed at a single Study Centre in Australia. Part 3 will take place at up to 5 study centres in Australia.

### Study Objectives:

This study has 3 Parts, with each Part having specific objectives. Part 1 will assess safety and tolerability in a multiple dose-escalation design, Part 2 is a food effect assessment, and Part 3 is a placebo-controlled assessment of safety and efficacy signals in the target population.

### Parts 1 and 2: Healthy Volunteers

### Primary objectives

Part 1: To evaluate safety and tolerability of multiple twice-daily (BID) doses of LYT-100 administered over 5 days.

Part 1: To determine time to steady state (up to Day 5) and to characterise the steady-state PK profile of LYT-100.

Part 1: To determine the maximum tolerated dose (MTD) for multiple BID doses of LYT-100 administered over 5 days.

### Secondary objectives

Part 1: To assess the pharmacokinetic (PK) profiles of multiple BID doses of LYT-100 administered over 5 days for dose proportionality.

Part 2: To descriptively compare the PK profile and compare the relative bioavailability of a single dose of LYT 100 administered at a dose below the MTD without food, to the equivalent dose with food.

### Part 3: Breast Carcinoma Patients with Secondary Lymphedema Following Axillary Node Dissection and/or Sentinel Lymph Node Biopsy

Part 3 is a 26-week randomized, double-blind, placebo-controlled assessment of LYT-100 at multiple study centres. Part 3 will be performed in breast carcinoma patients with secondary mild to moderate lymphedema following axillary node dissection and/or sentinel lymph node biopsy, with or without radiation, dosed with LYT-100 (dose to be determined from Part 1 outcomes) BID (with food) for 6 months.

### Part 1: Treatment Period

This is a randomised, double-blind, placebo-controlled, multiple ascending dose design to assess the safety, tolerability and PK profile of multiple doses of LYT-100 administered under fed conditions at steady state in healthy participants. Up to 5 dosing cohorts are planned in Part 1. Planned dose levels are as follows in **Table 16.**

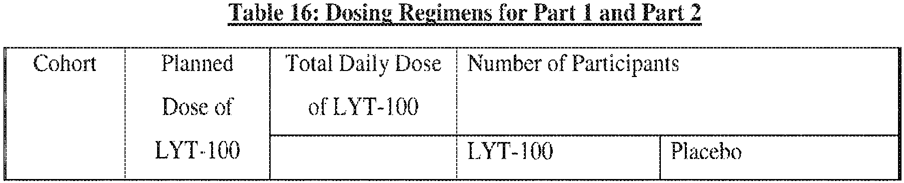

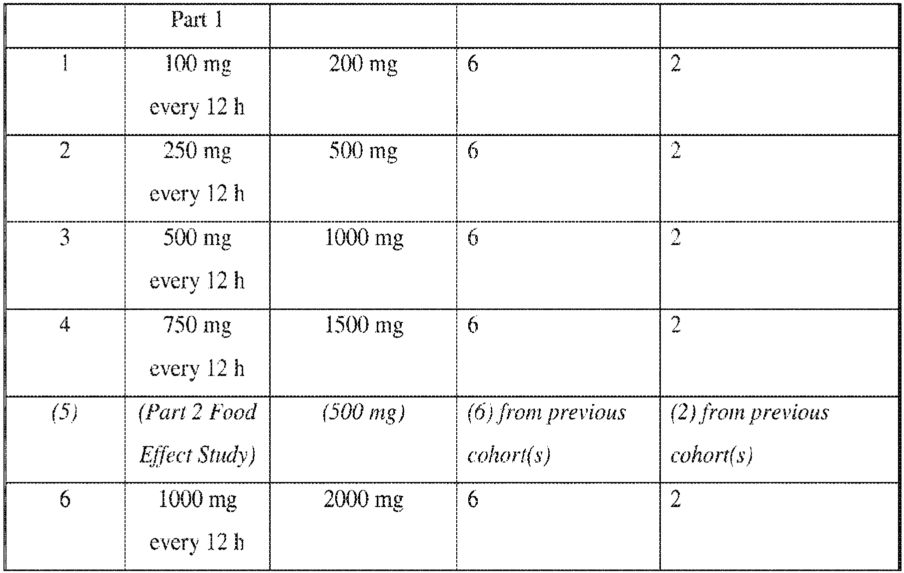

All Part 1 cohorts will be dosed every 12 hours with food for 5 days. Additional cohorts and intermediate doses may be selected in lieu of predefined doses as noted and in accordance with safety and tolerability responses, but doses will not exceed 1000 mg BID or a total daily dose of 2000 mg.

In cohort 6, three sentinel subjects (2 active and 1 placebo) will enrol and dose ≥48 hours in advance of the remaining 5 subjects (4 active and 1 placebo). If clinically significant safety signals assessed as > Mild/Grade 1 are observed in the 3 sentinel subjects in advance of dosing the remaining 5 subjects, the Safety Review Committee may meet to review safety data before the remaining 5 subjects are enrolled.

Up to 40 participants will be enrolled in Part 1 (n=6 LYT-100 and n=2 placebo in each cohort) unless additional intermediate cohorts are needed. Participants will be admitted to the Clinical Research Unit (CRU) on Day -1 and will be discharged on Day 7 in the absence of clinically significant safety signals, following completion of all Day 7 assessments and at the Investigator's discretion.

During the treatment period (Day 1 through Day 5), participants will be administered their assigned study medication BID, every 12 h ± 0.25 h (with approximately 240 mL of non-carbonated water), 30 minutes after the start of consumption of their standardised breakfast or dinner (12 h apart). A standardised lunch will be served ≥ 4 h post breakfast and ≥ 4 h prior to dinner. An evening snack will be served ≥ 3 h following evening study medication administration. No additional fluids will be allowed during the 1 h pre- and post-doses. On Day 6, subjects will replicate mealtimes as scheduled on Day 1-5. To ensure study drug dosing every 12 hours, here is an example of meal and dosing schedule in Part 1:

Breakfast: meal to be served 30 mins prior to AM dosing. Breakfast must be completed within 30 mins of start time.

Lunch: meal to be served at least 4 h post-AM study drug dose.

Dinner: meal to be served at least 11.5 h post-AM dose and served 30 minutes prior to PM study drug dose.

Evening snack: Snack to he served at least 15 h post-AM dose (at least 3 h post-PM dose).

Participants will return to the study centre for a follow-up visit 7 days after their final dose of study drug. For all cohorts in Part 1, the decision to escalate or modify the dose prior to dosing of the next Cohort will be determined by a Safety Review Committee (SRC).

### Part 2: Treatment Period

Eight (8) subjects completing Part 1 will participate in Part 2 (n=6 LYT-100 and n=2 placebo). An unblinded statistician will ensure that subjects receiving active treatment or placebo in Part 1 will maintain the same treatment allocation in Part 2, though the dose of active study treatment and number of matching placebo capsules may differ.

A single dose of 500 mg of LYT-100 or placebo will be administered on two days, separated by a minimum 7-day washout period. Four (4) subjects (3 active and 1 placebo) will be randomized to receive their single dose of Part 2 study treatment under fed conditions, while 4 subjects (3 active and 1 placebo) will be randomized to receive their single dose of Part 2 study treatment under fasted conditions.

Participants will return in Part 2 to the CRU following a minimum 7-day washout period to receive a single dose of 500 mg LYT-100 or placebo after crossing-over to the alternate fasted or fed single dose study treatment administration condition. Participants will be administered a single dose of their assigned treatment under fasting conditions (Cohort 5) in order to permit a comparison of the rate and extent of absorption of LYT-100 when given the equivalent dose under fed conditions (Cohort 5). The comparison will be based on Day 1, Day 2 and Day 3 PK plasma samples after the first dose of study drug on Day 1 under fast and fed conditions of Part 2.

Rescreening of participants in Part 2 prior to the first dose of study drug will be conducted according to the Schedule of Events for Part 2 to ensure that the participant continues to meet study eligibility criteria. A total of eight (8) participants will participate in Part 2 (n=6 LYT-100 and n=2 placebo).

Participants in Part 2 will receive the same treatment allocation of active or placebo while they were participating in Part 1, though the dose of active study treatment and number of matching placebo capsules may differ. Subjects will be randomized to one of two meal sequences as follows:
- Fasted-Fed in first dosing period of Part 1 and second dosing period of Part 2, respectively
- Fed-Fasted in first dosing period of Part 1 and second dosing period of Part 2, respectively

Subjects will be admitted to the CRU on Day -1 and will fast overnight for at least 10 h. On Day 1, a single dose of study drug, i.e., 500 mg LYT-100 or placebo will be administered with approximately 240 mL of non-carbonated water while either fasted or fed per randomization sequence.

On ***Fasted Days,*** meals will be provided as follows:
On Day 1, breakfast will be provided ≥ 4 h post-study drug administration. A standardised lunch will be served ≥ 4 h following breakfast, and dinner will be served ≥ 4 h following lunch. An evening snack will be served≥ 3 h following dinner. No additional fluids will be allowed during the 1 h pre- and post-dose. On Day 2, subjects will replicate mealtimes as scheduled on Day 1.

Mealtimes on Day 1 in relation to dosing in Part 2 are as follows:
- Breakfast: meal to be served 4 h post-AM study drug dose.
- Lunch: meal to be served at least 4 h following breakfast.
- Dinner: meal to be served at least 8 h following breakfast.
- Evening snack: snack to be served at least 11 h following breakfast.

On ***Fed Days,*** meals will be provided as follows:
On Day 1, a standardised breakfast will be provided 30 minutes prior to study drug administration. A standardised lunch will be served ≥ 4 h post breakfast and ≥ 4 h prior to dinner. An evening snack will be served ≥ 15 h following morning study medication administration. Fluids are restricted only during the 1 h pre- and post- morning dose. On Day 2, subjects will replicate mealtimes as scheduled on Day 1.

Mealtimes on Day 1 in relation to dosing in Part 2 are as follows:
- Breakfast: meal to be served 30 mins prior to AM dosing. Breakfast must be completed within 30 mins of start time.
- Lunch: meal to be served at least 4 h post-AM study drug dose.
- Dinner: meal to be served at least 11.5 h post-AM dose and served 30 minutes prior to PM study drug dose.
- Evening snack: Snack to be served at least 15 h post-AM dose

Participants will remain in the CRU until completion of the 48 h post-dose assessments following the single dose administration with fed or fasted as outlined in the Schedule of Events and in the absence of clinically significant safety signals, following completion of all Day 3 assessments and at the Investigator's discretion. Subjects will return after a ≥ 7-day washout period to participate in the alternate randomized meal sequence. They will return on Day 10 for their final study day visit.

### Part 1 and Part 2 Safety Monitoring and Follow Up

All participants in Part 1 and Part 2 will be monitored for safety (including assessment of chemistry, haematology and urinalysis parameters, electrocardiograms [ECOs], vital signs and adverse events [AEs]) and samples will be collected for assessment of PK at predefined time points pre and post-dose as delineated in the Schedule of Events.

All participants in Part 1 will be followed for at least 30 days and Part 2 will be followed for at least 10 days after the last administered dose of study drug.

In Part 1, participants will attend the CRU on an outpatient basis 7 days (± 1 day) following the last administered dose for safety assessments and a final safety follow up teleconference between site staff and the study participant will occur 30 days (± 3 days) after the last administered dose, at the discretion of the Investigator. If required, following the teleconference, an onsite visit to the CRU will be scheduled, at the Investigator's discretion.

In the case of premature discontinuation from the study, participants will return to the CRU and complete an early termination visit with assessments as delineated in the Schedule of Events. Following the early termination visit, participants will be contacted by telephone by site staff 30 days (± 3 days) in Part 1 and 10 days (± 3 days) in Part 2, post the last administered dose of study drug for a final safety review, at the Investigator's discretion.

### Safety Oversight for Part 1 and Part 2:

The study will be subject to oversight by a SRC comprised of the Principal Investigator (PI), medical monitor (MM), and Sponsor representative, at a minimum. Details of the roles and functioning of the SRC will be available in the SRC Charter.

### Part 1

The SRC will provide recommendations on the dose of LYT-100 for the next Cohort. The raw data will remain blinded. In the event that unblinding of an individual participant is required, every effort will be made to not compromise the overall blinded status of the study.

If an intolerable dose of LYT-100 is identified, the dose will not be further escalated and the previously tolerated dose will be considered the MTD or alternatively, an intermediate dose, lower than the intolerable dose, may be explored.

Escalation from one dose level to the next will occur after review of raw clinical safety data and approval by the SRC up to and including the onsite follow-up visit on Day 12 (7 days post last administration of study drug) for the last participant in the preceding cohort; cumulative data for earlier cohorts may also be reviewed.

As noted previously, in Cohort 6, three sentinel subjects (2 active and 1 placebo) will enroll and dose ≥48 hours in advance of the remaining 5 subjects (4 active and 1 placebo). If clinically significant safety signals assessed as > Mild/Grade 1 are observed in the 3 sentinel subjects in advance of dosing the remaining 5 subjects, the Safety Review Committee may meet to review safety data before the remaining 5 subjects are enrolled.

### Part 2

A single dose of 500 mg of LYT-100 or placebo will be administered on two days, separated by a minimum 7-day washout period.

### Stopping Rules for Part 1 and Part 2:

At any phase of the study, administration of study drug will be paused and participants will not receive further study drug until data review, recommendations and approval have been provided by the SRC.

Dose-limiting toxicity will be defined as 2 or more clinically significant AEs or abnormal laboratory values assessed as unrelated to intercurrent illness, or concomitant medications, which are determined by the Investigator to be related to the study drug and meet any of the following criteria:
- Two or more Grade 3 toxicities that are considered possibly or probably related to study drug.
- Any participant experiences a serious adverse event (SAE) that is considered possibly or probably related to study drug, or
- An AE or group of AEs that singularly or in aggregate suggests to the Investigator, Sponsor or Medical Monitor that the study drug is possibly or probably related, and it is poorly tolerated and further treatment per protocol may not be safe.

With observation of apparent dose-limiting toxicity, review by the SRC will take place as soon as possible to evaluate the events and determine next steps.

### Number of Participants (Planned):

Parts 1 and 2: Up to 40 healthy female and male adult participants (3:1 ratio), unless additional intermediate cohorts are needed.

Part 3: Up to 50 patients with breast cancer-related upper-limb unilateral secondary lymphedema (1:1 ratio).

### Main Criteria for Inclusion and Exclusion

### Part 1 (MAD) and Part 2 (single-dose fasting: Healthy Volunteers)

### Inclusion Criteria:

1. Male or female between 18 and 75 years old (inclusive) at the time of screening.
2. In good general health at screening, free from clinically significant unstable medical, surgical or psychiatric illness, at the discretion of the Investigator.
3. Participants have a body mass index (BMI) between ≥ 18.0 and ≤ 35.0 kg/m2 at screening.
4. Vital signs (measured in supine position after 5 minutes' rest) at screening:
5. Systolic blood pressure ≥90 and ≤140 mmHg;
6. Diastolic blood pressure ≥40 and ≤ 90 mmHg;
7. Heart rate ≥40 and ≤100 bpm;
8. Temperature ≥35.5°C and ≤ 37.5°C;
9. Vital signs may be repeated once, within a minimum of 10 minutes of the completion of the last set of vital signs (while maintaining supine position until the repeated set of vital signs are collected), if it is suspected that falsely high or low levels have been obtained.
10. No relevant dietary restrictions, and willing to consume standard meals provided and willing to avoid soy products while participating in the trial.
11. Willing to comply with all study procedures and requirements, including not driving or operating machinery for 12 h following study drug administration.
12. Willing to abstain from direct sun exposure from 2 days prior to dosing and until final study procedures have been conducted.

### Part 1 (MAD) and Part 2 (single-dose fasting): Healthy Volunteers

### Exclusion Criteria:

1. History or presence of malignancy at screening or baseline, with the exception of adequately treated localised skin cancer (basal cell or squamous cell carcinoma) or carcinoma in-situ of the cervix.
2. Clinically significant infection within 28 days of the start of dosing, or infections requiring parenteral antibiotics within the 6 months prior to screening.
3. Clinically significant surgical procedure within 3 months of screening, at the discretion of the Investigator.
4. Currently suffering from clinically significant systemic allergic disease at screening or baseline or has a history of significant drug allergies including a history of anaphylactic reaction (particularly reactions to general anaesthetic agents); allergic reaction due to any drug which led to significant morbidity; prior allergic reaction to pirfenidone.
5. Chronic administration (defined as more than 14 consecutive days) of immunosuppressants or other immune-modifying drugs within 3 months prior to study drug administration; corticosteroids are permitted at the discretion of the Investigator).
6. History or presence at screening or baseline of a condition associated with significant immunosuppression.
7. Positive test for hepatitis C antibody (HCV), hepatitis B surface antigen (HBsAg), or human immunodeficiency virus (HIV) antibody at screening.
8. Symptoms of dysphagia at screening or baseline or known difficulty in swallowing capsules.
9. Any condition at screening or baseline (e.g., chronic diarrhoea, inflammatory bowel disease or prior surgery of the gastrointestinal tract) that would interfere with drug absorption or any disease or condition that is likely to affect drug metabolism or excretion, at the discretion of the Investigator.
10. History or presence at screening or baseline of cardiac arrhythmia or congenital long QT syndrome.
11. QT interval corrected using Fridericia's formula (QTcF) > 450 msec. ECG may be repeated30 to 60 minutes apart from the first one collected at screening. If repeat ECG is ≤450 msec, the second ECG may be used to determine patient eligibility. However, if repeat ECG confirms QTcF remains >450msec, the subject is not eligible.
12. Use of tobacco or nicotine containing products in the previous 3 months prior to dosing or a positive urine cotinine test at Screening or Baseline.
13. Lack of willingness to abstain from the consumption of tobacco or nicotinecontaining products throughout the duration of the study and until completion of the final Follow-up visit.
14. Regular alcohol consumption defined as > 21 alcohol units per week (where 1 unit = 284 mL of beer, 25 mL of 40% spirit or a 125 mL glass of wine) or the Participant is unwilling to abstain from alcohol for 48 h prior to admission and 48 h prior to study visits.
15. Use of any prescription drugs (other than permitted contraception), over-the-counter (OTC) medication, nonsteroidal anti-inflammatory agents (NSAIDs), herbal remedies, supplements or vitamins within the 2 weeks prior to dosing or throughout the duration of the study, without prior approval of the Investigator and written approval of the Medical Monitor.
16. Paracetamol may be utilised, provided that the dose of Paracetamol does not exceed 2 g in any 24 h period.
17. Use of any of the following drugs within 28 days or 10 half-lives of that drug, whichever is longer, prior to study drug administration:
   a. Fluvoxamine, enoxacin, ciprofloxacin;
   b. Other inhibitors of CYP1A2 (including but not limited to methoxsalen or mexiletine);
   c. Contraceptives containing oestradiol, ethinyloestradiol or gestodene;
   d. Inducers of CYP1A2 (such as phenytoin), CYP2C9 or 2C19 (including but not limited to carbamazepine or rifampin);
   e. Any drug associated with prolongation of the QTc interval (including but not limited to moxifloxacin, quinidine, procainamide, amiodarone, sotalol).
18. Vaccination with a live vaccine within the 4 weeks prior to screening or that is planned within 4 weeks of dosing.
19. Exposure to any significantly immune suppressing drug within the 3 months prior to screening or 5 half-lives, whichever is longer.
20. Use of any investigational drug or device within 3 months prior to screening.
21. Consumption of grapefruit, grapefruit juice, Seville oranges, Seville orange juice, or any foods containing these ingredients, within 7 days prior to dosing or unwilling to abstain from these throughout the duration of the study.

### Dosage and Mode of Administration:

All participants in Parts 1 or 2 will be randomised 3:1 to receive either LYT-100 (deupirfenidone) formulated as powder in capsules, or placebo (a matching, inactive capsule containing methyl cellulose). All patients in Part 3 will be randomized to receive LYT-100 or placebo in a 1:1 ratio. The dosing regimen per study part and cohort is presented in **Table 17** below.

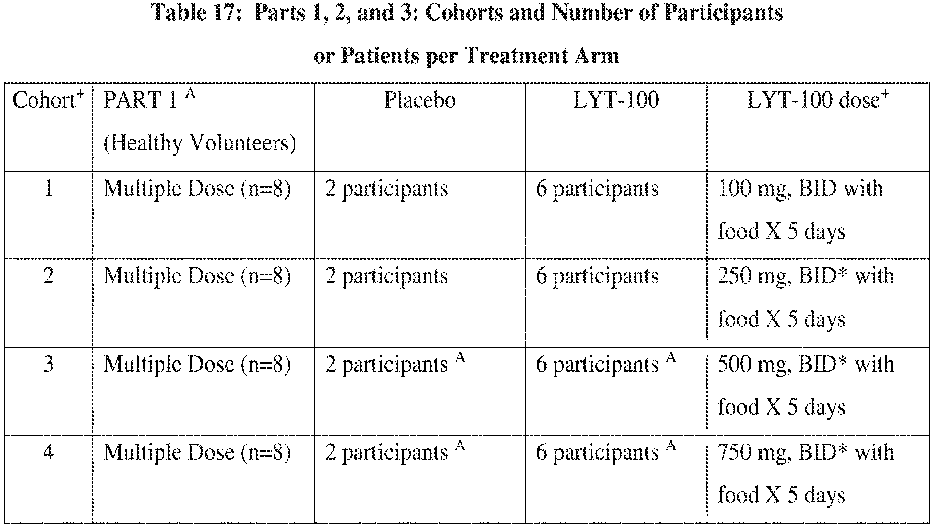

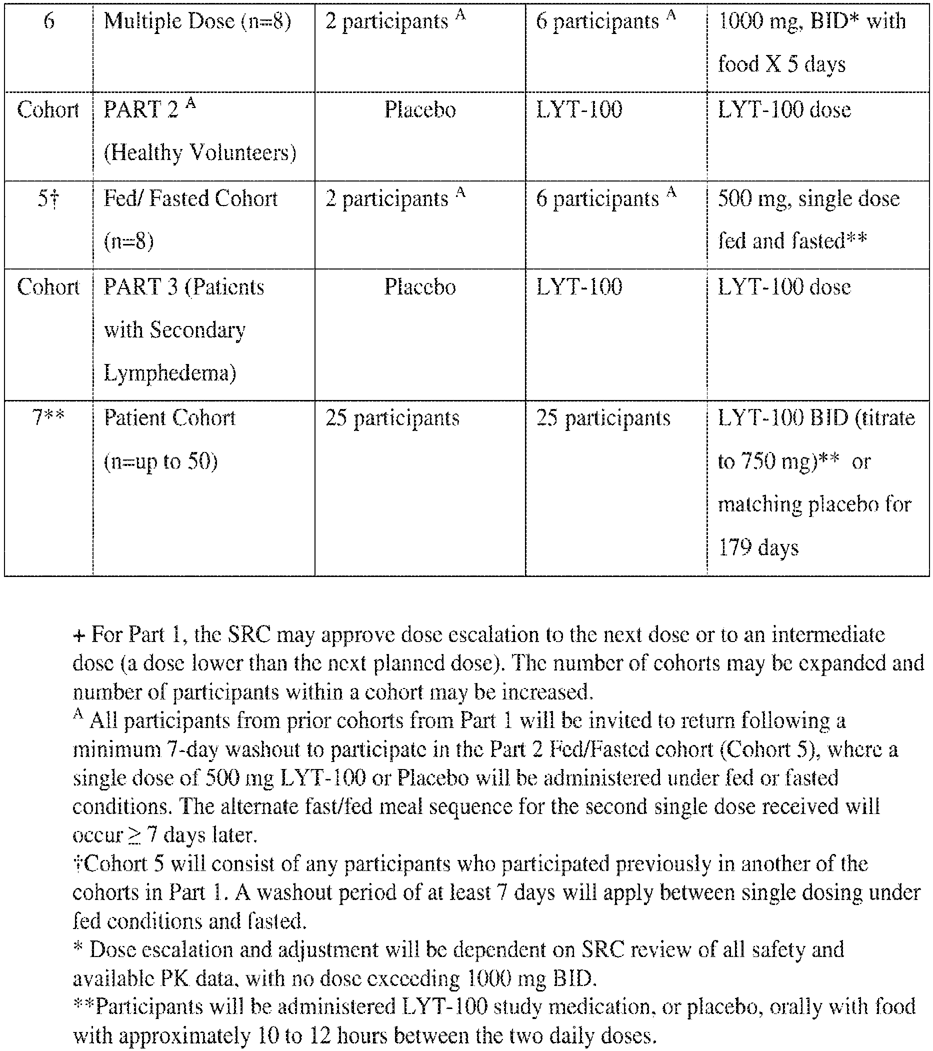

Doses may be adjusted according to safety and tolerability to avoid toxicity by adjusting to lower doses in response to patient safety and tolerability issues. If dosing titration is not well tolerated, adjustments to dosing may be made as follows: reductions to 250 mg, BID X 2 days (may be longer if needed), 500 mg BID X 2 days (may be longer if needed), 750 mg BID thereafter vs. matching placebo.

### Duration of Treatment with Study Medication:

- Part 1: 5 days
- Part 2: 2 single dose days
- Part 3: 26 weeks. Note that patients will each have a screening and post-treatment follow-up period.

### Criteria for Evaluation:

### Safety:

Safety and tolerability will be assessed throughout Part 1, Part 2 and Part 3 of the study by monitoring AEs, physical examination, vital signs, 12-lead ECGs, clinical laboratory values (haematology panel, multiphasic chemistry panel and urinalysis), and review of concomitant treatments/medication use.

### Pharmacokinetics:

Participants will provide blood samples at Baseline (Day -1) for the determination of CYP1A2, CYP2C9, CYP2C19, and CYP2D6 genotype to support exploratory PK analyses. Participants are required to provide consent for genotyping.

### Parts 1 and 2 (Healthy Participants)

In Part 1, blood samples (4 mL each) for PK will be collected for Cohorts 1, 2, 3, 4. and 6 at specified times (hours) at each as follows:
- Day 1 and Day 5: 0 (pre-dose), 0.5, 1, 1.5, 2, 2.5, 3, 3.5, 4, 6, 8, 12 (pre-dose), 13, 14, 15, 16, 18 h
- Day 2, Day 3 and Day 4: 0 (pre-moming-dose) and 12 h (pre-evening-dose)
- Day 6: 12, 18, 24 and 36 h post-last dose
- Day 7: 48 h (post-last dose)

Data will be used to assess the PK profiles of multiple BID doses of LYT-100 administered with food over 5 days for dose proportionality.

In Part 2, blood samples (4 mL each) for PK will be collected for Cohort 5 fed and fasted at specified times as follows:
- Day 1: 0 (pre-dose), 0.5, 1, 1.5, 2, 2.5, 3. 3.5, 4, 6, 8, 10, 12, 15. 18 h
- Day 2: 24 h post-dose
- Day 3: 48 h post-dose

Data will be used to descriptively compare the PK profile and compare the relative bioavailability of a single dose of LYT-100 administered at a dose below the MTD with food, to the equivalent dose without food.

Plasma concentration time data for LYT-100, and its metabolite(s) will be analysed using non-compartmental methods. Pharmacokinetics for Day 1 to 3 in Part 2 (fed state) will be compared to PK for Day 1 to Day 3 in Part 2 (fasted state). Plasma PK parameters (non-compartmental or compartmental analysis as appropriate) will include, but are not limited to:
- Following single dose: Tₘₐₓ, t_{lag}, Cₘₐₓ, AUC₀₋₄, AUC₀₋ₗₐₛₜ, t_{1/2}, AUC_{0-inf}, lambda z, %AUCₑₓₜ, CL/F, Vz/F, Cₘₐₓ/D, AUC₀₋ₗₐₛₜ/D, AUC_{0-inf}/D and MRT (D is dose)
- After repeated doses: C_{trough}, C_{trough}/D
- Following repeated doses on last day: Tₘₐₓ, Cₘₐₓ, C_{avg,} t_{lag}, t_{1/2}, AUC₀₋₄, AUC₀₋ₗₐₛₜ, AUC_{0-inf}, lambda z, %AUCₑₓₜ, CLss/F, Vzss/F, MRT, PTF, Rac(AUC), Rac(Cₘₐₓ), Cₘₐₓ/D, AUCₗₐₛₜ/D, AUC_{0-inf}/D and Rac (accumulation ratio).

### Study endpoints for the study are defined as follows:

### Part 1 and Part 2

- Safety and tolerability (AEs, physical examination, vital signs, ECGs, clinical laboratory parameters [haematology panel, multiphasic chemistry panel and urinalysis], and concomitant treatments).
- Pharmacokinetic parameters (AUC₍₀₋₁₂₎, AUC₍₀₋₂₄₎, t_{1/2} , VZ/F, CL/F*,* Cₘₐₓ, C₂₄, Cₘᵢₙ (T₂₄), and Tₘₐₓ).

### Part 3

### Primary endpoint:

- Safety and tolerability (AEs, physical examination, vital signs, ECGs, clinical laboratory parameters [haematology panel, multiphasic chemistry panel and urinalysis], and concomitant treatments).

### Secondary endpoint:

- Efficacy:
   ∘ Limb water content, by BIS.
   ∘ Limb volume (perometry).
   ∘ Tissue dielectric constant (MoistureMeterD).
   ∘ Tissue firmness (tonometry/SkinFibroMeter).
   ∘ Visual-analogue scales for pain, swelling, discomfort, and function.
- Infection
   ∘ Cellulitis
   ∘ Lymphangitis
- Health-related QoL:
   ∘ Lymphedema Symptom Intensity and Distress Survey-Arm (L-SIDS)
   ∘ Lymphedema Quality of Life Tool (LYMQOL)
- Population PK parameters.
- Fibrotic and Inflammatory biomarkers:
   ∘ Fibrotic and inflammatory biomarkers (G-CSF, MIG, FGF-2, IL-4, IL-10, lymphotoxin-α/TNF-β, leptin, IL-6, IL-1β, TNF-α, TGF-β1, MMP-9, TIMP-1, MCP-1).

### Statistical Methods

In Part 1 and Part 2, eight participants per cohort were chosen to adequately characterise the rate and extent of absorption as measured by select PK parameters, and to allow comparison of PK in a fed versus fasted state.

### General Statistical Plan

The analysis will be consistent with the study design, with assessment of each Part performed separately. The baseline for all variables will be the last measurement obtained prior to the participant receiving the first dose of study treatment.

Participant disposition (including the number and percent of participants/patients who are enrolled, who receive treatment, who prematurely discontinue and reasons for discontinuation, and who complete the study) will be tabulated by treatment group. Summary statistics for days of exposure and concentration of exposure will be provided by treatment group.

Adverse events, concomitant medications, clinical laboratory findings, physical examinations, ECGs and vital signs for each participant will be tabulated or summarised descriptively, where appropriate.

Demographic information will be presented for each participant and summarised. Treatment-emergent adverse events and laboratory, vital signs, and ECG parameters will be summarised. In addition, change from baseline will be summarised for laboratory and vital sign parameters. Shift tables will also be provided for clinical laboratory results. ECG results will be classified as normal and abnormal and summarised. ECG results for QTcF will also be classified as <450 msec. 450-500 msec or >500 msec. Changes in physical exams will be described.

### Study Populations

Analysis populations will be defined for each Part separately. In general:
- The Safety population is defined as all participants who receive LYT-100 or Placebo.
- The Full Analysis Set (FAS) population is defined as all randomized participants who received at least one dose of LYT-100 and had at least one post-baseline efficacy assessment. All efficacy endpoints will be assessed using this population.
- The Pharmacokinetic Population (PP) is defined as all participants who receive LYT-100 and for whom an evaluable concentration-time profile is available for the determination of at least one PK variable.
- The Fibrotic and Inflammatory Biomarker Population is defined as all patients in Part 3 only who receive LYT-100 and who provide biomarker and lymphedema assessment data.

### Analysis of PK in Parts 1 and 2

Analyses will be performed for each cohort separately. Determination of steady state for LYT-100 will be performed using Helmert Contrasts using trough concentrations at Days 2, 3, 4, 5, and 6. Pharmacokinetic parameter values will be listed and summarised by dose. Dose-linearity across the LYT-100 doses will be assessed. Comparison of the PK parameters in the fed and fasted states will be performed, and analysis of PK by gender may be performed if the data allow.

### Results for Study Part 1

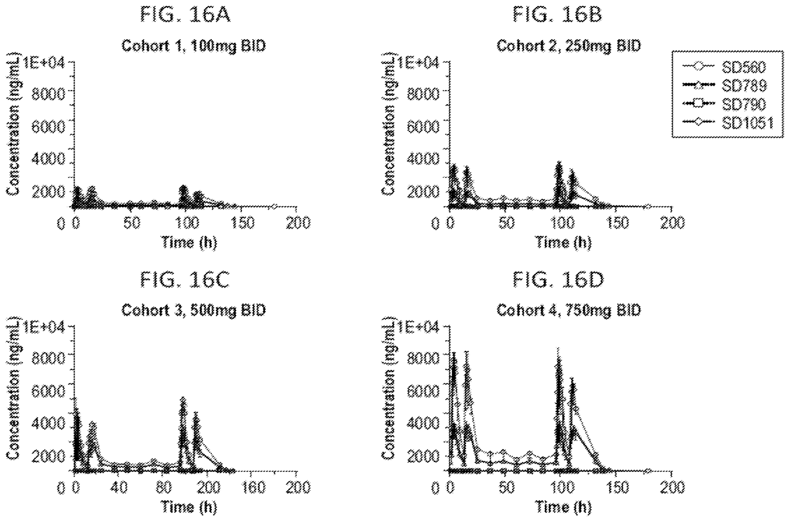

All cohorts (n=6 each) were dosed every 12 hours with food for 5 days at either 100, 250, 500, or 750 mg BID. **FIGs. 16A-16D** summarize the pharmacokinetics over 7 days (48 hours after last administration) for the active LYT-100 (deupirfenidone; SD-560) and the major metabolites, d₂-5-hydroxymethyl-pirfenidone (LYT-110; SD-790), non-deuterated 5-carboxy-pirfenidone (LYT-105; SD-789), and d₃-4'-hydroxy-pirfenidone (SD-1051). **FIG. 16A** shows the concentration in ng/mL of the active and each metabolite at each time point for the 100 mg BID Cohort 1. **FIG. 16B** shows the concentration in ng/mL of the active and each metabolite at each time point for the 250 mg BID Cohort 2. **FIG. 16C** shows the concentration in ng/mL of the active and each metabolite at each time point for the 500 mg BID Cohort 3. **FIG. 16D** shows the concentration in ng/mL of the active and each metabolite at each time point for the 750 mg BID Cohort 4. In each case, the data demonstrated increased exposure to drug and metabolites.

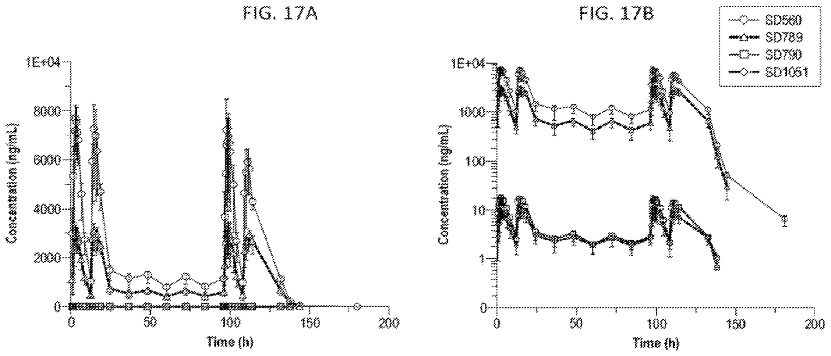

**FIG. 17A** and **17B** (linear and log scale concentration axes, respectively) provide the concentration in ng/mL of the active and each metabolite at each time point for the 750 mg BID Cohort 4. The exposure of LYT-100 (SD-560) was greatest, followed by 5-carboxy-pirfenidone (SD-789). Exposures for d₂-5-hydroxymethyl-pirfenidone (SD-790) and d₃-4'-hydroxy-pirfenidone (SD-1051) were approximately equal to each other, and more than two orders of magnitude less than that of the parent or SD-789, consistent with previous cohorts. Cₘₐₓ was similar to previous cohorts, and C_{trough} value was roughly constant, and no obvious accumulation was observed.

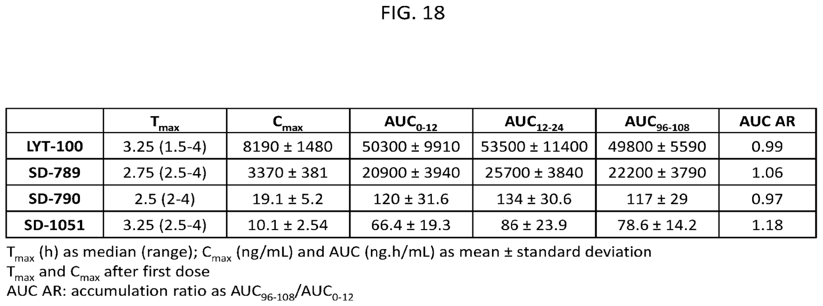

Pharmacokinetic data for Cohort 4 (Tₘₐₓ, Cₘₐₓ, AUC₀₋₁₂, AUC₁₂₋₂₄, AUC₉₆₋₁₀₈, and AUC accumulation ratio (AUC₉₆₋₁₀₈/AUC₀₋₁₂)) for the active and each metabolite is provided in **FIG. 18****.** The data demonstrate that no major accumulation of LYT-100 or its metabolites occurred over the length of the study. The data also confirmed that SD-789 was the major metabolite, and had an average ratio to the parent (M/P) by AUC of 0.45 (0.37-0.50). The minor metabolites SD-790 and SD-1051 had M/P of 0.0025 (0.0020-0.0029) and 0.0017 (0.0012-0.0022), respectively.

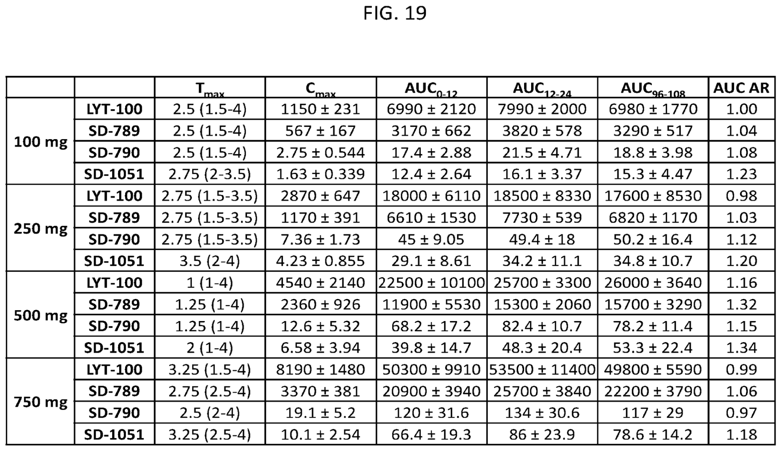

Pharmacokinetic data for LYT-100 and each metabolite (SD-789, SD-790, and SD-1051) are provided for each dosing Cohort (100 mg, 250 mg, 500 mg, and 750 mg BID; Cohorts 1-4, respectively) in **FIG. 19****,** which demonstrated similar accumulation ratios (~1) across all dose groups.

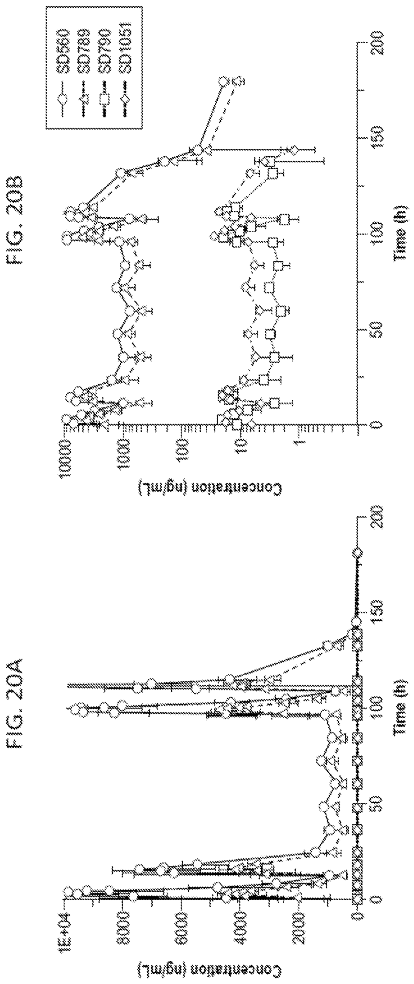

Pharmacokinetic data for LYT-100 and each metabolite (SD-789, SD-790, and SD-1051) are provided for Cohort 6 (1000 mg BID) in **FIGs. 20A** and **20B** (linear and log scale concentration axes, respectively), which demonstrated similar results as for previous cohorts. The exposure of LYT-100 (SD-560) was greatest, followed by 5-carboxy-pirfenidone (SD-789). Cₘₐₓ was similar to previous cohorts, and C_{trough} value was roughly constant, and no obvious accumulation was observed.

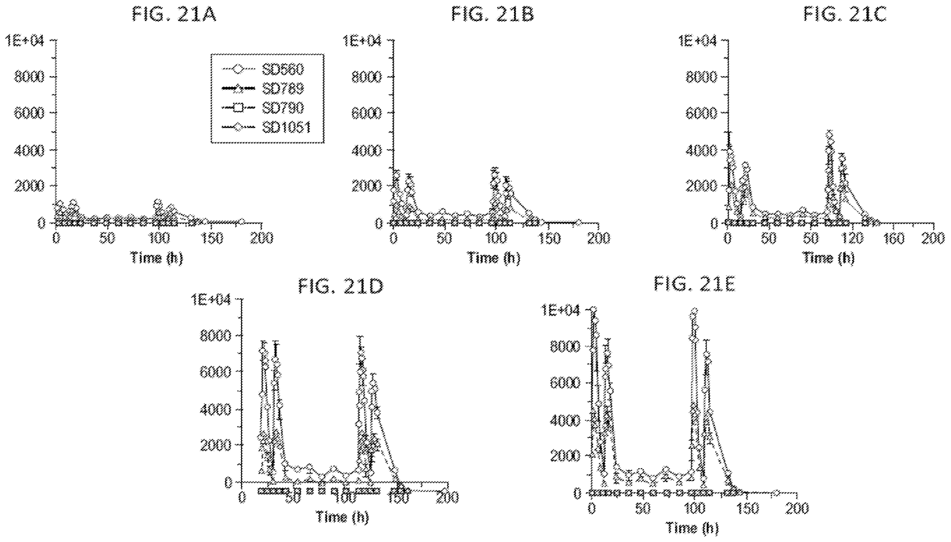

Pharmacokinetic data for LYT-100 and each metabolite (SD-789, SD-790, and SD-1051) are provided for each dosing Cohort (100 mg, 250 mg, 500 mg, 750 mg, and 1000 mg BID) in **FIGs. 21A-21E****,** respectively, which demonstrated increased exposure to drug and metabolites with dose.

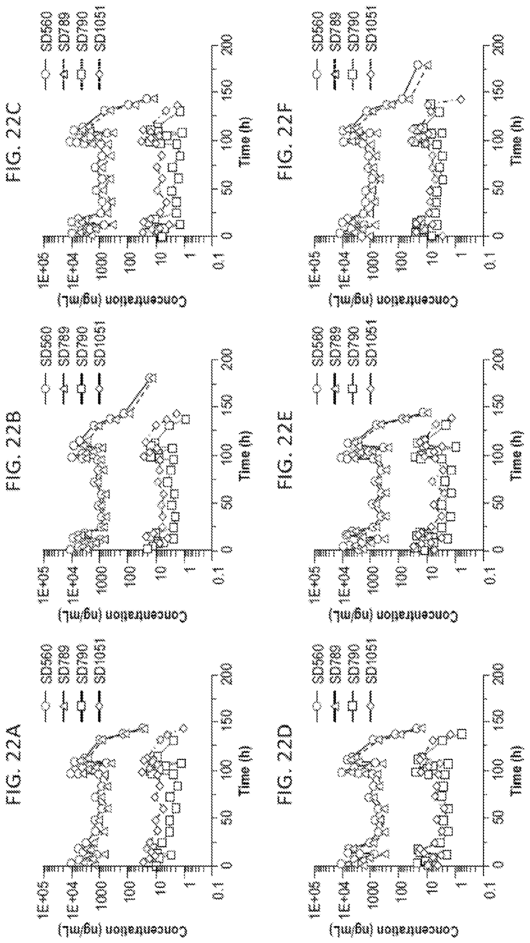

Pharmacokinetic data for LYT-100 and each metabolite (SD-789. SD-790, and SD-1051) are provided for each of the six subjects of Cohort 6 (1000 mg BID) in **FIGs. 22A-22F****,** which demonstrated little variation across subjects.

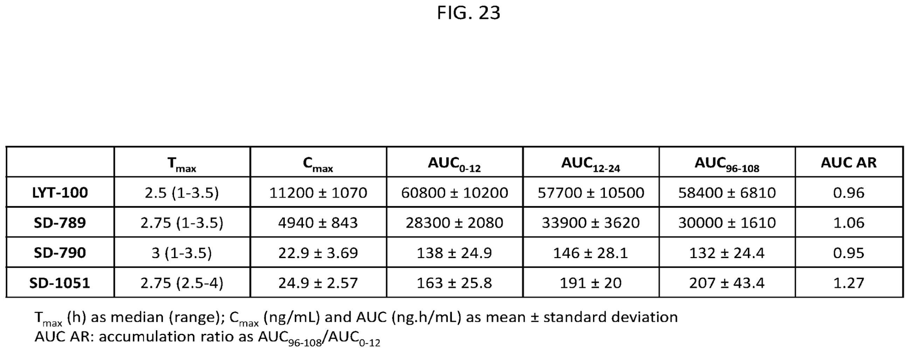

Pharmacokinetic data for Cohort 6 (Tₘₐₓ, Cₘₐₓ, AUC₀₋₁₂, AUC₁₂₋₂₄, AUC₉₆₋₁₀₈, and AUC accumulation ratio (AUC₉₆₋₁₀₈/AUC₀₋₁₂)) for the active and each metabolite is provided in **FIG. 23****.** The data demonstrate that no major accumulation of LYT-100 or its metabolites occurred over the length of the study. The data also confirmed that SD-789 was the major metabolite, and had an average ratio to the parent (M/P) by AUC of 0.56 (0.44-0.66). The minor metabolites SD-790 and SD-1051 had M/P of 0.0028 (0.0018-0.0038) and 0.0037 (0.0030-0.0058), respectively.

Pharmacokinetic data for LYT-100 and each metabolite (SD-789, SD-790, and SD-1051) are provided for each dosing Cohort (100 mg, 250 mg, 500 mg, 750 mg, and 1000 mg BID) in **FIG. 24****,** which demonstrated similar accumulation ratios (~1) across all dose groups.

The dose dependent AUC was evaluated for LYT-100 and SD-789 across all dose Cohorts using the AUC₉₆₋₁₀₈ data points. The data **(****FIG. 25****)** demonstrated linear dose proportionality for both parent and major metabolite.

Data across the Cohorts was compared against data from Huang et al. ("Pharmacokinetics, Safety and Tolerability of Pirfenidone and its Major Metabolite after Single and Multiple Oral Doses in Healthy Chinese Subjects under Fed Conditions." Drug Res (Stuttg) *63*, 388-395; 2013) at 200 mg BID pirfenidone, extrapolated to 100, 250, 500, 750, 1000 mg assuming dose proportionality, and comparing to AUC₀₋₁₂ and Cₘₐₓ, LYT-100 and SD-789 only. With the exception of the 500 mg dose, there was an increase for LYT-100 AUC over that of SD-559 and a decrease for SD-789 Cₘₐₓ over that of SD-559 **(****FIG. 26****).**

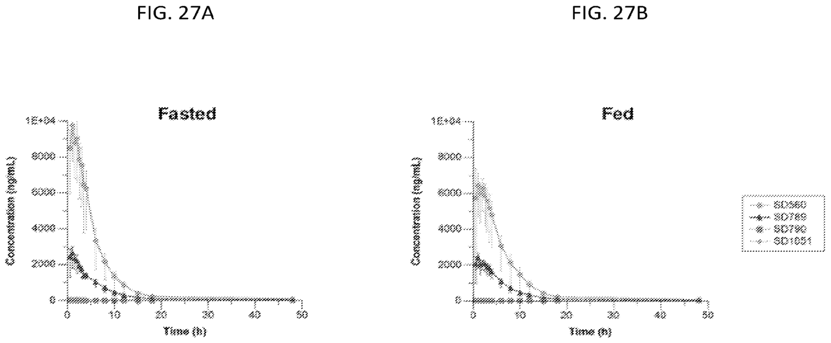

Pharmacokinetic data for LYT-100 and each metabolite (SD-789, SD-790, and SD-1051) are provided for dosing Cohort 5 (500 mg single dose; fasted and fed states) in **FIGs. 27A** and **27B** (fasted and fed, respectively) which demonstrated a similar exposure profile as in the other Cohorts, but with a markedly lower fed Cₘₐₓ.

Pharmacokinetic data for Cohort 5 (Tₘₐₓ, Cₘₐₓ, AUC_{0-inf}) for the active and each metabolite in fasted and fed subjects following a single 500 mg dose is provided in FIG. 28. The data demonstrated that in the fed state, the Cₘₐₓ and AUC of LYT-100 were decreased relative to that achieved when subjects were dosed in the fasted state (23% and 19% decrease in Cₘₐₓ and AUC, respectively). Pirfenidone demonstrates a much greater food effect on Cmax (49% decrease in fed state) For LYT-100, there was no food effect on Cₘₐₓ or AUC of LYT-100 metabolites. In contrast to food effects on pirfenidone (median Tₘₐₓ shift from 0.5 to 3), there was no food effect on the Tₘₐₓ of either LYT-100 or its metabolites.

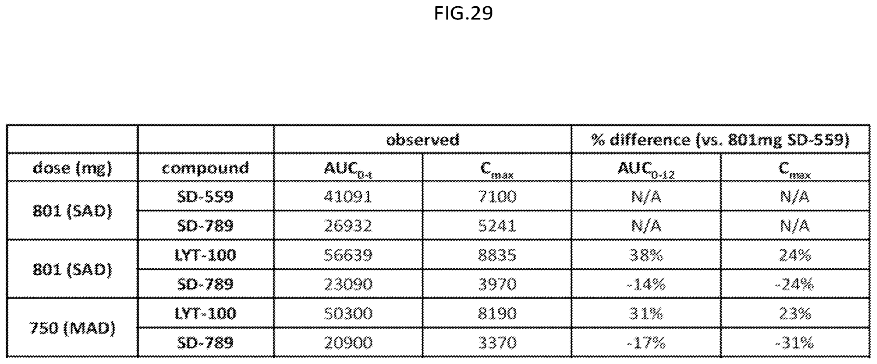

A comparison of data from the 750 mg MAD dose, pirfenidone at 750 mg, and the SAD dose of LYT-100 extrapolated to 750 mg **(****FIG. 29****).** The 750 mg MAD data reiterated observations in the SAD study, and demonstrated that there was higher exposure to LYT-100 than SD-559 for the same dose, and lower exposure to SD-789 after LYT-100 vs. SD-559 dosing at same dose level. This data confirms the deuterium kinetic isotope effect for LYT-100 vs. SD-559 on metabolic profile.

### Adverse Event Profile

Based on the blinded data obtained in the MAD study, LYT-100 was well tolerated over five days of BID dosing up to 1000 mg (2000 mg daily dose). There were no clinically relevant findings in vital signs, electrocardiograms, biochemistry, hematology, urinalysis, or coagulation parameters. In total, 33 subjects experienced a total of 72 adverse events (AEs). All AE's were graded as mild and transient, and less than half of these AEs (33 of 72) were considered possibly related to treatment. The most common AE was headache (25% of subjects). Further, in contrast to pirfenidone (Phase I study, PIPF-005), LYT-100 did not demonstrate a dose-related increase in adverse events based on the MAD blinded study data. For example, the low 250 mg BID dose had the most AEs, while the high dose (1000 mg) had the lowest (two incidences of headache). There were no dose limiting toxicities observed, and no maximally tolerated dose was reached.

Overall, single doses of LYT-100 and pirfenidone were well tolerated and have a comparable safety profile. No clinically significant differences were observed between the 2 treatments in terms of type, severity, or frequency of treatment emergent adverse events. The most common adverse event following either treatment was headache. Of interest, although administration of the 801 mg dose of LYT-100 resulted in greater drug exposure than with the same pirfenidone dose, the incidence of gastrointestinal and nervous system adverse events was not increased with LYT-100 administration as compared to pirfenidone (e.g., headache, nausea, abdominal discomfort). No significant changes in laboratory parameters, vital signs, or ECGs were observed following either treatment.

### Example 8: Efficacy and Safety of LYT-100 for Treatment of Coronavirus Infection

A study will be conducted to evaluate the efficacy and safety of LYT-100 on coronavirus infection. By administering LYT-100 as a treatment program, alone or in addition to standard treatment, the treatment program will treat the symptoms of coronavirus infection. The adult subjects will be stratified according to whether the onset time is ≤14 days and randomly divided into groups of 1:1, receiving treatment of LYT-100 or placebo orally as described in **Table 18.** The course will be 4 weeks or more.

Formulations: LYT-100 is manufactured in accordance with International Conference on Harmonization (ICH) Q7 Good Manufacturing Practice (GMP) and the United States FDA current GMP. The drug product, LYT-100 DIC (drug in capsule) is a Swedish-orange, hard-gelatin capsule directly filled with LYT-100 drug substance for oral use. The dose strengths of the capsules used for clinical trial will range from 50 mg to 250 mg, based on specified fill weights of the drug substance. A matching placebo will be manufactured using identical capsules filled with microcrystalline cellulose.

Storage conditions: The drug product, LYT-100 DIC will be packaged in high-density polyethylene (HDPE) bottles with a white, polypropylene screw cap with an induction sealed, aluminum inner-seal. The drug product bottles should be stored in a secure area with access limited to authorized staff, at controlled room temperature (20 C to 25 C).

Stability Data: The stability of LYT-100 drug substance and LYT-100 DIC is being monitored by ongoing stability programs in accordance with ICH stability guidelines.

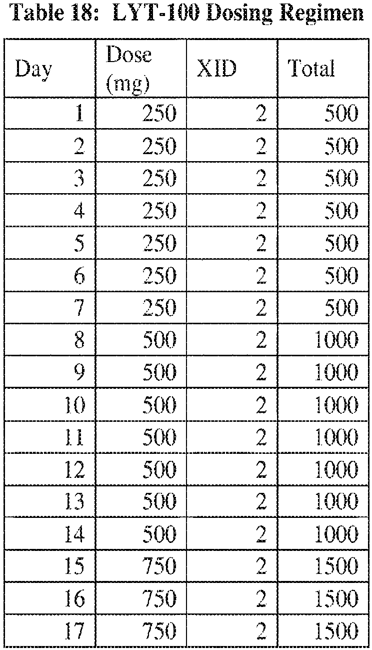

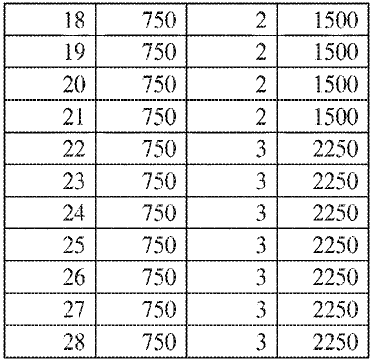

The primary outcome measures include: lesion area of chest CT image at 4 weeks (chest CT); absolute change in pulse oxygen from baseline (finger pulse oxygen with at 4 weeks); blood gas (Absolute change in blood gas from baseline in 4 weeks); absolute change in total score of Kings Brief Interstitial Lung Disease (KBILD) questionnaire for interstitial disease from baseline at week 4. Secondary outcome measures include: death within 4 weeks due to respiratory problems; time to disease progression or death within 4 weeks; changes of lymphocyte count in blood within 4 weeks; absolute change in viral nucleic acid from baseline within 4 weeks; pulmonary fibrosis survival symptoms absolute changes in dyspnea score from baseline within 4 weeks; changes in blood inflammatory indexes within 4 weeks; and absolute change in cough scores for pulmonary fibrosis symptoms from baseline in 4 weeks.

### Patient Main Inclusion Criteria

Main inclusion criteria include:
1. Female or male ≥ 18 years old.
2. Clinically diagnosed patients with new type of coronavirus pneumonia include: on the basis of meeting the criteria for suspected cases, one of the following pathogenic evidence:
   a. real-time fluorescent RT-PCR of respiratory specimens or blood specimens for detection of new coronavirus nucleic acid; or
   b. respiratory or blood specimens are genetically sequenced and highly homologous to known new coronaviruses.
3. The time interval between the suspected neocoronary pneumonia case and the random enrollment is determined within 4 days to 7 days according to the history symptoms and chest CT imaging.
4. Cough, diarrhea, or other related symptoms can be used. The imaging changes are mainly based on chest CT.
5. Study exclusion criteria: (1) AST and ALT> 1.5 x ULN at visit 1; (2) bilirubin> 1.5 x ULN at visit 1; (3) Cockcroft-Gault formula at visit 1 Calculated creatinine clearance rate <30 mL / min; (4) patients with potential chronic liver disease (Child Pugh A, B or C liver injury; (5) previous treatment with nintedanib, LYT100, or pirfenidone; Screening visit (Visit 1) 1 month or 6 half-life (whichever is greater) received other research drug treatment; (7) Based on ATS / ERS / JRS / ALAT 2011 guidelines (P11-07084) IPF diagnosis; (8) Obvious pulmonary hypertension (PAH) defined by any of the following standards: I. Clinical / echocardiographic evidence of previously obvious right heart failure; II. Medical history including right heart catheter showing a heart index ≤ 21 / min / m2; III. required Parenteral administration of epoprostenol / treprostinil for the treatment of PAH; (9) other clinically significant pulmonary abnormalities considered by the investigator; (10) major extrapulmonary physiological limitations (such as chest wall deformities, large amounts of pleural effusion); (11) cardiovascular disease, any of the following diseases: I. severe hypertension within 6 months of visit 1 with uncontrollable (≥160 / 100 mmHg); II. myocardial infarction within 6 months of visit 1; III. unstable angina within 6 months of visit 1; (12) history of severe central nervous system (CNS) events; (13) Known allergies to the test drug; (14) Other diseases that may interfere with the testing process or judged by the investigator may interfere with the trial participation or may put the patient at risk when participating in the trial; (15) Pregnancy, women who are breastfeeding or planning a pregnancy; (16) Patients unable to understand or follow the test procedures, including completing the questionnaires themselves without help.

### Example 9: A Interventional study of LYT-100 for the Treatment of Novel Coronavirus

A study will be conducted to evaluate the efficacy and safety of LYT-100 on coronavirus infection (open-label dose-titration, safety and exploratory trial of Deupirfenidone (LYT-100) in patients with moderate to and severe Coronavirus followed by a Phase 2b randomized, double-blind LYT-100 treatment of novel Coronavirus).

### Part 1

In the first part of the trial, primary objectives of the study are to evaluate safety, tolerability of multiple doses via dose-titration and determine the optimal doses of LYT-100 in patients with SARS-CoV-2 infection.

The secondary objectives of the study include the following: (1) A composite endpoint of adverse outcomes defined as progression to next level of respiratory disease severity, initiation of mechanical ventilation, or all cause fatality at 14- and 30-days is explored; (2) Efficacy signals of LYT-100 on progression of disease as follows:
(2A) Outcomes are explored including 14- and 30-day all cause fatality, duration of mechanical ventilation, length of ICU and hospital stays [if more than one hospital admission then account for each one separately (separately record days on ICU and on other ward(s)] and 28-day ventilator free days among survivors, for possible use as outcomes (individually or as composites) in a subsequent definitive comparative randomized controlled trial.
(2B) Clinical and routinely measured laboratory-based measures of severity are described including Sequential organ failure assessment (SOFA) score and its component determinants, CBC including NLR (neutrophil to lymphocyte ratio), CRP, Liver, kidney, clotting factors (with fibrinogen and d-dimer), oxygenation (including pulse oximetry, daily amount of O2/total number of days, PaO2/FiO2 ratio), ESR, presepsin, procalcitonin, ferritin, and dialysis.
(2C) Time to SARS-CoV-2 RT-PCR status change (positive/negative) from a nasopharyngeal and/or oropharyngeal swab is reported if tests are being repeated.
(2D) Blood samples are collected for experimental biomarker assessment of surrogate outcomes once each day during hospitalization period. Biomarkers may be assayed in an iterative manner but is likely to include those known to reflect pulmonary inflammation and fibrosis, including but not limited to, G-CSF. MIG, FGF-2, IL-4, IL-10, lymphotoxin α/TNF-β, leptin, IL-6, IL-1β, TNF-a, TGF-β1, MMP-9, TIMP-1, MCP-1, LDH, Troponin and N-terminal pro B-type natriuretic peptide (NT-proBNP), creatinine kinase.
(2E) Time to resolution of all signs and symptoms of disease (e.g., pulse, rate, blood pressure, need for oxygen supplementation, cough and dyspnoea severity and duration) is monitored and reported.
(2F) Chest X-ray and/or computed tomography (CT Scan) respiratory findings (frequency and type of imaging to be determined by the standard of care and investigator) are monitored and reported.
(2G) Pharmacokinetics - plasma concentration time data for LYT-100, and its metabolites, are monitored and reported.

Additional exploratory end point objectives include exploration of: (1) Murray lung injury score; (2) Respiratory function at Day 56 (TLCO, gas transfer, pulmonary fibrosis survival symptoms) if follow up is possible; and (3) Change in patient weight from the start of therapy until Day 28 and Day 56, if follow up is possible.

### Part 2

In the second the part of the study, patients with moderate to severe coronavirus severity are randomized in a 3:1 manner. As part of the Primary Study Objectives the optimal dosing schedule is provided, and the study objectives are re-evaluated and confirmed. An amendment to Part 2 of the study is planned to be updated if any changes are indicated. The primary trial endpoint is a composite endpoint of adverse outcome(s) at 14- and 28-days defined as: progression to next level of respiratory disease severity, initiation of mechanical ventilation, or all cause fatality.

As part of the Secondary Study Objectives the following parameters are evaluated. (1) Outcomes are evaluated including 14- and 30-day all cause fatality, duration of mechanical ventilation, length of ICU and hospital stays [if more than one hospital admission then account for each one separately (separately record days on ICU and on other ward(s)] and 30-day ventilator free days among survivors, for possible use as outcomes (individually or as composites) in a subsequent definitive comparative randomized controlled trial. (2) Clinical and routinely measured laboratory-based measures of severity are evaluated including Sequential organ failure assessment (SOFA) score and its component determinants, CBC, including NLR (neutrophil to lymphocyte ratio), CRP. Liver, kidney, clotting factors (with fibrinogen and d-dimer), oxygenation (including pulse oximetry, daily amount of O2/total number of days, PaO2/FiO2 ratio), ESR, presepsin, procalcitonin, ferritin, and dialysis. (3) Time to SARS-CoV-2 RT-PCR status change (positive/negative) from a nasopharyngeal and/or oropharyngeal swab is determined if tests are being repeated. (4) Blood samples are evaluated for experimental biomarker assessment of surrogate outcomes once each day, during hospitalization period. The nature of these biomarkers is yet to be determined and may be assayed in an iterative manner but is likely to include those known to reflect pulmonary inflammation and fibrosis, including G-CSF, MIG, FGF-2, IL-4, IL-10, lymphotoxin α/TNF-β, leptin, IL-6, IL-1β, TNF-a, TGF-β1, MMP-9, TIMP-1, MCP-1, LDH, Troponin and N-terminal pro B-type natriuretic peptide (NT-proBNP), and creatinine kinase. (5) Time to resolution of all signs and symptoms of disease (e.g., pulse, rate, blood pressure, need for oxygen supplementation, cough and dyspnoea severity and duration) is measured. (6) Chest X-ray and/or computed tomography (CT Scan) respiratory findings (frequency and type of imaging to be determined by the standard of care and investigator) are evaluated. (7) Safety, tolerability of multiple doses via dose-titration and determine the optimal doses of LYT-100 in patients with SARS-CoV-2 infection is evaluated. (8) Pharmacokinetics - plasma concentration time data for LYT-100, and its metabolite(s) is evaluated. Additional Exploratory Endpoints optionally include (1) Assessment of the Murray lung injury score progression (2) Determination of the respiratory function at Day 56 (e.g., TLCO, gas transfer, pulmonary fibrosis survival symptoms) if follow up is possible (3) Determination of the change in patient weight from the start of therapy until Day 28 and Day 56, if follow up is possible (4) Determination of fertility at Day 56, if follow up is possible (5) Determination of Anosmia (loss of sense of smell); (6) Determination of Hypogeusia (reduced ability to taste)

This study is conducted in patients who test positive for COVID-19 and is conducted at multiple study centres. Screening period does not exceed 48 hours. Only patients who meet all the applicable inclusion and none of the applicable exclusion criteria per study part are enrolled.

### Treatment Period

This study will be conducted in 2 parts.

Part 1 is an open-labelled study of LYT-100 in a lower number of patients (such as between 30 and 40) with moderate and severe COVID-19. The intent of Part I is to establish a safe and tolerable dose titration schedule. The data generated from Part 1 of the study is used to inform and reassess the sample size estimates for the randomized portion of this trial protocol. Specific clinical outcomes data includes median time to the composite endpoint in LYT-100 treated patients, as well as median time to death.

Part 2 of this protocol is a randomized, double-blind, placebo-controlled trial of 246 patients with moderate and severe COVID-19. Patients are randomized in a 3:1 manner with a larger number of patients (e.g., 150 to 200) in the LYT-100 treatment group and patients (e.g., 50-80) in the placebo group.

For Part 1 and Part 2, patients are enrolled on to the trial until written informed consent has been received. Screening is performed up to 48 hours prior to administration of the first dose of study medication for both study parts. Only participants who meet all the applicable inclusion criteria and none of the applicable exclusion criteria per study part will be enrolled. Patients who discontinue the trial may be replaced.

To ensure patient safety and identify early efficacy signals, two interim analyses are incorporated into the Part 2 trial design. Interim analyses are performed when one third and two thirds of the total events have occurred using the early stopping criteria of O'Brien and Fleming.

This is a non- randomized, open label clinical study to assess the safety, tolerability and efficacy (survivability) following LYT-100 administration with food (solid or nutritional supplements, whenever possible). Planned cohorts are as follows in **Table 19A-B.**

**Table 19A. Dosing Regimen Part 1**

| **PART 1: Participants receiving Open-label LYT-100** | | | | | |
|---|---|---|---|---|---|
| Single doses are as follows: | | | | | |
| 250 mg = 1 capsule / 500 mg=2 capsules/750 mg=3 capsules | | | | | |
| Day 1 | Day 2 | Day 3 through Day 7 | Day 8 through Day 28 | *⁴Optional Day 29 to Day 56* | *⁴Optional Day 57 to Day 84* |
| ²LYT-100 250 mg on Day 1 (first dose) | ²LYT-100 500 mg BID X 1 day | ²LYT-100 750 mg BID X 5 days, or maintain prior dose | ² LYT-100 750 mg BID X 3 week. or maintain prior dose | *^{2,3}LYT-100 750 mg BID X 4 weeks, or maintain prior dose* | *^{2,3}LYT-100 750 mg BID X 4 weeks, or maintain prior dose* |
| LYT-100 500 mg on Day 1 (second dose) | | | | | |
| **Moderate COVID-19¹** | | | | | |
| Hospitalized meeting criteria in the table below | | | | | |
| Number of patients with moderate severity = 21 (dropouts may be replaced) | | | | | |
| **Severe COVID-19¹** | | | | | |
| Hospitalized and meeting criteria in the table below | | | | | |
| Number of patients with severe severity = 10 (dropouts may be replaced) | | | | | |

| | | | | | |
|---|---|---|---|---|---|
| Patients will be stratified by disease severity ²Participants will be administered LYT-100 medication, or placebo, orally, preferably with food (solid or nutritional supplements, whenever possible) or through a nasogastric or gastric tube, approximately 12 h apart ³Dosing may be increased to 750 mg TID on a case by case basis if safety and tolerability are established; however, if questionable, dose should not to exceed BID without checking PK bloods collected 2-3 hours post any daily dose prior to planned dose increased to 750 mg TID. ⁴ Optional dosing is allowed following the first 28 days on study drug with evidence of any degree of fibrosis on radiographic imaging and per PIs medical judgement. Following screening and confirmation of eligibility, patients will begin their study drug treatment period on Day 1 and continue the treatment period through Day 28. Continuation of study medication beyond Day 28 and up to Day 56 or Day 84 is optional in Part 1 and Part 2 and is allowed with evidence of any degree of fibrosis on radiographic imaging and per PIs medical judgement. Patients will be seen on the ward or in the ICU daily during hospital stay. A visit will occur upon discharge from the hospital. Post-treatment follow-up visits will occur at Day 42 (Week 6) and Day 56 (Week 8) post Day 1 of study drug. If patient continues medication beyond Day 28, optional study visits will apply every 4 weeks and 2 weeks post last day of treatment, e.g., Day 56 (+/- Day 58 if treatment ends on Day 56), and Day 84 (+/- Day 86 if treatment ends on Day 84). | | | | | |

**Table 19B. Dosing Regimen Part 2**

| **PART 2: Number of Participants receiving Double-blind LYT-100, and placebo (final dosing regimen is established upon completion of Part 1)** | | | | | | |
|---|---|---|---|---|---|---|
| Single doses are as follows: 250 mg = 1 capsule / 500 mg=2 capsules/750 mg=3 capsules | | | | | | |
| | Day 1 | Day 2 | Day 3 through Day 7 | Day 8 through Day 28 | *⁴Optional Day 29 to Day 56* | *⁴Optional Day 57 to Day 84* |
| **Cohort** | ²LYT-100 250 mg or matching Placebo on Day 1 (first dose) | ²LYT-100 500 mg BID or matching Placebo X 1 day | ²LYT-100 750 mg BID or matching Placebo X 5 days, or maintain prior dose | ^{2,3}LYT-100 750 mg BID or matching Placebo X 3 week, or maintain prior dose | *^{2,3}LYT-100 750 mg BID or matching Placebo X 4 weeks, or maintain prior dose* | *^{2,3}LYT-100 750 mg BID or matching Placebo X 4 weeks, or maintain prior dose* |
| | LYT-100 500 mg on Day 1 (second dose) | | | | | |
| 1 | **Moderate COVID-19+** | | | | | |
| | Hospitalized on the regular ward or semi-critical ward and meeting criteria in the table below | | | | | |
| | Number of patients with moderate severity receiving LYT-100 or placebo = 3:1 | | | | | |
| 2 | **Severe COVID-19+** | | | | | |
| | Hospitalized in the semi-critical ward and/or ICU and meeting criteria in the table below | | | | | |
| | Number of patients with severe severity receiving LYT-100 or placebo = 3:1 | | | | | |

| | | | | | | |
|---|---|---|---|---|---|---|
| ¹ Patients will be stratified by disease severity ²Participants will be administered LYT-100 medication, or placebo, orally, preferably with food (solid or nutritional supplements, whenever possible) or through a nasogastric or gastric tube, approximately 12 h apart ³Dosing may be increased to 750 mg TID on a case by case basis if safety and tolerability are established; however, if questionable, dose should not to exceed BID without checking PK bloods collected 2-3 hours post any daily dose prior to planned dose increased to 750 mg TID. ⁴ Optional dosing is allowed following the first 28 days on study drug with evidence of any degree of fibrosis on radiographic imaging and per PIs medical judgement. | | | | | | |

Following screening and confirmation of eligibility, patients begin their study drug treatment period on Day 1 and continue the treatment period through Day 28. Continuation of study medication beyond Day 28 and up to Day 56 or Day 84 is optional in Part 1 and Part 2 and is allowed with evidence of any degree of fibrosis on radiographic imaging and per PIs medical judgement.

Patients are seen on the ward or in the ICU daily during hospital stay. A visit occurs upon discharge from the hospital. Post-treatment follow-up visits occur at Day 42 (Week 6) and Day 56 (Week 8) post Day 1 of study drug. If patient continues medication beyond Day 28, optional study visits apply every 4 weeks and 2 weeks post last day of treatment, e.g., Day 56 (+/- Day 58 if treatment ends on Day 56), and Day 84 (+/- Day 86 if treatment ends on Day 84).

Documentation of study variables will be recorded by use of three means. A monitoring report form inclusive of enrolment criteria, adverse events, concomitant medications and treatment outcomes information (e.g., 14- day, 28-day, 56-day or 84-day) and post-treatment outcomes will be completed. Patient severity of illness scores and routine physiologic and laboratory monitoring variables will be documented. Drug dosing and monitoring forms will be completed.

### Case Report form

A case report form will be developed for each patient, which will include:
(1) Date and time of first positive SARS-CoV-2 RT-PCR.
(2) Time to SARS-CoV2 RT-PCR negativity from a nasopharyngeal and/or oropharyngeal swab.
(3) Time to resolution of all signs and symptoms of disease.
Symptoms at enrollment, including fever, maximum temperature (≤37.3, 37.3-38, 38-39, >39), dyspnea, chest tightness, cough (dry or productive), sputum (color, amount, culture), rhinorrhea, anorexia, fatigue, vomiting, headache, dizziness, diarrhea, and myalgia.
(4) Echocardiogram reports.
(5) Risk factors, including known or repeated exposure, hypertension (characterized by the need for active pharmacotherapy), diabetes (characterized by the need for regular insulin injections or active pharmacotherapy), active cancer, cancer in remission, respiratory disease (requiring active pharmacotherapy, e.g. asthma, COPD), cardiovascular or metabolic disease requiring active pharmacotherapy, immunosuppression, auto-immune disease requiring active pharmacotherapy, hemopathies, organ dysfunction, coagulation dysfunction, and co-morbid illnesses as defined by Charlson.
(6) Outpatient and inpatient medications at time of admission.
(7) Daily documentation of SOFA score, adverse events, tolerability, and presence of criteria for septic shock (sepsis 3 definitions), ARDS (Berlin definition), laboratory confirmed infections, and renal failure (Rifle criteria).
(8) Time to and presence of respiratory disease progression from baseline through day 14, 28 and optional Day 56 and optional Day 84.
(9) Respiratory rate.
(10) Oxygen saturation of blood on room air.
(11) Forced expiratory volume in one second (FEV1) (if being assessed by investigator).
(12) Forced vital capacity (FVC) (if being assessed by investigator).
(13) FEV1/FVC ratio (if being assessed by investigator) pulmonary fibrosis survival symptoms.
(14) Absolute changes in dyspnea score from diagnosis to Day 28 and Day 56.
(15) Absolute change in cough score from diagnosis to Day 28 and Day 56.
(16) Need for supplemental oxygen at Day 28 and Day 56.
(17) Symptoms directed physical examination and sequential organ failure assessment.
(18) Liver function exam and tests: Hepatomegaly, AST, ALT, total bilirubin.
(19) Spleen exam and Tests: splenomegaly, kidney (serum creatinine, eGFR, urea). Creatinine kinase, LDH.
(20) Pulse rate and arterial blood pressure out to Day 28, and optional Day 56 and optional Day 84.
(21) Chest x-ray and /or computed tomography (CT scan), +/- contrast to evaluate the following from Baseline to Day 28, Day 56 and Optional Day 84. Scans need to be done from the level of the upper thoracic inlet to the inferior level of the costophrenic angle. Readings of radiologic assessments will include the number of involved segments **(Table 20A).**

**Table 20A: Involved Segments**

| | |
|---|---|
| **Location** | **Distribution** |
| □ Unilateral | □ Central |
| □ Bilateral | □ Peripheral |
| | o Central + Peripheral |
| **Lesion Development** | **Main Features** |
| o Single lesion | o Ground glass opacity |
| o Multiple lesion | □ Consolidation |
| □ Diffuse | □ Linear opacity |
| | □ Mixed type |
| **Interstitial Change** | □ |
| □ Septal thickening | |
| □ Crazy paving pattern | |
| □ Pleural thickening | |
| □Pleural effusion | |
| □ Lymphadenopathy | |
| □ Total lesion to lung ratio | |

(22) Lung lobe involvement for each of five lobes **(Table 20B).**

**Table 20B: Lung Lobe Involvement**

| **Lung lobe involvement - for each of the five lung lobes** | |
|---|---|
| None 0% | 0 |
| Minimal (1%-25%) | 1 |
| Mild (26%-50%) | 2 |
| Moderate (51%-75%) | 3 |
| Severe (76%-100%) | 4 |
| | |
| None 0% | 0 |
| Minimal (1%-25%) | 1 |
| Mild (26%-50%) | 2 |
| Moderate (51%-75%) | 3 |
| Severe (76%-100%) | 4 |
| | |
| None 0% | 0 |
| Minimal (1%-25%) | 1 |
| Mild (26%-50%) | 2 |
| Moderate (51%-75%) | 3 |
| Severe (76%-100%) | 4 |
| | |
| None 0% | 0 |
| Minimal (1%-25%) | 1 |
| Mild (26%-50%) | 2 |
| Moderate (51%-75%) | 3 |
| Severe (76%-100%) | 4 |
| | |
| None 0% | 0 |
| Minimal (1%-25%) | 1 |
| Mild (26%-50%) | 2 |
| Moderate (51%-75%) | 3 |
| Severe (76%-100%) | 4 |
| **TOTAL LUNG SEVERITY SCORE (0-20):** | |

23. Mechanical ventilation (regardless of whether mechanical ventilation was implemented or not), meeting any one of the following criteria: presence of bradypnea, apnea, or respiratory arrest; acute respiratory distress syndrome; tachypnea (RR ≥30 times / minute for ≥ 55 years of age and with history of chronic respiratory or cardiovascular disease, diabetes, or hypertension; or RR>25 for patients ≥ 55 years of age with no respiratory or cardiovascular disease, diabetes, or hypertension or < 55 years of age); vital capacity <15 mLJkg; respiratory minute ventilation >101/min; respiratory acidosis, with PCO2 > 50 mm Hg, pH <7.35; hypoxemia with arterial partial pressure of oxygen (PAO2) < 55 mm Hg; and pulse oximeter with SPO2 <90%.
24. Survival status at 14, 28, 56, and optional 84-days (alive in hospital, alive post-discharge, death).

### Clinical information

The following variables are recorded: Hospital and ICU admissions and discharge dates and times; Admission APACHE II and III scores; Daily extreme (lowest and highest) blood pressure, temperature, heart rate, respiratory rate, full blood counts (including lymphocyte count), lactate, arterial gas (pH, pO₂, pCO₂, bicarbonate), ALT, bilirubin, serum sodium, potassium, creatinine, glucose, vasopressor doses, and FIO₂. Time to first and subsequent Ventilation, status (yes/no) and duration; Requirement for continuous renal replacement therapy (CRRT); Fibrotic and inflammatory biomarkers (G-CSF, MIG, FGF-2, IL-4, IL-10, lymphotoxin-α/TNF-β, leptin, IL-6, IL-1β, TNF-α, TGF-β1, MMP-9, TIMP-1, MCP-1, LDH, Troponin and N-terminal pro B-type natriuretic peptide (NT-proBNP), creatinine kinase); Additional Markers: C-reactive protein, ESR, presepsin, procalcitonin, ferritin, clotting factors (including PT, APTT, thrombin clotting time, fibrinogen and d-dimer); Haematology panels; CBC with differential counts including: Lymphocyte count; Leukocyte count; Neutrophil count; Platelets; and Eosinophil count; Self-rating depression scale out to 28 days; Change in patient weight from the start of therapy randomization until Day 28 and Day 56; Serology (IgM/IgG for SARS-CoV2) analyses; Safety, tolerability of LYT-100; Adverse events; Concomitant medication needs; Biochemistry panels; Urinalysis panels; Physical examinations; vital signs; ECGs; and Echocardiogram.

Pharmacokinetics - PK samples will be collected at the same time as routine blood samples, time of blood collection and time of last dose will be recorded. Plasma concentration data for LYT-100. and its metabolite(s) will be analysed at a minimum of 3 separate days. Blood samples (4 ml each) for population pharmacokinetics will be collected at baseline and on Days 7, 14 and 28 (and Days 56 and 84 if patient continues on longer termed, optional treatment). Each participant will provide 3 samples at one of the time points in reference to dosing and without duplicating the same time interval as follows more than once: Pre-dose, Post-dose times: 1 to <2 h; 2 to <4 h; 1 to <2 h; 4 to <8 h; and 8 to 12 h

### Outcomes

The primary outcomes of the study are definition of the optimal dosing of LYT-100 in patients with SARS-CoV-2 infection admitted to ICU, along with assessment of the safety of this dosing and the feasibility of the study protocol.

Secondary outcomes, assessed for the purpose of determining suitability and a power calculation for a subsequent definitive comparative trial, are: 14-day all cause fatality; 28-day all cause fatality; Duration of mechanical ventilation; Length of ICU and hospital stay; 28-day ventilator free days among survivors; Requirement for renal dialysis; Time to SARS-CoV-2 RT-PCR status change (positive/negative) from a nasopharyngeal and/or oropharyngeal swab if tests are being repeated; (viii) Occurrence of adverse events; Time to resolution of septic shock (Sepsis 3 definition); Time to resolution of ARDS (Berlin definition); and Time to resolution of organ failures, as defined by the daily Sequential organ failure assessment (SOFA) score and its component determinants

Tertiary outcomes are the results of any biochemical or other laboratory tests undertaken for the purpose of understanding the mechanism of action of LYT-100.

All patients are monitored for the following screening, clinical efficacy and safety assessments of LYT-100 at time points delineated in the Schedule of Events **Table 21** and **Table 22.**

**Table 21. Schedule of Events for Cohorts 1 and 2 Receiving 28-Days of Treatment**

| | **Screening** | **Baseline** | **Part 1: Treatment Phase LYT-100, N=31Part 2: Treatment Phase LYT-100 vs. Placebo Phase, N=246** | | | | | | | | **Hospital Discharge** | **Post-treatment Phase** | |
|---|---|---|---|---|---|---|---|---|---|---|---|---|---|
| | **Days** | **Day** | **Days** | | | | | | | | | **Days** | |
| **Study Day** | **-2 to Day -1** | **-1** | **1** | **4** | **7** | **10** | **14** | **21** | **28** | **30** | **Hosp D/C** | **42** | **56** |
| Visit Number | 1 | 2 | 3 | 4 | 5 | 6 | 7 | 8 | 9 | 10 | DC | 11 | 12 |
| Demographics | | X | | | | | | | | | | | |
| Pertinent Medical History/ Risk Factors | X | | | | | | | | | | | | |
| Symptom Checklist including Cough and Dyspnoea | X | X | | X | X | X | X | X | | X | X | X | X |
| APACHE II (including Glasgow Coma Scale) | | X | | | | | | | | | | | |
| Charlson Comorbidity | | X | | | | | | | | | | | |
| SARS-CoV-2 RT-PCR from a nasopharyngeal and/or oropharyngeal swab | X | | | | | | X | | | X | X | | X |
| Height (cm) | | X | | | | | | | | | | | |
| Murray Lung injury score | | X | | | | | | | | X | X | | |

**Table 22. Schedule of Events for Cohorts 1 and 2 Receiving 56- or 84-Days of Treatment**

| | **Screenin g** | **Baselin e** | **Part 1: Treatment Phase LYT-100, N=31 Part 2: Treatment Phase LYT-100 vs. Placebo Phase, N=246** | | | | | | | | | | | **Post-treatmen t Phase** | | |
|---|---|---|---|---|---|---|---|---|---|---|---|---|---|---|---|---|
| | **Days** | **Day** | **Days** | | | | | | | | **Hospital Discharg e** | **Optional Rx Days** | | **Days** | | |
| **Study Day** | **-2 to Day -1** | **-1** | **1** | **4** | **7** | **10** | **1 4** | **2 1** | **28** | **30** | **Hosp DC** | **56** | **84** | **58*** | **86**** | |
| Visit Number | 1 | 2 | *N* A | 3 | 4 | 5 | 6 | 7 | *N*/ A | 8 | DC | 9 | 10 | 10 | 11 | |
| Demographics | X | | | | | | | | | | | | | | | |
| Pertinent Medical History/ Risk Factors | X | | | | | | | | | | | | | | | |
| Symptom Checklist including Cough and Dyspnoea | X | X | | | X X | X | X | X | | X | X | | | X | X | |
| APACHE II (including Glasgow Coma Scale) | | X | | | | | | | | | | | | | | |
| Charlson Comorbidity | | X | | | | | | | | | | | | | | |
| SARS-CoV-2 RT-PCR from a nasopharyngeal and/or oropharyngeal swab | X | | | | | | X | | | | X | X | | | X | |
| Height (cm) | | X | | | | | | | | | | | | | | |
| Murray Lung injury score | | X | | | | | | | | X | | X | X | | | |
| Study Drug Administration and Accountability | | | X | X | X | X | X | X | X | X (only if continuing ) | | X (only if continuin g | X (only if continuin g | | | |

| **Study Day** | **-2 to Day -1** | **-1** | **1** | **4** | **7** | **1 0** | **1 4** | **2 1** | **28** | **30** | **Hosp DC** | **56** | **84** | **58*** | **86**** | |
|---|---|---|---|---|---|---|---|---|---|---|---|---|---|---|---|---|
| Physical Exam / Organ Failure Assessment | X | X² | | | X ² | | X ² | | | X² | | X² | X² | X² | X² | |
| Weight (kg) | X | X | | | X | | X | | | X | | X | X | X | X | |
| Body mass index | X | | | | | | | | | | | X | X | X | X | |
| Chest X-ray and/or CT Scan | X | X | | | X | | | | | X | | X | X | X | X | |
| Respiratory Disease Progression Assessment | | X | | | XX | X | X | X | | X | | X | X | X | X | |
| Pulmonary Function Tests (FEV, FCC) | X | X | | | X | | X | | | X | | X | X | X | X | |
| Respiratory Function | X | X | | | X | | X | | | X | | X | X | X | X | |
| Pulmonary Fibrosis Survival Symptoms | X | X | | | X | | X | | | X | | X | X | X | X | |
| Arterial Blood Pressure | X | X | | | X | | X | | | X | | X | X | | | |
| Standard 12-lead ECG ³ | X | | | | X | | X | | | X | | X | X | X | X | |
| Echocardiogra m | | X | | | | | X | | | X | | X | X | | | |
| Vital signs (BP, PR)³ | X | X | | | X | | X | | | X | | X | X | | | |
| Respiratory Rate³ | X | X | | X | X | X | X | X | | X | | X | X | X | X | |
| Tympanic temperature | X | X | | X | X | X | X | X | | X | | X | X | X | X | |
| Haematology | X | X | | | X | | X | | | X | | X | X | X | X | |
| Blood chemistry | X | X | | | X | | X | | | X | | X | X | X | X | |
| Coagulation | X | X | | | X | | X | | | X | | X | X | X | X | |
| Urinalysis | X | X | | | X | | X | | | X | | X | X | X | X | |
| Pregnancy test | Serum | Urine | | | | | | | | | | | | Urin e | Urine | |
| Biomarkers | | X | | | X | | X | | | X | | X | X | X | X | |
| Additional Markers | | X | | | X | | X | | | X | | X | X | X | X | |
| PK blood samples | | X | | | X | | X | | | X | | X | X | | | |
| Adverse Events/Mortalit y | | | | | | | | | | | | X | X | X | X | |
| Prior & Concomitant Meds | X (in last week) | X | | | | | | | | | | X | X | X | X | |

| | | | | | | | | | | | | | | | | |
|---|---|---|---|---|---|---|---|---|---|---|---|---|---|---|---|---|
| * 56-Day Treatment ** 84-Day Treatment | | | | | | | | | | | | | | | | |

### Study Inclusion Criteria

(1) Positive result of RT-PCR for SARS-CoV-2 RNA result from a clinical specimen deemed clinically associated with the current episode of illness, warranting hospital admission as per investigator's judgement, or already hospitalized (central and/or local laboratory COVID-19 tests are accepted from any biological material source).
(2) Features of respiratory illness including symptoms of cough or shortness of breath or radiologic evidence of lower respiratory tract infection
(3) Written Ethics Committee approved informed consent from the patient or Legally Authorized Representative prior to any study procedures.
(4) Male or female, aged 18 and older at the time of screening. No upper age limit.
(5) Willing to comply with all study procedures and requirements.
(6) Meeting one of the moderate or severe COVID-19 severity classifications at the time of enrollment (Table 23).
(7) Laboratory parameters (CBC, biochemistry) deemed by the investigator as acceptable to enrol the patient.
(8) Women of childbearing potential (WOCBP) must be non-pregnant and non-lactating, and must agree to use an acceptable, highly effective double contraception or be abstinent from heterosexual intercourse for 90 days following last dose of study medication.
(9) Male participants must be surgically sterile (> 30 days since vasectomy with no viable sperm), abstinent, or if engaged in sexual relations with a WOCBP, the participant and his partner must be surgically sterile (e.g., tubal occlusion, hysterectomy, bilateral salpingectomy, bilateral oophorectomy) or using an acceptable, highly effective contraceptive method from screening until study completion, including the follow-up period and an additional 12 weeks after the last dose.
(10) Males must not donate sperm for at least 90 days after the last dose of study drug.
(11) Partners of male patients and female patients should report pregnancy occurring within 90 days from cessation of study drug.
(12) Patients are allowed to be on any other medication for their comorbidities except from those clearly outlined in the exclusion criteria.
(13) Patients are allowed to be on any and all medication (on and off label) to treat COVID-19 respiratory disease, that the investigator deems clinically relevant in combination with the study drug.
(14) Patients are allowed to concomitantly receive any standard of care as per investigators' clinical decision. Standard of care may encompass any approved, on or off label medication apart from the ones specifically outlined in the exclusion criteria. Concomitant medication may not be other investigational agents(s).

**Table 23. COVID-19 Severity Classifications**

| **Moderate COVID-19** | **Severe COVID-19** | |
|---|---|---|
| Must have at least two more of the following | c. SOFA score of ≥2 points | |
| a. Requiring some non-invasive oxygen supplementation via nasal cannula to keep oxygen saturation ≥94% on room air | | o PaO₂/FiO₂ <300), |
| | | o Fraction of inspired oxygen concentration (FiO₂) exceeding 0% to maintain an oxygen saturation of >90% in arterial blood, or |
| b. Radiographic evidence of pneumonia | | |
| *clinically relevant increase over UNL will be determined by the investigator at the site based on internal standard of care for COVID-19 patients | | o Mechanically ventilated |
| | | o Radiographic evidence of pneumonia |

### Exclusion Criteria

Exclusion criteria includes: Prior condition unrelated to SARS-CoV-2 infection congestive heart failure requiring therapy, prior interstitial lung disease (e.g., idiopathic pulmonary fibrosis, COPD, etc.), other chronic hypoxia conditions, or other co-morbidity associated with life expectancy less than 1 year, ejection fraction if estimated <45%; History of anaphylactic reaction (particularly reactions to general anaesthetic agents); allergic reaction due to any drug which led to significant morbidity; prior allergic reaction to pirfenidone; sepsis confirmed by hemoculture, septic/hypovolemic/cardiogenic/ncurogenic shock; known symptoms of dysphagia or known difficulty in swallowing capsules and total gastrectomy; patient undergoing dialysis; and total gastrectomy and/or inability to adequate ingest (either orally or enterally) study medication, or tolerate oral or enteral feeding.

Use of any of the following drugs is not recommended: Fluvoxamine, enoxacin, ciprofloxacin, other inhibitors of CYP1A2 (including but not limited to methoxsalen or mexiletine), inducers of CYP1A2 (such as phenytoin), CYP2C9 or 2C19 (including but not limited to carbamazepine or rifampin), and drugs associated with substantial prolongation of the QTc interval (including but not limited to moxifloxacin, quinidine, procainamide, amiodarone, sotalol). Note that QTc prolongation is not an all / nothing drug effect, and specifically that administration of hydroxychloroquine does not preclude participation in this trial but does mandate measurement of the QTc every 6 hours.

For baseline LFTs 2.5.0 x UNL investigators may use their own judgement to clarify and assign causality and determine eligibility to initiate study medication will be taken off study medication.

Dosage and Mode of Administration: Part 1 is open-label treatment and patients will receive LYT-100 (deupirfenidone) formulated as powder in 250 mg capsules. Part 2 is double-blind trial. Patients will receive LYT-100 (deupirfenidone) formulated as powder in 250 mg capsules or matching placebo.

Duration of Treatment: Patients are treated up to 28 consecutive days. Continuation of study medication beyond Day 28 in Part 1 and Part 2 is at the discretion of the principal investigator. Further decisions to adjust dosing titration and confirm extension of dosing in Part 2 beyond Day 28 in all patients will be made pending observations collected in Part 1. Follow-up of 28 days post last dose are implemented at Day 42 and Day 56. If additional dosing option up to Day 56 or Day 84 is extended, post-study treatment will occur two-weeks following the end of treatment, e.g., Day 58 and Day 86, respectively.

### Statistical Methods

The target sample size for Part 1 of the study is 31 [CH8] patients. This sample size achieves an 80% power to detect a 20% difference (P1-P0) in LYT-100 toxicity (all grades) - using a two-sided binomial test and a two sided p value of 5%. The study power assumes that the population proportion of patients receiving LYT-100 who develop toxicity is 30%.

The target sample size for Part II was based on the hypothesis that LYT-100 versus the placebo control group will significantly reduce the risk of treated patients progressing to next level of respiratory disease severity, initiating mechanical ventilation, or death. The overall sample size in this study is expected to be 246 subjects (with 163 events), randomized in a 3 to 1 manner into the experimental and placebo control group (61 in the placebo control group and 185 in the treatment group). Assuming an alpha of 5% (two tailed), the study will have an 80% power to detect a 40% reduction (i.e. HR = 0.60) in the risk of death, mechanical ventilation or worsening of disease between the experimental and control group. The final target sample size also assumes that 10% of patients enrolled into each group will drop out of the study or be lost to follow up.

To ensure patient safety and identify early efficacy signals, two interim analyses will be incorporated into the trial design. Interim analyses will be performed when one third (approx. 53) and two thirds (approx. 110) of the total events have occurred and the early stopping criteria of O'Brien and Fleming will be used. Specifically, the threshold for early stopping at the first interim will be p < 0.005 and p < 0.014 at the second interim analysis. If the study is not stopped early all patients have been enrolled, the final threshold for statistically significance will be p < 0.045, which is consistent with the O'Brien and Fleming approach. The Drug Safety and Data Monitoring Board (DSMB) will monitor patient safety and efficacy and make decisions at each interim on stopping the trial early for efficacy, futility or continuing to full enrolment. An independent statistician will perform each interim analysis provide the results to the DSMB.

All efficacy analyses will be based on the ITT population unless otherwise specified. Baseline patient and clinical data as well as all secondary and exploratory endpoints will be presented descriptively as means, medians, or proportions with appropriate measures of variance (i.e. range, 95%CI). For the primary composite endpoint, survival curves will be generated by the method of Kaplan-Meier and compared with the log-rank test from the day of randomization. Patients who are alive and discharged from the hospital or are lost to follow up will be censored. In a supporting analysis, an adjusted hazard ratio for the risk of death, MH or disease worsening between the experimental and control group will be estimated using multivariate Cox Proportional Hazard regression. To identify the set of baseline factors with the greatest potential for association with the primary endpoint, those with a p-value of 0.25 or less in a simple univariate Cox regression with the dependent variable, will be retained for further consideration. This is a recommended approach for removing weak prognostic covariates so that a more manageable set of variables can be analyzed via multivariate techniques. After this univariate screening process, the final independent variables with a p < 0.05 will be retained in the final model through a backwards elimination process.

The binary secondary endpoints (e.g. overall mortality by Day 28, respiratory depression by Day 28) are presented as odds ratios with appropriate 95% confidence intervals. The analysis also includes a univariate comparison of patient and clinical variables at baseline as well as a comparison of the primary composite primary endpoint at Day 28 and 56.

Secondary endpoints include: Pulmonary Function Tests, Radiographic imaging (Chest X-ray and/or CT Scans), Pharmacodynamics Biomarker assessments, Safety/tolerability and Pharmacokinetics. Change from Baseline to each post-Baseline visit through Day 28 on-treatment effect and up to Day 56 post-treatment effect on the primary and secondary endpoints are evaluated in an exploratory analysis.

Exploratory correlational analysis is undertaken to identify patient factors and potential biomarkers that are associated with overall survival and successful resolution of all signs and symptoms of the disease. All the statistical analysis is performed using SAS, version 9.2. (SAS, Cary, NC).

The baseline for all variables is the last measurement obtained prior to the participant receiving the first dose of study treatment. Participant disposition (including the number and percent of patients who are enrolled, who receive treatment, who prematurely discontinue and reasons for discontinuation, and who complete the study) are tabulated by Cohort. Summary statistics for days of exposure and concentration of exposure will be provided by Cohort.

Adverse events, concomitant medications, clinical laboratory findings, physical examinations, ECGs, Echocardiograms and vital signs for each participant is tabulated or summarized descriptively, where appropriate.

Demographic information is presented for each participant and summarized. Treatment-emergent adverse events and laboratory, vital signs, ECG parameters, and Echocardiograms are summarized. In addition, change from baseline is summarized for laboratory and vital sign parameters. Shift tables are provided for clinical laboratory results. ECG results will be classified as normal and abnormal and summarized. ECG results for QTcF are classified as <450 msec, 450-500 msec or >500 msec. Changes in physical exams are described in the final study report.

The "Intent-to-Treat" or "ITT" study population refers to all randomized patients summarized by randomized treatment. The Safety population is defined as all participants who received at least one dose of LYT-100. The "Per-protocol population" refers to all randomized patients in the experimental group who receive at least 7 days of active therapy or placebo. The Pharmacokinetic Population (PP) is defined as all participants who receive LYT-100 and for whom an evaluable concentration-time profile is available for the determination of at least one PK variable. The Pharmacodynamic Population is defined as all patients who receive LYT-100 or placebo and who provide biomarker and efficacy assessment data.

### Example 10: An Interventional study of LYT-100 for the Treatment of Novel Coronavirus

A study will be conducted to evaluate the efficacy and safety of LYT-100 on coronavirus infection.

The primary study objective of this study is to evaluate survivability of 2019 Novel Coronavirus up to 28 days post-diagnosis. The secondary study objectives are to evaluate: safety and tolerability; time to respiratory disease progression from onset of treatment; chest X-ray and computed tomography (CT Scan) to evaluate abnormal changes in lung tissues, nodules and other structures out to 28 days; respiratory function: respiratory rate, oxygen saturation of blood on room air, FEV1, FVC; laterality of chest findings; the number of lung segments involved; pulmonary fibrosis survival symptoms (absolute changes in dyspnea score from baseline within 28 days from diagnosis); pulmonary fibrosis survival symptoms (absolute change in cough score within 28 days from diagnosis); fever; need for supplemental oxygen; arterial blood gas analyses (ABGs); sequential organ failure assessment (liver function tests: AST, ALT, Total bilirubin, and kidney - serum creatinine, eGFR, urea); recovery time over 28 days; time to SARS-CoV-2 RT-PCR negativity from a nasopharyngeal and/or oropharyngeal swab; self-rating depression scale out to 28 days; fibrotic and inflammatory biomarkers (G-CSF, MIG, FGF-2, IL-4, IL-10, lymphotoxin-α/TNF-β, leptin, IL-6, IL-1β, TNF-α, TGF-β1, MMP-9, TIMP-1, MCP-1); C-reactive protein, presepsin, procalcitonin, ferritin; and lymphocyte count

This study will be conducted in patients who test positive for COVID-19 and will be conducted at multiple study centres. This is a randomized, double-blind, placebo-controlled, dose design to assess the safety, tolerability and survivability following test article administration of either LYT-100 or placebo (7:1 ratio active treatment vs. placebo) administered with food. Cohorts are planned with varying severity of COVID-19. Planned cohorts are as follows in **Table 24.**

**Table 24. Dosing Regimen**

| Cohort | Number of Participants receiving LYT-100 | | | | Number of Participants receiving Placebo |
|---|---|---|---|---|---|
| | Single doses are as follows: | | | | |
| | 250 mg=1 capsule/500 mg=2 capsules/750 mg=3 capsules | | | | |
| | *LYT-100 250 mg BID X 1 week | *LYT-100 500 mg BID X 1 week | *LYT-100 750 mg BID X 1 week | *+LYT-100 750 mg TID X 1 week, or maintain prior dose | Matching Placebo |
| | | | | | • 1 capsule BID at Week 1 |
| | | | | | • 2 capsules BID at Week 2 |
| | | | | | • +3 capsules TID at Week 3 |
| 1 | Up to 100 patients | | | | 15 patients |
| Mild COVID-19 | | | | | |
| 2 | Up to 100 patients | | | | 15 patients |
| Moderate COVID-19 | | | | | |
| 3 | Up to 100 patients | | 15 patients | | |
| Severe COVID-19 | | | | | |
| TOTAL | 300 patients | | 45 patients | | |
| *All patients will be titrated on LYT-100 with food as follows: | | | | | |
| Week 1: 250 mg BID X 7-days | | | | | |
| Week 2: 500 mg BID X 7-days | | | | | |
| Week 3: 750 mg BID X 7-days | | | | | |
| +Week 4: 750 mg TID X 7-days, if tolerated dosing at Week 3, otherwise maintain dosing at 750 mg BID; *options are allowed to reduce dosing further to achieve optimal tolerability* | | | | | |
| Up to 345 patients will be enrolled in the United States, Italy and China. | | | | | |

During the treatment period (Day 1 and up to 28 days), participants will be administered LYT-100 medication BID and up to TID (with water), and preferably with food, approximately 12 h apart.

Following screening and confirmation of eligibility, patients will begin their study drug medication, starting the titration with twice / day dosing of either LYT-100 study drug, 750 mg or placebo BID on a full stomach for up to 28 days.

Patients will be seen for Baseline visit on Day -1, On-treatment visits at Days 1, 4, 7, 10, 14, 21 and 28 (Week 4), and Post-treatment follow-up visits at Day 42 (Week 6) and Day 56 (Week 8).

All patients will be monitored for the following clinical efficacy and safety assessments of LYT-100 at time points delineated in the Schedule of Events as described herein. Monitoring will include Survivability (Respiratory disease related mortality); Pulmonary Function Tests (FEV1 and FVC) and respiratory rate; Pulse rate and arterial blood pressure; Chest X-ray; CT scan (with or without contrast); Biomarker assessments (T- cells, CD8 + T cells, IL-1ra, IL-1β, IL-2R, IL-4, IL-6, IL-8, IL-10, IFN-γ, MCP-3 and TNF-a, C-reactive protein, ferritin, procalcitonin, presepsin); Pharmacokinetics; and Safety (Adverse Events, Concomitant medication needs. Haematology panels - CBC with differential counts including leukocyte count, lymphocyte count, neutrophil count, platelet and eosinophil count , Biochemistry panels, Urinalysis panels, Physical Examinations, Vital Signs, ECGs , and Echocardiogram - Ejection Fraction).

Inclusion/exclusion criteria and Coronavirus Severity Scale are described in Example 5. All participants will be randomised 7:1 to receive either LYT-100 (deupirfenidone) formulated as powder in capsules, or placebo (a matching, inactive capsule containing methyl cellulose). Patients will be treated up to 28 consecutive days. Follow up of 28 days post last dose will be implemented.

Evaluation will follow parameters essentially as described in Example 5. In brief, criteria for Evaluation will include Pulmonary Function Tests (FEV, FVC) and Pharmacodynamic Biomarker assessments (T- cells CD8 + T cells, IL-1ra, IL-1β, IL-2R, IL-4, IL-6, IL-8, IL-10, IFN-γ, MCP-3, TNF-a) Additional markers include C-reactive protein, Ferritin, Procalcitonin, Presepsin. Safety Assessment will include Adverse Events, Concomitant medication needs, Haematology panels, CBC with differential counts, Leukocyte count, Lymphocyte count, Neutrophil count, Eosinophil count, Platelets, Biochemistry panels, Urinalysis panels, Physical Examinations, Vital Signs, ECG, and Echocardiogram to determine Ejection Fraction. Pharmacokinetics will be based on Blood samples (4 mL each) for population pharmacokinetics will be collected at specified times at any visit from Day 1 to Day 28 of the study. Each participant will provide up to a minimum of 1 sample at each time point in reference to dosing: Pre-dose; Post-dose times: to <2 h; 2 to <4 h; 4 to <8 h; 8 to 12 h, **Table 15** includes certain respiratory features can be recorded from chest imaging.

### Example 11: Treatment of Patients with COVID-19 Respiratory Disease.

This study is a phase 2 multi-center randomized, double-blind, parallel arm, placebo-controlled study being conducted to evaluate the safety and efficacy of LYT-100 compared to placebo in post-acute adult patients with post-acute COVID-19 respiratory complications who were treated with, but no longer require mechanical ventilation (MV), extracorporeal membrane oxygenation (ECMO), non-invasive ventilation (NIV) (i.e., continuous positive airway pressure (CPAP), bilevel positive airway pressure (BiPAP)), high-flow nasal oxygen therapy (HFNO), or a combination thereof, other high flow supplemental oxygen with FiO2 ≥35% (i.e., venturi or nonrebreather mask at flow rate ≥ 8 lpm) or combination thereof; and may or may not require regular nasal cannula O₂ supplementation at the time of screening/enrolment. This study will be conducted in patients who test positive for COVID-19 and will aim to address issues in post-acute patients with COVID-19 infection. The study will be performed in approximately 26 clinical sites in Europe, the US, and Asia. Other regions may be added as the pandemic mandates.

After informed consent is obtained, patients will enter a screening phase for no more than 5 days, to determine eligibility. Patients are required to have all screening assessments, including a training session for the 6-minute walk test (6MWT), and meet all inclusion/exclusion criteria for the trial prior to randomization. Up to 168 eligible patients who no longer require mechanical ventilation, extracorporeal membrane oxygenation, non-invasive ventilation, continuous positive airway pressure or high-flow nasal oxygen therapy for at least 72 hours prior to the screening visit, will be randomized to receive LYT-100 or placebo, in a ratio of 1:1, respectively, in conjunction with institutional standard of care for COVID-19. Two 6MWT will be performed at baseline, with the longest distance recorded for the baseline measure. These tests can take place on Days -1 and/or 1, prior to dosing.

Patients randomized will be assigned to stratification levels as shown in **Table 25.** The primary analysis will be based on all data. Subgroup analysis may be performed as the data warrant, although the sample size for subgroups may not be sufficient for definitive conclusions to be drawn.

**Table 25: Baseline Randomization Stratification Levels**

| | | | | | |
|---|---|---|---|---|---|
| **6MWT 10 to <100 m** | | **6MWT 100 to 200 m** | | **6MWT >200 m** | |
| **Prior Mechanical Ventilation/Prior ECMO Status** | | **Prior Mechanical Ventilation/Prior ECMO Status** | | **Prior Mechanical Ventilation/Prior ECMO Status** | |
| **Yes** | **No** | **Yes** | **No** | **Yes** | **No** |
| **Age ≥ 65** | | **Age ≥ 65** | | **Age ≥ 65** | |
| **Yes** | **No** | **Yes** | **No** | **Yes** | **No** |

Standard of care will be administered as per investigator's medical judgement, based on the severity of the disease, and according to local government and/or institutional guidelines. LYT-100 or placebo will be administered daily for up to 91 days. Patients will start participation in the study while either hospitalized, or at time of hospital discharge / post-discharge from hospital for COVID-19 respiratory disease.

The timing of randomization is dependent on type of qualifying respiratory support and date of initial confirmed COVID-19 diagnosis, as described below in **Table 26.**

**Table 26. Timing of Randomization Relative to COVID-19 Diagnosis and Qualifying Respiratory Support**

| **Qualifying Respiratory Support** | **Day of Randomization Relative to:** | |
|---|---|---|
| | **Last Day of Qualifying Respiratory Support** | **Initial Confirmed Diagnosis of COVID-19** |
| NIV, HFNO or other High Flow Oxygen | Within 42 days | Within 56 days |
| ECMO or MV | Within 42 days | Within 90 days |

Treatment assignments will remain blinded for the duration of the study to the patient, study staff, and sponsor. Patients will be seen either on the hospital ward and/or in the ICU daily during hospital stay, or at discharge, a rehabilitation ward, an outpatient clinic, or the patient's home post-discharge. Note that for patients who were hospitalized and are discharged during the study, these patients will continue with study visits according to the Schedule of Events. Study follow-up visits will occur at Days 14, 28, 56, and 91, as well as 14-days post-completion of study treatment.

COVID-19 respiratory disease is a multi-faceted indication, and thus this clinical study includes a number of objectives (and corresponding endpoints) to adequately describe the effects of study treatment. To that end, there are primary and key secondary objectives that form the basis for study success, while other secondary objectives and exploratory objectives will inform overall conclusions and allow for assessment of a preponderance of evidence as to the benefit of LYT-100 in this population.

### Primary Objective

A primary objective of the study is to evaluate the effect of LYT-100 on the change in distance walked on the six-minute walk test (6MWT) performed in line with the American Thoracic Society/European Respiratory Society Guidelines.

### Key Secondary Objectives

The key Secondary Objective is to evaluate the effect of 91 days of treatment with LYT-100 compared to placebo on the Modified Borg Dyspnea Scale (mBDS).

### Other Secondary Objectives

To evaluate the effect of 91 days of treatment with LYT-100 compared to placebo on:
(1) Saint George Respiratory Questionnaire-I (SGRQ-I)
(2) Dyspnoea-12
(3) The WHO Ordinal Scale for Clinical Improvement
(4) Quality of Life assessment as collected using the SF-36 (v2), including 8 domains and 2 component scores (SF-36)
(5) Number of lung segments involved using CT Scan and QLF assessment
(6) Inflammatory, fibrosis and other biomarkers
(7) Development of an acute respiratory exacerbation, i.e., severe respiratory failure requiring MV, ECMO, NIV, CPAP and/or HFNO; or pulmonary exacerbations, classified as severe and nonsevere
(8) Severity of lung lobe(s) involvement, using CT Scan
(9) Pulse oximetry
(10) Chest X-ray
(11) Length of hospital stay
(12) All-cause mortality
(13) Use of supplemental oxygen from Baseline to hospital discharge and number of days that continue post-discharge
(14) All-cause rehospitalizaton

### Exploratory Objectives

Exploratory Objectives are to evaluate the effect of 91 days of treatment with LYT-100 compared to placebo on:
(1) Severity of lung lobe involvement using CT scan;
(2) oxygen saturation (SpO2) as determined by pulse oximetry;
(3) Chest X-ray;
(4) Length of hospital stay;
(5) All-cause mortality;
(6) Use of supplemental oxygen from Baseline to hospital discharge and number of days that continue post-discharge;
(7) Use of intubation and mechanical ventilation from Baseline to hospital discharge and number of days that continue post-discharge;
(8) Need for continuous positive airway pressure (CPAP), extracorporeal membrane oxygenation (ECMO) or mechanical ventilation (MV) during hospitalization;
(9) Number of days in the intensive care unit during hospital stay;
(10) Days from original COVID-19 diagnosis by PCR to discharge from hospital; and
(11) Date of the first negative COVID-19 PCR test.

### Safety Objective:

To evaluate the safety and tolerability of LYT-100 up to 91 days of treatment and 14-day follow-up post-study drug treatment.

### Pharmacokinetic Objectives:

To evaluate the plasma concentration vs. time data via population pharmacokinetic analysis of LYT-100 and its metabolite(s). Blood samples for population pharmacokinetics will be collected at Study Days 14, 28, 56, and 91. When pharmacokinetic levels are collected, the last dose of study medication taken prior to blood collection will be recorded, including the date and the time. Each participant will provide up to a minimum of 1 sample at each time point in reference to dosing: Pre-dose, 1 to <2 h, 2 to <4 h, 4 to <8 h, 8 to 12 h. Sites should avoid on-treatment duplications of the same time point interval within the same patient. The following is an example of blood collection options:
- Day 14 (4 to <8 h sample collected)
- Day 28 (pre-dose sample collected; patient should bring medication with them to the visit)
- Day 56 (1 to <2 h, 2 to <4 h sample, can collect two time points at a visit if patient plans to be at the site for extended time)
- Day 91 (8 to 12 h, again patient should bring medication with them to the visit if next dose is due shortly after the blood collection).
For trough or later time point intervals such as 8 to 12 hours, the patient should be instructed to hold their next study medication dose and bring it with them to their study visit(s) to accommodate the longer time intervals required from their last dose taken.

### Study Drug Dosage and Mode of Administration:

Patients will receive LYT-100 (deupirfenidone) formulated as powder in 250 mg capsules or matching placebo. Dosing will be provided as in **Table 27.** An initial dosage of 500 mg BID will be given the first 3 days of dosing, followed by titration up to 750 mg BID thereafter. Participants will take LYT-100 study medication, or placebo, orally with or without food (preferably with food, (solid or nutritional supplements, whenever possible), with approximately 10 to 12 hours between the two daily doses.

**Table 27: Dosing Regimens for Days 1 through 3 and Thereafter**

| Day 1 to Day 3 | Day 4 through Day 91 |
|---|---|
| LYT-100 500 mg BID or matching Placebo x 3 days | LYT-100 750 mg BID or matching Placebo x 88 days |

Doses may be adjusted according to safety and tolerability to avoid toxicity by adjusting to lower doses in response to patient safety and tolerability issues. If dosing titration is not well tolerated, adjustments to dosing may be made as follows: reduction to 250 mg, BID X 2 days (may be longer if needed), 500 mg, BID X 2 days (may be longer if needed), 750 mg, BID thereafter. Alternative dosing schedules for higher (2000 mg/day) dosing are provided in **Table 28.**

**Table 28: Alternative Dosing Regimens for Days 1 through 3, Days 4 through 7, and Thereafter**

| Day 1 to Day 3 | Day 4 through Day 7 | Day 8 through Day 91 |
|---|---|---|
| LYT-100 500 mg BID or matching Placebo | LYT-100 750 mg BID or matching Placebo | LYT-100 1000 mg BID or matching Placebo |
| LYT-100 500 mg BID or matching Placebo | LYT-100 1000 mg BID or matching Placebo | LYT-100 1000 mg BID or matching Placebo |

If dosing titration is not well tolerated, adjustments to dosing may be made as follows: reduction to 250 mg. BID X 2 days (may be longer if needed), 500 mg, BID X 2 days (may be longer if needed), 750 mg, BID X 2 days (may be longer if needed), and 1000 mg, BID thereafter.

All patients will be monitored following screening for completion of clinical efficacy and safety assessments of LYT-100 at time points delineated herein.

### Number of Participants (Planned):

Up to 168 patients with post-acute, documented COVID-19 will be randomized to receive LYT-100 or placebo in a ratio of 1:1, respectively.

### Inclusion criteria:

Inclusion criteria include:
(1) Positive result of a validated molecular RT-PCR diagnostic test or SARS-CoV-2 RNA result from a clinical specimen deemed clinically associated with the current episode of illness, warranting hospital admission as per investigator's judgement, or previously hospitalized (central and/or local laboratory COVID-19 tests are accepted from any biological material source).
(2) Hospitalization for COVID -19 respiratory disease for at least 1-day and requiring at least one of the listed treatment modalities for at least 24 hours: MV, ECMO, NIV, HFNO, other high flow oxygen with FiO2 ≥35% and flow rates ≥ 81pm during hospitalization.
(3) Male or female, aged ≥18 to ≤80 years of age, inclusively at the time of informed consent.
(4) Able to bear weight and ambulate a minimum of 10 m distance (use of inhaled oxygen permitted).
(5) Enrollment within 42 days from initial confirmed diagnosis of COVID-19
(6) COVID-19 pneumonia on imaging (chest X-ray or CT Scan) with a minimum of two lobe involvement.
(7) Shortness of breath ≥ grade 3 on mBDS dyspnea scale
(8) Laboratory parameters (CBC, biochemistry) deemed by the investigator as acceptable to enroll the patient.
(9) Patients can be on other medication (on and off label), apart from the ones specifically outlined in the Exclusion Criteria, to treat COVID-19 respiratory disease, that the investigator deems clinically relevant in combination with the study drug
(10) Patients are allowed to concomitantly receive any standard of care as per investigators' clinical decision; standard of care may encompass any approved, on or off label medication apart from the ones specifically outlined in the exclusion criteria. Concomitant medication may be other investigational agents apart from the ones specifically outlined in the Exclusion Criteria.

### Exclusion Criteria

Excluded from this study are the following:
(1) Patients with pre-existing chronic respiratory condition(s), obstructive or restrictive, for which the patient is actively taking concomitant medication are excluded. Patients with history of Idiopathic Pulmonary Fibrosis (IPF), lung cancer, pulmonary arterial hypertension, other interstitial lung diseases, severe cardiac insufficiency (grade IV) are excluded irrespective of whether they are actively being medicated for those conditions or not.
(2) Pre-existing co-morbid conditions preventing outcome assessments, e.g., neurological, medical, orthopedic injury/disability, disease or condition that would prevent inability to transfer and walk for 6 minutes, prior to confirmed COVID-19 diagnosis.
(3) Co-morbid diagnosis of tuberculosis.
(4) Unstable angina or myocardial infarction in the last month prior to screening.
(5) Pre-existing co-morbidity associated with life expectancy less than 1 year.
(6) Present life expectancy less than 3 days and/or lactate dehydrogenase (LDH) > 360.
(7) Body mass index ≥ 40 kg/m².
(8) Resting heart rate of ≥ 120 bpm, systolic blood pressure of > 180 mm Hg and a diastolic blood pressure of > 100 mm Hg and/or or a fall in pulse oximetry oxygen, saturation (SpO₂) to <80% upon ambulation.
(9) Patients on MV, ECMO, NIV, and/or HFNO or other high flow supplemental oxygen FiO2 ≥greater than or equal to 35% and ≥8 lpm within the last 72 hours prior to screening.
(10) Sepsis, septic/hypovolemic/cardiogenic/neurogenic shock.
(11) History of anaphylactic reaction (particularly reactions to general anaesthetic agents; allergic reaction due to any drug which led to significant morbidity; prior allergic reaction to pirfenidone or LYT-100.
(12) Known symptoms of dysphagia or known difficulty in swallowing capsules and/or total gastrectomy.
(13) Patients undergoing dialysis or with a history of dialysis, or anticipated to require dialysis within 72 hours of screening.
(14) Use of any of the following drugs (a.) fluvoxamine, enoxacin, ciprofloxacin; (b.) Treatment with steroids under institutional protocols and guidelines to manage COVID-19 pneumonia/respiratory symptoms is allowed. Use of steroids off-label to treat non-COVID-19 related conditions is not allowed; (c.) any Investigational agents; (d.) other inhibitors of CYP1A2 (including but not limited to ciprofloxacin, grapefruit, enoxacin, methoxsalen or mexiletine); (e.) inducers of CYP1A2 (such as phenytoin or nicotine), CYP2C9 or 2C19 (including but not limited to carbamazepine or rifampicin); (f.) drugs associated with substantial risk for prolongation of the QTc interval (including but not limited to moxifloxacin, quinidine, procainamide, amiodarone, sotalol). QTc prolongation is not an all/nothing drug effect, and specifically the administration of hydroxychloroquine does not preclude participation in this trial but does mandate measurement of the QTc every 6 hours. until deemed necessary in accordance with investigator judgement; and (g.) Immunosuppressants or other immune-modifying drugs are not permitted, except for corticosteroids at the discretion of the Investigator as part of standard of care for COVID-19 respiratory pathology, and remdesivir where it is provided as part of standard of care for the treatment of COVID-19. The use of dexamethasone for COVID-19 respiratory disease was supported by the recent RECOVERY trial (Horby et al 2020). Dexamethasone may be administered up to a total daily dose of 6 mg either by oral or intravenous routes for up to 10 days or until hospital discharge, whichever comes first. If the use of other corticosteroids in place of dexamethasone is also part of local standard of care, the following drugs may be administered in the place of dexamethasone up to 3 weeks post discharge from the ICU: prednisone (1 mg/kg/day), methylprednisolone (1/mg/kg/day), or hydrocortisone (160 mg). Any significantly immune-suppressing drug used off-label, including experimental therapies as part of a clinical trial must be discontinued at least 3 months prior to screening or 5 half-lives, whichever is longer.
(15) Vaccination with a live vaccine is not permitted during the period from 4 weeks prior to screening to 4 weeks after the last dose.
(16) For baseline AST ≥2.5xULN, ALT ≥2.5xULN and/or total bilirubin >1.5xULN (in the absence of history of Gilbert syndrome and/or the absence of alkaline phosphatase ≥2xULN or clinical evidence of cholestasis), or severe hepatic impairment.
(17) Patients requiring surgery due to an acute onset condition prior to enrolment/randomization.
(18) Use of tobacco and nicotine products and/or a positive cotinine test at screening.
(19) Pirfenidone or nintedanib
(20) Patients experiencing tachypnea. For the purposes of this study, tachypnea is defined as a resting respiratory rate ≥ 25 per minute.

### Duration of Study and Study Treatment

Over two days, patients will be screened and complete baseline assessments. Randomization occurs on the day following the Baseline Visit/assessment, i.e., shortly prior to the first dose of double-blind study medication.

All patients will be treated up to 91 consecutive days. Follow-up of study assessment post-randomization is at Days 14, 28, 56 and Day 91.

Note that for patients who were hospitalized and are discharged during the study, these patients will continue with study visits according to the Schedule of Events.

A follow-up post-treatment completion visit will occur within 14 days of last day of study treatment. The total duration of the study for each participant is up to 113 days, including screening and baseline of 5 days, treatment period of 91 days and off-treatment safety follow-up of 14 days including 3-day study window for last visit.

### Efficacy Endpoints

The efficacy endpoints are consistent with the primary and secondary objectives of the study. All analyses will be performed at each protocol-scheduled time point (i.e., Days 14, 28, 56, and 91). It is possible the effect of LYT-100 may increase over time, and thus the effect (benefit) could be greater at Day 91 than earlier time points. The effect-time profile for each endpoint is of primary interest and will be tabulated.

### Primary efficacy endpoint

The 6-minute walk test (6MWT) is the primary efficacy endpoint. All patients will complete a 6MWT test according to the Schedule of Assessments and aligned with the American Thoracic Society/European Respiratory Society Guidelines with COVID-19 modifications provided in the 6MWT study procedural manual. The 6MWT measures the distance a patient can quickly walk on a flat, hard surface in a period of 6 minutes. It evaluates the global and integrated response of all the systems involved during exercise. This is a self-paced test. Patients choose their own intensity of exercise and may stop and rest during the test. Changes from baseline in the 6MWT distance will be assessed using covariates in a mixed model. Dyspnea and fatigue are also assessed prior to the 6MWT and end of the test.

### Key Secondary efficacy endpoints

The Modified Borg Dyspnea Questionnaire (mBDQ) is a measure of dyspnea. The Modified Borg Dyspnea Scale (mBDS) is a 0 to 10 rated numerical score used to measure dyspnea as reported by the patient during submaximal exercise and is routinely administered during six-minute walk testing (6MWT). The mBDS is a self-administered unidimensional assessment tool that analyzes breathlessness under exertion. Improvements in the Borg (where 0 indicates no shortness of breath and 10 indicates maximal shortness of breath) may suggest improvement in dyspnea. Changes over time will be derived.

### Other Secondary and Exploratory Efficacy Endpoints

The Saint George Respiratory Questionnaire-I (SGRQ-I) is an idiopathic pulmonary fibrosis disease-specific instrument designed to measure impact on overall health, daily life, and perceived well-being in patients with interstitial lung disease. There are 34 self-completed items with 3 domain component scores (Symptoms, Activities, and Impacts). Higher scores indicate more limitations. Changes from baseline in the component scores of the SGRQ-I will be assessed as secondary efficacy endpoint. Further, a responder analysis will be performed in which the proportion of patients experiencing an increase of ≥4 units (vs < 4 units) will be assessed.

The Dyspnoea-12 (D-12) is an instrument used to assess the effect of dyspnea in interstitial lung disease and COPD, and is used in reference to the patient's breathlessness experience "these days", i.e., current at baseline and at follow-up visits, and unlike other dyspnea tools, it has no reference to activity. The tool has 12 descriptor items ranging from none (0), mild (1), moderate (2) or severe (3). An overall score of breathlessness severity is provided that encompasses seven physical items and five affective items. Total scores range from 1 to 36 whereby the higher score correlates with greater severity

The WHO Ordinal Scale for Clinical Improvement will be assessed for changes from baseline to improvement over time (e.g., of ≥ one point). as well as a responder analysis (improvement by at least 1 point and also for at least 2 points). For example, the WHO Ordinal Scale for Clinical Improvement will be assessed for changes over time for varying response point thresholds (1-point change, 2-point change), and will also be analyzed on a continuum. This ordinal scale is a nine-category ordinal scale with the following categories: (0) Uninfected (No clinical or virological evidence of infection); (1) Ambulatory (No limitation of activities); (3) Hospitalized Mild Disease (Hospitalized, no oxygen therapy); (4) Oxygen by mask or nasal prongs; (5) Hospitalized Severe Disease (Non-invasive ventilation or high-flow oxygen; (6) Intubation and mechanical ventilation; (7) Ventilation + additional organ support - pressor, RRT, ECMO; (8) Dead (Death).

The SF-36 (v2) is a self-administered questionnaire containing 36 items that measure functional status, well-being and overall evaluation of health in 8 domains. It requires approximately 5-10 minutes to complete and uses scaled, ordinal responses (e.g., All of the time, Most of the time, A good bit of the time, etc.) Changes in the standardized component scores and domain scores will be calculated The domain scores and component summaries are as follows:
- SF-36 v2 scaled domains
- Vitality
- Physical functioning
- Bodily pain
- General health perception
- Physical role functioning
- Emotional role functioning
- Social role functioning
- Mental health
- SF-36 v2 Mental Component Summary
- SF-36 v2 Physical Component Summary

Changes in the standardized component scores and domain scores will be calculated. The SF-36 will be assessed via analysis of each of the 8 domains as well as the 2 component scores. The outcomes will be used to allow for a more thorough understanding of the treatment benefit and may be explored via correlation analyses with the 6MWT and other endpoints, as the data warrant.

Qualitative assessments will be collected from chest CT Scans without contrast. The proportion of patients with 'normal' chest X-ray or CT scan images will be defined in a responder analysis. Patients with pre-existing chest pathology pre-COVID-19 should be considered 'normal' for those pre-existing items/findings. Chest CT scans will be performed according to the CT Scan manual. Lung lobe involvement will be assessed for each of five lobes via the central read with the terms defined by the Fleischner Society (Hanscll et al 2018). The CT images will be assessed for the presence of ground-glass opacity (hazy areas of increased attenuation without obscuration of the underlying vasculature), consolidation (homogeneous opacification with obscuration of the underlying vasculature), reticular pattern (consisting of either coarse linear or curvilinear opacity or fine subpleural reticulation without substantial ground-glass opacity), mixed pattern (combination of consolidation, ground-glass opacity, and reticular opacity in the presence of architectural distortion), and honeycomb pattern (clustered cystic air spaces). The distribution of abnormalities will be classified as predominantly subpleural (involving mainly the peripheral one-third of the lung), central (involving mainly the central two-third of the lung), or diffuse. CT scans may also be analyzed with semi-Quantitative Lung Fibrosis (QLF) QLF added and Computer-Aided Diagnosis (CAD) QLF Scores (Kim et al, 2010). Scans will be centrally read for these parameters. The total score for each of the four findings per lobe (range 0 to 25), each finding across the five lobes (range 0-25) and total score for the whole lung for all findings together (0-100) will be derived accordingly. Changes from baseline in this total score will be assessed. A responder analyses will be performed, in which the proportion of patients showing pulmonary inflammation, fibrosis, ground glass opacities, fibrosis, consolidation, reticulation/bronchiectasis, and other radiographic abnormality improvement(s) by determining at least a 2-point score improvement for each abnormality, across the five lobes (range 0-25) will be derived. For each of the five lobes, an assessment of lung lobe involvement using CT Scan will be assessed. Shifts in these characteristics over the course of treatment will be of interest.

Changes in biomarkers will be assessed, including C-reactive protein (CRP), lymphocyte count, d-dimer, cardiac troponin, ferritin, lactate dehydrogenase (LDH), 1L-6, TGF-β1, TNF-α, IL-1β, PDGF- β, GCSF, and VEGF. Correlation of changes in biomarkers with clinical endpoints (e.g., 6MWT, mBDS) will be assessed where relevant in an exploratory fashion.

The proportion of patients with 'normal' chest X-ray will be defined in a responder analysis. Patients with pre-existing chest pathology pre-COVID-19 should be considered 'normal' for those pre-existing items/findings.

Pulse oximetry will be performed at rest and changes over time will be determined.

Number of hospital stay days and time to all-cause mortality will be assessed. Date of (original) admission, date of (original) discharge and rehospitalization(s) and subsequent discharge(s) will be collected.

Other clinical outcomes will be assessed, as follows:
Use of supplemental oxygen from Baseline to hospital discharge and number of days that continue post-discharge.

Percentage of patients who develop severe respiratory failure requiring mechanical ventilation (MV), extracorporeal membrane oxygenation (ECMO), NIV, continuous positive airway pressure (CPAP) and/or high flow oxygen via nasal cannula (HFNO).

Percentage of patients who develop respiratory exacerbations, classified as either severe or non-severe.

Percentage of patients who experience rehospitalization for any cause, while on treatment.

Use of supplemental oxygen from Baseline to end of treatment.

The number of days of in the intensive care unit during the hospital stay.

The number of days before discharge from hospital was the patient originally diagnosed with COVID-19 by PCR.

The data of the first negative COVID-19 PCR test.

### Safety Endpoints

Safety and tolerability endpoints include: Adverse events, concomitant medications, clinical laboratory findings (chemistry, hematology, coagulation, urinalysis), physical examinations, ECGs, and vital signs. These will be tabulated or summarized descriptively, where appropriate. Outcomes such as mortality and duration of hospital stay are considered efficacy outcomes in the context of the study objectives and disease being studied but will be included in the overall discussion of the safety and tolerability of LYT-100.

### Population PK

Using plasma concentration versus time data, population pharmacokinetic analysis will be performed to assess the exposure-response characteristics of LYT-100 and its metabolite(s).

### Statistical Methods

### Randomization Stratification:

Up to 168 patients will be randomized to receive LYT-100 or placebo in a 1:1 ratio. The randomization will be stratified by 6MWT baseline and risk factors (Table 25). A sample size reestimation (SSRE) may be performed by an unblinded statistician when approximately 50 patients have completed through Day 91. The conditional power of the study to detect a statistically significant difference for the primary efficacy endpoint may be estimated, and the sample size may be increased according to the observed conditional power. A detailed DSMB charter will include details of the safety monitoring and this SSRE, including the thresholds for sample size increases for the different categories of CP (e.g., 20% to 50%, 50% to 70%).

### Sample Size

The study will utilize a suite of endpoints to measure patient clinical function and health-related quality of life. The sample size was based on both the 6MWT (a measure of patient clinical function) and the mBDS, a measure of patient HRQoL.

The target sample size is based on the hypothesis that LYT-100 versus the placebo control group will significantly improve the "90-day time-distance profile" as measured via changes from baseline in the 6MWT distance walked as collected at multiple on-treatment time points. Because this study is being performed in patients with active COVID-19, there may be limitations at investigational centers to facilitate collection of some data points. Thus, this study assumes that missing data may occur due to COVID-19 (e.g., patients may be unable to travel due to COVID restrictions) and thus may lead to missing data. The study has been powered accordingly.

Specifically, group sample sizes of 84 per arm (total = 168) achieve 85% power to detect a difference of 30 meters in the change from baseline in the 6MWT, using a mixed model for repeated measures (MMRM) with at least 2 time points, an autoregressive covariance structure, an assumed standard deviation of 75 meters, estimated correlation between observations on the same subject of 0.50, and an alpha of 5%.

The sample size above assumes the difference of 30 meters change from baseline is observed at each time point measured, which may or may not necessarily reflect the actual time course of treatment benefit (response profile over time) in the target population. Due to the unknown time-distance response profile, and as there is no single time point of primary interest (rather, it is the full 90-day time-distance profile that is of primary interest), the treatment benefit at each measured time point will also be tested independently.

### Study Populations

The Safety Population is defined as all randomized patients who received at least one dose of study treatment (LYT-100). All safety endpoints will be assessed using this population. Patients will be analyzed according to the treatment they actually received, regardless of which treatment they were randomized to.

The Intent to Treat (ITT) population is defined as all randomized participants. All efficacy endpoints will be assessed using this population, with primary conclusions regarding study drug effect be based on the ITT population outcomes. Patients will be analyzed according to the treatment group they were randomized to, regardless of which treatment they actually received.

The Full Analysis (FAS) population is defined as all randomized participants who received at least one dose of LYT-100 and had at least one post-baseline assessment for the 6-minute walk test. All efficacy endpoints will be assessed using this population.

The Per-Protocol (PP) population is defined as all randomized patients who completed 91 days of treatment with at least 80% compliance and have at least one post-baseline 6MWT outcome. Patients who died or discontinued due to TEAE will be included in this population.

The Pharmacokinetic (PK) Population is defined as all participants who receive LYT-100 and provide at least one plasma concentration measure. This population will be determined by the study pharmacokineticist. A population PK analysis describing the exposure-response characteristics will be performed. As this study includes sparse sampling, PK data from prior clinical trials may be used to develop the PopPK model accordingly. Note that pre-dose concentrations at Baseline are assumed to be zero.

### Study Treatment Period

Over two to five days, patients will be screened and complete baseline assessments. Randomization (Day 1) occurs on the day following the Baseline Visit/assessment.

All patients will be treated up to 91 consecutive days during the study treatment period. Study assessments are at Days 14, 28, 56 and Day 91 ± 3 days.

A follow-up post-treatment completion visit will occur within 14 days of last day of study treatment ± 3 days.

The total duration of the study for each participant is up to 113 days, including screening and baseline of 2 to 5 days, treatment period of 91 days and off-treatment safety follow-up of 14 days including 3-day study window for last visit.

Where possible, assessments should be conducted in order of least invasive to most invasive. Study manuals will be described for certain endpoints (e.g., 6MWT, Chest x-ray, CT Scans) that will define how procedures will be performed. The sections below describe the general procedures to be performed each day.

In general, patient-reported quality of life scales (e.g., SF-36 v2, mBDS, SGRQ-I) should be performed at the beginning of each visit, with the remaining procedures (e.g., blood collections, 6MWT) performed after.

Throughout patient participation, patients should be warned to avoid exposure to sunlight and avoid sunlamps. They should be encouraged to wear sunscreen and protective clothing on a daily basis to avoid potential for skin reactions.

Throughout the conduct of the study, patients and study staff should take appropriate precautions to mitigate or minimize infection risks. This includes the use of personal protective equipment (masks, gowns, gloves) and hand washing, as appropriate for the situation.

### Screening (Day -2 to -5)

Over two to five days, patients will be screened and complete baseline assessments. Randomization occurs on the day following the Baseline Visit/assessment. Patients are required to have all screening assessments, including training for the 6-minute walk test, and meet all inclusion/exclusion criteria for the trial prior to randomization.

At Screening, participants will be provided with full information concerning details of the study assessments and procedures. They will also be provided with an informed consent form (ICF). Prior to being asked to sign the consent form, participants will be given time to review study information and ask any questions.

After the Informed Consent Form is obtained (written, electronic, or oral), screening assessments will be carried out as follows:
- RT-PCR diagnostic test / SARS-CoV-2 RNA (may predate the screening period)
- Eligibility - inclusion/exclusion criteria
- Recording of demographic information
- Pertinent Medical History
- Physical examination
- Sequential Organ Failure Assessment (SOFA) should be included in any physical
- exam where indicated on the Schedule of Events. The SOFA score is a simple and objective
- score that allows for calculation of both the number and the severity of organ dysfunction in six organ systems (respiratory, coagulation, liver, cardiovascular, renal, and neurologic).
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 12-lead ECG (after participant has been resting in a supine position for at least 5 minutes and prior to blood draws and assessment of vital signs)
- Chest x-ray
- Modified Borg Dyspnoca Scale (mBDS) - conduct this at beginning of visit
- Confirmation of prior requirement for NIV, ECMO, MV, and/or HFNO during hospitalization, with current need ruled out
- Laboratory testing (hematology, biochemistry, coagulation, urinalysis, urine cotinine, and FSH (for post-menopausal females only)). A urine cotinine test will be performed at screening, Day 28, and Day 91 to measure the amount of cotinine in urine for patients exposed to nicotine.
- Urine pregnancy test (WOCBP only)
- Pre-treatment adverse events
- Prior and concomitant medications - include medications 30 days prior to screening.

Note: AEs and changes in concomitant medications will be recorded from the time of informed consent. They will be recorded, and 'pre-treatment' will be noted for AEs and concomitant medications taken prior to first dose of study medication.

### Baseline (Day -1)

The following assessments will be carried out:
- Abbreviated symptom-directed physical examination
- Measurement of height and weight, and calculation of Body Mass Index
- Vital signs (blood pressure, heart rate, respiratory rate, SpO₂ (after participant has been resting in a supine position for at least 5 minutes and prior to blood draws), oral, contactless, or lympanic body temperature (same temperature measurement should be used throughout the study)

- Charlson Comorbidity Index
- Chest CT Scan (If a CT scan without contrast was obtained within 3 days of the baseline CT scan, and there have been no significant changes in signs and symptoms of respiratory complications in those 3 days, then an additional CT scan would not need to be collected. However, the prior CT scan must have been done without contrast).
- 6-minute Walk Test - Two tests must be conducted at baseline, with the longest distance recorded for the baseline measure. These tests can take place on Days -1 and/or 1, prior to dosing. There should be a minimum of 30 minutes between the two tests. The second test can also be performed on Day 1.
- Modified Borg Dyspnoea Scale - collect this at beginning of visit
- Dyspnoea-12- collect this at beginning of visit
- St. George's Respiratory Questionnaire-I (SGRQ-I) - collect this at beginning of visit
- WHO Ordinal Scale for Clinical Improvement
- SF-36 (v2)- collect this at beginning of visit
- Clinical Outcome Check (i.e. have any of the following been required since the last visit: HFNO, NIV, ECMO MV)
- Biomarkers - time of biomarker sample collection should be consistent across study days, occurring always either before noontime (morning) or after noontime (afternoon)
- Pre-treatment adverse events
- Concomitant medications.

### Study Treatment Period

The study treatment period includes visits at Day 1, 14, 28, 56, and 91.

### Day 1 (Day of First Dose of Study Medication)

Participants will be randomized prior to dosing on Day 1. Dosing should be performed AFTER all assessments below are performed.
Post-treatment vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
Post-treatment Modified Borg Dyspnoea Scale - collect this at beginning of visit
Study Drug Administration/Accountability - doses will be taken per day, approximately 10 to 12 hours apart and preferably with meals
Clinical Outcome Check (i.e. have any of the following been required since the last visit: HFNO, NIV, ECMO MV)
6-minute walk test (if a second test was not conducted on Day -1)
Adverse Events / Mortality
Concomitant medications.

### Day 14

Assessments will be conducted as follows:
- Abbreviated symptom-directed physical examination
- Sequential Organ Failure Assessment (SOFA)
- Weight and Body Mass Index calculation
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 12-lead ECG (after participant has been resting in a supine position for at least 5 minutes and prior to blood draws and assessment of vital signs)
- 6-minute Walk Test - conduct this at end of visit
- Modified Borg Dyspnoea Scale - collect this at beginning of visit
- Dyspnoea-12 - collect this at beginning of visit
- Laboratory testing (hematology, biochemistry, coagulation, urinalysis, urine cotinine)
- Clinical Outcome Check (i.e. have any of the following been required since the last visit: HFNO, NIV, ECMO MV)
- Biomarkers - time of biomarker sample collection should be consistent across study days, occurring always either before noontime (morning) or after noontime (afternoon)
- Study Drug Administration/Accountability - doses will be taken per day, approximately 10 to 12 hours apart and preferably with meals
- PK blood sample- collect last dose taken prior to blood collection; one PK is collected at visit and must be recorded at corresponding time interval: Hour 0, Hour 1 to <2, Hour 2 to <4, Hour 4 to <8, Hour 8 to <12. Avoid on-treatment duplications of the same time point interval within the same patient
- Adverse Events / Mortality
- Concomitant medications.

### Day 28

Assessments will be conducted as follows:
- Weight and calculation of Body Mass Index
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 6-minute Walk Test - conduct this at end of visit
- Modified Borg Dyspnoea Scale - collect this at beginning of visit
- Dyspnoea-12 - collect this at beginning of visit
- St. George's Respiratory Questionnaire-I (SGRQ-I) - collect this at beginning of visit
- WHO Ordinal Scale for Clinical Improvement
- SF-36 (v2)- collect this at beginning of visit
- Clinical Outcome Check (i.e. have any of the following been required since the last visit: HFNO, NIV. ECMO MV)
- Urine pregnancy test (WOCBP only)
- Urine Cotinine
- Biomarkers - time of biomarker sample collection should be consistent across study days, occurring always either before noontime (morning) or after noontime (afternoon)
- Study Drug Administration/Accountability - doses will be taken per day, approximately 10 to 12 hours apart and preferably with meals
- PK blood sample - collect last dose taken prior to blood collection; one PK is collected at visit and must be recorded at corresponding time interval: Hour 0, Hour 1 to <2, Hour 2 to <4, Hour 4 to <8, Hour 8 to <12. Avoid on-treatment duplications of the same time point interval within the same patient
- Adverse Events / Mortality
- Concomitant medications.

### Day 56

Assessments will be conducted as follows:
- Weight and calculation of Body Mass Index
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 6-minute Walk Test - conduct this at end of visit
- Modified Borg Dyspnoea Scale - collect this at beginning of visit
- Dyspnoea-12 - collect this at beginning of visit
- SF-36 (v2)- collect this at beginning of visit
- Clinical Outcome Check (i.e. have any of the following been required since the last visit: HFNO, NIV, ECMO MV)
- Laboratory testing (hematology, biochemistry, coagulation, urinalysis, urine cotinine)
- Urine pregnancy test (WOCBP only)
- Biomarkers - time of biomarker sample collection should be consistent across study days, occurring always either before noontime (morning) or after noontime (afternoon)
- Study Drug Administration/Accountability - doses will be taken per day, approximately 10 to 12 hours apart and preferably with meals
- Hour 0, Hour 1 to <2, Hour 2 to <4, Hour 4 to <8, Hour 8 to <12. Avoid on-treatment duplications of the same time point interval within the same patient
- Adverse Events / Mortality
- Concomitant medications.

### Day 91

Assessments will be conducted as follows:
- Abbreviated symptom-directed physical examination
- Sequential Organ Failure Assessment (SOFA)
- Weight and calculation of Body Mass Index
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 12-lead ECG (after participant has been resting in a supine position for at least 5 minutes and prior to blood draws and assessment of vital signs)
- Charlson Comorbidity Index
- Chest CT scan
- Chest x-ray
- 6-minute Walk Test - conduct this at end of visit
- Modified Borg Dyspnoea Scale - collect this at beginning of visit
- Dyspnoea-12 - collect this at beginning of visit
- St. George's Respiratory Questionnaire-I (SGRQ-I) - collect this at beginning of visit
- WHO Ordinal Scale for Clinical Improvement
- SF-36 (v2)- collect this at beginning of visit
- Clinical Outcome Check (i.e. have any of the following been required since the last visit: HFNO, NIV, ECMO MV)
- Clinical laboratory testing (hematology, biochemistry, coagulation, urinalysis, urine cotinine)
- Urine pregnancy test (WOCBP only)
- Biomarkers - time of biomarker sample collection should be consistent across study days, occurring always either before noontime (morning) or after noontime (afternoon)
- Study Drug Administration/Accountability - doses will be taken per day, approximately 10 to 12 hours apart and preferably with meals
- PK blood sample- collect last dose taken prior to blood collection; one PK is collected at visit and must be recorded at corresponding time interval: Hour 0, Hour 1 to <2. Hour 2 to <4, Hour 4 to <8, Hour 8 to <12. Avoid on-treatment duplications of the same time point interval within the same patient
- Adverse Event / Mortality
- Concomitant medications.

### Post-Treatment/Early Termination (ET) or Day 105 End of Study

End of study is when the last visit and last assessment(s) is conducted. Assessments will be conducted as follows:
- Abbreviated symptom-directed physical examination
- Sequential Organ Failure Assessment (SOFA)
- Weight and calculation of Body Mass Index
- Vital signs (blood pressure, heart rate, respiratory rate, SpO₂ (after participant has been resting in a supine position for at least 5 minutes and prior to blood draws), oral, contactless, or tympanic body temperature (same temperature measurement should be used throughout the study)
- 12-lead ECG (after participant has been resting in a supine position for at least 5 minutes and prior to blood draws and assessment of vital signs)
- Chest x-ray
- 6-minute Walk Test - conduct this at end of visit
- Modified Borg Dyspnoea Scale - collect this at beginning of visit
- Dyspnoea-12 - collect this at beginning of visit
- SF-36 (v2)- collect this at beginning of visit
- Clinical Outcome Check (i.e. have any of the following been required since the last visit: HFNO, NIV. ECMO MV)
- Biomarkers - time of biomarker sample collection should be consistent across study days, occurring always either before noontime (morning) or after noontime (afternoon)
- Laboratory testing (hematology, biochemistry, coagulation, urinalysis, urine cotinine)
- Urine pregnancy test (WOCBP only)
- PK blood sample- collect PK only if early terminated while on study treatment; collect last dose taken prior to blood collection; one PK is collected at visit and must be recorded at corresponding time interval: Hour 0, Hour 1 to <2, Hour 2 to <4, Hour 4 to <8, Hour 8 to <12. Avoid on-treatment duplications of the same time point interval within the same patient
- Adverse Events / Mortality
- Concomitant medications.
- Biochemistry parameters to be tested are:
   - C-reactive protein (CRP- collected with biomarkers)
   - Urea (U)
   - Creatinine (CREAT)
   - Total Bilirubin (BILI) and Direct Bilirubin (BILIDIR)
   - Urate (URATE)
   - Albumin (ALB)
   - Globulin (GLOBUL)
   - Alkaline Phosphatase (ALP)
   - Creatine phosphokinase (CPK)
   - Troponin 1 (TROP1- collected with biomarkers)
   - Aspartate Aminotransferase (AST)
   - Alanine Aminotransferase (ALT)
   - Gamma- glutamyl transpeptidase (GGT)
   - Glucose (GLU) (fasting labs only)
   - Sodium (NA)
   - Potassium (K)
   - Calcium (CA)
   - Chloride (CL)
   - Phosphate (PHOS)
   - Bicarbonate (BICARB)
   - Lactate dehydrogenase (LDH- collected with biomarkers)
   - Ferritin (collected with biomarkers)
   - Transforming Growth Factor Beta 1 (TGF-β1)
   - Tumor necrosis factor alpha (TNF-α)
   - Interleukin 6 (IL-6)
   - Interleukin 1 beta (IL-1β)
   - Platelet-derived growth factor- β (PDGF- β)
   - Granulocyte colony-stimulating factor (GCSF)
   - Vascular endothelial growth factor (VEGF) Coagulation parameters to be tested are:
   - INR
   - Prothrombin time
   - APTT
   - d-dimer (collected with biomarkers)
- Urinalysis. A urinalysis test (dipstick) will be performed for each participant. Urinary analysis will be performed at Screening and other times according to the study schedule. If abnormality is noted for protein, blood, nitrite or leukocyte esterase (and at the discretion of the Investigator) a microscopic examination of RBC, WBC, bacteria and casts will be performed.
- Macroscopic urinalysis parameters to be tested are:
   - pH (PH)
   - Specific Gravity (SPGRAV)
   - Protein (PROT)
   - Glucose (GLUC)
   - Ketones (KETONES)
   - Total Bilirubin (BILI)
   - Occult Blood (OCCBLD)
   - Nitrite (NITRITE)
   - Urobilinogen (UROBIL)
   - Leukocytes (WBC)
   - Cotinine presence
The present application is a divisional application based on European Patent Application No. 21766819.3 (PCT Application No PCT/IB2021/052145). The following numbered clauses, which correspond to the claims of PCT/IB2021/052145 as filed, form part of the present disclosure and in particular form further aspects of the invention.
1. A method of treating viral respiratory disease or infection, the method comprising administering to a subject in need thereof an effective amount of: 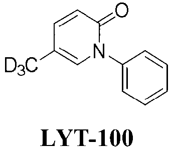 to the subject, wherein the treating is effective in preventing the development of, halting the progression of, or slowing the progression of one or more of lung fibrosis, respiratory complications of the viral respiratory disease or infection, respiratory symptoms of the viral respiratory disease or infection, or pulmonary dysfunction in the subject.
2. The method of clause 1, wherein the viral respiratory infection is caused by infection with human influenza virus H1N1, human influenza virus H7N9, SARS-CoV, SARS-CoV-2, MERS-CoV, HCoV-HKU1, or HCoV-OC43
3. The method of clause 1, wherein the viral respiratory disease is COVID-19 or a complication thereof.
4. The method of clause 3, wherein the complication is COVID-19 related pneumonia.
5. The method of clause 3, wherein the complication is COVID-19 related acute respiratory distress syndrome (ARDS).
6. The method according to any of the preceding clauses, wherein the treating is effective in preventing the development of, halting the progression of, or slowing the progression of pulmonary fibrosis.
7. The method according to any of the preceding clauses, wherein the subject exhibits reduced respiratory symptoms of the viral respiratory disease or infection, wherein the respiratory symptoms are cough, dyspnea, or both.
8. The method according to any of the preceding clauses, wherein the subject exhibits reduced evidence of one or more of lung fibrosis, respiratory complications, or pulmonary dysfunction relative to a subject who has not been treated with LYT-100, as measured by a chest X-ray or a CT scan, or as evidenced by a pulmonary function test result.
9. The method of clause 8, wherein the pulmonary function test result is a statistically significant improvement in the distance walked on the six-minute walk test (6MWT) compared to a baseline 6MWT result in the treated subject.
10. The method according to any of the preceding clauses, wherein treatment is initiated within 90 days from a confirmed COVID-19 diagnosis.
11. The method according to any of the preceding clauses, wherein treatment is initiated within 60 days from a confirmed viral respiratory disease diagnosis.
12. The method according to any of the preceding clauses, wherein treatment is initiated within 42 days from a confirmed viral respiratory disease diagnosis.
13. The method according to any of the preceding clauses comprising administering an effective amount of LYT-100 daily for up to 3 months.
14. The method according to any of the preceding clauses comprising administering an effective amount of LYT-100 daily for up to 12 months.
15. The method according to any of the preceding clauses, comprising daily administration of a total daily dose of between 200 mg and 2250 mg LYT-100.
16. The method according to any of the preceding clauses, comprising daily administration of a total daily dose of 1000 mg of LYT-100.
17. The method according to any of the preceding clauses, comprising daily administration of a total daily dose of 1500 mg of LYT-100.
18. The method according to any of the preceding clauses, comprising daily administration of a total daily dose of 2000 mg of LYT-100.
19. The method according to any of the preceding clauses, wherein the LYT-100 is administered without regard to food.
20. The method according to any of the preceding clauses, wherein the LYT-100 is administered without food.
21. The method according to any one of clauses 1-18, wherein the LYT-100 is administered with solid food or with a nutritional supplement.
22. The method according to any of the preceding clauses, wherein the LYT-100 is administered orally.
23. The method according to any of the preceding clauses, wherein the LYT-100 is administered in tablet or capsule form.
24. The method according to any of the preceding clauses, wherein the LYT-100 is formulated as powder in 250 mg capsules.
25. The method according to any of the preceding clauses, wherein daily administration is twice daily.
26. The method according to clause 24, wherein the LYT-100 is administered orally without food in two daily doses of 100 mg each.
27. The method according to clause 24, wherein the LYT-100 is administered orally without food in two daily doses of 250 mg each.
28. The method according to clause 24, wherein the LYT-100 is administered orally without food in two daily doses of 500 mg each.
29. The method according to clause 24, wherein the LYT-100 is administered orally without food in two daily doses of 750 mg each.
30. The method according to clause 24, wherein the LYT-100 is administered orally without food in two daily doses of 1000 mg each.
31. The method according to any one of clauses 1-23, wherein daily administration is three times daily.
32. The method according to clause 31, wherein the LYT-100 is administered orally without food in three daily doses of 100 mg each.
33. The method according to clause 31, wherein the LYT-100 is administered orally without food in three daily doses of 250 mg each.
34. The method according to clause 31, wherein the LYT-100 is administered orally without food in three daily doses of 500 mg each.
35. The method according to clause 31, wherein the LYT-100 is administered orally without food in three daily doses of 750 mg each.
36. The method according to any one of clauses 1-35, wherein the LYT-100 is administered without dose escalation.
37. The method according to any one of clauses 1-36, wherein administering the LYT-100 does not comprise an initial titration.
38. The method according to any one of clauses 1-25, wherein the LYT-100 is administered orally without food in two daily doses of 1000 mg each without dose escalation.
39. The method according to any one of clauses 1-25, wherein the LYT-100 is administered in two daily doses in an amount of 500 mg each on days 1 through 3, and in two daily doses in an amount of 750 mg each thereafter.
40. The method according to any one of clauses 1-25, comprising administering the LYT-100 in two daily doses in an amount of 250 mg each for two days; in two daily doses in an amount of 500 mg each for two days; and in two daily doses in an amount of 750 mg each thereafter.
41. The method according to any one of clauses 1-25, wherein the LYT-100 is administered in two daily doses in an amount of 500 mg each on days 1 through 3, and in two daily doses in an amount of 1000 mg each thereafter.
42. The method according to any one of clauses 1-25, wherein the LYT-100 is administered in two daily doses in an amount of 500 mg each on days 1 through 3, in two daily doses in an amount of 750 mg each on days 4 through 7, and in two daily doses in an amount of 1000 mg each thereafter.
43. The method according to any one of clauses 1-25, comprising administering the LYT-100 in two daily doses in an amount of 250 mg each for two days; in two daily doses in an amount of 500 mg each for two days; and in two daily doses in an amount of 1000 mg each thereafter.
44. The method according to any one of clauses 1-25, comprising administering the LYT-100 in two daily doses in an amount of 250 mg each for two days; in two daily doses in an amount of 500 mg each for two days; in two daily doses in an amount of 750 mg each for two days, and in two daily doses in an amount of 1000 mg each thereafter.
45. The method according to any one of the preceding clauses, wherein the LYT-100 is administered in conjunction with at least one additional therapeutic agent selected from the group consisting of antivirals, anti-inflammatories, convalescent plasma, and immune globulins.
46. The method according to any one of the preceding clauses, wherein the LYT-100 is administered and the subject is also maintaining another treatment relating to the viral respiratory disease.
47. The method of clause 46, wherein the another treatment comprises one or more non-pharmacological treatments selected from the group consisting of mechanical ventilation, non-invasive ventilation, O₂ supplementation, extra corporeal membrane oxygenation (ECMO), continuous positive airway pressure (CPAP), respiratory rehabilitation, and exercise.
48. The method according to any of the preceding clauses, wherein one or more of the following applies to the subject prior to treatment with LYT-100:
   the subject has a positive COVID-19 RT-PCR or SARS-CoV-2 RNA diagnostic test result;
   the subject is or was hospitalized for COVID-19 respiratory disease for at least 1-day, and required at least one of the listed treatment modalities for at least 24 hours: mechanical ventilation (MV), extra-corporeal membrane oxygenation (ECMO), non-invasive ventilation (NIV), high flow nasal oxygen (HFNO), and or other high flow oxygen with FiO2 ≥35% and flow rates ≥ 8 lpm;
   the subject has a confirmed diagnosis of COVID-19;
   the subject exhibits COVID-19 pneumonia as confirmed by imaging, including by chest X-ray or high-resolution computerized tomography with a minimum of two lobe involvement.
49. The method according to any of the preceding clauses, wherein the subject exhibits one or more of the following prior to treatment: shortness of breath ≥ grade 3 on mBDS dyspnea scale fever >38C°; cough; elevated CRP ≥ 10.0 mg/dL; oxygen saturation ≤93% at rest; requires supplemental oxygen; requires high-flow oxygen or non-invasive ventilation.
50. The method according to any of the preceding clauses, wherein the subject does not have one or more of the following:
   a pre-existing respiratory condition unrelated to the viral respiratory disease;
   a resting heart rate of ≥ 120 bpm, systolic blood pressure of > 180 mm Hg and a diastolic blood pressure of > 100 mm Hg;
   a fall in pulse oximetry oxygen saturation (SpO₂) to <80% upon ambulation;
   a lactate dehydrogenase (LDH) value >360;
   body mass index ≥ 40 kg/m²
   a history of anaphylactic reaction including reactions to general anaesthetic agents;
   a history of allergic reaction to any drug which led to significant morbidity;
   a history of prior allergic reaction to pirfenidone or LYT-100;
   sepsis;
   septic, hypovolemic, cardiogenic, or neurogenic shock.
51. The method according to any of the preceding clauses, wherein the subject: is not intubated or mechanically ventilated; does not require extracorporeal membrane oxygenation (ECMO); is not undergoing dialysis; and does not have a history of dialysis; and wherein the subject has not been intubated, mechanically ventilated, or received ECMO within 72 hours prior to initiating treatment.
52. The method according to any of the preceding clauses, wherein the subject is not currently taking any of the following: fluvoxamine, enoxacin, or ciprofloxacin; nonsteroidal antiinflammatory drugs (NSAIDs); inhibitors of CYP1A2; inducers of CYP1A2, CYP2C9 or 2C19; tobacco or nicotine containing products; and any drug associated with substantial risk for prolongation of the QTc interval.
53. The method according to any of the preceding clauses, wherein an improvement of 25 meters or more in a 6-minute walk test (6-MWT) over monthly increments through day 91 of treatment is achieved.
54. The method according to any of the preceding clauses, wherein at least a 1.5-point decrease in the Modified Borg Dyspnea Scale is achieved.
55. The method according to any of the preceding clauses, wherein one or more of the following is achieved in the subject:
   an improvement in blood oxygenation level in the human subject over 91 days of treatment, as determined by pulse oximetry;
   a 10 unit drop in an overall score in the Short Form Health Survey (SF-36 v2) over 91 days of treatment;
   an increase of ≥2 units in the overall St. George's Respiratory Questionnaire (SGRQ-I) score over 91 days of treatment.
   an improvement in pulmonary inflammation;
   an improvement in fibrosis;
   an improvement in other radiographic abnormalities, as measured by determining at least a 2-point score improvement over 91 days of treatment on high resolution computed tomography (HRCT) scans, from the level of the upper thoracic inlet to the inferior level of the costophrenic angle over 28, 56, and 91 days of treatment utilizing the following scoring system: a) number of involved segments; and b) lung lobe involvement for each of five lobes via the semi-Quantitative Lung Fibrosis (QLF) added and Computer-Aided Diagnosis (CAD) QLF Scores.
56. The method according to any of the preceding clauses, wherein an improvement in the score on one or more of the following is achieved in the subject: Dyspnoea-12; Patient-reported quality of life (SF-36 v2); St. George's Respiratory Questionnaire (SGRQ-I); the World Health Organization (WHO) Ordinal Scale for Improvement.
57. The method according to any of the preceding clauses, wherein a reduction in fibrosis or fibrotic lesions on high resolution computed tomography (HRCT) is achieved in the subject.
58. The method according to clause 57, wherein the fibrotic lesion reduced in the subject is one or more of ground glass opacity, reticular patterning, honeycombing, traction bronchiectasis, bronchiolectasis, interlobular septal thickening, ill-defined margins, air bronchogram, "crazy-paving" pattern, thickening of the adjacent pleura, nodules, cystic changes, pleural effusion, or lymphadenopathy.
59. The method according to any of the preceding clauses, wherein at least a 25% reduction in one or more of the following biomarkers in achieved: C-Reactive Protein (CRP); d-dimer; cardiac troponin; ferritin; lactate dehydrogenase (LDH); interleukin-6 (IL-6); TGF-β1; TNF-α; IL-1β; PDGF- β; GCSF; VEGF.
60. The method according to any of the preceding clauses, wherein an improvement by 2 points or higher on the WHO Ordinal Scale for Clinical Improvement is achieved.
61. A method of treating post-acute sequelae of infection with SARS-CoV-2, the method comprising administering to a subject in need thereof an effective amount of: 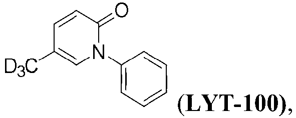 wherein the post-acute sequelae of infection are treated in the subject.
62. The method according to clause 61, wherein the post-acute sequelae comprise one or more of post-acute respiratory complications, impaired lung function, pulmonary dysfunction, and pulmonary fibrosis.
63. The method according to clause 61, wherein the post-acute sequelae comprise progressive pulmonary fibrosis.
64. The method according to clause 63, wherein a reduction in fibrosis or fibrotic lesions on high resolution computed tomography (HRCT) is achieved in the subject.
65. The method according to any one of clauses 61-64, wherein the administering is initiated during an acute phase of the SARS-CoV-2 infection.
66. The method according to any one of clauses 61-65, wherein the administering is initiated during an acute phase of the SARS-CoV-2 infection and continued during a post-acute phase of the SARS-CoV-2 infection.
67. The method according to any one of clauses 61-64, wherein the administering is initiated during a post-acute phase of the SARS-CoV-2 infection.
68. The method of any one of clauses 61-67, wherein the subject with one or more post-acute respiratory complications was previously treated with, but no longer requires mechanical ventilation (MV), extracorporeal membrane oxygenation (ECMO), non-invasive ventilation (NIV), high-flow nasal oxygen therapy (HFNO), or high flow supplemental oxygen with FiO2 ≥35%.
69. The method of any one of clauses 61-67, wherein the subject has not been treated with mechanical ventilation or extracorporeal membrane oxygenation.
70. The method of any one of clauses 61-69, wherein the subject requires regular nasal cannula O₂ supplementation.
71. The method of any one of clauses 61-69, wherein the subject does not require regular nasal cannula O₂ supplementation.
72. The methods of any one of clauses 61-71, wherein the administering begins while the subject is hospitalized for SARS-CoV-2 infection, at the time of discharge from the hospital for SARS-CoV-2 infection, or post-discharge from the hospital for SARS-CoV-2 infection.
73. The method according to any one of clauses 61-72, wherein the LYT-100 is administered orally without food in two daily doses of 500 mg each, 750 mg each, or 1000 mg each.
74. The method according to any one of clauses 61-73, wherein the LYT-100 is administered orally with or without food in an initial dose of 500 mg BID for the first 3 days of dosing, followed by titration up to 750 mg BID thereafter for at least three months.
75. The method according to any one of clauses 61-73, wherein the LYT-100 is administered orally with or without food in an initial dose of 500 mg BID for the first 3 days of dosing, followed by titration up to 1000 mg BID thereafter for at least three months.
76. The method according to any one of clauses 61-73, wherein the LYT-100 is administered orally with or without food in an initial dose of 500 mg BID for the first 3 days of dosing, followed by titration up to 750 mg BID for days 4 through 7, followed by titration up to 1000 mg BID thereafter for at least three months.
77. The method according to any of the preceding clauses, wherein the subject is human.
78. The method according to any of the preceding clauses, wherein the subject is an adult.

## Claims

**1.** A compound for use in the treatment of a viral respiratory disease or infection in a subject wherein the compound is 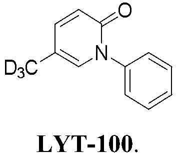 wherein the treatment is effective in preventing the development of, halting the progression of, or slowing the progression of one or more of lung fibrosis, respiratory complications of the viral respiratory disease or infection, respiratory symptoms of the viral respiratory disease or infection, or pulmonary dysfunction in the subject.

**2.** The compound for use according to claim 1, wherein:
(i) the viral respiratory infection is caused by infection with human influenza virus H1N1, human influenza virus H7N9, SARS-CoV, SARS-CoV-2, MERS-CoV, HCoV-HKU1, or HCoV-OC43; or
(ii) the viral respiratory disease is COVID-19 or a complication thereof.

**3.** The compound for use according to any of the preceding claims, wherein:
(i) the treatment is effective in preventing the development of, halting the progression of, or slowing the progression of pulmonary fibrosis; and/or
(ii) the subject exhibits reduced respiratory symptoms of the viral respiratory disease or infection, wherein the respiratory symptoms are cough, dyspnea, or both; and/or
(iii) the subject exhibits reduced evidence of one or more of lung fibrosis, respiratory complications, or pulmonary dysfunction relative to a subject who has not been treated with LYT-100, as measured by a chest X-ray or a CT scan, or as evidenced by a pulmonary function test result.

**4.** The compound for use according to any of the preceding claims, wherein said treatment is treatment initiated within 60 days from a confirmed viral respiratory disease diagnosis.

**5.** The compound for use according to any of the preceding claims, wherein said treatment comprises daily administration of a total daily dose of 200 mg - 2500 mg LYT-100.

**6.** The compound for use according to claim 5, wherein said treatment comprises daily administration of a total daily dose of between 250 mg - 2500 mg LYT-100.

**7.** The compound for use according to claim 5 or claim 6, wherein said treatment comprises daily administration of a total daily dose of between 2400 mg - 2500 mg LYT-100.

**8.** The compound for use according to any of claims 1- 5, wherein said treatment comprises daily administration of a total daily dose of between 200 mg and 2250 mg LYT-100.
8. The compound for use according to any of the preceding claims, wherein the compound is for oral administration.

**9.** The compound for use according to any of the preceding claims, wherein the compound is for administration in tablet or capsule form.

**11.** The compound for use according to any of the preceding claims, wherein said treatment comprises daily administration of LYT-100, and said daily administration is twice daily.

**13.** The compound for use according to any one of claims 1-10, wherein said treatment comprises daily administration of LYT-100, and said daily administration is three times daily.

**14.** The compound for use according to any one of the preceding claims, wherein:
(i) said administration is without dose escalation; or
(ii) said treatment does not comprise an initial titration.

**15.** The compound for use according to claim 2, wherein said treatment is the treatment of post-acute sequelae of infection with SARS-CoV-2

**16.** The compound for use according to claim 14, wherein:
(i) the treatment is for initiation during an acute phase of the SARS-CoV-2 infection; or
(ii) the treatment is for initiation during an acute phase of the SARS-CoV-2 infection and continuation during a post-acute phase of the SARS-CoV-2 infection; or
(iii) the treatment is for initiation during a post-acute phase of the SARS-CoV-2 infection.
